# EUROPEAN PATENT SPECIFICATION

(11) **EP 0 952 997 B1**
(45) Date of publication and mention of the grant of the patent: **11.08.2004**
(21) Application number: 98902510.1
(22) Date of filing: 13.01.1998
(51) Int. Cl.: C08F 10/00, C08F 4/70, C08F 4/82, C07F 15/04, C07D 207/335, C07C 225/14, C07C 229/30, C07C 237/16, C07C 327/44

(54) **POLYMERIZATION OF OLEFINS**
POLYMERISATION VON OLEFINEN
POLYMERISATION D'OLEFINES

(30) Priority: 14.01.1997 US 35190 P
(43) Date of publication of application: 03.11.1999
(73) Proprietor: E.I. DU PONT DE NEMOURS AND COMPANY, Wilmington, Delaware 19898 (US)
(72) Inventor: JOHNSON, Lynda, Kaye, Wilmington, DE 19803 (US); BENNETT, Alison, Margaret, Anne, Wilmington, DE 19803 (US); ITTEL, Steven, Dale, Wilmington, DE 19810 (US); WANG, Lin, Hockessin, DE 19707 (US); PARTHASARATHY, Anju, Glenmoore, PA 19343 (US); HAUPTMAN, Elisabeth, Wilmington, DE 19809 (US); SIMPSON, Robert, D., Philadelphia, PA 19119 (US); FELDMAN, Jerald, Hockessin, DE 19707 (US); COUGHLIN, Edward, Bryan, Wilmington, DE 19809 (US)
(74) Representative: Towler, Philip Dean
(86) International application number: PCT/US1998/000610
(87) International publication number: WO 1998/030609

(56) References cited:
- WO-A-96/23010
- DE-A- 4 415 725
- US-A- 5 395 811

## Description

### FIELD OF THE INVENTION

Olefins are polymerized by a catalyst system that includes a nickel[II] complexes of selected monoanionic bidentate ligands. Some of these complexes are also novel.

### TECHNICAL BACKGROUND

Polymers of ethylene and other olefins are important items of commerce, and these polymers are used in a myriad of ways, from low molecular weight polyolefins being used as a lubricant and in waxes, to higher molecular weight grades being used for fiber, films, molding resins, elastomers, etc. In most cases, olefins are polymerized using a catalyst, often a transition metal compound or complex. These catalysts vary in cost per unit weight of polymer produced, the structure of the polymer produced, the possible need to remove the catalyst from the polyolefin, the toxicity of the catalyst, etc. Due to the commercial importance of polymerizing olefins, new polymerization catalysts are constantly being sought.

### SUMMARY OF THE INVENTION

This invention concerns a process for the polymerization of an olefin selected from one or more of R⁶⁷CH=CH₂, cyclopentene, a styrene, a norbornene or H₂C=CH (CH₂)ₛCO₂R⁷⁷, comprising, contacting, at a temperature of about -100°C to about +200°C, R⁶⁷CH=CH₂, cyclopentene, a styrene, a norbornene, or H₂C=CH (CH₂)ₛCO₂R⁷⁷, optionally a Lewis acid, and a compound of the formula: 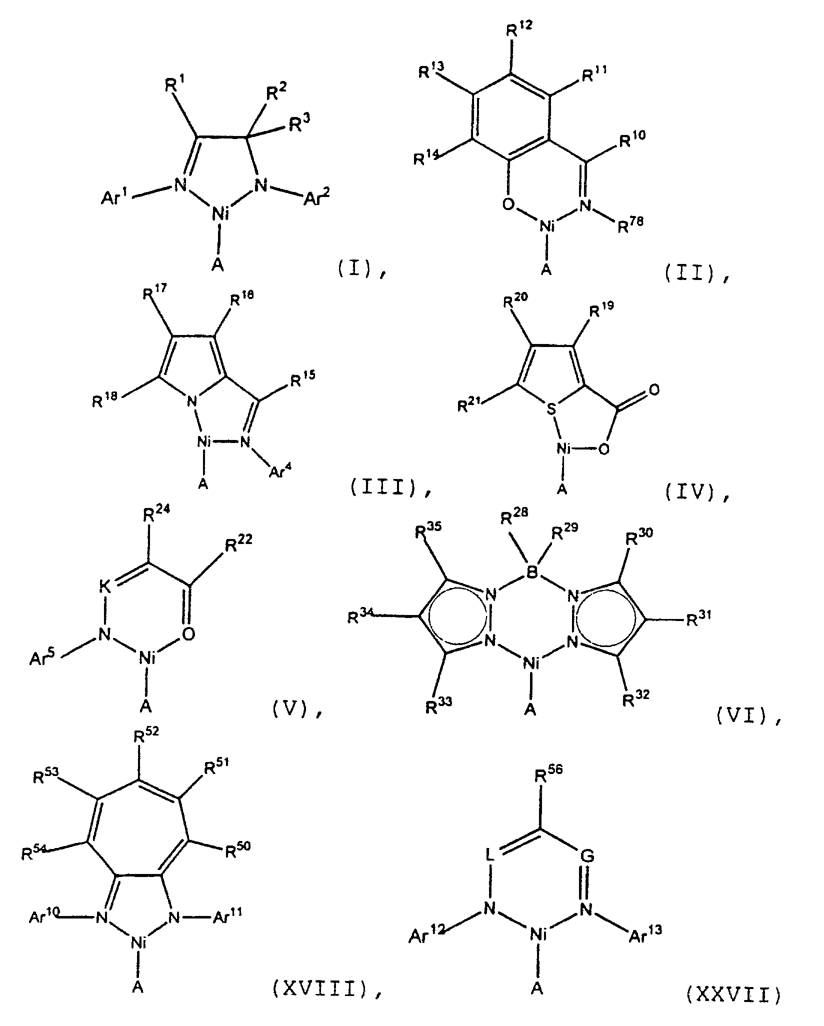 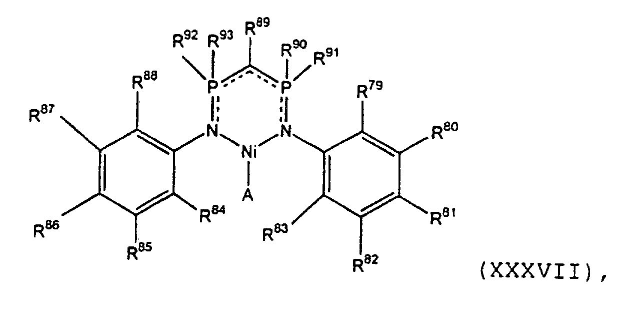 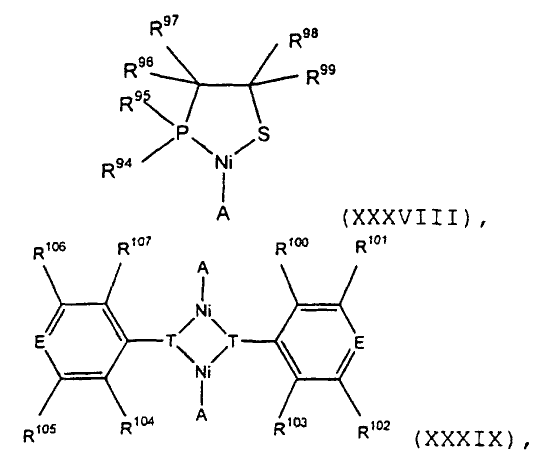 or 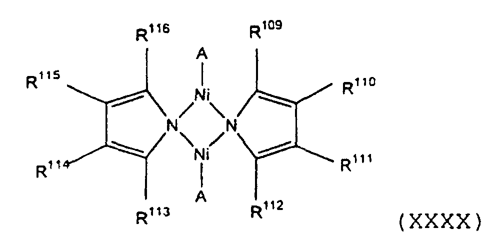 wherein:
Ar¹, Ar², Ar⁴, Ar⁵, Ar¹⁰, Ar¹¹, Ar¹² and Ar¹³ are each independently aryl or substituted aryl;
R¹ and R² are each independently hydrogen, hydrocarbyl, substituted hydrocarbyl, or R¹ and R² taken together form a ring, and R³ is hydrogen, hydrocarbyl or substituted hydrocarbyl or R¹, R² and R³ taken together form a ring;
A is a π-allyl or π-benzyl group;
R¹⁰ and R¹⁵ are each independently hydrogen, hydrocarbyl or substituted hydrocarbyl;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵ R⁵⁰, R⁵¹, R⁵², R⁵³ and R⁵⁴ are each independently hydrogen, hydrocarbyl, substituted hydrocarbyl, an inert functional group, and provided that any two of these groups vicinal to one another taken together may form a ring;
K is N or CR²⁷;
R²² is hydrocarbyl, substituted hydrocarbyl, -SR¹¹⁷, -OR¹¹⁷, or -NR¹¹⁸₂, R²⁴ is hydrogen, a functional group, hydrocarbyl or substituted hydrocarbyl, and R²⁷ is hydrocarbyl or substituted hydrocarbyl, and provided that R²² and R²⁴ or R²⁴ and R²⁷ taken together may form a ring;
R¹¹⁷ is hydrocarbyl or substituted hydrocarbyl;
each R¹¹⁸ is independently hydrogen, hydrocarbyl or substituted hydrocarbyl;
G and L are both N or G is CR⁵⁷ and L is CR⁵⁵;
R⁵⁵, R⁵⁶ and R⁵⁷ are each independently hydrogen, hydrocarbyl or substituted hydrocarbyl, or any two of R⁵⁵, R⁵⁶ and R⁵⁷ taken together form a ring;
R⁶⁷ is hydrogen, alkyl or substituted alkyl;
R⁷⁷ is hydrocarbyl or substituted hydrocarbyl;
R⁷⁹ is hydrocarbyl or substituted hydrocarbyl;
R⁷⁹, R⁸⁰, R⁸¹, R⁸², R⁸³, R⁸⁴, R⁸⁵, R⁸⁶, R⁸⁷, R⁸⁸ and R⁸⁹ are each independently hydrogen, hydrocarbyl, substituted hydrocarbyl, or a functional group;
R⁹⁰, R⁹¹, R⁹² and R⁹³ are each independently hydrocarbyl or substituted hydrocarbyl;
R⁹⁴ and R⁹⁵ are each independently hydrocarbyl or substituted hydrocarbyl;
R⁹⁶, R⁹⁷, R⁹⁸, and R⁹⁹ are each independently hydrogen, hydrocarbyl, substituted hydrocarbyl or a functional group;
both of T are S (sulfur) or NH (amino);
each E is N (nitrogen) or CR¹⁰⁸ wherein R¹⁰⁸ is hydrogen, hydrocarbyl, substituted hydrocarbyl or a functional group;
R¹⁰⁰, R¹⁰¹ , R¹⁰², R¹⁰³, R¹⁰⁴, R¹⁰⁵, R¹⁰⁶, and R¹⁰⁷ are each independently hydrogen, hydrocarbyl, substituted hydrocarbyl, or a functional group;
R¹⁰⁹ , R¹¹⁰ , R¹¹¹ , R¹¹² , R¹¹³ , R¹¹⁴ , R¹¹⁵ and R¹¹⁶ are each independently hydrogen, hydrocarbyl, substituted hydrocarbyl or a functional group;
s is an integer of 1 or more; and
R²⁸ and R²⁹ are each independently hydrogen, hydrocarbyl or substituted hydrocarbyl;
and provided that when H₂C=CH (CH₂)ₛCO₂R⁷⁷ is present, R⁶⁷CH=CH₂ is also present.

This invention also concerns a process for the polymerization of an olefin selected from one or more of R⁶⁷CH=CH₂, a styrene, a norbornene or H₂C=CH(CH₂)ₛCO₂R⁷⁷, comprising, contacting, at a temperature of about -100°C to about +200°C, R⁶⁷CH=CH₂, cyclopentene, a styrene, a norbornene, or H₂C=CH (CH₂)ₛCO₂R⁷⁷, optionally a Lewis acid, and a compound of the formula: 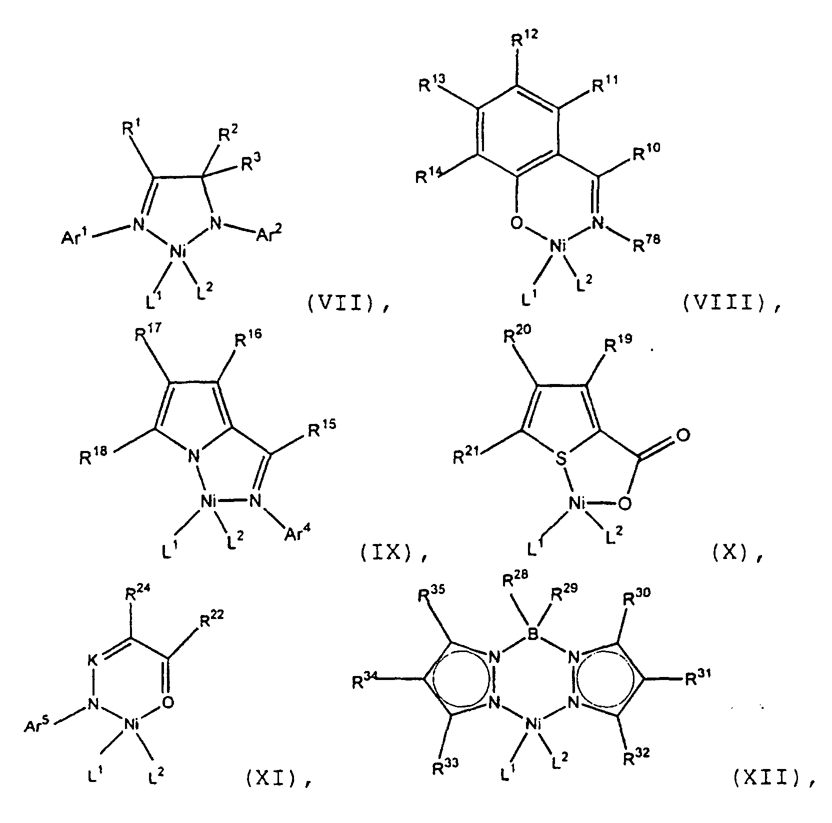 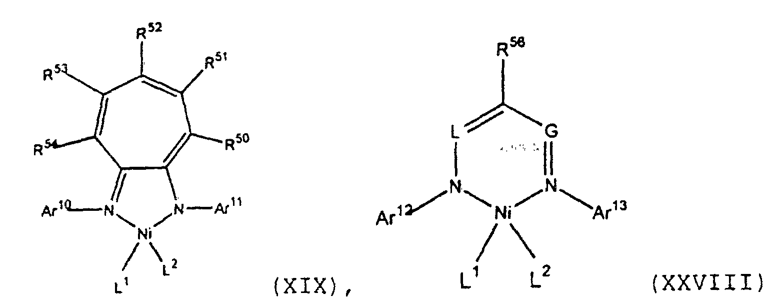 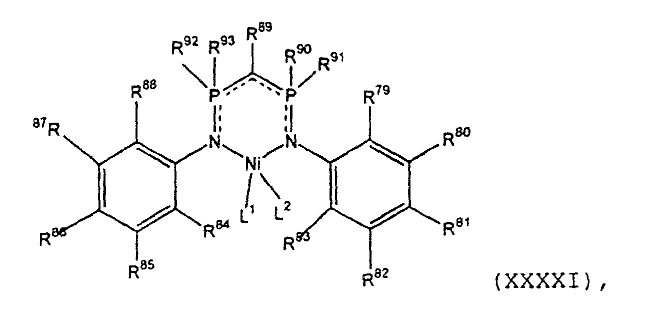 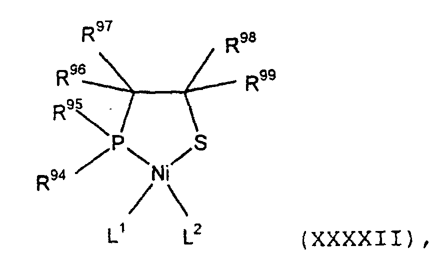 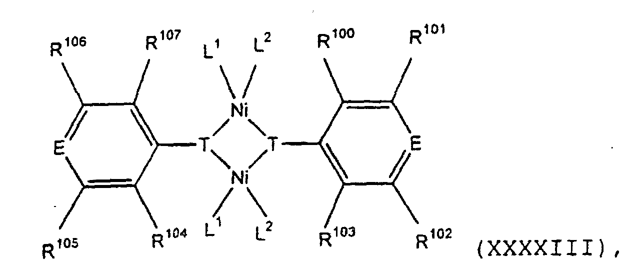 or 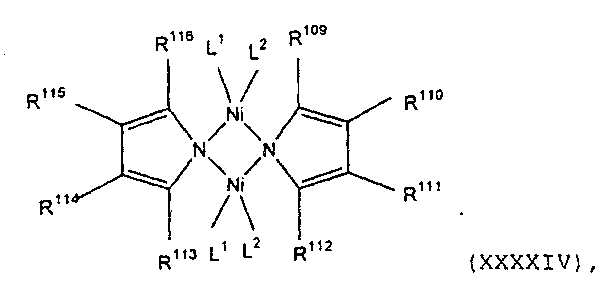 wherein:
L¹ is a neutral monodentate ligand which may be displaced by said olefin, and L² is a monoanionic monodentate ligand, or L¹ and L² taken together are a monoanionic bidentate ligand, provided that said monoanionic monodentate ligand or said monoanionic bidentate ligand may add to said olefin;
Ar¹, Ar², Ar⁴, Ar⁵, Ar¹⁰, Ar¹¹, Ar¹² and Ar¹³ are each independently aryl or substituted aryl;
R¹ and R² are each independently hydrogen, hydrocarbyl, substituted hydrocarbyl, or R¹ and R² taken together form a ring, and R³ is hydrogen, hydrocarbyl or substituted hydrocarbyl or R¹, R² and R³ taken together form a ring;
R¹⁰ and R¹⁵ are each independently hydrogen, hydrocarbyl or substituted hydrocarbyl;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ and R⁵⁴ are each independently hydrogen, hydrocarbyl, substituted hydrocarbyl, an inert functional group, and provided that any two of these groups vicinal to one another taken together may form a ring;
K is N or CR²⁷;
R²² is hydrocarbyl, substituted hydrocarbyl, -SR¹¹⁷, -OR¹¹⁷, or -NR¹¹⁸₂, R²⁴ is hydrogen, a functional group, hydrocarbyl or substituted hydrocarbyl, and R²⁷ is hydrocarbyl or substituted hydrocarbyl, and provided that R²² and R²⁴ or R²⁴ and R²⁷ taken together may form a ring;
R¹¹⁷ is hydrocarbyl or substituted hydrocarbyl;
each R¹¹⁸ is independently hydrogen, hydrocarbyl or substituted hydrocarbyl;
G and L are both N or G is CR⁵⁷ and L is CR⁵⁵;
R⁵⁵, R⁵⁶ and R⁵⁷ are each independently hydrogen, hydrocarbyl or substituted hydrocarbyl, or any two of R⁵⁵, R⁵⁶ and R⁵⁷ taken together form a ring;
R⁶⁷ is hydrogen, alkyl or substituted alkyl;
R⁷⁷ is hydrocarbyl or substituted hydrocarbyl;
R⁷⁸ is hydrocarbyl or substituted hydrocarbyl; R⁷⁹, R⁸⁰, R⁸¹, R⁸², R⁸³, R⁸⁴, R⁸⁵, R⁸⁶, R⁸⁷, R⁸⁸ and R⁸⁹ are each independently hydrogen, hydrocarbyl, substituted hydrocarbyl, or a functional group;
R⁹⁰, R⁹¹, R⁹² and R⁹³ are each independently hydrocarbyl or substituted hydrocarbyl;
R⁹⁴ and R⁹⁵ are each independently hydrocarbyl or substituted hydrocarbyl;
R⁹⁶, R⁹⁷, R⁹⁸, and R⁹⁹ are each independently hydrogen, hydrocarbyl, substituted hydrocarbyl or a functional group;
both of T are S (sulfur) or NH (amino);
each E is N (nitrogen) or CR¹⁰⁸ wherein R¹⁰⁸ is hydrogen, hydrocarbyl, substituted hydrocarbyl or a functional group;
R¹⁰⁰, R¹⁰¹, R¹⁰², R¹⁰³, R¹⁰⁴, R¹⁰⁵, R¹⁰⁶, and R¹⁰⁷ are each independently hydrogen, hydrocarbyl, substituted hydrocarbyl, or a functional group;
R¹⁰⁹, R¹¹⁰, R¹¹¹, R¹¹², R¹¹³, R¹¹⁴, R¹¹⁵ and R¹¹⁶ are each independently hydrogen, hydrocarbyl, substituted hydrocarbyl or a functional group;
s is an integer of 1 or more; and
R²⁸ and R²⁹ are each independently hydrogen, hydrocarbyl or substituted hydrocarbyl;
and provided that when H₂C=CH(CH₂)ₛCO₂R⁷⁷ is present, R⁶⁷CH=CH₂ is also present.

Also described herein is a compound of the formula: 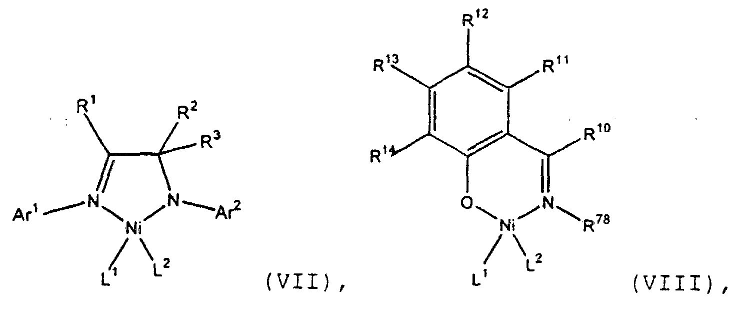 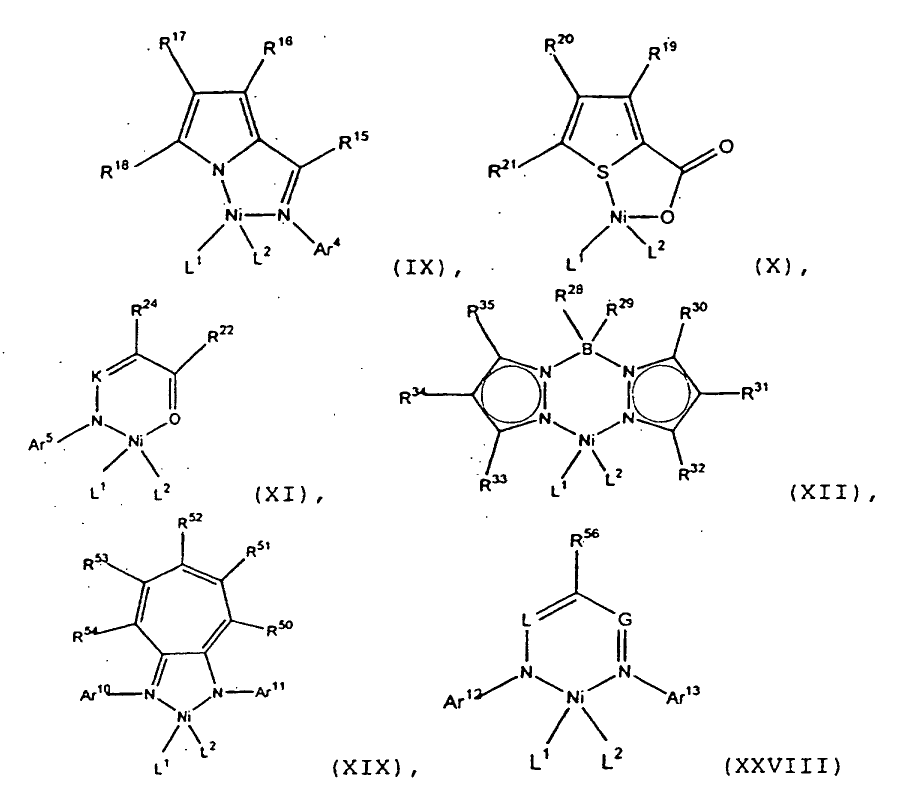 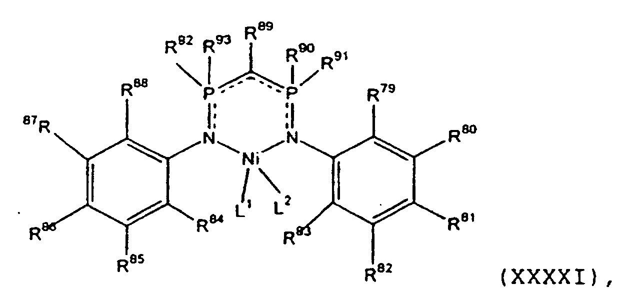 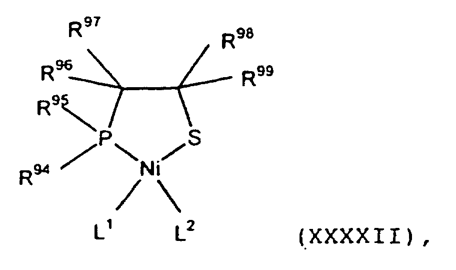 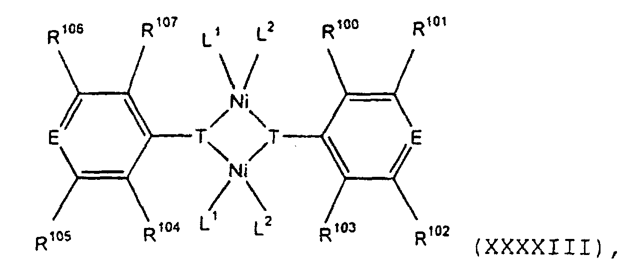 or 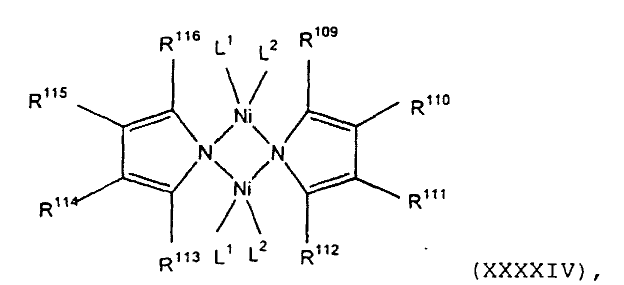 wherein:
L¹ is a neutral monodentate ligand which may be displaced by said olefin, and L² is a monoanionic monodentate ligand, or L¹ and L² taken together are a monoanionic bidentate ligand, provided that said monoanionic monodentate ligand or said monoanionic bidentate ligand may add to said olefin;
Ar¹, Ar²,Ar⁴, Ar⁵, Ar¹⁰, Ar¹¹, Ar¹² and Ar¹³ are each independently aryl or substituted aryl;
R¹ and R² are each independently hydrogen, hydrocarbyl, substituted hydrocarbyl, or R¹ and R² taken together form a ring, and R³ is hydrogen, hydrocarbyl or substituted hydrocarbyl or R¹, R² and R³ taken together form a ring;
R¹⁰ and R¹⁵ are each independently hydrogen, hydrocarbyl or substituted hydrocarbyl;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ and R⁵⁴ are each independently hydrogen, hydrocarbyl, substituted hydrocarbyl, an inert functional group, and provided that any two of these groups vicinal to one another taken together may form a ring;
K is N or CR²⁷;
R²² is hydrocarbyl, substituted hydrocarbyl, -SR¹¹⁷, -OR¹¹⁷, or -NR¹¹⁸₂, R²⁴ is hydrogen, a functional group, hydrocarbyl or substituted hydrocarbyl, and R²⁷ is hydrocarbyl or substituted hydrocarbyl, and provided that R²² and R²⁴ or R²⁵ and R²⁷ taken together may form a ring;
R¹¹⁷ is hydrocarbyl or substituted hydrocarbyl;
each R¹¹⁸ is independently hydrogen, hydrocarbyl or substituted hydrocarbyl;
G and L are both N or G is CR⁵⁷ and L is CR⁵⁵;
R⁵⁵ R⁵⁶ and R⁵⁷ are each independently hydrogen, hydrocarbyl or substituted hydrocarbyl, or any two of R⁵⁵, R⁵⁶ and R⁵⁷ taken together form a ring;
R⁷⁸ is hydrocarbyl or substituted hydrocarbyl;
R⁷⁹, R⁸⁰, R⁸¹, R⁸², R⁸³, R⁸⁴, R⁸⁵, R⁸⁶, R⁸⁷, R⁸⁸ and R⁸⁹ are each independently hydrogen, hydrocarbyl, substituted hydrocarbyl, or a functional group;
R⁹⁰, R⁹¹, R⁹² and R⁹³ are each independently hydrocarbyl or substituted hydrocarbyl;
R⁹⁴ and R⁹⁵ are each independently hydrocarbyl or substituted hydrocarbyl;
R⁹⁶, R⁹⁷, R⁹⁸, and R⁹⁹ are each independently hydrogen, hydrocarbyl, substituted hydrocarbyl or a functional group;
both of T are S (sulfur) or NH (amino);
each E is N (nitrogen) or CR¹⁰⁸ wherein R¹⁰⁸ is hydrogen, hydrocarbyl, substituted hydrocarbyl or a functional group;
R¹⁰⁰, R¹⁰¹, R¹⁰², R¹⁰³, R¹⁰⁴, R¹⁰⁵, R¹⁰⁶, and R¹⁰⁷ are each independently hydrogen, hydrocarbyl, substituted hydrocarbyl, or a functional group;
R¹⁰⁹, R¹¹⁰, R¹¹¹, R¹¹², R¹¹³, R¹¹⁴, R¹¹⁵ and R¹¹⁶ are each independently hydrogen, hydrocarbyl, substituted hydrocarbyl or a functional group; and
R²⁸ and R²⁹ are each independently hydrogen, hydrocarbyl or substituted hydrocarbyl.

Also disclosed herein is a compound of the formula 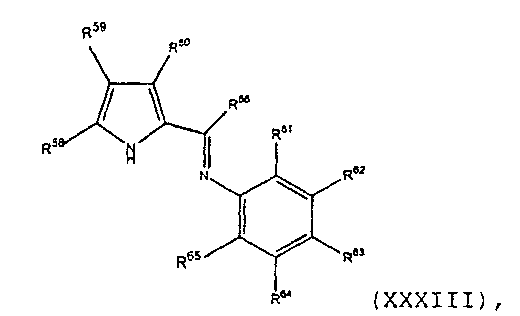 wherein:
R⁵⁸, R⁵⁹, R⁶⁰, R⁶², R⁶³ and R⁶⁴ are each independently hydrogen, hydrocarbyl, substituted hydrocarbyl, or a functional group, and provided that any two of these groups vicinal to one another taken together may form a ring, or if vicinal to R⁶¹ or R⁶⁵ form a ring with them;
R⁶⁶ is hydrogen, hydrocarbyl or substituted hydrocarbyl; and
R⁶¹ and R⁶⁵ are each independently hydrocarbyl containing 2 or more carbon atoms, or substituted hydrocarbyl containing 2 or more carbon atoms, and provided that R⁶¹ and R⁶⁵ may form a ring with any group vicinal to it.

This invention also concerns a compound of the formula 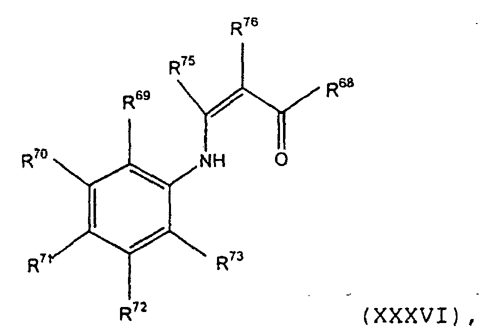 wherein:
R⁶⁸ is hydrocarbyl, substituted hydrocarbyl, - SR¹¹⁷, -OR¹¹⁷, or -NR¹¹⁸₂, R⁷⁶ is hydrogen, a functional group, hydrocarbyl or substituted hydrocarbyl, and R⁷⁵ is hydrocarbyl or substituted hydrocarbyl, and provided that R⁶⁸ and R⁷⁶ or R⁷⁵ and R⁷⁶ taken together may form a ring;
R¹¹⁷ is hydrocarbyl or substituted hydrocarbyl;
each R¹¹⁸ is independently hydrogen, hydrocarbyl or substituted hydrocarbyl;
R⁷⁰ ,R⁷¹ and R⁷² are each independently hydrogen, hydrocarbyl, substituted hydrocarbyl or a functional group;
R⁶⁹ and R⁷³ are hydrocarbyl containing 3 or more carbon atoms, substituted hydrocarbyl containing 3 or more carbon atoms or a functional group;
and provided that any two of R⁷⁰, R⁷¹, R⁷², R⁶⁹ and R⁷³ vicinal to one another together may form a ring.

### DETAILS OF THE INVENTION

Herein, certain terms are used. Some of them are:
- A "hydrocarbyl group" is a univalent group containing only carbon and hydrogen. If not otherwise stated, it is preferred that hydrocarbyl groups herein preferably contain 1 to about 30 carbon atoms.
- By "substituted hydrocarbyl" herein is meant a hydrocarbyl group which contains one or more substituent groups which are inert under the process conditions to which the compound containing these groups is subjected. The substituent groups also do not substantially interfere with the process. If not otherwise stated, it is preferred that substituted hydrocarbyl groups herein contain preferably 1 to about 30 carbon atoms. Included in the meaning of "substituted" are heteroaromatic rings.
- By "(inert) functional group" herein is meant a group other than hydrocarbyl or substituted hydrocarbyl which is inert under the process conditions to which the compound containing the group is subjected. The functional groups also do not substantially interfere with any process described herein that the compound in which they are present may take part in. Examples of functional groups include halo (fluoro, chloro, bromo and iodo), ether such as -OR²⁵, -CO₂R²⁵, -NO₂, and -NR²⁵₂, wherein R²⁵ is hydrocarbyl or substituted hydrocarbyl. In cases in which the functional group may be near a nickel atom the functional group should not coordinate to the metal atom more strongly than the groups in compounds which are shown as coordinating to the metal atom, that is they should not displace the desired coordinating group.
- By a "polymerization process" herein (and the polymers made therein) is meant a process which produces a polymer with a degree of polymerization (DP) of about 5 or more, preferably about 10 or more, more preferably about 40 or more [except where otherwise noted, as in P in compound (XVII)]. By "DP" is meant the average number of repeat (monomer) units in the polymer.
- By "aryl" herein is meant a monovalent radical whose free valence is to a carbon atom of an aromatic ring. Unless otherwise noted herein, preferred aryl groups contain carbocyclic rings, but heterocyclic rings are also included within the definition of "aryl". The aryl radical may contain one ring or may contain 2 or more fused rings, such as 9-anthracenyl or 1-naphthyl. Unless otherwise stated aryl groups preferably contain 5 to 30 carbon atoms.
- By "substituted aryl" herein is meant an aryl radical substituted with one or more groups that do not interfere with the synthesis of the compound or the resulting polymerization. Suitable substituents include alkyl, aryl such as phenyl, halo, alkoxy, ester, dialkylamino and nitro. Unless otherwise stated, substituted aryl groups contain 5 to about 30 carbon atoms.
- By a "monoanionic ligand" is meant a ligand with one negative charge.
- By a "neutral ligand" is meant a ligand that is not charged.
- "Alkyl group" and "substituted alkyl group" have their usual meaning (see above for substituted under substituted hydrocarbyl). Unless otherwise stated, alkyl groups and substituted alkyl groups preferably have 1 to about 30 carbon atoms.
- By a styrene herein is meant a compound of the formula 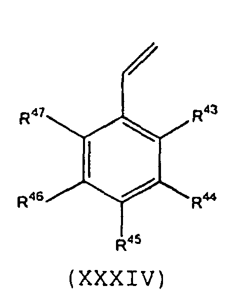 wherein R⁴³, R⁴⁴, R⁴⁵, R⁴⁶ and R⁴⁷ are each independently hydrogen, hydrocarbyl, substituted hydrocarbyl or a functional group, all of which are inert in the polymerization process. It is preferred that all of R⁴³, R⁴⁴, R⁴⁵, R⁴⁶ and R⁴⁷ are hydrogen. Styrene (itself) is a preferred styrene.
- By a norbornene is meant ethylidene norbornene, dicyclopentadiene, or a compound of the formula 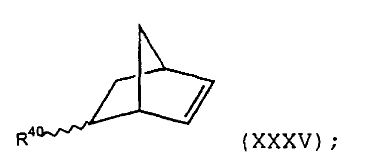 wherein R⁴⁰ is hydrogen or hydrocarbyl containing 1 to 20 carbon atoms. It is preferred that R⁴⁰ is hydrogen or alkyl, more preferably hydrogen or n-alkyl, and especially preferably hydrogen. The norbornene may be substituted by one or more hydrocarbyl, substituted hydrocarbyl or functional groups in the R⁴⁰ or other positions, with the exception of the vinylic hydrogens, which remain. Norbornene (itself), dimethyl endo-norbornene-2,3-dicarboxylate, t-butyl 5-norbornene-2-carobxylate are preferred norbornenes and norbornene (itself) is especially preferred.
- By a π-allyl group is meant a monoanionic ligand with 3 adjacent sp² carbon atoms bound to a metal center in an η³ fashion. The three sp² carbon atoms may be substituted with other hydrocarbyl groups or functional groups. Typical π-allyl groups include 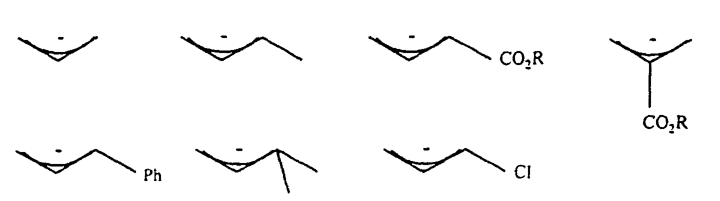 wherein R is hydrocarbyl. By a π-benzyl group is meant π-allyl ligand in which two of the sp² carbon atoms are part of an aromatic ring. Typical π-benzyl groups include 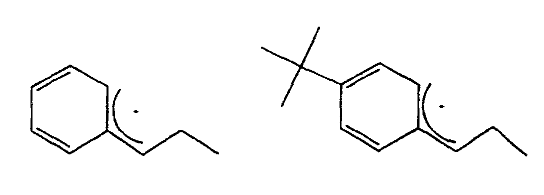 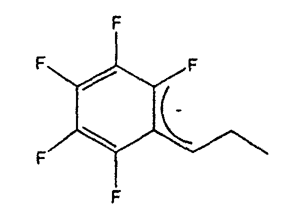 and

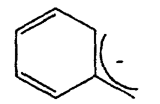

π-Benzyl compounds usually initiate polymerization of the olefins fairly readily even at room temperature, but π-allyl compounds may not necessarily do so. Initiation of π-allyl compounds can be improved by using one or more of the following methods:
- Using a higher temperature such as about 80°C.
- Decreasing the bulk of the monoanionic ligand, such as aryl being 2,6-dimethylphenyl instead of 2, 6-diisopropylphenyl.
- Making the π-allyl ligand more bulky, such as using 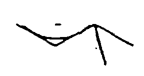 rather than the simple π-allyl group itself.
- Having a Lewis acid or a material that acts as a Lewis acid present while using a π-allyl or π-benzyl group, especially a functional π-allyl or π-benzyl group. Relatively weak Lewis acids such as triphenylborane, tris(pentafluorophenyl)borane, tris(3,5-trifluoromethylphenyl)borane, and poly(methylaluminoxane) are preferred. Suitable functional groups include chloro and ester.

Lewis acids may or may not be present in the process of the invention.

Lewis acids may also be optionally present when compounds containing L¹ and/or L² are present in the polymerization, even when L² is not a π-allyl or π-benzyl group. It is believed that the Lewis acid, if present, may help to remove L¹ (if present) from the nickel atom, thereby facilitating the coordination of the olefin to the Ni atom. If a compound containing L¹ and/or L² does not act as a polymerization catalyst, it is suggested that a Lewis acid, such as those mentioned above be added to the process to determine if polymerization will then take place. Such testing requires minimal experimentation, and is illustrated in the Examples. Not surprisingly, with any particular set of polymerization process ingredients, some Lewis acids may be more effective than others.

The olefin or olefins are preferably selected from ethylene; a styrene; a norbornene; an α-olefin, cyclopentene; H₂C=CH(CH₂)₅CO₂R⁷⁷ and ethylene; ethylene and α-olefin; a styrene and a norbornene; and 2 or more norbornenes.

In preferred olefins herein, R⁶⁷ is hydrogen or n-alkyl containing 1 to 20 carbon atoms (an α-olefin), more preferably n-alkyl containing 1 to 8 carbon atoms, or more preferably hydrogen (e.g., ethylene) or methyl (e.g., propylene), and especially preferably hydrogen. A combination of ethylene and H₂C=CHR⁶⁷ wherein R⁶⁷ n-alkyl containing 1 to 8 carbon atoms is also preferred, and a combination of ethylene and propylene is more preferred. It is also preferred that s is 2 or more, and/or R⁷⁷ is alkyl, especially preferably methyl or ethyl. When H₂C=CH(CH₂)ₛCO₂R⁷⁷ is present as one of the olefins, it is preferred that R⁶⁷ is hydrogen.

While not all homopolymers and copolymers of the olefinic monomers useful herein can be made using the polymerization processes described herein, most homopolymers and many copolymers can be made. The following homopolymers can be readily made in these polymerization processes: polyethylene, polystyrene, a polynorbornene, poly-α-olefins (often lower molecular weight polymers obtained), polycyclopentene (often lower molecular weight polymers obtained). Attempted homopolymerization of functionalized norbornenes often does not proceed, nor do homopolymerizations of compounds of the formula H₂C=CH(CH₂)ₛCO₂R⁷⁷. Many copolymers can be made, including ethylene/α-olefins, styrene/norbornene copolymers, copolymers of 2 or more norbornenes including functionalized norbornenes, copolymers of ethylene and cyclopentene, copolymers of ethylene and a norbornene, and copolymers of ethylene and H₂C=CH (CH₂)ₛCO₂R⁷⁷ .

Not every variation of every nickel complex listed in the various polymerizations will make every one of the polymers listed immediately above. However, many of them will make most if not all of these types of polymers. While no hard and fast rules can be given, it is believed that for polymerizations which include ethylene and/or α-olefins, steric hindrance about the nickel atom caused by substituent groups is desirable for making polymers, especially higher molecular weight polymers, while for polymers containing one or more of a styrene and/or a norbornene such steric hindrance is not as important.

The Ni[II] complexes that are useful herein for the polymerization of ethylene contain a bidentate monoanionic ligand (other than a combined L¹ and L²)in which the coordinating atoms are 2 nitrogen atoms, a nitrogen atom and an oxygen atom, a phosphorous atom and a sulfur atom, or an oxygen atom and a sulfur atom. Compounds of formulas (I) through (VI), (XVIII), (XXVII), and (XXXVII)-(XXXX) can be made by reaction of 2 moles of the anionic form of the ligand with one mole of the appropriate nickel allyl or benzyl precursor (XXI ) , 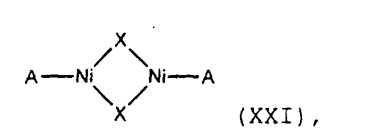 wherein X is preferably chlorine or bromine and A is a π-allyl or π-benzyl group, to form the nickel compound (see Examples 17-40 and 469-498).

Compounds of formulas (VII) through (XII), (XIX), (XXVIII), and (XXXXI)-(XXXXIV) can be synthesized by protonation of a suitable Ni[0] or Ni[II] precursor by the neutral ligand or by reaction of a suitable Ni[II] precursor with the anionic form of the ligand.
Examples of suitable Ni[0] and Ni[II] precursors include Ni(1,9-cyclooctadiene)₂,
(N,N,N'N'-tetramethylethylenediamine)NiMe₂, 2,2'-bipyridineNiMe₂, (MePPh₂)₃NiMe₂, [Ni(OMe)Me(PPh₃)]₂, [Ni(OMe)Me(PMe₃)₂, NiBr₂,
N,N,N'N'-tetramethylethylenediamine)Ni (acetylacetonate)₂, (1,2-dimethoxyethane)NiBr₂, N,N,N'N'-tetramethylethylenediamine)Ni(CH₂=CHCO₂CH₃)₂, (pyridine)₂Ni(CH₂=CHCO₂CH₃)₂, and (acetylacetonate)Ni(Et)(PPh₃). The addition of phosphine or ligand "sponges" such as CuCl, BPh₃ or tris(pentafluorophenyl)borane may aid such reactions.

Some of the nickel compounds herein such as (XXXIX), (XXXX), (XXXXIII) and (XXXXIV) may exist as "dimers" or monomers, or in equilibrium between the two. The dimer contains two nickel atoms, each nickel atom being coordinated to L¹ and L², wherein L¹ and L² combined may be a bidentate monoanionic ligand such as a π-allyl or π-benzyl group, and both Ni atoms "share" coordination to each of the other ligands present. As described herein, depiction of the monomeric compound also includes the dimeric compound, and vice versa. Whether any particular nickel compound is (predominantly) a monomer or dimer, or both states are detectable will depend on the ligands present. For instance it is believed that as the ligands become more bulky, especially about the nickel atom, the tendency is to form a monomeric compound.

Ligands for compounds (I) and (VII) of the formula 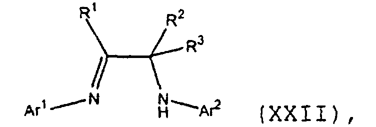 can be made by reaction of an alpha-diimine of the formula Ar¹N=CR¹-CR²=NAr² with one equivalent of a compound of the formula R³Li, see for instance M. G. Gardner, et al., Inorg. Chem., vol. 34 p. 4206-4212 (1995). In another case, a ligand of the formula 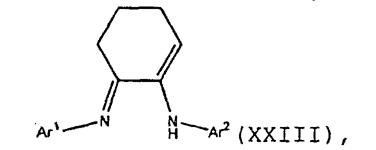 can be made by the condensation of 1,2-cyclohexadione with the corresponding aromatic amine(s), see for instance R. van Asselt, et. al, Recl. Trav. Chim. Pays-Bas, vol. 113, p. 88-98 (1994). Note that in (XXIII) R¹, R² and R³ taken together form a ring, with R² and R³ both "part of" a double bond to the same carbon atom. These ligands can then be converted to their corresponding nickel complexes by the methods described above.

Compounds of the formula (II) can be made by the reaction of a ligand of the formula 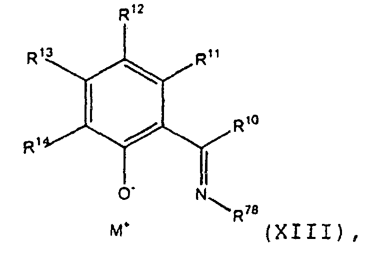 while compounds of the formula (VIII) can be made from the protonated form of (XIII). (XIII) can be made from the corresponding salicylaldehyde (when R¹⁰ is hydrogen) and aromatic amine, followed by reaction with an alkali metal base (such as an alkali metal hydride) to form the aryloxide.

(III) and (IX) can be made by reacting pyrrole-2-carboxyaldehyde with the appropriate aromatic amine to form the pyrrole-2-imine, followed by reaction with a strong base to form the pyrrole anion, and then reaction with the nickel precursors described above to form the nickel [II] complex.

Similarly, (IV) and (X) can be formed from an alkali metal thiophene-2-carboxylate and the nickel precursors described above.

When K is CR²⁷ the ligand for (V) and (XI) can be made by the reaction of the corresponding ketone (which may contain other functional groups) with an aromatic amine to give 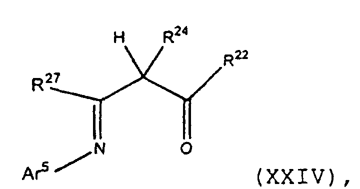 which is a tautomer of

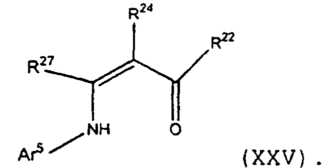

Useful ketones for making (V) and (XI) include ethyl acetoacetate, ethyl 2-ethylacetoacetate, isobutyl acetoacetate, t-butyl acetoacetate, S-t-butyl acetoacetate, allyl acetoacetate, ethyl 2-methylacetoacetate, methyl 2-chloroacetoacetate, ethyl 2-chloroacetoacetate, methyl 4-chloroacetoacetate, ethyl 4-chloroacetoacetate, ethyl 4,4,4-trifluoroacetoacetate, S-methyl 4,4,4-trifluoro-3-oxothiobutyrate, 2-methoxyethyl acetoacetate, methyl 4-methoxyacetoacetate, methyl propionylacetate, ethyl propionyl acetate, ethyl isobutyrylacetate, methyl 4,4-dimethyl-3-oxopentanoate, ethyl bytyrylacetate, ethyl 2,4-dioxovalerate, methyl 3-oxo-6-octenoate, dimethyl 1,3-acetonedicarboxylate, diethyl 1,3-acetonedicarboxylate, di-t-butyl 1,3-acetonedicarboxylate, dimethyl 3-oxoadipate, diethyl 3-oxopimelate, dimethyl acetylsuccinate, diethyl acetylsuccinate, diethyl 2-acetylglutarate, methyl 2-cyclopentatecarboxylate, ethyl 2-cyclopentanecarboxylate, ethyl 4-methyl-2-cyclohexanone-1-carboxylate, ethyl 4-methyl-2-cyclohexanone-1-carboxylate, ethyl 3-(1-adamantyl)-3-oxopropionate, methyl 2-oxo-1-cycloheptanecarboxylate, N-t-butylacetoamide, 2-chloro-N,N-dimethylacetoacetamide, 4,4,4-trifluoro-1-phenyl-1,3-butanedione, 4,4,4-trifluoro-1-(2-naphthyl)-1,3-butanedione, 2-acetyl-1-tetralone, ethyl 2-benzylacetoacetonate, methyl 1-benzyl-4-oxo-3-piperidinecarboxylate hydrochloride, benzyl acetoacetate, acetoacetanilide, o-acetoacetotoluide, N-(2,4-dimethylphenyl)-3-oxobutyramide, o-acetoacetanisidide, 4'-chloroacetoacetanilide, and 1,1,1-trifluoro-3-thianoylacetone.

When K is N in (V) and (XI), and R²⁴ is nitrile, the ligand can made by the reaction of R²²C (O) CH₂C_{N} with the diazonium salt of the corresponding arylamine, see for instance V.P. Kurbatov, et al., Russian Journal of Inorganic Chemistry, vol. 42, p. 898-902(1997). This paper also reviews methods of making ligands wherein K is CR²⁷ .

The boron containing ligands needed for compounds (VI) and (XII), 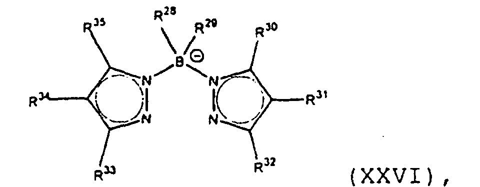 can be made by known procedures, see for instance S. Trofimenko, Prog. Inorg. Chem., vol. 34, p. 115-210 (1986) and S. Trofimenko, Chem. Rev., vol. 93, p. 943-980 (1993).

The synthesis of the tropolone-type ligands required for (XVIII) and (XIX) are described in J. J. Drysdale, et al., J. Am. Chem. Soc., vol. 80, p. 3672-3675 (1958); W. R. Brasen, et al., vol. 83, p. 3125-3138 (1961); and G. M. Villacorta, et al., J. Am. Chem. Soc., vol. 110, p. 3175-3182 (1988). These can be reacted as described above to form the corresponding nickel complex.

The ligand for (XXVII) and (XXVIII), 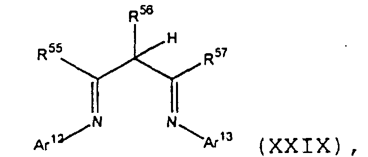 or either of its tautomers, 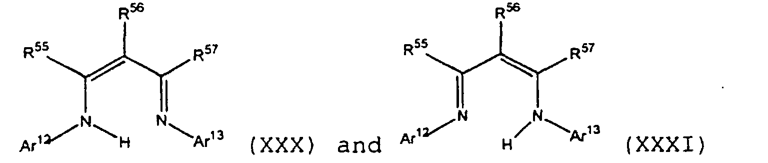 can be made by reaction of the appropriate α,χ-dioxo compound such as a 1,3-dione or 1,3-dial or similar compound with the appropriate aromatic amine, see for instance J. E. Parks, et al., Inorg. Chem., vol. 7, p. 1408 (1968); R. H. Holm, Prog. Inorg. Chem., vol. 14, p. 241 (1971); and P. C. Healy, et al., Aust. J. Chem., vol. 32, p. 727 (1979).

If the ligand precursor may form a tautomer, the ligand itself may usually be considered a tautomer. For instance, the monoanionic ligand derived from (XXIX) and it tautomers may be written as

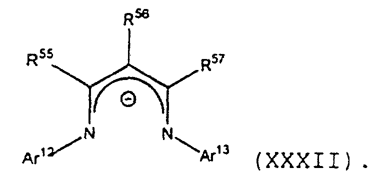

In (XXVII) and (XXVIII) when L and/or G is N, the ligand can be made by the method described in Y.A. Ibrahim, et al., Tetrahedron, vol. 50, p. 11489-11498 (1994) and references described therein.

The ligands for (XXXVII) and (XXXXI) can be made by methods described in Phosphorous, Sulfur and Silicon, vol. 47, p. 401 et seq. (1990), and analogous reactions.

The ligands for (XXXVIII) and (XXXXII) can be made by reacting R₂PLi (from R₂PH and n-BuLi) with propylene sulfide to form R₂CH₂CH(CH₃)SLi, and analogous reactions.

The ligands for (XXXIX) and (XXXXIII), and for (XXXX) and (XXXXIV) are commercially available. Those used herein were bought from Aldrich Chemical Co., Inc., Milwaukee, WI, U.S.A.

In the compounds (and ligands in those compounds) (I) through (XII), (XVIII), (XIX), (XXVII), (XXVIII), and (XXXVII)-(XXXXIV), certain groups are preferred. When present, they are:
R¹ and R² are both hydrogen; and/or
R³ is alkyl or aryl containing 1 to 20 carbon atoms, more preferably R³ is t-butyl; and/or
R¹, R² and R³ taken together are 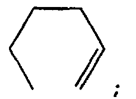 and/or
Ar¹, Ar², Ar³, Ar⁴, Ar⁵, Ar¹⁰ and Ar¹¹ are each independently 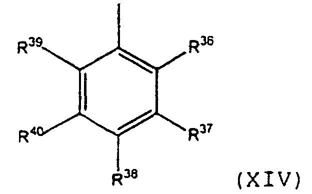 wherein R³⁶, R³⁷, R³⁸, R³⁹ and R⁴⁰ are each independently hydrogen, hydrocarbyl, substituted hydrocarbyl or a functional group, provided that any 2 of R³⁶, R³⁷, R³⁸, R³⁹ and R⁴⁰ that are vicinal to one another taken together may form a ring (for example the group 9-anthracenyl), and it is especially preferred that R³⁶ and R³⁹ are halo, phenyl or alkyl containing 1 to 6 carbon atoms, and it is more preferred that R³⁶ and R³ are methyl, bromo, chloro, t-butyl, hydrogen, or isopropyl; and/or
R⁷⁸ is Ar³, which is aryl or substituted aryl;
Ar¹, Ar², Ar³, Ar⁴, Ar⁵, Ar¹⁰ and Ar¹¹ are each independently 2-pyridyl or substituted 2-pyridyl;
if a π-allyl group is 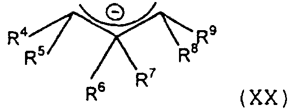
then R⁴, R⁵, R⁶, and R⁸ are hydrogen; and/or
R⁴, R⁵, R⁶, R⁷, R⁸ and R⁹ are hydrogen; and/or
R⁴, R⁵, R⁶, and R⁷ are hydrogen and R⁸ and R⁹ are methyl;
one of R⁷ or R⁹ is -CO₂R⁴¹ or chloro, and the other is hydrogen, and wherein R⁴¹ is hydrocarbyl, preferably alkyl containing 1 to 6 carbon atoms; and/or
R¹¹, R¹², R¹³ and R¹⁴ are each independently chloro, bromo, iodo, alkyl, alkoxy, hydrogen or nitro; and/or
R¹¹ and R¹² taken together form an aromatic carbocyclic 6-membered ring; and/or
R¹⁴ and R¹² are both chloro, bromo, iodo, t-butyl or nitro; and/or
R¹¹ and R¹³ are methoxy;
R¹⁴ is hydrogen and R¹² is nitro; and/or
one or more of R¹¹, R¹², R¹³ and R¹⁴ are hydrogen; and/or
R¹⁶, R¹⁷, R¹⁸, R¹⁹, R²⁰, and R²¹ are hydrogen; and/or
R¹⁶, R¹⁷, R¹⁸, R¹⁹, and R²⁰ are hydrogen and R²¹ is methyl; and/or
K is CR²⁷; and/or
R²⁷ is hydrogen, hydrocarbyl, substituted hydrocarbyl, or a functional group; and/or
R²⁷ is alkyl, more preferably methyl; and/or
R²⁴ is hydrogen, alkyl, cyano, or halo, more preferably hydrogen; and/or
R²² is hydrocarbyl or -OR¹¹⁷, , wherein R¹¹⁷ is hydrocarbyl, more preferably alkyl containing 1 to 6 carbon atoms, or R²² is phenyl; and/or
R³² and R³³ are both alkyl containing 1 to 6 carbon atoms or phenyl, more preferably isopropyl; and/or
R²⁸ and R²⁹ are both hydrogen or phenyl; and/or
R³⁰, R³¹, R³⁴ and R³⁵ are all hydrogen; and/or
R³¹ and R³² taken together and R³³ and R³⁴ taken together are both a 6-membered aromatic carbocyclic ring having a t-butyl group vicinal to the R³² and R³³ positions; and/or
R⁵⁰, R⁵¹, R⁵², R⁵³ and R⁵⁴ are hydrogen; and/or
L is CR⁵⁵ wherein R⁵⁵ is hydrocarbyl, hydrogen, or substituted hydrocarbyl; and/or
G is CR⁵⁷ wherein R⁵⁷ is hydrocarbyl, hydrogen or substituted hydrocarbyl; and/or
more preferably R⁵⁵ and R⁵⁷ are both alkyl or fluorinated alkyl, more preferably methyl; and/or
R⁵⁶ is hydrogen; and/or
Ar¹² and Ar¹³ are both 2,6-diisopropylphenyl; and/or
R⁷⁹, R⁸⁰, R⁸¹, R⁸², R⁸³, R⁸⁴ R⁸⁵, R⁸⁶, R⁸⁷, R⁸⁸ and R⁸⁹ are each independently hydrogen or alkyl; and/or
R⁹⁰, R⁹¹, R⁹² and R⁹³ are each independently hydrocarbyl, more preferably aryl, and especially preferably phenyl; and/or
R⁹⁴ and R⁹⁵ are each independently hydrocarbyl; and/or
R⁹⁶, R⁹⁷, R⁹⁸, and R⁹⁹ are each independently hydrogen or hydrocarbyl; and/or
E is N or CR¹⁰⁸; and/or
R¹⁰⁸ is hydrogen or hydrocarbyl; and/or
R¹⁰⁰, R¹⁰¹, R¹⁰², R¹⁰³, R¹⁰⁴, R¹⁰⁵, R¹⁰⁶, and R¹⁰⁷ is each independently hydrogen, hydrocarbyl,or halo; and/or
R¹⁰⁹, R¹¹⁰, R¹¹¹, R¹¹², R¹¹³, R¹¹⁴, R¹¹⁵ and R¹¹⁶ are each independently hydrogen or hydrocarbyl.

Specific preferred compounds (I)-(IV) and (VI) are given in Table A. The same groupings shown in the Table are preferred for the analogous compounds (VII)-(X) and (XII). In all of these compounds, where applicable, R⁴, R⁵, R⁶, R⁸, R⁹ [in (XX) above], R¹⁵, R¹⁶, R¹⁷, R¹⁸, R¹⁹, R²⁰, R³⁰, and R³⁵ are all hydrogen (with the exceptions in the footnotes), R¹⁰ is hydrogen or methyl, R²¹ is hydrogen or methyl, and R⁷ is -CO₂CH₃ (with the exceptions in the footnotes). In compounds wherein L₁ and L₂ appear, and especially in compounds of formula (VIII) it is preferred that L₁ is a nitrile, such as benzonitrile, p-methylbenzonitrile methyl nitrile, or pyridine or a substituted pyridine such as 2,6-dimethyl pyridine. A preferred L₂ is an alkyl group, especially methyl. L₁ and L₂ taken together may be a π-allyl or π-benzyl group, but for all compounds in which L₁ and L₂ are present (i.e. combined) it is preferred that they are not a π-allyl or π-benzyl group.

Table B give specific preferred compounds for (V), (XXXVII) and (XXXIX) as well as the corresponding compounds (XI), (XXXXI) and (XXXXIII) respectively. In all of these compounds Ar⁵ is 2,6-diisopropylphenyl, K is CCH₃, R²⁴, R⁷⁹, R⁸⁰, R⁸² , R⁸⁵, R⁸⁷ , R⁸⁸, and R⁸⁹ are hydrogen, R⁹⁰, R⁹¹, R⁹², and R⁹³ are phenyl.

In a specific preferred compounds (XXVII) and the corresponding (XXVIII), Ar¹² and Ar¹³ are 2,6-diisopropylphenyl, L and G are CCH₃, and R⁵⁶ is hydrogen.

In a specific preferred compound (XXXVIII) and the corresponding (XXXXVII), R⁹⁴ and R⁹⁵ are each cyclohexyl, R⁹⁶ , R⁹⁷ and R⁹⁸ are hydrogen, and R⁹⁹ is methyl.

In a specific preferred compound (XXXX) and the corresponding (XXXXIV), R¹¹⁰, R¹¹¹, R¹¹⁴ and R¹¹⁵ are hydrogen and R¹⁰⁹, R¹¹², R¹¹³ and R¹¹⁶ are methyl.

In a preferred compound (VIII), R¹⁰ is hydrogen or methyl; R⁷⁸ is Ar³, which is aryl or substituted aryl; and R¹¹, R¹², R¹³ and R¹⁴ are each independently chloro, bromo, iodo, alkyl, alkoxy, hydrogen or nitro or R¹¹ and R¹² taken together form a 6-membered carbocyclic ring and R¹³ and R¹⁴ are hydrogen.

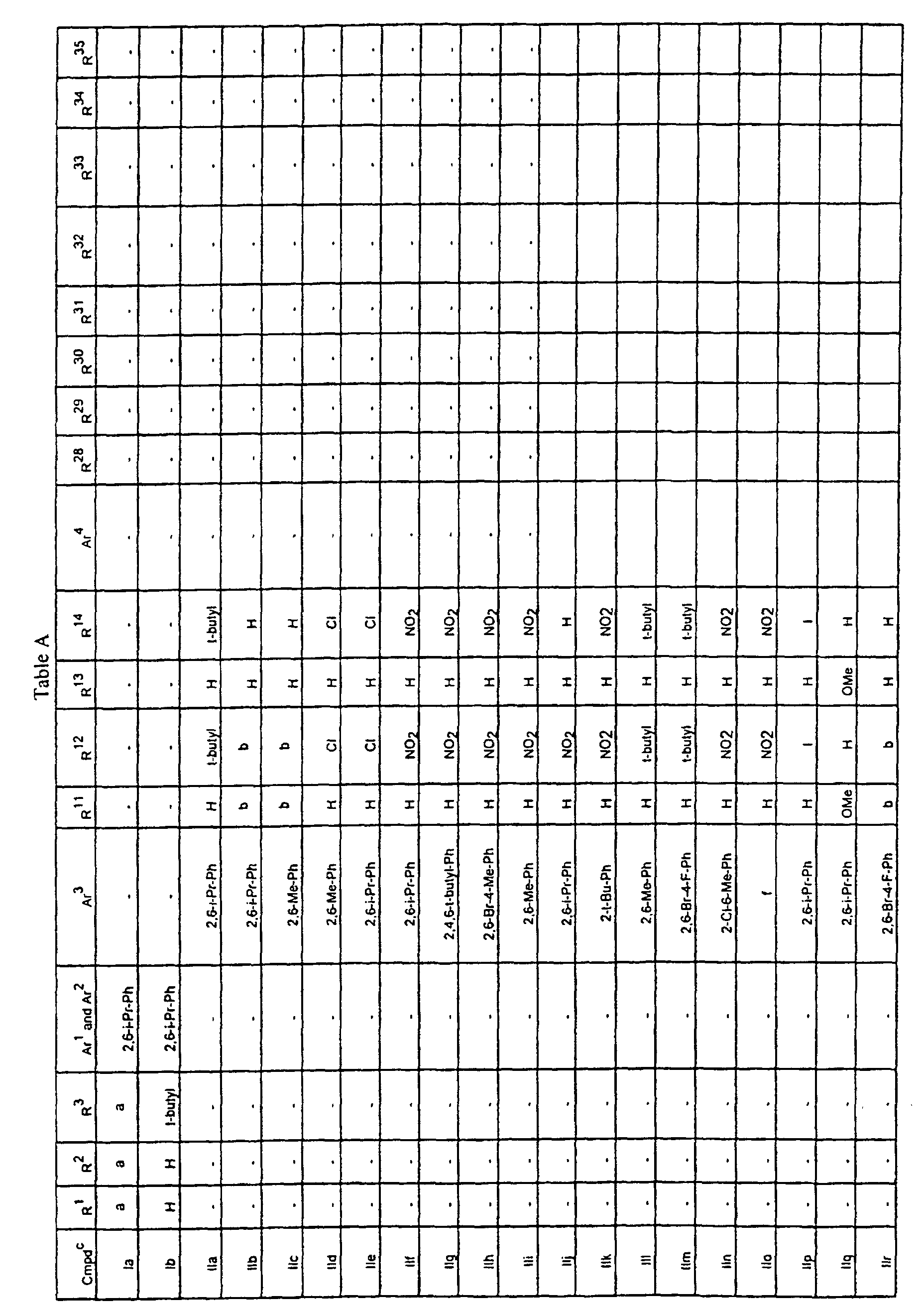

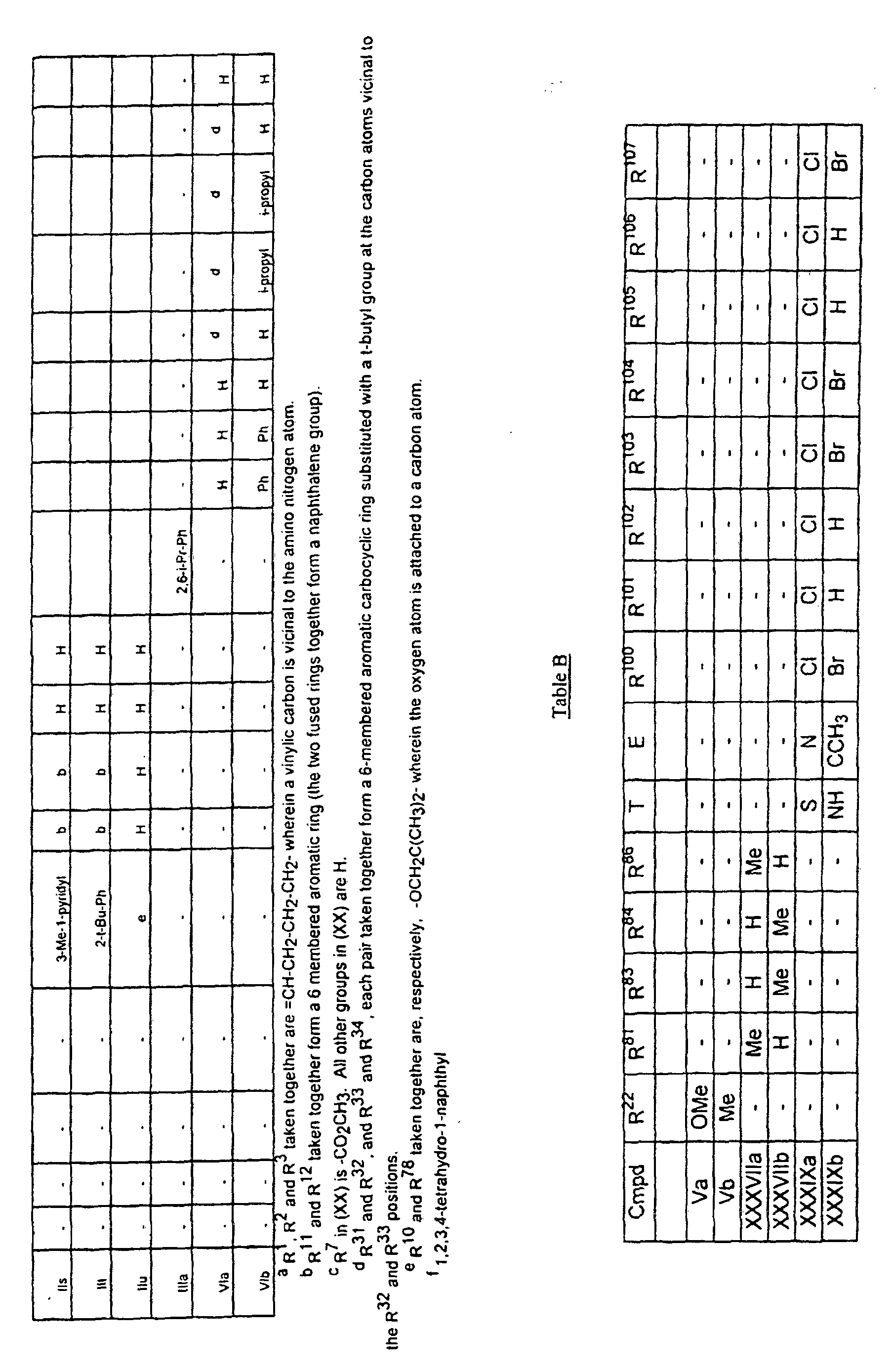

For clarity, the structures of compounds (Ia), (IIb) and (VIa) are shown below; 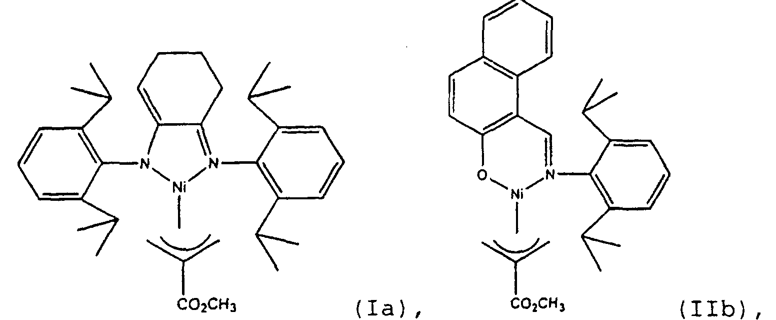 and

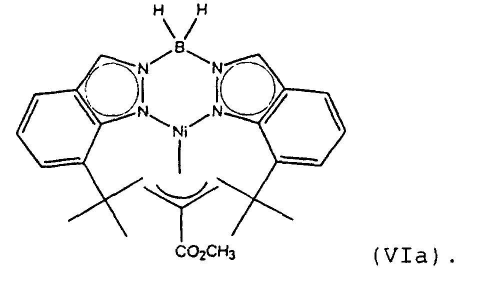

In (XXXIII) it is preferred that:
R⁵⁸, R⁵⁹, R⁶⁰, R⁶², R⁶³ and R⁶⁴ are each independently hydrogen, hydrocarbyl, substituted hydrocarbyl, or a functional group, and provided that any two of these groups vicinal to one another taken together may form a ring;
R⁶⁶ is hydrogen, hydrocarbyl or substituted hydrocarbyl; and
R⁶¹ and R⁶⁵ are each independently hydrocarbyl containing 2 or more carbon atoms, or substituted hydrocarbyl containing 2 or more carbon atoms.
R⁵⁸, R⁵⁹, R⁶⁰, R⁶², R⁶³ and R⁶⁴ are each hydrogen; and/or
R⁶⁶ is hydrogen; and/or
R⁶¹ and R⁶⁵ are each independently alkyl, and more preferred that both are isopropyl or methyl.

In a preferred compound or ligand (XVIII) and (XIX):
R⁵⁰, R⁵¹, R⁵², R⁵³ and R⁵⁴ are hydrogen; and/or
Ar¹⁰ and Ar¹¹ are 2,6-dialkyl substituted phenyl, more preferably 2,6-dimethylphenyl or 2,6-diisopropylphenyl.

Monoanionic ligands which the olefins herein may add to include hydride, alkyl, substituted alkyl, aryl, substituted aryl, or R²⁶C(=O)- wherein R²⁶ is hydrocarbyl or substituted hydrocarbyl, and groups π-allyl and π-benzyl groups such as η³-C₈H₁₃, see for instance J. P. Collman, et al., Principles and Applications of Organotransition Metal Chemistry, University Science Book, Mill Valley, CA, 1987. Such groups are also described in World Patent Application WO 96/23010.

In compound (XXXVI) it is preferred that R⁶⁸ is -OR¹¹⁷ or aryl, and/or R⁷⁵ is hydrocarbyl or substituted hydrocarbyl, and/or R⁷⁶ is hydrogen, hydrocarbyl, substituted hydrocarbyl or a functional group, more preferably hydrogen, hydrocarbyl or substituted hydrocarbyl.

In the second polymerization process described herein a nickel[II] complex such as any one of (VII)-(XII), (XIX), (XXVIII) or (XXXXI)-(XXXXIV) is either added to the polymerization process or formed in situ in the process. In fact, more than one such complex may be formed during the course of the process, for instance formation of an initial complex and then reaction of that complex to form a living ended polymer containing such a complex.

An example of such a complex which may be formed initially in situ is 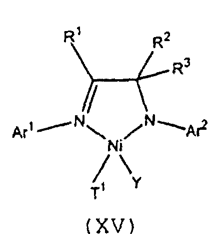 wherein R¹ through R³, Ar¹ and Ar² are as defined above, T¹ is hydride, alkyl, or R⁴²C(=O)- wherein R⁴² is hydrocarbyl or substituted hydrocarbyl or any other monoanionic ligand which ethylene may add to, and Y is a neutral ligand, or T¹ and Y taken together are a bidentate monoanionic ligand which ethylene may add to. Similar complexes may also be formed with the ligands in (VIII)-(XII), (XIX), (XXVIII) and (XXXXI)-(XXXXIV). Such complexes may be added directly to the process or formed in situ.

After the olefin polymerization has started, the complex may be in forms such as 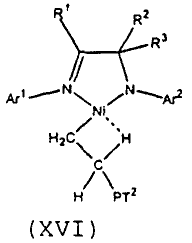 and 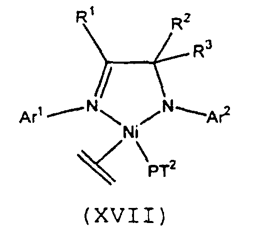 wherein R¹ through R³, Ar¹ and Ar² are as defined above, P is a divalent (poly)olefin group (the specific olefin shown in (XVI) and (XVII) is ethylene], -(CH₂)ₓ- wherein x is an integer of 2 or more, and T² is an end group, for example the groups listed for T¹ above. (XVI) is a so-called agostic form complex. Similar complexes may also be formed with the ligands in (VIII)-(XII), (XI), (XXVIII) and (XXXXI)-(XXXXIV). Analogous compounds with other olefins in place of ethylene also may be formed. In all the polymerization processes herein, the temperature at which the olefin polymerization is carried out is about -100°C to about +200°C, preferably about 0°C to about 150°C, more preferably about 25°C to about 100°C. The olefin concentration at which the polymerization is carried out is not critical, atmospheric pressure to about 275 MPa being a suitable range for ethylene and propylene.

The polymerization processes herein may be run in the presence of various liquids, particularly aprotic organic liquids. The catalyst system, olefin, and polyolefin may be soluble or insoluble in these liquids, but obviously these liquids should not prevent the polymerization from occurring. Suitable liquids include alkanes, cycloalkanes, selected halogenated hydrocarbons, and aromatic hydrocarbons. Hydrocarbons are the preferred solvent. Specific useful solvents include hexane, toluene, benzene, chloroform, methylene chloride, 1,2,4-trichorobenzene, p-xylene, and cyclohexane.

The catalysts herein may be "heterogenized" by coating or otherwise attaching them to solid supports, such as silica or alumina. Where an active catalyst species is formed by reaction with a compound such as an alkylaluminum compound, a support on which the alkylaluminum compound is first coated or otherwise attached is contacted with the nickel compound precursor to form a catalyst system in which the active nickel catalyst is "attached" to the solid support. These supported catalysts may be used in polymerizations in organic liquids, as described in the immediately preceding paragraph. They may also be used in so-called gas phase polymerizations in which the olefin(s) being polymerized are added to the polymerization as gases and no liquid supporting phase is present.

Included herein within the definitions of all the polymerization processes are mixtures of starting materials that lead to the formation in situ of the nickel compounds specified in all of the polymerization processes.

In the Examples all pressures are gauge pressures.

Quantitative ¹³C NMR data for the polymers was obtained using a 10 mm probe on typically 15-20% solutions of the polymer and 0.05M Cr(acetylacetonate)₃ in 1,2,4-trichlorobenzene are 120-140°C. For a full description of determination of branching by ¹³C and ¹H NMR, and for a definition of branches, see World Patent Application 96/23010, which is hereby included by reference.

In the Examples, the following abbreviations are used:
Am - amyl
Bu - butyl
Cy - cyclohexyl
E - ethylene
Et - ethyl
GPC - gel permeation chromatography
Me - methyl
MI - melt index
Mn - number average molecular weight
Mw - weight average molecular weight
MW - molecular weight
N - norbornene
P - propylene
PE - polyethylene
PDI - polydispersity, Mw/Mn
PMAO - poly(methylaluminoxane)
Pr - propyl
RI - refractive index
rt - room temperature
S - styrene
TCB - 1,3,5-trichlorobenzene
THF - tetrahydrofuran
Tm - melting point
tmeda - N,N,N',N'-tetramethylethylenediamine
TO - turnovers, moles of monomer polymerized per mole of catalyst (nickel compound) used

### Examples 1-16

### Ligand Syntheses

Ligand syntheses and deprotonations were carried out according to the general procedures given below unless stated otherwise. The general procedure for imine synthesis is based upon published procedures for the synthesis of N-aryl-substituted imines given in the following reference: Tom Dieck, H.; Svoboda, M.; Grieser, T. *Z. Naturforsch* **1981,** *36b,* 823 - 832. The synthesis of ArN=CH-CH(*t*-Bu)-N(Ar) (Li) [Ar = 2,6-(*i*-Pr)₂C₆H₃] is based on the published synthesis of (*t*-Bu)N=CH-CH(*t*-Bu)-N(*t*-Bu)(Li): Gardiner, M. G.; Raston, C. L. *Inorg. Chem.* **1995,** *34*, 4206 - 4212. The synthesis of ArN=C(Me)-CH=C(Me)-NH(Ar) [Ar = 2,6-(*i*-Pr)₂C₆H₃] was published in WO Pat. Appl. 96/23010, and it was deprotonated according to the general procedure given below. The bis(pyrazolyl)borate anions that were used to synthesize complexes 18 and 19 were provided by S. Trofimenko (DuPont) and were synthesized according to the procedures published in the following review: Trofimenko, S. *Chem. Rev.* **1993,** *93*, 943.

General Procedure for Imine Synthesis. In a fume hood, formic acid catalyst was added to a methanol solution of the aldehyde and the aniline (∼1.1 - 1.2 equiv). The reaction mixture was stirred and the resulting precipitate was collected on a frit and washed with methanol. The product was then dissolved in Et₂O or CH₂Cl₂ and stirred over Na₂SO₄ overnight. The solution was filtered through a frit with Celite® and the solvent was removed in vacuo to yield the product.

### General Procedure for the Synthesis of Sodium Salts.

The protonated forms of the ligands were dissolved in anhydrous THF in the drybox. Solid NaH was slowly added to the solution, and then the reaction mixture was stirred overnight. The next day, the solution was filtered through a frit with dry Celite®. The solvent was removed and the resulting powder was dried in vacuo. With some exceptions (e.g., Example 1), the sodium salts were not soluble in pentane and were further purified by a pentane wash.

### Example 1

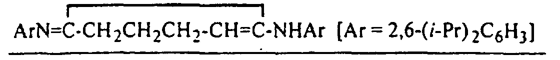

A drop of formic acid was added to a solution of 1,2-cyclohexanedione (0.25 g, 2.2 mmol) and 2,6-diisopropylaniline (0.85 mL, 4.5 mmol) in 5 mL of methanol. The reaction mixture was stirred at rt for 3 days. The white solid thus formed was filtered, washed with a small amount of methanol and dried under vacuum. After recrystallization from hot methanol, the product (0.4 g; 41% yield; mp 81-83 °C) was isolated as white crystals: ¹H NMR (CDCl₃, 300 MHz, rt): δ 7.28 - 7.08 (m, 6, Haryl), 6.45 (s, 1, N*H*), 4.84 (t, 1, J = 4.6, -C*H*=CNHAr), 3.30 (septet, 2, J = 6.88, C*H*Me₂), 2.86 (septet, 2, J = 6.87, C' *H*Me₂), 2.22 (m, 4, ArN=CC*H*_{*2*}C*H*_{*2*}-), 1.75 (m, 2, C*H*_{*2*}C*H*=CNHAr), 1.24 and 1.22 (d, 12 each, CH*Me*_{*2*} and C'H*Me*_{*2*}); ¹³C NMR (CDCl₃, 300 MHz, rt) δ 162.1, 147.3, 145.8, 139.6, 137.0, and 136.4 (ArNH-*C-C*=NAr, Ar: Cᵢₚₛₒ, C_{*o*}; Ar' : Cipso, C_{*o*}), 126.5, 123.4, 123.3 and 122.9 (Ar: C_{*p*}, C_{*m*}; Ar': C_{*p*}, C_{*m*}), 106.0 (ArNHC=CH-), 29.3, 28.4 and 28.3 (ArNHC=CH-CH₂CH₂CH₂C=NAr), 24.2 and 23.30 (*C*HMe2, *C*'HMe₂), 23.25 and 22.9 (CH*Me*₂, C'H*Me*_{*2*}).

The sodium salt was cleanly synthesized according to the above general procedure: ¹H NMR (300 MHz, THF-*d*_{*8*}): no THF coordinated.

### Example 2

### ArN=CH-CH(t-Bu)-N(Ar)(Li) [Ar = 2,6-(i-Pr)₂C₆H₃]

In a nitrogen-filled drybox, *t*-BuLi (7.81 mL of a 1.7 M solution in pentane) was filtered through a short plug of dry Celite® into a round bottom flask. The flask was cooled to -35°C in the drybox freezer. The diimine ArN=CH-CH=NAr [Ar = 2,6-(*i*-Pr₂)C₆H₃] was added as a solid over a period of 15 min to the cold *t*-BuLi solution. The reaction mixture was stirred for ∼2 h to give a viscous red solution. The solution was diluted with pentane and then filtered through a frit with Celite®. The resulting clear solution was concentrated under vacuum and then cooled in the drybox freezer to -35°C. An orange powder was obtained (3.03 g, 51.8%, 1st crop): ¹H NMR (THF-*d*_{*8*}, 300 MHz, rt) δ 8.29 (s, 1, C*H*=N), 7.08 (d, 2, J = 7.4, Ar: H_{*m*}), 7.00 (t, 1, J = 7.0, Ar: H_{*p*}), 6.62 (m, 2, Ar: H_{*m*}), 6.14 (t, 1, J = 7.4, Ar: H_{*p*}), 4.45 (s, 1, C*H*(*t*-Bu)), 3.08 (br septet, 2, C*H*Me₂), 3.05 (septet, 2, J = 6.8, C*H*Me₂), 1.35 (d, 3, J = 6.7, CH*Me*_{*2*}), 1.13 (d, 3, J = 7.0, CH*Me*_{*2*}), 1.13 (br s, 12, CH*Me*_{*2*}), 1.02 (d, 3, J = 6.7, CH*Me*_{*2*}), 0.93 (s, 9, C*Me*_{*3*}); ¹³C NMR (THF-*d*_{*8*}, 75 MHz, rt) δ 184.5 (N=*C*H), 161.9 and 150.1 (Ar, Ar': Cᵢₚₛₒ), 139.7, 139.5 (br), 139.0 (br) and 137.3 (Ar, Ar': C_{*o*}), 125.0, 124.0, 123.5, 122.4 and 112.2 (Ar, Ar': C_{*m*} and C_{*p*}), 80.8 (CH(*t*-Bu)), 41.5 (*C*Me₃), 29.3, 28.6, 27.8 (br), 26.5 (br), 26.3, 25.9, 25.6, 25.0 and 23.3 (br) (Ar, Ar': *C*H*Me*_{*2*}; C*Me*_{*3*}) .

### Example 3

### [2-(OH)-3,5-(t-Bu)₂C₆H₂]-CH=NAr [Ar = 2,6-(i-Pr)₂C₆H₃]

The general procedure for imine synthesis was followed using 10.1 g (43.0 mmol) of 3,5-di-*t*-butyl-2-hydroxybenzaldehyde and 9.91 g (55.9 mmol, 1.30 equiv) of 2,6-diisopropylaniline. A light yellow powder (10.5 g, 62.1%) was isolated: ¹H NMR (CDCl₃, 300 MHz, rt) δ 13.50 (s, 1, O*H*), 8.35 (s, 1, C*H*=NAr), 7.56 (d, 1, J = 2.7, H_{aryl}), 7.22 (m, 4, H_{aryl}), 3.08 (septet, 2, J = 6.8, C*H*Me₂), 1.55 (s, 9, C*Me*_{*3*}), 1.39 (s, 9, C'*Me*_{*3*})*,* 1.23 (d, 12, J = 6.7, CH*Me*_{*2*}).

The sodium salt was cleanly synthesized according to the above general procedure: ¹H NMR (300 MHz, THF-*d*_{*8*}): 0.63 equiv of THF coordinated.

### Example 4

### [2-(OH)-3,5-(t-Bu)₂C₆H₂]-CH=NAr [Ar = 2,6-Me₂C₆H₃]

The general procedure for imine synthesis was followed using 3.05 g (13.0 mmol) of 3,5-di-*t*-butyl-2-hydroxybenzaldehyde and 1.89 g (15.6 mmol, 1.20 equiv) of 2,6-dimethylaniline. A yellow powder (2.00 g, 45.6%) was isolated: ¹H NMR (CDCl₃, 300 MHz, rt, OH resonance not assigned) δ 8.34 (s, 1, C*H*=NAr), 7.50 and 7.16 (d, 1 each, H_{aryl}), 7.10 (d, 2, Ar: H_{*m*}), 7.01 (t, 1, Ar: H_{*p*}), 2.22 (s, 6, Ar: *Me*), 1.49 (s, 9, C*Me*_{*3*}), 1. 34 (s, 9, C'*Me*_{*3*}).

The sodium salt was cleanly synthesized according to the above general procedure: ¹H NMR (300 MHz, THF-*d*_{*8*}) : 0.51 equiv of THF coordinated.

### Example 5

### [2-(OH)-3,5-(t-Bu)₂C₆H₂]-CH=NAr [Ar = 2,6-Br₂-4-F-C₆H₂]

The general procedure for imine synthesis was followed using 2.12 g (9.05 mmol) of 3,5-di-*t*-butyl-2-hydroxybenzaldehyde and 1.89 g (10.8 mmol, 1.20 equiv) of 2,6-dibromo-4-fluoroaniline. A yellow powder (1.11 g, 25.5%) was isolated: ¹H NMR (CDCl₃, 300 MHz, rt, OH resonance not assigned) δ 8.45 (s, 1, C*H*=NAr), 7.54 (d, 1, H_{aryl}), 7.40 (d, 2, J_{HF} ~9, Ar: H_{*m*}), 7.19 (d, 1, H_{aryl}), 1.50 (s, 9, C*Me*_{*3*}), 1.35 (s, 9, C'*Me*_{*3*}).

The sodium salt was cleanly synthesized according to the above general procedure: ¹H NMR (300 MHz, THF-*d*_{*8*}) : 0.58 equiv of THF coordinated.

### Example 6

### [2-(OH)-3,5-(NO₂)₂C₆H₂]-CH=NAr [Ar = 2,6-(i-Pr)₂C₆H₃]

The general procedure for imine synthesis was followed using 4.98 g (23.5 mmol) of 3,5-dinitro-2-hydroxybenzaldehyde and 4.16 g (23.5 mmol) of 2,6-diisopropylaniline. A yellow powder (6.38 g, 73.1%) was isolated: ¹H NMR (CDCl₃, 300 MHz, rt, OH resonance not assigned) δ 9.06 (d, 1, H_{aryl}), 8.52 (d, 1, H_{aryl}), 8.31 (d, 1, J ~ 6, C*H*=NAr), 7.40 (t, 1, Ar: H_{*p*}), 7.30 (d, 2, Ar: H_{*m*}), 2.96 (septet, 2, C*H*Me₂), 1.25 (d, 12, CH*Me*_{*2*}).

The sodium salt was cleanly synthesized according to the above general procedure: ¹H NMR (300 MHz, THF-*d*_{*8*}): 0.57 equiv of THF coordinated.

### Example 7

### [2-(OH)-3,5-(NO₂)₂C₆H₂]-CH=NAr [Ar = 2,4,6-(t-Bu)₃C₆H₂]

The general procedure for imine synthesis was followed using 3.00 g (14.1 mmol) of 3,5-dinitro-2-hydroxybenzaldehyde and 3.88 g (14.9 mmol, 1.06 equiv) of 2,4,6-tris(*t*-butyl)aniline. A yellow powder (4.78 g, 74.5%) was isolated: ¹H NMR (CDCl₃, 300 MHz, rt, OH resonance not assigned) δ 9.09 (d, 1, H_{aryl}), 8.41 (d, 1, H_{aryl}), 8.16 (d, 1, J ∼ 12, C*H*=NAr), 7.48 (s, 1, Ar: H_{*m*}), 1.38 (s, 18, C*Me*_{*3*}), 1.36 (s, 9 C'*Me*_{*3*}).

The sodium salt was cleanly synthesized according to the above general procedure: ¹H NMR (300 MHz, THF-*d*_{*8*}): 2 equiv of THF coordinated.

### Example 8

### (2-(OH)-3,5-(NO₂)₂C₆H₂]-CH=NAr [Ar = 2,6-Me₂C₆H₃]

The general procedure for imine synthesis was followed using 3.11 g (14.7 mmol) of 3,5-dinitro-2-hydroxybenzaldehyde and 1.96 g (16.1 mmol, 1.10 equiv) of 2,6-dimethylaniline. A yellow powder (3.63 g, 78.4%) was isolated: ¹H NMR (CDCl₃, 300 MHz, rt, OH resonance not assigned) δ 9.05 (d, 1, H_{aryl}), 8.52 (d, 1, H_{aryl}), 8.42 (d, 1, J ~ 9, C*H*=NAr), 7.22 (m, 3, Ar: H_{*p*} and H_{*m*}), 2.36 (s, 6, Ar: *Me*).

The sodium salt was cleanly synthesized according to the above general procedure: ¹H NMR (300 MHz, THF-*d*_{*8*}): 0.25 equiv of THF coordinated.

### Example 9

### [2-(OH)-3,5-(NO₂)₂C₆H₂]-CH=NAr [Ar = 2,6-Br₂-4-Me-C₆H₂]

The general procedure for imine synthesis was followed using 3.10 g (14.6 mmol) of 3,5-dinitro-2-hydroxybenzaldehyde and 4.64 g (17.5 mmol, 1.20 equiv) of 2,6-dibromo-4-methylaniline. A yellow powder (5.15 g, ∼76.8%) was isolated. The ¹H NMR spectrum of the product showed the presence of methanol, so the powder was dissolved in THF in the drybox under a nitrogen atmosphere and the solution was placed over molecular sieves for several days. The solution was then filtered through a frit with Celite® and the solvent was removed in vacuo: ¹H NMR (CDCl₃, 300 MHz, rt; OH resonance not assigned; ∼ 1 equiv of THF is present) δ 8.95 (d, 1, J = 2.8, H_{aryl}), 8.76 (s, 1, C*H*=NAr), 8.71 (d, 1, J = 2.8, H_{aryl}), 7.43 (s, 2, Ar: H_{*m*}), 2.31 (s, 3,Ar: *Me*).

The sodium salt was cleanly synthesized according to the above general procedure: ¹H NMR (300 MHz, THF-d₈) : 3 equiv of THF coordinated.

### Example 10

### [2-Hydroxynaphthyl]-CH=NAr [Ar = 2,6-(i-Pr)₂C₆H₃]

The general procedure for imine synthesis was followed using 20.1 g (117 mmol) of 2-hydroxy-1-naphthaldehyde and 24.8 g (140 mmol, 1.20 equiv) of 2,6-diisopropylaniline. A yellow-gold powder (30.8 g, 79.5%) was isolated: ¹H NMR (CDCl₃, 300 MHz, rt) δ 15.30 (d, 1, O*H*), 9.15 (d, 1, C*H*=N), 8.08 (d, 1, H_{naphthyl}), 7.98 (d, 1, H_{naphthyl}), 7.88 (d, 1, H_{naphthyl}), 7.60 (t, 1, H_{naphthyl}), 7.45 (t, 1, H_{naphthyl}), 7.35 (m, 3, Ar: H_{*m*} and H_{*p*}), 7.29 (d, 1, H_{naphthyl}), 3.20 (septet, 2, C*H*Me₂), 1.33 (d, 12, CH*Me*_{*2*}).

The sodium salt was cleanly synthesized according to the above general procedure ¹H NMR (300 MHz, THF-*d*_{*8*}): 0.5 equiv of THF coordinated.

### Example 11

### [2-Hydroxynaphthyl]-CH=NAr [Ar = 2,6-Me₂C₆H₃]

The general procedure for imine synthesis was followed using 33.7 g (196 mmol) of 2-hydroxy-1-naphthaldehyde and 28.4 g (235 mmol, 1.20 equiv) of 2,6-dimethylaniline. A golden yellow powder (47.2 g, 87.5%) was isolated: ¹H NMR (CDCl₃, 300 MHz, rt, OH resonance not assigned) δ 9.23 (d, 1, N=C*H*), 8.4 - 7.1 (m, 9, H_{aryl}), 2.41 (s, 6, Ar: *Me*).

The sodium salt was cleanly synthesized according to the above general procedure: ¹H NMR (300 MHz, THF-*d*_{*8*}): 0.5 equiv of THF coordinated.

### Example 12

### [2-(OH)-3,5-Cl₂C₆H₂]-CH=NAr [Ar = 2,6-(i-Pr)₂C₆H₃]

The general procedure for imine synthesis was followed using 8.67 g (45.4 mmol) of 3,5-dichloro-2-hydroxybenzaldehyde and 9.66 g (54.5 mmol, 1.20 equiv) of 2,6-diisopropylaniline. A light yellow powder (10.7 g, 67.3%) was isolated: ¹H NMR (CDCl₃, 300 MHz, rt) δ 13.95 (s, 1, O*H*), 8.20 (s, 1, C*H*=NAr), 7.50 (d, 1, H_{aryl}), 7.18 - 6.83 (m, 3, H_{aryl}), 7.23 (d, 1, H_{aryl}), 2.89 (septet, 2, C*H*Me₂), 1.16 (d, 12, CH*Me*_{*2*}); ¹³C NMR (CDCl₃, 75 MHz, rt) δ 165.1 (N=*C*H), 156.1, 145.0, 138.7, 132.9, 129.8, 128.6, 126.2, 123.4, 123.0 and 119.7 (C_{aryl}), 28.3 (*C*HMe₂), 23.6 (CH*Me*_{*2*}).

The sodium salt was cleanly synthesized according to the above general procedure: ¹H NMR (300 MHz, THF-*d*_{*8*}) : 0.5 equiv of THF coordinated.

### Example 13

### [2-(OH)-3,5-Cl₂C₆H₂]-CH=NAr [Ar = 2,6-Me₂C₆H₃]

The general procedure for imine synthesis was followed using 16.2 g (85.0 mmol) of 3,5-dichloro-2-hydroxybenzaldehyde and 11.3 g (93.5 mmol, 1.10 equiv) of 2,6-dimethylaniline. A yellow powder (18.2 g, 72.7%) was isolated: ¹H NMR (CDCl₃, 300 MHz, rt) δ 14.15 (s, 1, O*H*), 8.43 (s, 1, N=C*H*) , 7.65 (d, 1, J = 2.5, H_{aryl}), 7.41 (d, 1, J = 2.5, H_{aryl}), 7.30 - 7.18 (m, 3, Ar: H_{*m*} and H_{*p*}), 2.35 (s, 6, *M*e).

The sodium salt was cleanly synthesized according to the above general procedure: ¹H NMR (300 MHz, THF-*d*_{*8*}): 0.33 equiv of THF coordinated.

### Example 14

### [2-(OH)-5-(NO₂)C₆H₂]-CH=NAr [Ar = 2,6-(i-Pr)₂C₆H₃]

The general procedure for imine synthesis was followed using 5.22 g (31.2 mmol) of 5-nitro-2-hydroxybenzaldehyde and 6.65 g (37.5 mmol, 1.20 equiv) of 2,6-diisopropylaniline. A yellow powder (4.39 g, 43.1%) was isolated: ¹H NMR (CDCl₃, 300 MHz, rt, OH resonance not assigned) δ 8.38 (s, 1, C*H*=NAr)*,* 8.35 (d, 1, J = 3, H'ₘ to hydroxy), 8.30 (dd, 1, J = 9, 3, H_{*m*} to hydroxy), 7.23 (s, 3, Ar: H_{*m*} and H_{*p*}), 7.15 (d, 1, J = 9, H_{*o*} to hydroxy), 2.93 (septet, 2, C*H*Me₂), 1.20 (s, 12, CH*Me*_{*2*}).

The sodium salt was cleanly synthesized according to the above general procedure: ¹H NMR (300 MHz, THF-*d*_{*8*}): 0.25 equiv of THF coordinated.

### Example 15

### Pyrrole-2-(CH=NAr) [Ar = 2,6-(i-Pr)₂C₆H₃]

The general procedure for imine synthesis was followed using 5.00 g (52.6 mmol) of pyrrole-2-carboxaldehyde and 10.3 g (57.9 mmol, 1.1 equiv) of 2,6-diisopropylaniline. The compound was isolated as an off-white powder: ¹H NMR (CDCl₃, 300 MHz, rt) δ 10.96 (s, 1, N*H*), 8.05 (s, 1, N=C*H*), 7.26 (s, 3, Ar: H_{*m*}, H_{*p*}), 6.68, 6.29 and 6.24 (m, 1 each, H_{pyrrole}), 3.17 (septet, 2, J = 6.9, C*H*Me₂), 1.20 (d, 12, J = 7.2, CH*Me*_{*2*}); ¹³C NMR (CDCl₃, 75 MHz, rt) δ 152.6 (N=*C*H), 148.5, 138.9 and 129.9 (pyrrole: Cᵢₚₛₒ; Ar: Cᵢₚₛₒ, C_{*o*}), 124.5, 124.0, 123.2, 116.5 and 109.9 (pyrrole: 3 CH carbons and Ar: C_{*m*}, C_{*p*}), 27.9 (*C*HMe₂), 23.6 (CH*Me*_{*2*}).

The sodium salt was cleanly synthesized according to the above general procedure: ¹H NMR (300 MHz, C₆D₆/THF-*d*_{*8*}): 1 equiv of THF coordinated.

### Example 16

### (Ar) (H)N-C(Me)=CH-C(O)OMe [Ar = 2,6-(i-Pr)₂C₆H₃]

Concentrated HCl (2 drops) was added to a solution of methylacetoacetate (5.2 mL; 48.5 mmol) and 2,6-diisopropylaniline (8.58 g, 48.5 mmol) in methanol. The reaction mixture was stirred at room temperature for 30 h. The product (5.95 g; 45% yield; mp 125-127°C) was filtered, washed with a small amount of methanol, and then dried under vacuum. Additional product (3.79 g, 28%; mp 115-122°C) was isolated from the mother liquor: ¹H NMR (300 MHz, CDCl₃, rt): δ 9.78 (br s, 1, N*H*), 7.29 (t, 1, J = 8.1, Ar: H_{*p*}), 7.17 (d, 2, J = 8.2, Ar: H_{*m*}), 4.71 (s, 1, =C*H*), 3.70 (s, 3, OMe), 3.10 (septet, 2, J = 6.8, C*H*Me₂), 1.61 (s, 3, =C*Me*), 1.22 (d, 6, J = 6.8, CH*Me*Me'), 1.1.5 (d, 6, J = 6.7, CHMe*Me'*).

The sodium salt was cleanly synthesized according to the above general procedure: ¹H NMR (300 MHz, THF-*d*_{*8*}): no THF coordinated.

### Examples 17 - 40

### Synthesis of Nickel Complexes

General Synthesis of Nickel Allyl Initiators. A mixture of two equiv of the appropriate anionic ligand and one equiv of [(allyl)Ni(µ-X)]₂ (X = Cl or Br) was dissolved in THF. The reaction mixture was stirred for several h before being filtered. The solvent was removed in vacuo to yield the desired product. Depending on the solubility of the product, further purification was often carried out by dissolving the product in Et₂O or pentane and filtering again or washing the product with Et₂O or pentane. Due to ease of characterization and, especially, ease of initiation in the presence of a Lewis acid, typically allyl = (a) H₂CC(CO₂Me)CH₂. However, other allyl derivatives were also synthesized and their polymerization activity explored; these include allyl = (b) H₂CCHCH₂, (c) H₂CCHCHMe, (d) H₂CCHCMe₂, (f) H₂CCHCHCl, and (g) H₂CCHCHPh. The [(allyl)Ni(µ-X)]₂ precursors were synthesized according to the procedures published in the following reference: Wilke, G.; Bogdanovic, B.; Hardt, P.; Heimbach, P; Keim, W.; Kroner, M.; Oberkirch, W.; Tanaka, K.; Steinrucke, E.; Walter, D.; Zimmermann, H. *Angew. Chem. Int. Ed. Engl.* **1966,** *5*, 151-164.

Complexes **1 - 20** were synthesized according to the above general procedure and their structures, syntheses and characterization follow:

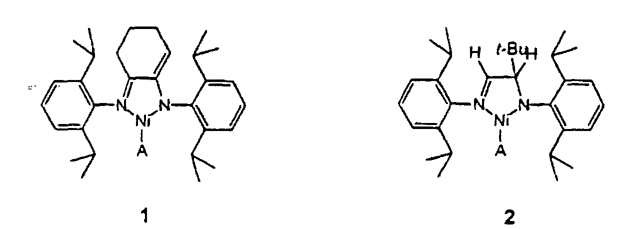

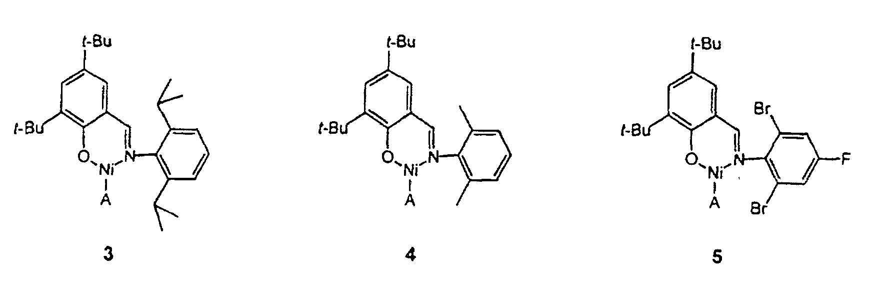

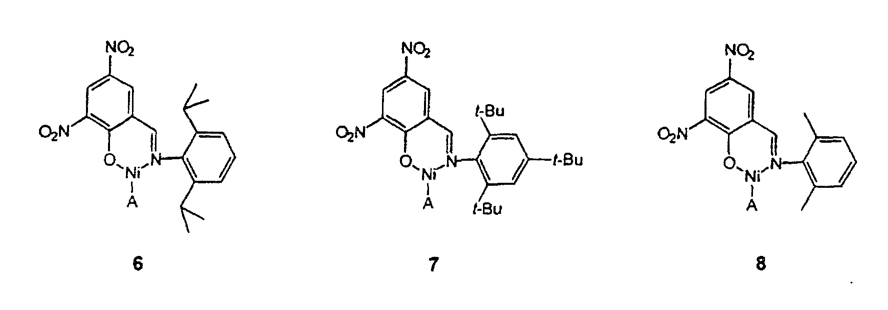

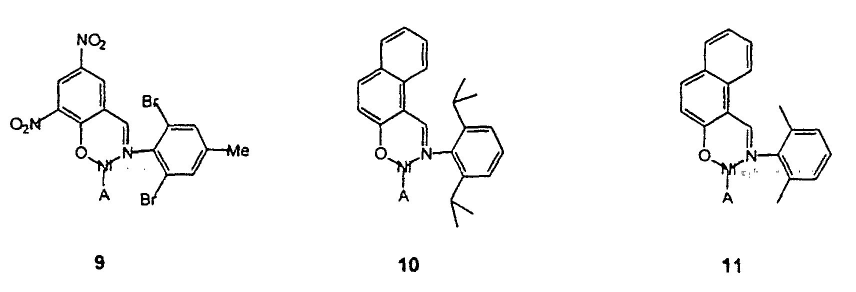

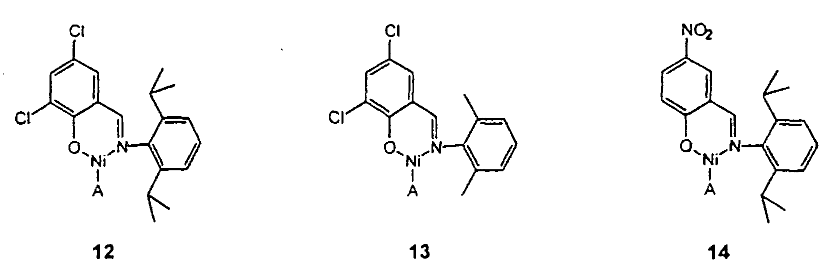

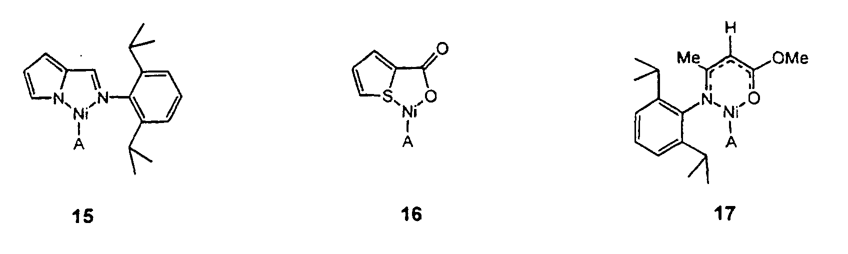

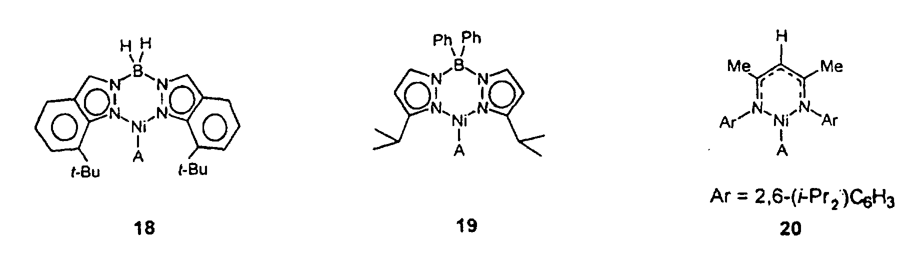

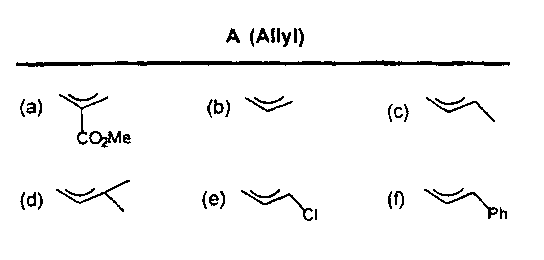

### Example 17

Complex 1a. Two equiv (610 mg, 1.35 mmol) of the sodium salt of the ligand were reacted with one equiv (321 mg, 0.674 mmol) of [(allyl)Ni(µ-Br)]₂ (allyl = H₂CC(CO₂Me)CH₂) to give 655 mg (82.6% yield) of a deep purple powder.

### Example 18

Complex 1d. Two equiv (667 mg, 1.47 mmol) of the sodium salt of the ligand were reacted with one equiv (306 mg, 0.737 mmol) of [(allyl)Ni(µ-Br)]₂ (allyl = H₂CCHCMe₂) to give a purple solid: ¹H NMR (C₆D₆, 300 MHz, rt, *H*_{*2*}CCHCMe₂ resonances not assigned) δ 7.25 - 6.79 (m, 6, H_{aryl}), 4.93 (t, 1, J = 4.6, ArNHC=C*H*-), 4.56 (br m, 1, H₂CC*H*CMe₂), 3.48 (septet, 2, J = 6.9, C*H*Me₂), 2.99 (septet, 2, J = 6.9, C'*H*Me₂), 2.07 (m, 2, Cy: C*H*_{*2*}), 1.92 (m, 2, Cy: C*H*_{*2*}), 1.42 (m, 2, Cy: C*H*_{*2*}), 1.2 - 1.1 (doublets, 24, C*H*Me₂, C'*H*Me₂), 0.72 and 0.61 (br s, 3 each, H₂CCHC*MeMe*').

### Example 19

Complex 2a. Two equiv (1.08 g, 2.44 mmol) of the lithium salt of the ligand were reacted with one equiv (581 mg, 1.22 mmol) of [(allyl)Ni(µ-Br)]₂ (allyl = H₂CC(CO₂Me)CH₂) to yield 1.35 g (93.8% yield) of a red powder. ¹H NMR spectrum in C₆D₆ is complex.

### Example 20

Complex 3a. Two equiv (4.01 g, 8.71 mmol) of the sodium salt of the ligand were reacted with one equiv (2.07 g, 4.35 mmol) of [(allyl)Ni(µ-Br)]₂ (allyl = H₂CC(CO₂Me)CH₂) to yield 3.61 g (75.2% yield) of a golden yellow powder. ¹H NMR (C₆D₆, 300 MHz, rt) δ 7.84 (s, 1, N=*CH*) , 7.44 and 6.92 (d, 1 each, H_{aryl}), 7.20 (m, 3, Ar: H_{*m*}, H'_{*m*} and H_{*p*}), 3.88 (d, 1, *H*H'CC(CO₂Me)CHH'), 3.86 (septet, 1, C*H*Me₂), 3.80 (s, 3, OMe), 3.04 (septet, 1, C'*H*Me₂), 2.91 (s, 1, HH'CC(CO₂Me)C*H*H'), 1.89 (m, 1, H*H*'CC(CO₂Me)CHH'), 1.43 (s, 1, HH'CC(CO₂Me)CH*H'*), 1.41 and 1.25 (s, 9 each, C*Me*_{*3*} and *C'Me*_{*3*}), 1.37, 1.27, 1.16 and 1.02 (d, 3 each, CH*MeMe'* and C'H*MeMe'*); ¹³C NMR (CD₂Cl₂, 75 MHz, rt) δ 166.6 (N=CH), 167.4, 164.7, 153.0, 141.3, 140.9, 139.9, 136.5, 117.7 and 110.9 (H₂C*C*(CO₂Me)CH₂; Ar: Cᵢₚₛₒ, C_{*o*}, C'_{*o*}; Ar': Cᵢₚₛₒ, C_{*o*}, C_{*m*}, C'_{*m*}), 130.2, 127.9, 126.8, 124.0 and 123.9 (Ar: Cₘ, C'_{*m*}, C_{*p*}; Ar': C_{*p*} and C'_{*o*}), 59.8 and 47.0 (H₂*C*C(CO₂Me)*C*H₂), 53.1 (CO₂*Me*), 35.9 and 34.3 (*C*Me₃ and *C*'Me₃), 31.6 and 30.0 (C*Me*_{*3*} and C'*Me*_{*3*}), 29.0, 28.5, 25.7, 25.6, 23.3 and 22.7 (CH*MeMe'* and *C*'H*MeMe'*).

Single crystals were formed by cooling a pentane solution of the complex to -35°C in the drybox freezer. The structure of the compound was solved by X-ray crystallography and is in agreement with the proposed structure.

### Example 21

Complex 4a. Two equiv (834 mg, 2.11 mmol) of the sodium salt of the ligand were reacted with one equiv (501 mg, 1.05 mmol) of [(allyl)Ni(µ-Br)]₂ (allyl = H₂CC(CO₂Me)CH₂) to give 935 mg (89.7% yield) of a golden yellow powder: ¹H NMR (THF-*d*_{*8*}, 300 MHz, rt) δ 7.94 (s, 1, N=C*H*), 7.40 (d, 1, H_{aryl}), 7.13 - 6.92 (m, 3, Ar: Hₘ, H'ₘ, H_{*p*}), 7.00 (d, 1, H_{aryl}), 3.78 (s, 3, OMe), 3.76 (d, 1, *H*H'CC(CO₂Me)CHH'), 2.80 (s, 1, HH'CC(CO₂Me)C*H*H'), 2.45 (s, 3, Ar: *Me),* 2.10 (s, 3, Ar: *Me'*), 1.85 (d, 1, H*H*'C(CO₂Me)CHH'), 1.60 (t, 1, HH'CC(CO₂Me)CH*H'*), 1.40 and 1.24 (s, 9 each, C*Me*_{*3*} and C'*Me*_{*3*}); ¹³C NMR (CD₂Cl₂, 75 MHz, rt) δ 166.2 (N=*C*H), 167.3, 164.3, 155.1, 141.1, 136.2, 130.0, 129.4, 118.0 and 110.3 (H₂C*C*(*C*O₂Me)CH₂, Ar: Cᵢₚₛₒ, C_{*o*}, C'_{*o*}; Ar': Cᵢₚₛₒ, C_{*o*}, C_{*m*}, C'_{*m*}), 129.8, 128.5, 128.4, 127.8 and 125.6 (Ar: C_{*m*}, C'_{*m*}, C_{*p*}; Ar': C_{*p*}, C'_{*o*}), 57.8 and 47.7 (H₂C*C*(CO₂Me)*C*H₂), 52.8 (OMe), 35.7 and 34.1 (*C*Me₃ and *C'*Me₃), 31.4 and 29.4 (C*Me*_{*3*} and C'*Me*_{*3*}), 19.0 and 18.4 (Ar: *Me* and *Me'*).

### Example 22

Complex 5a. Two equiv (390 mg, 0.709 mmol) of the sodium salt of the ligand were reacted with one equiv (169 mg, 0.355 mmol) of [(allyl) Ni (µ-Br)]₂ (allyl = H₂CC(CO₂Me)CH₂) to give 189 mg (45.8% yield) of a golden yellow powder: ¹H NMR (CDCl₃, 300 MHz, rt, broad resonances) δ 7.80 (br s, 1, N=C*H*) , 7.50 (br s, 1, H_{aryl}), 7.42 (br s, 1, Ar: H_{*m*}, H'_{*m*}), 6.96 (br s, 1, H_{aryl}), 3.92 (br s, 1, *H*H'CC(CO₂Me)CHH'), 3.86 (br s, 3, OMe), 2.84 (br s, 1, HH'CC(CO₂Me)C*H*H'), 1.98 and 1.76 (br s, 1 each, H*H*'CC(CO₂Me)CH*H*'), 1.43 and 1.29 (br s, 9 each, C*Me*_{*3*} and C'*Me*_{*3*}).

### Example 23

Complex 6a. Two equiv (900 mg, 2.10 mmol) of the sodium salt of the ligand were reacted with one equiv (500 mg, 1.05 mmol) of [(allyl)Ni(µ-Br)]₂ (allyl = H₂CC(CO₂Me)CH₂) to give 864 mg (77.9% yield) of a golden yellow powder: ¹H NMR (C₆D₆, 300 MHz, rt) δ 8.40 (d, 1, J = 3.0, H_{aryl}), 7.66 (d, 1, J = 3.0, H_{aryl}), 7.12 (s, 1, N=C*H*), 7.10 - 6.90 (m, 3, Ar: H_{*m*}, H'_{*m*}*,* H_{*p*}), 4.05 (m, 1, *H*H'CC(CO₂Me)CHH'), 3.49 (septet, 1, J = 6.9, C*H*Me₂), 3.21 (s, 3, OMe), 2.96 (septet, 1, J = 6.8, C'*H*Me₂), 2.67 (s, 1, HH'CC(CO₂Me)C*H*H'), 2.23 (m, 1, H*H'*CC(CO₂Me)CHH'), 1.34 (br s, 1, HH'CC(CO₂Me)CH*H'*), 1.36, 1.15, 0.95 and 0.84 (d, 3 each, J = 6.8, CH*MeMe'*, C'H*MeMe*').

### Example 24

Complex 6f. Two equiv (267 mg, 0.621 mmol) of the sodium salt of the ligand were reacted with one equiv (105 mg, 0.310 mmol) of [(allyl)Ni(µ-Cl)]₂ (allyl = H₂CCHCHCl) to give 245 mg (78.3% yield) of a golden yellow powder: ¹H NMR spectrum in C₆D₆ is complex.

### Example 25

Complex 7a. Two equiv (926 mg, 1.49 mmol) of the sodium salt of the ligand were reacted with one equiv (354 mg, 0.745 mmol) of [(allyl)Ni(µ-Br)]₂ (allyl = H₂CC(CO₂Me)CH₂) to give 861 mg (94.4% yield) of a golden yellow powder: ¹H NMR (C₆D₆, 300 MHz, rt) δ 8.43 (d, 1, J = 2.6, H_{aryl}), 7.81 (d, 1, J = 2.9, H_{aryl})*,* 7.48 (s, 2, Ar: H_{*m*}), 7.45 (s, 1 , N=C*H*), 4.12 (d, 1, J = 2.9, *H*H'C(CO₂Me)CHH'), 3.28 (s, 3, O*Me*), 2.84 (s, 1, HH'C(CO₂Me)C*H*H'), 2.44 (t, 1, J = 2.4, H*H'*C(CO₂Me)CHH'), 1.58, 1.41 and 1.28 (s, 9 each, *CMe*_{*3*}*,* C'*Me*_{*3*}, C"*Me*_{*3*}), 1.31 (d, 1, J = 1.1, HH' C (CO₂Me) CH*H*') ; ¹³C NMR (C₆D₆, 75 MHz, rt) δ 166.4 (N=*C*H), 165.5, 162.9, 151.0, 148.1, 144.9, 139.4, 138.8, 134.21, 120.5 and 113.4 (H₂C*C*(*C*O₂Me)CH₂; Ar: Ciₚₛₒ, C_{*o*}, C*'*_{*o*}, C_{*p*}; Ar': Cᵢₚₛₒ, C_{*o*}, Cₘ, C'ₘ), 134.16, 126.1, 125.1 and 124.7 (Ar: C_{*m*}, C'_{*m*} and Ar': C_{*p*} and C'_{*o*}), 63.3 and 49.0 (H₂C(CO₂Me)*C*H₂), 52.4 (OMe), 37.2 (*C*Me₃), 34.8, 34.4 and 31.4 *(CMe*_{*3*}*,* C'*Me*_{*3*} and C'*'Me*_{*3*}), (*C*'Me₃ and *C*"Me₃ overlap with CMe₃ or C*Me*_{*3*} or C'*Me*_{*3*} resonances).

### Example 26

Complex 8a. Two equiv (529 mg, 1.49 mmol) of the sodium salt of the ligand were reacted with one equiv (354 mg, 0.745 mmol) of [(allyl)Ni(µ-Br)]₂ (allyl = H₂CC(CO₂Me)CH₂) to give 662 mg (94.1% yield) of a golden yellow powder: ¹H NMR (CD₂Cl₂, 300 MHz, rt) δ 8.80 (d, 1, H_{aryl}), 8.40 (d, 1, H_{aryl}), 8.08 (s, 1, N=C*H*) , 7.14 (m, 3, Ar: H_{*m*}, H'_{*m*}, H_{*p*}), 3.82 (d, 1, *H*H'CC(CO₂Me)CHH'), 3.88 (s, 3, OMe), 3.00 .(s, 1, HH'C(CO₂Me)C*H*H'), 2.46 (s, 3, Ar: *Me*), 2.16 (m, 1, H*H'* CC (CO₂Me) CHH' ) , 2.14 (s, 3, Ar: *Me*'), 1.91 (s, 1, HH'CC(CO₂Me)CH*H*').

### Example 27

Complex 9a. Two equiv (1.46 g, 2.09 mmol) of the sodium salt of the ligand were reacted with one equiv (497 mg, 1.05 mmol) of [(allyl)Ni(µ-Br)]₂ (allyl = H₂CC(CO₂Me)CH₂) to give 1.42 g (96.0% yield) of a red powder: ¹H NMR (CD₂Cl₂, 300 MHz, rt) δ 8.79 (d, 1, H_{aryl}), 8.44 (d, 1, H_{aryl}), 8.06 (s, 1, N=C*H*), 7.51 and 7.49 (s, 1 each, Ar: H_{*m*}, H'_{*m*}), 3.96 (d, 1, *H*H'CC(CO₂Me)CHH'), 3.85 (s, 3, OMe), 3.65 (br s, ~ 1.25 equiv THF), 3.00 (s, 1, HH'CC(CO₂Me)C*H*H'), *2*.37 (s, 3, Ar: *Me*), 2.23 (m, 1, H*H'*CC(CO₂Me)CHH' ) , 2.13 (s, 1, HH'CC(CO₂Me)CH*H'*), 1.85 (br s, ∼ 1.25 equiv THF); ¹³C NMR (CD₂Cl₂, 75 MHz, rt) δ 168.3 (N=*C*H), 166.0, 163.6, 148.9, 142.7, 140.5, 134.3, 122.0, 117.3, 116.7 and 114 . 8 (H₂C*C*(*C*O₂Me)CH₂; Ar: Cᵢₚₛₒ, C_{*o*}, C'_{*o*}, C_{*p*}; Ar': Cᵢₚₛₒ, C_{*o*}, C_{*m*}, C'_{*m*}), 136.1, 133.5, 133.5 and 126.3 (Ar: C_{*m*}, C'_{*m*}; Ar': C_{*p*}, C'_{*o*}); 72.6 (br, THF), 61.2 and 51.4 (H₂*C*C(CO₂Me)*C*H₂), 53.6 (OMe), 34.9 (br, THF), 20.8 (Ar: *Me*).

### Example 28

Complex 10a. Two equiv (490 mg, 1.3 mmol) of the sodium salt of the ligand were reacted with one equiv (300 mg, 0.63 mmol) of [(allyl)Ni(µ-Br)]₂ (allyl = H₂CC(CO₂Me)CH₂) to give 259 mg (∼41% yield) of a yellow-green powder. About 12.5% of the isolated sample consists of a second species whose NMR spectrum is consistent with a (ligand)₂Ni(II) complex. The remainder is the allyl complex: ¹H NMR (C₆D₆, 300 MHz, rt) δ 8.75 (s, 1, N=C*H*) , 7.50 - 6.90 (m, 8, H_{aryl}), 6.03 (d, 1, J = 9.2, H_{aryl}), 4.16 (d, 1, J = 3.0, *H*H'CC(CO_{*2*}Me)CHH'), 3.92 (septet, 1, J = 6.9, C*H*Me₂), 3.33 (s, 3, OMe), 3.27 (septet, 1, J = 6.8, C'*H*Me₂), 2.83 (s, 1, HH'CC(CO_{*2*}Me)C*H*H'), 2.77 (dd, 1, J = 3.4, 1.6, H*H*'CC(CO₂Me)CHH'), 1.47 (dd, 1, J = 1.5, 0.9, HH'CC(CO_{*2*}Me)CH*H*'), 1.36, 1.20, 1.02 and 0.92 (d, 3 each, J = 6.5 - 6.8, CH*MeMe'*, C'H*MeMe'*). [Proposed (ligand)₂Ni(II) complex: δ 8.19 (s, 2, N=C*H*), 7.50 - 6.90 (m, 16, H_{aryl}), 6.12 (d, 2, H_{aryl}), 4.54 (septet, 4, J = 6.98, C*H*Me₂), 1.53 (d, 12, J = 6.8 CH*Me*Me'), 1.18 (d, 12, CHMe*Me'*).]

### Example 29

Complex 11a. Two equiv (487 mg, 1.32 mmol) of the sodium salt of the ligand were reacted with one equiv (314 mg, 0.660 mmol) of [(allyl)Ni(µ-Br)]₂ (allyl = H₂CC(CO₂Me)CH₂) to give 351 mg (~61.5% yield) of a yellow-green powder. About 17% of the isolated product consists of a second species whose NMR spectrum is consistent with a (ligand)₂Ni(II) complex; the remainder is the allyl complex: ¹H NMR (C₆D₆, 300 MHz, rt) δ 8.64 (s, 1, N=C*H*), 7.41 - 6.93 (m, 8, H_{aryl}), 6.05 (d, 1, J = 9.2, H_{aryl}), 4.07 (d, 1, J = 3.3, *H*H'CC(CO₂Me)CHH'), 3.30 (s, 3, OMe), 2.65 (s, 1, HH'CC(CO₂Me)C*H*H'), 2.28 (s, 3, Ar: *Me*), 2.16 (s, 4, Ar: *Me'* and H*H'*CC(CO₂Me)CHH'), 1.41 (br s, 1, HH'CC(CO₂Me)CH*H*'. [Proposed (ligand)₂Ni complex: δ 8.01 (s, 2, N=C*H*), 2.66 (s, 12, Ar: *Me*).]

### Example 30

Complex 11b. Two equiv (179 mg, 0.484 mmol) of the sodium salt of the ligand were reacted with one equiv (101 mg, 0.242 mmol) of [(allyl)Ni(µ-Br)]₂ (allyl = H₂CCHCMe₂) to give an orange-yellow powder (176 mg, 90.4%): ¹H NMR (C₆D₆, 300 MHz, rt) δ 8.65 (s, 1, N=C*H*), 7.48 - 6.94 (m, 9, H_{aryl}), 5.14 (dd, 1, J = 13.0, 7.9, H₂CC*H*CMe₂), 2.34 (s, 3, Ar: *Me*), 2.08 (s, 3, Ar: *Me*') 1.40 (d, 1, J = 7.7, *H*H'CCHCMe₂), 1.36 (d, 1, J = 13.1, H*H*'CCHCMe₂), 1.13 and 1.02 (s, 3 each, H₂CCHC*MeMe'*).

### Example 31

Complex 12a. Two equiv (862 mg, 2.11 mmol) of the sodium salt of the ligand were reacted with one equiv (501 mg, 1.05 mmol) of [(allyl)Ni(µ-Br)]2 (allyl = H₂CC(CO₂Me)CH₂) to give 951 mg (∼88.8% yield) of a yellow-green powder. About 10% of the isolated product consists of a second species whose NMR spectrum is consistent with a (ligand)₂Ni(II) complex; the remainder is the allyl complex: ¹H NMR (C₆D₆, 300 MHz, rt) δ 7.40 (s, 1, N=C*H*), 7.38 - 6.98 (m, 5, H_{aryl}), 4.13 (d, 1, J = 2.9, *H*H'CC(CO₂Me)CHH'), 3.61 (septet, 1, J = 6.9, C*H*Me₂), 3.27 (s, 3, OMe), 3.03 (septet, 1, J = 6.8, C'*H*Me₂), 2.78 (s, 1, HH'CC(CO₂Me)C*H*H'), 2.16 (t, 1, J = 1.7, H*H'*CC(CO₂Me)CHH'), 1.38 (br s, 1, HH'CC(CO₂Me)C*H*H'), 1.34, 1.16, 0.94 and 0.83 (d, 3 each, J = 6.6 - 7.0, CH*MeMe'*, C'H*MeMe'*); ¹³C NMR (C₆D₆, 75 MHz, rt, diagnostic resonances) δ 165.2 (N=*C*H), 61.9 and 48.7 (H₂*C*C(CO₂Me)*C*H₂), 52.3 (OMe), 28.7 and 28.4 (*C*HMe₂; *C'*HMe₂), 25.3, 25.3, 22.8 and 22.6 (CH*MeMe'*, C'H*MeMe'*). [Proposed (ligand)₂Ni complex: ¹H NMR (C₆D₆) δ 7.20 - 6.36 (m, 12, N=C*H* and H_{aryl}), 4.49 (septet, 4, J = 6.9, C*H*Me₂), 1.42 and 1.13 (d, 12 each, J = 7.0, *CHMeMe' )* ¹³C NMR (C₆D₆) δ 29.6: (*C*HMe₂), 24.4 and 23.6 (CH*MeMe'*).]

### Example 32

Complex 13a. Two equiv (491 mg, 1.26 mmol) of the sodium salt of the ligand were reacted with one equiv (300 mg, 0.632 mmol) of [(allyl)Ni(µ-Br)]₂ (allyl = H₂CC(CO₂Me)CH₂) to give 469 mg (∼82.4% yield) of a green powder. ∼13% of the isolated product consists of a second species whose NMR spectrum is consistent with a (ligand)₂Ni(II) complex; the remainder is the allyl complex: ¹H NMR (C₆D₆, 300 MHz, rt) δ 7.37 (d, 1, J = 2.6, H_{aryl}), 6.98 (s, 1, N=C*H*), 6.98 - 6.86 (m, 3, H_{aryl}), 6.56 (d, 1, J = 2.0, H_{aryl}), 4.05 (d, 1, J = 2.6, *H*H'CC(CO_{*2*}Me)CHH'), 3.23 (s, 3, OMe), 2.60 (s, 1, HH'CC(CO₂Me)C*H*H', overlaps with Ar: Me of dimer), 2.09 and 2.03 (s, 3 each, Ar: *Me, Me'*), 2.06 (m, 1, H*H'*CC(CO₂Me)CHH'), 1.31 (s, 1, HH'CC(CO₂Me)CH*H'*). [Proposed (ligand)₂Ni(II) complex: δ 2.60 (s, Ar: Me).]

### Example 33

Complex 14a. Two equiv (772 mg, 2.11 mmol) of the sodium salt of the ligand were reacted with one equiv (501 mg, 1.05 mmol) of [(allyl)Ni(µ-Br)]₂ (allyl = H₂CC(CO₂Me)CH₂) to give 891 mg (87.4% yield) of a yellow-orange powder: ¹H NMR (CD₂Cl₂, 300 MHz, rt) δ 8.25 (d, 1, Ar': H_{*o*}), 8.16 (dd, 1, Ar': H_{*p*}) , 7.98 (s, 1, N=C*H*), 7.24 (m, 3, Ar: Hₘ, *H'*_{*m*}*,* H_{*p*}), 6.90 (d, 1, Ar': H_{*m*}), 3.92 (d, 1, *H*H'CC(CO₂Me)CHH'), 3.86 (s, 3, OMe), 2.99 (septet, 1, C*H*Me₂), 3.02 (s, 1, H*H'*CC(CO₂Me)*CH*H' 2.98 (septet, 1, C'*H*Me₂), 2.08 (m, 1, H*H'*CC(CO₂Me)CHH'), 1.66 (t, 1, HH'CC(CO₂Me)CH*H'*), 1.39, 1.31, 1.17 and 1.01 (d, 3 each, CH*MeMe'* and C'H*MeMe'*).

### Example 34

Complex 15a. Two equiv (1.09 g, 3.13 mmol) of the sodium salt of the ligand were reacted with one equiv (743 mg, 1.56 mmol) of [(allyl)Ni(µ-Br)]₂ (allyl = H₂CC(CO₂Me)CH₂ to give 858 mg (66.7% yield) of a yellow-orange powder: ¹H NMR (C₆D₆, 300 MHz, rt) δ 7.20 - 7.00 (m, 5, N=C*H*; Ar: H_{*m*}, *H'*_{*m*}*,* H_{*p*}; H_{pyrrole}), 6.77 (m, 1, H_{pyrrole}), 6.42 (m, 1, H_{pyrrole}), 3.84 (m, 1, *H*H'CC(CO₂Me)CHH'), 3.65 (septet, 1, J = 6.8, C*H*Me₂), 3.30 (s, 3, OMe), 3.19 (septet, 1, J = 6.9, C'*H*Me₂), 2.85 (m, 1, HH'CC(CO₂Me)C*H*H'), 2.20 (d, 1, J = 0.89, H*H'*CC(CO₂Me)CHH'), 1.89 (d, 1, J = 0.89, HH'CC(CO₂Me)CH*H'*), 1.24, 1.18, 1.05 and 0.92 (d, 3 each, J = 6.8 - 7.1, CH*MeMe'*, C'H*MeMe'*); ¹³C NMR (C₆D₆, 75 MHz, rt) δ 162.5 (N=*C*H), 166.2, 148.7, 141.5, 141.4, 141.3, 140.8, 126.5, 123.43, 123.39, 118.8, 114.0 and 109.6 (H₂C*C*(*C*O₂Me)CH₂); C_{aryl}; C_{pyrrole}), 54.0 and 50.3 (H₂*C*C(CO₂Me)*C*H₂), 52.1 (OMe), 28.4 and 28.3 (*C*HMe₂, *C*'HMe₂), 25.1, 24.9, 23.0 and 22.5 (CH*MeMe'* and C'H*MeMe*').

### Example 35

Complex 16a. Two equiv (323 mg, 2.15 mmol) of the sodium salt of the ligand were reacted with one equiv (511 mg, 1.07 mmol) of [(allyl)Ni(µ-Br)]₂ (allyl = H₂CC(CO₂Me)CH₂) to give 322 mg (62.6% yield) of an off-white (slightly red) powder.

### Example 36

Complex 17a. Two equiv (987 mg, 3.32 mmol) of the sodium salt of the ligand were reacted with one equiv (789 mg, 1.66 mmol) of [(allyl)Ni(µ-Br)]₂ (allyl = H₂CC (CO₂Me) CH₂) to give 1.14 g (79.4% yield) of a bright yellow-orange powder: ¹H NMR (C₆D₆, 300 MHz, rt) δ 7.06 (br s, 3, H_{aryl}), 4.86 (d, 1, J = 1.2, ArNC (Me) C*H*CO₂Me), 4.04 (septet, 1, J = 6.7, C*H*Me₂), 3.90 (d, 1, J = 3.0, *H*H'CC(CO₂Me)CHH'), 3.37 and 3.36 (s, 3 each, (OMe)allyl and (OMe)_{ligand}), 3.24 (septet, 1, J = 7.0, C'*H*Me₂), 2.66 (s, 1, HH'CC(CO₂Me)C*H*H'), 2.01 (m, 1, H*H'*CC(CO₂Me)CHH'), 1.44 (s, 3, ArNC(*Me*)CHCO₂Me), 1.36, 1.29, 1.17 and 1.03 (d, 3 each, J = 6.2 - 6.9, CH*MeMe*', C' H*MeMe'*) *,* 1.14 (br s, 1, HH'CC(CO₂Me)CH*H'*); ¹³C NMR (C₆D₆, 75 MHz, rt) δ 170.5, 169.4, 166.7, 151.5, 147.3, 141.1, 140.1, 125.4, 123.7 and 109.2 (Ar: Cᵢₚₛₒ, C_{*o*}, C_{*o*}', C_{*m*}, C_{*m*}' , C_{*p*}; H₂C*C*(CO₂Me)CH₂; ArN*C*(Me)CH*C*O₂Me), 80.2 (ArNC(Me)*C*HCO₂Me), 60.8 and 46.3 (H₂*C*C(CO₂Me)*C*H₂), 52.0 and 50.9 (H₂CC(CO₂*Me*)CH₂, ArNC(Me)CHCO₂*Me*), 28.4 and 28.1 (*C*HMe₂, *C'*HMe₂), 24.5, 24.3, 24.3 and 23.6 (CH*MeMe'*, C'H*MeMe'),* 23.2 (ArNC (*Me*) CHCO₂Me) .

### Example 37

Complex 18a. Two equiv (1.20 g, 2.14 mmol) of the thallium salt of the ligand were reacted with one equiv (508 mg, 1.07 mmol) of [(allyl)Ni(µ-Br)]₂ (allyl = H₂CC(CO₂Me)CH₂) to give 730 mg (72.2% yield) of a red powder: ¹H NMR spectrum in C₆D₆ is complex.

### Example 38

Complex 19a. Two equiv (435 mg, 1.03 mmol) of the potassium salt of the ligand were reacted with one equiv (245 mg, 0.514 mmol) of [(allyl)Ni(µ-Br)]2 (allyl = H₂CC(CO₂Me)CH₂) to give 309 mg (60.4% yield) of a golden yellow powder. Some impurities are present, but the majority of the product is the allyl complex: ¹H NMR (CD₂Cl₂, 300 MHz, rt) δ 7.44 (s, 2, H_{pyrazole}), 7.4 - 7.0 (m, 10, H_{aryl}), 6.00 (s, 2, H_{pyrazole}), 3.91 (s, 3, OMe), 3.50 (s, 2, *H*H'CC(CO₂Me)C*H*H'), 2.96 (septet, 2, J = 6.8, C*H*Me₂), 1.27 (d, 6, J = 7.0, CH*Me*Me'), 1.19 (d, 6, J = 7.0, CHMe*Me*'), 0.90 (s, 2, H*H'*CC(CO₂Me)CH*H'*).

### Example 39

Complex 20a. Two equiv (583 mg, 1.32 mmol) of the sodium salt of the ligand were reacted with one equiv (315 mg, 0.662 mmol) of [(allyl)Ni(µ-Br)]₂ (allyl = H₂CC(CO₂Me)CH₂) to give 407 mg (53.6% yield) of a bright yellow-green powder: ¹H NMR (C₆D₆, 300 MHz, rt) δ 7.11 (m, 6, H_{aryl}), 5.04 (s, 1, NC(Me)C(*H*)C(Me)N), 4.04 (septet, 2, C*H*Me₂), 3.40 (septet, 2, C'*H*Me₂), 3.35 (s, 3, OMe), 2.29 (s, 2, *H*H'CC(CO₂Me)C*H*H'), 1.95 (s, 2, H*H*'CC(CO₂Me)CH*H*'), 1.62 (s, 6, NC(*Me*)C(H)C(*Me*)N), 1.38, 1.32, 1.20 and 1.07 (d, 6 each, CH*MeMe'*, C'H*MeMe'*).

### Example 40

Complex 20b. Two equiv (296 mg, 0.672 mmol) of the sodium salt of the ligand were reacted with one equiv (90.8 mg, 0.336 mmol) of [(allyl)Ni(µ-Cl)]₂ (allyl = H₂CCHCH₂) to give 151 mg (43.4% yield) of a bright yellow-orange powder: ¹H NMR (C₆D₆, 300 MHz, rt) δ 7.14 - 7.02 (m, 6, H_{aryl}), 5.84 (m, 1, H₂CC*H*CH₂), 5.04 (s, 1, NC(Me)C(*H*)C(Me)N), 4.05 (septet, 2, J = 6.9, C*H*Me₂), 3.43 (septet, 2, J = 6.9, C'*H*Me₂), 1.79 (d, 2, J = 12.8, *H*H'CCHC*H*H'), 1.64 (s, 6, NC(*Me*)C(H)C(*Me*)N), 1.53 (d, 2, J = 6.8, H*H*' CCHCH*H*'), 1.39 1.29, 1.21 and 1.10 (d, 6 each, J = 6.8 - 7.1, CH*MeMe'*, C' H*MeMe'*).

### Examples 41 - 130

### Ethylene and Propylene Polymerization Procedures and Reactions

The results of ethylene and propylene polymerizations catalyzed by complexes **1-20** under various reaction conditions (see general procedures and Table 1 below) are reported in Tables 2-5. The polymers were characterized by NMR, GPC, and DSC analysis. A description of the methods used to analyze the amount and type of branching in polyethylene samples by ¹³C NMR spectroscopy is given in WO Pat. Appl. 96/23010. GPC's were run in trichlorobenzene at 135°C and calibrated against polystyrene standards.

### General Procedure for the Screening of Ethylene Polymerizations by Nickel Allyl Initiators at 6.9 MPa Ethylene.

In the drybox, a glass insert was loaded with the isolated allyl initiator. The insert was cooled to -35°C in the drybox freezer, 5 mL of solvent (typically C₆D₆ or CDCl₃) was added to the cold insert, and the insert was cooled again. A Lewis acid cocatalyst [typically BPh₃ or B(C₆F₅)₃] was often added to the cold solution, and the insert was then capped and sealed. Outside of the drybox, the cold tube was placed under ethylene (typically 6.9 MPa) and allowed to warm to rt as it was shaken mechanically for approximately 18 h. An aliquot of the solution was used to acquire a ¹H NMR spectrum. The remaining portion was added to ~20 mL of MeOH in order to precipitate the polymer. The polyethylene was isolated and dried under vacuum.

### General Procedure for the Screening of Ethylene Polymerizations by Nickel Allyl Initiators at 28-35 kPa Ethylene with Polymethylaluminoxane (PMAO) Cocatalyst.

In the drybox, the nickel complex was placed in a Schlenk flask and dissolved in ∼ 20 mL of toluene. The flask was sealed, removed from the drybox and attached to an ethylene line where it was purged with first nitrogen and then ethylene. After purging with ethylene, PMAO was quickly added to the reaction mixture and the flask was placed under 28-35 kPa of ethylene. After being stirred overnight, the reaction mixture was quenched with ~15 mL of a solution of concentrated HCl in methanol (10:90 volume percent solution). The polymer was collected on a frit, washed with methanol and then acetone and then dried in vacuo overnight.

### General Procedure for the Screening of Propylene Polymerization by Nickel Allyl Initiators at 48 kPa Propylene with Polymethylaluminoxane (PMAO) Cocatalyst.

In the drybox, the nickel complex was placed in a Schlenk flask and dissolved in ∼ 10 mL of toluene. The flask was sealed, removed from the drybox and attached to an ethylene line where it was purged with first nitrogen and then propylene. After purging with propylene, PMAO was quickly added to the reaction mixture and the flask was placed under ∼ 48 kPa of propylene. After being stirred overnight, the reaction mixture was quenched with ∼ 10 mL of a solution of concentrated HCl in methanol (10:90 volume percent solution). The polymer was collected on a frit, washed with methanol and then acetone and then dried in vacuo overnight.

### General Procedure for the Screening of Propylene Polymerization by Nickel Allyl Initiators at 48 kPa Propylene with B(C₆E₅)₃ Cocatalyst.

In the drybox, the nickel complex was placed in a Schlenk flask and dissolved in ~10 mL of CH₂Cl₂. Two equiv of B(C₆F₅)₃ were dissolved in a minimal amount of CH₂Cl₂ and the solution was transferred to the Schlenk flask. The flask was sealed, removed from the drybox and attached to an ethylene line where it was purged with first nitrogen and then propylene. The flask was placed under ∼48 kPa of propylene and the reaction mixture was stirred overnight and then was quenched with ∼10 mL of a solution of concentrated HCl in methanol (10:90 volume percent solution). The polymer was collected on a frit, washed with methanol and then acetone and then dried in vacuo overnight

### General Procedure for the Screening of Propylene Polymerization by Nickel Allyl Initiators at 600 kPa Propylene with B(C₆F₅)₃ Cocatalyst.

In the drybox, the nickel complex was placed in a vessel and dissolved in ~20 mL of CH₂Cl₂. Two equiv of B(C₆F₅)₃ were dissolved in 10 mL of CH₂Cl₂ and placed in a separate vessel. Both vessels were sealed and removed from the drybox. The solution of the nickel complex was transferred to a 100 mL Parr reactor under vacuum and the solution of B(C₆F₅)₃ was transferred to the addition port of the same reactor. The B(C₆F₅)₃ solution was forced into the reactor ~600 kPa of propylene. The reactor pressure was maintained at 600 kPa and the reaction mixture was stirred for 3 h. Next, the reaction mixture was quenched with ∼ 10 mL of a solution of concentrated HCl in methanol (10:90 volume percent solution). If polymer was present, it was collected on a frit, washed with methanol and then acetone and then dried in vacuo overnight. Oligomers were characterized by GC analysis.

**Table I**

| Reaction Conditions Used in Ethylene and Propylene Polymerizations^{*a*} | |
|---|---|
| A | 5 mL C₆D₆, rt, 18 h, 6.9 MPa E, 2 equiv BPh₃ |
| B | 5 mL CDCl₃, 80°C, 18 h, 6.9 MPa E, 1 equiv B(C₆F₅)₃ |
| C | 5 mL C₆D₆, 80°C. 18 h, 6.9 MPa E. 2 equiv BPh₃ |
| D | 5 mL C₆D₆, rt, 18 h, 6.9 MPa E, I equiv B(C₆F₅)₃ |
| E | 5 mL C₆D₆, 80°C, 18 h, 6.9 MPa E, 2 equiv B[3,5-C₆H₃-(CF₃)₂]₃ |
| F | 5 mL CDCl₃, rt, 18 h, 6.9 MPa E, 2 equiv B[3,5-C₆H₃-(CF₃)₂]₃ |
| G | 5 mL CDCl₃, rt. 18 h, 6.9 MPa E, 2 equiv B(C₆F₅)₃ |
| H | 5 mL CDCl₃, 80°C, 18 h, 6.9 MPa E, 2 equiv B(C₆F₅)₃ |
| I | 20 mL toluene, rt, overnight, 28-35 kPa E, excess PMAO |
| J | 10 mL toluene, rt, overnight, 48 kPa P, excess PMAO |
| K | 10 mL CH₂Cl₂, rt, overnight, 48 kPa P, 2 equiv B(C₆F₅)₃ |
| L | 30 mL CH₂Cl₂, rt, 3 h, 600 kPa P, 2 equiv B(C₆F₅)₃ |

| | |
|---|---|
| ^{*a*}Abbreviations. E: Ethylene; P: Propylene; PMAO: Polymethylaluminoxane. | |

**Table 2**

| Polymerization of Ethylene by Compounds **1-20** at 6.9 MPa Ethylene | | | | | | | |
|---|---|---|---|---|---|---|---|
| Conditions A (5 mL C₆D₆, rt, 18 h, 2 equiv BPh₃) | | | | Conditions B (5 mL CDCl₃, 80°C, 18 h, 1 equiv B(C₆F₅)₃) | | | |
| Ex. | Cmpd ^{*b*} | PE (g)^{*c*} | TO^{*d*} | Ex. | Cmpd ^{*b*} | PE(g)^{*c*} | TO^{*d*} |
| 41 | **1a** | 0.43^{*e*} | 260 | 61 | **1a** | 0.40^{*f*} | 240 |
| 42 | **2a** | *a* | *a* | 62 | **2a** | 1.2 | 730 |
| 43 | **3a** | 4.1 | 2400 | 63 | **3a** | *a* | *a* |
| 44 | **4a** | 12.7 | 7500 | 64 | **4a** | *a* | *a* |
| 45 | **5a** | 5.1 | 3000 | 65 | **5a** | *a* | *a* |
| 46 | **6a** | 1.3^{*g*} | 590 | 66 | **6a** | 1.09 | 650 |
| 47 | **7a** | *a* | *a* | 67 | **7a** | 0.14 | 81 |
| 48 | **8a** | 0.29 | 170 | 68 | **8a** | 2.65 | 1600 |
| 49 | **9a** | 0.70 | 410 | 69 | **9a** | 2.5 | 1500 |
| 50 | **10a** | 0.33 | 200 | 70 | **10a** | *a* | *a* |
| 51 | **11a** | *a* | *a* | 71 | **11a** | 0.24 | 140 |
| 52 | **12a** | 0.15 | 87 | 72 | **12a** | 0.16 | 95 |
| 53 | **13a** | *a* | *a* | 73 | **13a** | 0.66 | 390 |
| 54 | **14a** | 1.1 | 640 | 74 | **14a** | 1.2 | 730 |
| 55 | **15a** | 0.14 | 80 | 75 | **15a** | 0.21 | 120 |
| 56 | **16a** | *a* | *a* | 76 | **16a** | 2.3 | 1400 |
| 57 | **17a** | 0.52 | 310 | 77 | **17a** | 1.33 | 780 |
| 58 | **18a** | 0.35 | 590 | 78 | **18a** | *a* | *a* |
| 59 | **19a** | 0.53 | 310 | 79 | **19a** | *a* | *a* |
| 60 | **20a** | 0.14 | 81 | 80 | **20a** | *a* | *a* |

| | | | | | | | |
|---|---|---|---|---|---|---|---|
| ^{a}Less that 0.1 g of polyethylene was isolated. | | | | | | | |
| ^{*b*}0.06 mmol | | | | | | | |
| ^{c}PE: Polyethylene. | | | | | | | |
| ^{d}TO: number of turnovers per metal center = (moles ethylene consumed, as determined by the weight of the isolated polymer or oligomers) divided by (moles catalyst). | | | | | | | |
| ^{*e*}1 equiv of B(C₆F₅)₃ was used (Conditions D in Table 1). | | | | | | | |
| ^{*f*}5 mL C₆D₆ and 2 equiv BPh₃ were used (Conditions C in Table 1). | | | | | | | |
| ^{*g*}1 equiv BPh₃ was used. | | | | | | | |

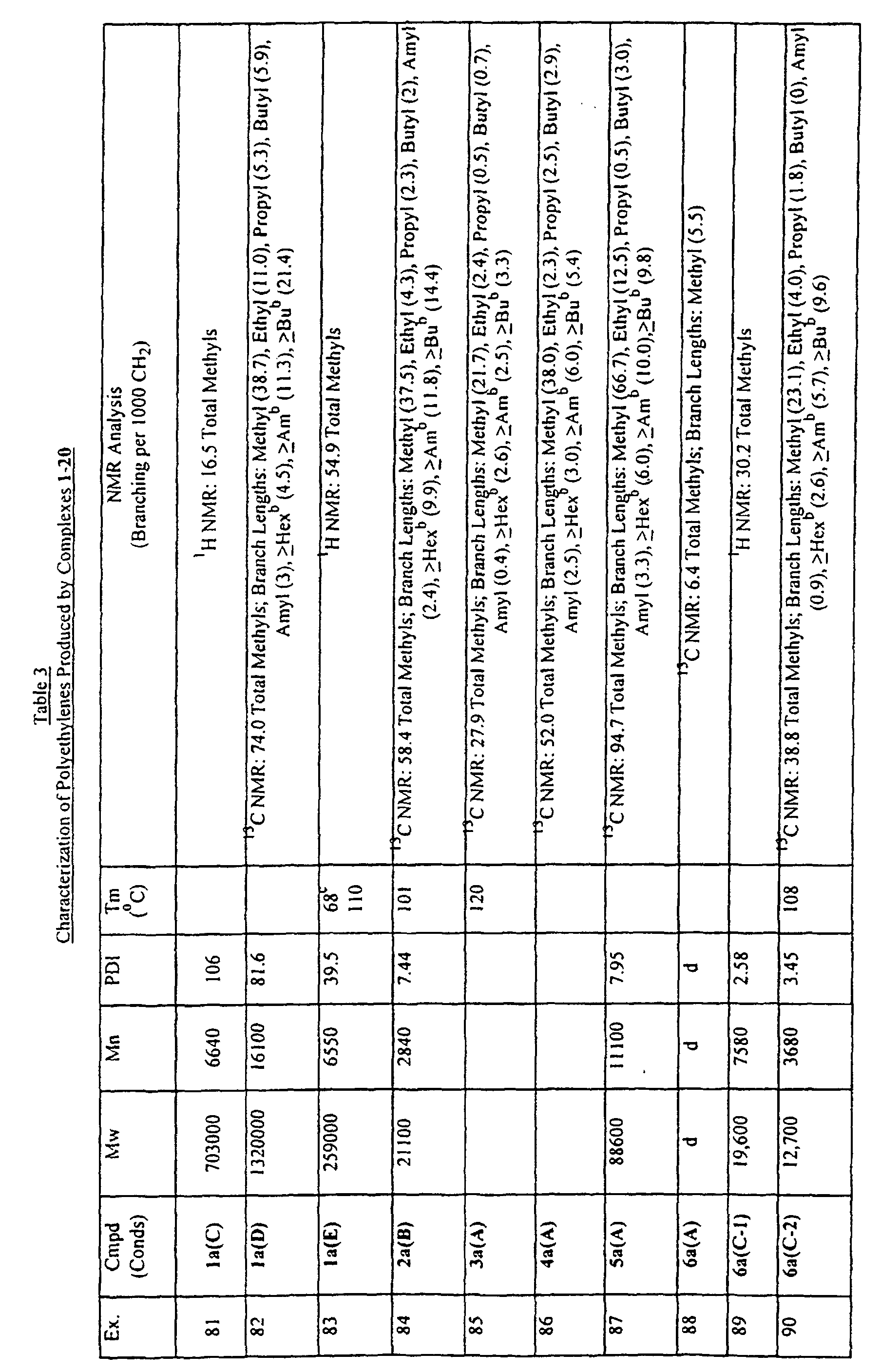

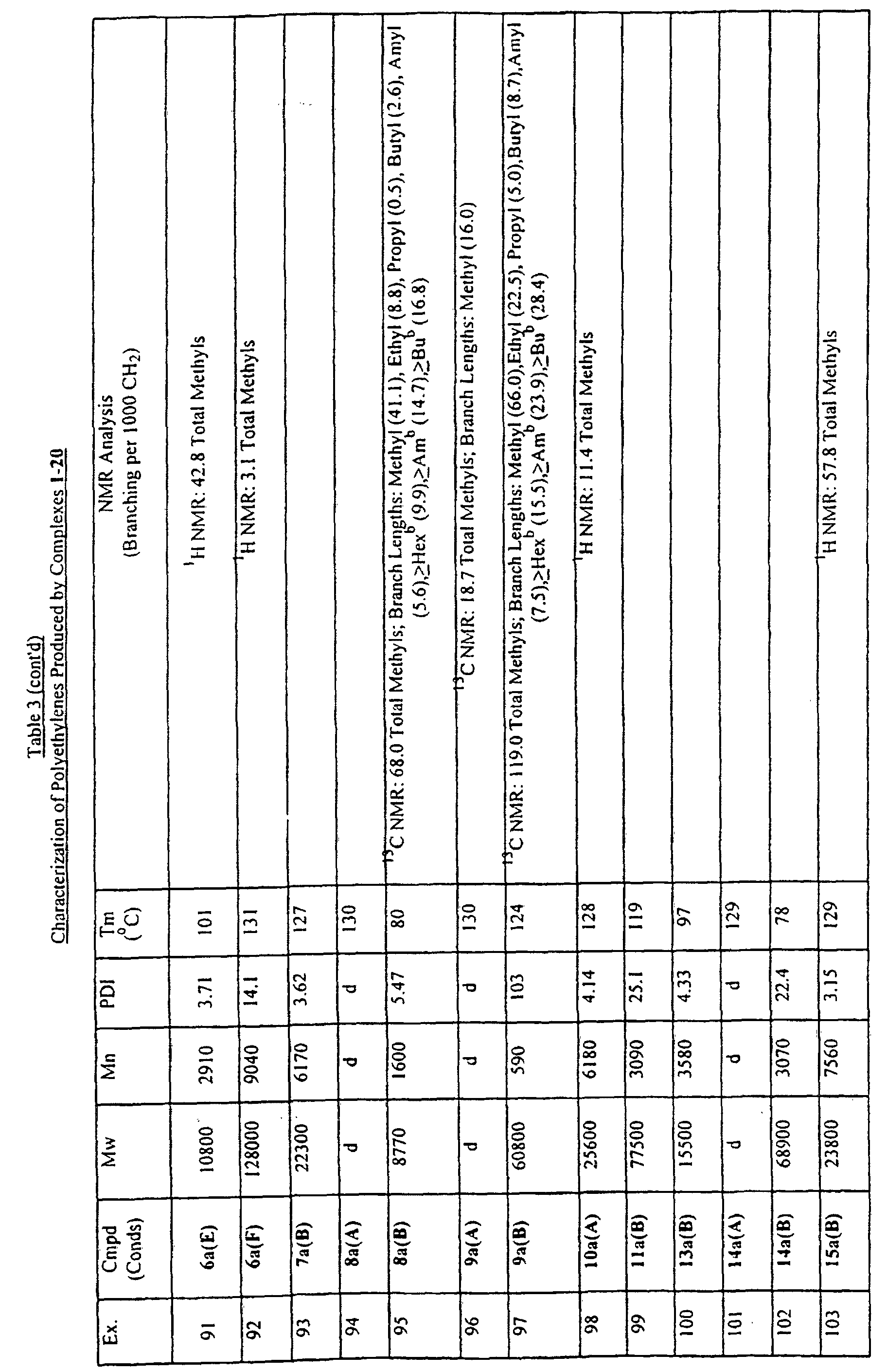

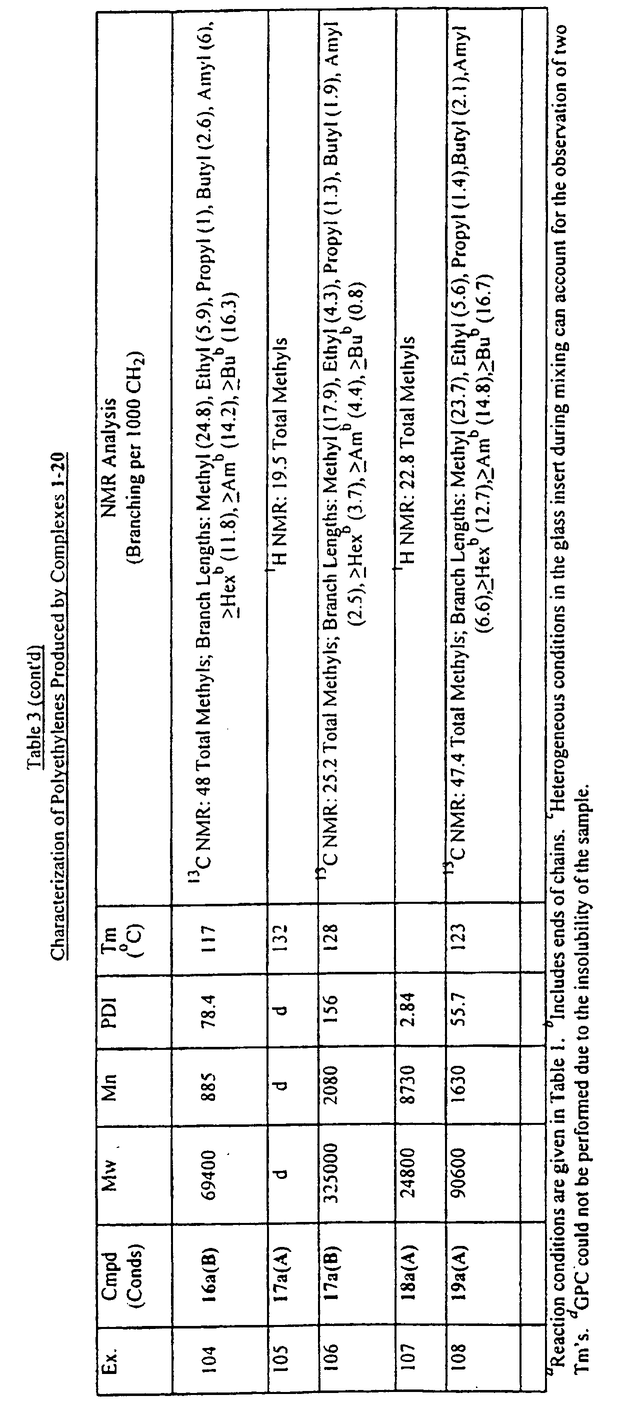

**Table 4**

| Polyethylene Yields: Demonstration of Effects of Reaction Conditions and Reproducibility of Yields Using Rapid Screening Techniques with Compound 6 | | | | | | |
|---|---|---|---|---|---|---|
| | | Polyethylene Yield (g) | | | | |
| Ex. | Reaction Conditions^{b} | Run 1 | Run 2 | Run 3 | Run 4 | Run 5 |
| 109 - 113 | A: 5 mL C₆D₆, rt, 18 h, 6.9 MPa E, 2 equiv BPh₃ | a | 0.10 | 0.10 | a | 1.3^{c} |
| 114 - 115 | B: 5 mL CDCl₃, 80°C, 18 h, 6.9 MPa E, 1 equiv B(C₆F₅)₃ | 0.10 | 0.28 | | | |
| 116 - 118 | C: 5 mL C₆D₆, 80°C, 18 h, 6.9 MPa E, 2 equiv BPh₃ | 9.50 | 9.55 | 0.49^{d} | | |
| 119 - 121 | G: 5 mL CDCl₃, rt, 18 h, 6.9 MPa E, 2 equiv B(C₆F₅)₃ | a | 0.65 | 7.78 | | |
| 122 - 123 | H: 5 mL CDCl₃, 80 °C, 18 h, 6.9 MPa E, 2 equiv B(C₆F₅)₃ | 1.09 | 1.09 | | | |

| | | | | | | |
|---|---|---|---|---|---|---|
| ^{*a*}less than 0.1 g of polyethylene was isolated. | | | | | | |
| ^{*b*}E: Ethylene; H²(CO₂Me)CH₂ initiator was used unless otherwise noted. | | | | | | |
| ^{*c*}1 equiv of BPh₃ was used. | | | | | | |
| ^{*d*}H₂CCHCHCl allyl initiator was used. | | | | | | |

**Table 5**

| Polymerization of Ethylene and Propylene at Low Pressures | | | | | | |
|---|---|---|---|---|---|---|
| Ex. | Cmpd (mmol) | Conds^{a} | Gas (kPa)^{b} | Polymer (g) | TO^{c} | Tₘ (°C) |
| 124 | **1a** (0.11) | **I** | E (27-35) | 2.91 | 970 | d |
| 125 | **6a** (0.12) | **I** | E (27-35) | 0.42 | 128 | 125 |
| 126 | **15a** (0.15) | **I** | E (27-35) | 0.11 | 27 | 121^{e} |
| 127 | **20a** (0.11) | **I** | E (27-35) | 0.27 | 88 | f |
| 128 | **1a** (0.06) | **J** | P (48) | 1.84 | 730 | g |
| 129 | **1a** (0.06) | **K** | P (48) | 0.21 | 81 | h |
| 130 | **6a** (0.06) | **L** | P (600) | 11.2ⁱ | 4400ⁱ | j |

| | | | | | | |
|---|---|---|---|---|---|---|
| ^{*a*}Reaction conditions are defined in Table 1. | | | | | | |
| ^{*b*}E: Ethylene. P: Propylene. | | | | | | |
| ^{*c*}TO: number of turnovers per catalyst center = (moles monomer consumed, as determined by the weight of the isolated polymer or oligomers) divided by (moles catalyst). | | | | | | |
| ^{d}Clear, rubbery amorphous PE. | | | | | | |
| ^{e}White, rubbery PE. | | | | | | |
| ^{f}white, crystalline PE. | | | | | | |
| ^{g}Clear rubbery PP. | | | | | | |
| ^{h}Clear sticky PP. | | | | | | |
| ⁱ14 mL of liquid oligomers were isolated. A density of 0.8 g/mL was assumed. | | | | | | |
| ^{j}GC analysis indicates that pentamers, hexamers and heptamers predominate. | | | | | | |

### Examples 131-136

### Styrene and Norbornene Homo- and Copolymerizations

In the subsequent examples describing polymerizations of styrene and norbornene, all manipulations were carried out in a nitrogen-purged drybox. Anhydrous solvents were used. The styrene (99+%, Aldrich, inhibited with 4-*tert*-butylcatechol) was degassed, filtered through basic alumina and inhibited with phenothiazine (98+%, Aldrich, 50 ppm) before use. The norbornene was purified by vacuum sublimation. Tacticities of polystyrenes were measured according to the following reference: T Kawamura et al., *Macromol. Rapid Commun.* **1994,** *15*, 479-486.

### General Procedure for Styrene Polymerizations.

The nickel complex (0.03 mmol) was slurried in dry toluene (6 mL) and styrene (1.3 mL, 1.18 g, 11.3 mmol) was added. Two equiv of B(C₆F₅)₃ were then added with vigorous stirring. The resulting mixture was shaken at rt in the dark for 16 h after which time the sample was removed from the drybox and MeOH was added to precipitate the polymer. The solid polymer was isolated, redissolved in CHCl₃ and reprecipitated with MeOH to remove catalyst impurities. The product was then collected on a frit, washed with MeOH and finally with a MeOH/acetone/Irganox® 1010 solution.

### General Procedure for Norbornene Polymerizations.

The nickel complex (0.03 mmol) was slurried in dry toluene (6 mL) and norbornene (1.6 g, 17.0 mmol) was added. Two equiv of B(C₆F₅)₃ were then added with vigorous stirring. The resulting mixture was shaken at rt. After 16 h, the sample was removed from the drybox and MeOH was added to precipitate the polymer. The solid polymer was isolated. The polymer was redissolved or swollen with solvent in order to remove catalyst impurities and then reprecipitated with MeOH. The product was then collected on a frit, washed with MeOH and finally with an acetone/2% Irganox® 1010 solution.

General Procedure for Styrene/Norbornene Copolymerizations. The nickel complex (0.03 mmol) was slurried in dry toluene (5 mL) and a mixture of norbornene (1.17 g, 12.4 mmol) and styrene (1.4 mL, 1.27 g, 12.2 mmol) in toluene (3 mL) was added. Two equiv of B(C₆F₅)₃ were then added with vigorous stirring. The resulting mixture was shaken at rt in the dark for 5 h. The sample was then removed from the drybox and MeOH was added to precipitate the polymer. The isolated polymer was dissolved (CHCl₃) and reprecipitated (MeOH) to remove the catalyst residue. The product was stirred overnight in acetone to remove polystyrene and then filtered, washed with MeOH and finally with an acetone/2% Irganox® 1010 solution.

**Table 6**

| Styrene (S) and Norbornene (N) Homo- and Copolymerizations | | | | | | | | |
|---|---|---|---|---|---|---|---|---|
| | | | | TO^{*a*} | | | | |
| Ex. | Cmpd | Monomers | Yield (%) | S | N | Mn^{*b*} | PDI | % S |
| 131 | **3a** | S | 79 | 300 | - | 2140 | 1.9 | 100^{*c*} |
| 132 | **6a** | S | 54 | 200 | - | 3390 | 1.8 | 100^{*c*} |
| 133 | **3a** | N | >95 | - | 570 | *d* | *d* | Ñ |
| 134 | **6a** | N | >95 | - | 570 | *d* | *d* | Ñ |
| 135 | **3a** | S, N | 21 | 13 | 170 | 9580 | 2.5 | 8 |
| 136 | **6a** | S, N | 7 | 4 | 56 | 15500 | 2.0 | 7 |

| | | | | | | | | |
|---|---|---|---|---|---|---|---|---|
| ^{*a*}Number of turnovers: TO = (moles monomer consumed, as determined by the weight of the isolated polymer) divided by (moles catalyst). | | | | | | | | |
| ^{*b*}Mₙ (GPC, TCB 120°C, polystyrene standards). | | | | | | | | |
| ^{*c*}¹³C NMR spectroscopy (CDCl₃) indicates enrichment in meso diad units relative to atactic polystyrene. | | | | | | | | |
| ^{*d*}Within 30 min the reaction mixture completely solidified and attempts to redissolve the polymer were unsuccessful. The insolubility of the polymer product indicates that an addition polymer of norbornene was formed. | | | | | | | | |

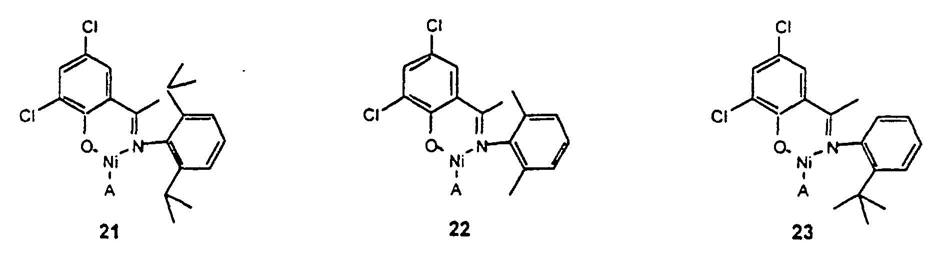

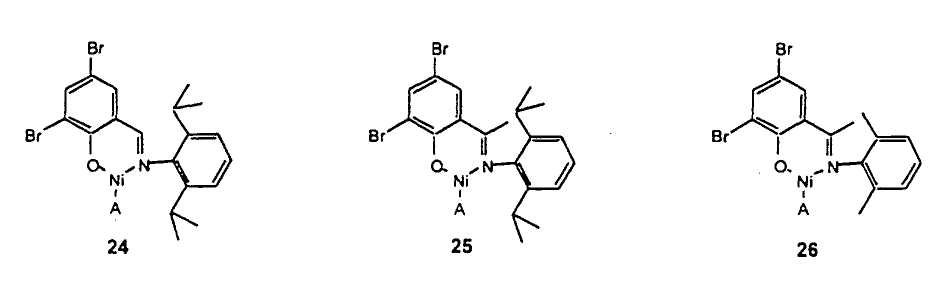

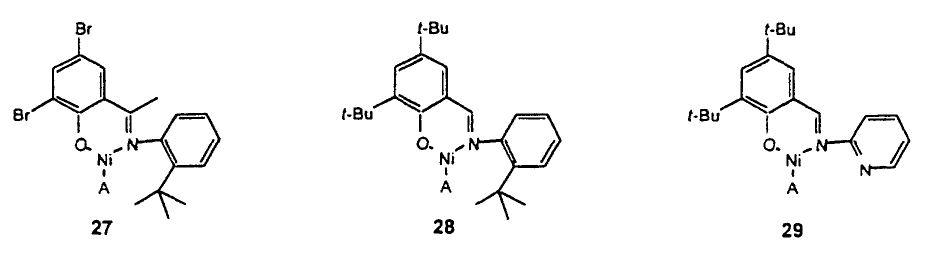

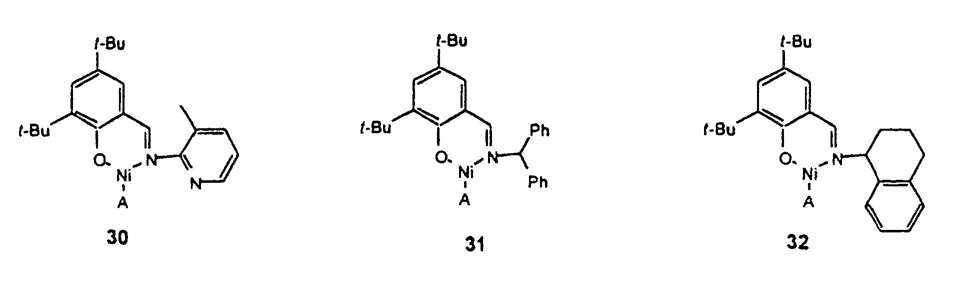

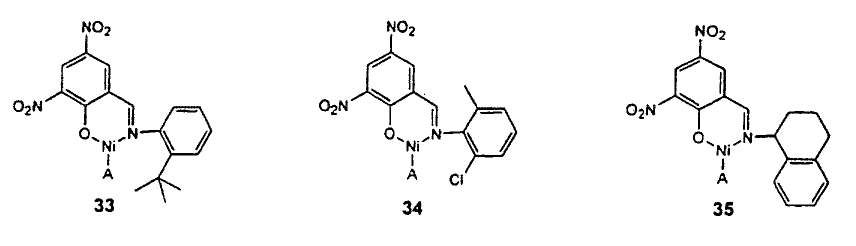

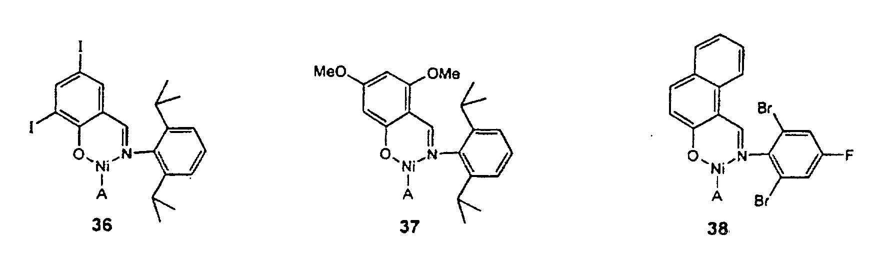

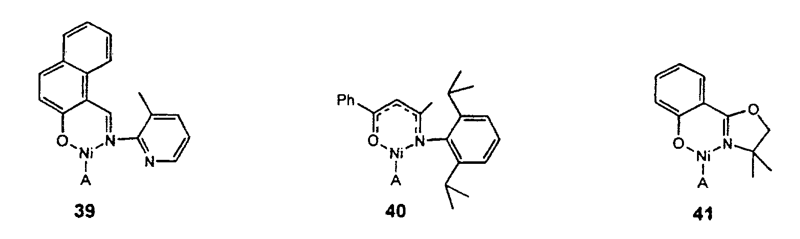

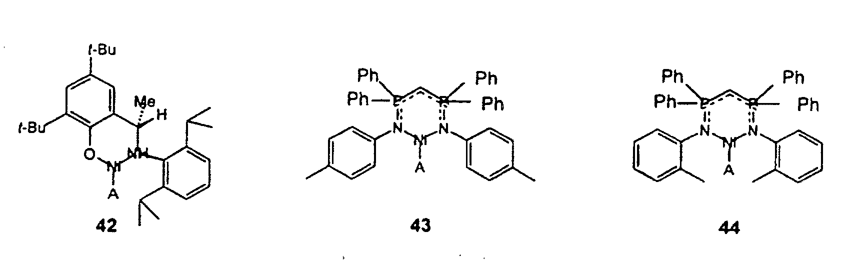

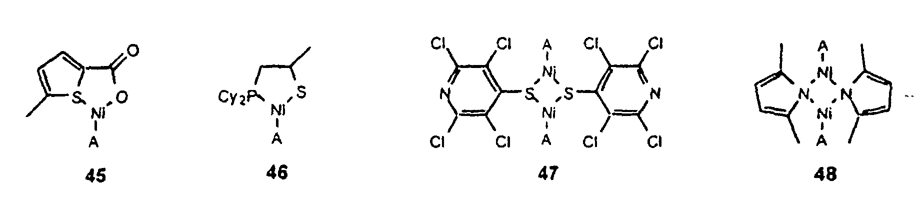

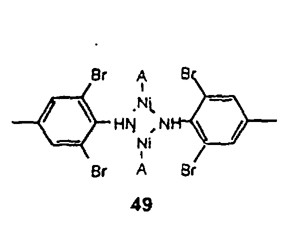

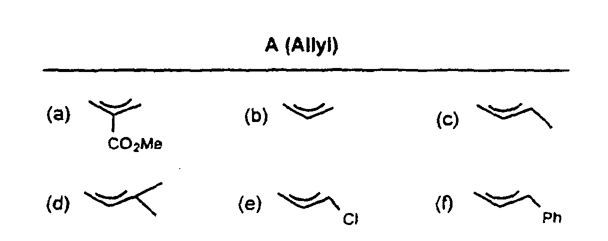

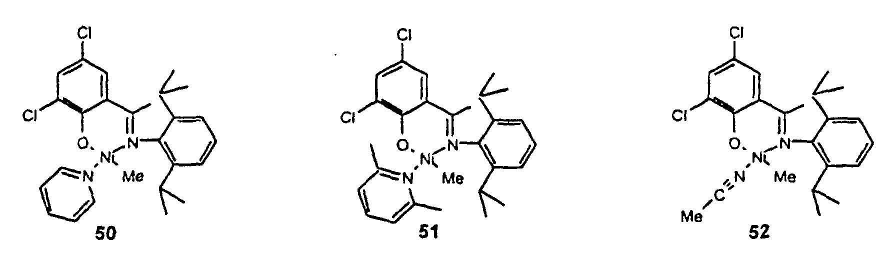

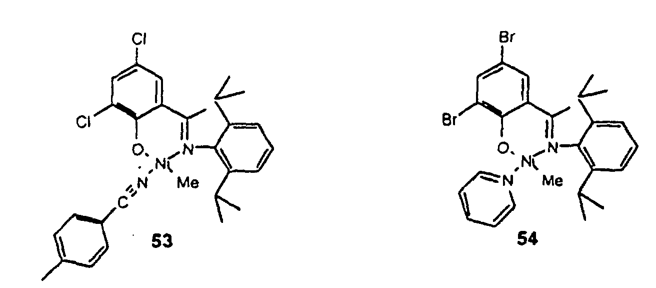

Compounds **21-54.** The syntheses and characterization of compounds **21-60** and their ligand precursors are reported in Examples 467 to 498. These compounds are used in the following Examples.

### Examples 137 - 187

### Styrene Homopolymerizations and Styrene/Norbornene Copolymerizations

In the subsequent examples describing polymerizations of styrene and norbornene, all manipulations were carried out in a nitrogen-purged drybox. Anhydrous solvents were used. The styrene (99+%, Aldrich, inhibited with 4-*tert*-butylcatechol) was degassed, filtered through basic alumina and inhibited with phenothiazine (98+%, Aldrich, 50 ppm) before use. The norbornene was purified by vacuum sublimation. Tacticities of polystyrenes were measured according to the following reference: T Kawamura et al., *Macromol. Rapid Commun.* **1994,** *15*, 479-486.

General Procedure for Styrene Polymerizations (Table 7). The nickel complex (0.03 mmol) was slurried in dry toluene (6 mL) and styrene (1.6 mL, 14 mmol) was added. Two equiv of B(C₆F₅)₃ were then added with vigorous stirring. The resulting mixture was shaken at rt in the dark for 5 h after which time the sample was removed from the drybox and MeOH was added to precipitate the polymer. The solid polymer was isolated, redissolved in CHCl₃ and reprecipitated with MeOH to remove catalyst impurities. The product was then collected on a frit, washed with MeOH and finally with a MeOH/ acetone/Irganox® 1010 solution. The polymer was then dried under vacuum.

General Procedure for Styrene/Norbornene Copolymerizations (Table 8). The nickel complex (0.03 mmol) was slurried in dry toluene (5 mL) and a mixture of norbornene (1.41 g, 15 mmol) and styrene (1.7 mL, 15 mmol) in toluene (3 mL) was added. Two equiv of B(C₆F₅)₃ were then added with vigorous stirring. The resulting mixture was shaken at rt in the dark overnight. The sample was then removed from the drybox and added to MeOH to precipitate the polymer. The product was stirred overnight in acetone to remove polystyrene and then filtered, washed with MeOH and finally with an acetone/2% Irganox 1010 solution. The polymer was then dried under vacuum.

**Table 7**

| **Styrene Homopolymerizations** | | | | | | |
|---|---|---|---|---|---|---|
| Ex. | Cmpd | Yield (%) | TO^{a} | Mₙ^{b} | PDI | Tacticity |
| 137 | **1a** | e | e | | | |
| 138 | **2a** | e | e | | | |
| 139 | **4a** | 41 | 192 | 16,000 | 2.0 | enriched in meso diads^{c} |
| 140 | **5a** | 61 | 282 | 14,900 | 2.1 | enriched in meso diads^{c} |
| 141 | **8a** | 15 | 70 | 2,390 | 3.8 | enriched in meso diads^{c} |
| 142 | **9a** | 0.7 | 3.2 | | | |
| 143 | **14a** | 36 | 170 | 2,010 | 5.4 | enriched in meso diads^{c} |
| 144 | **15a** | 31 | 144 | 1,350 | 2.4 | enriched in meso diads^{c} |
| 145 | **16a** | e | e | | | |
| 146 | **17a** | 9 | 42 | 5,800 | 2.2 | enriched in meso diads^{c} |
| 147 | **21a** | 72 | 336 | 770 | 2.7 | enriched in meso diads^{c} |
| 148 | **22a** | 66 | 304 | 760 | 2.7 | enriched in meso diads^{c} |
| 149 | **24a** | 73 | 340 | 1,010 | 2.9 | enriched in meso diads^{c} |
| 150 | **28a** | 48 | 221 | 730 | 2.9 | enriched in meso diads^{c} |
| 151 | **31a** | 57 | 265 | 2,230 | 1.8 | enriched in meso diads^{c} |
| 152 | **32a** | 78 | 362 | 14,900 | 2.1 | enriched in meso diads^{c} |
| 153 | **33a** | 26 | 122 | 830 | 2.3 | enriched in meso diads^{c} |
| 154 | **35a** | 6 | 29 | 18,800 | 5.1 | highly isotactic |
| 155 | **35a**^{**d**} | 29 | 134 | 4,150 | 6.8 | highly isotactic |
| 156 | **39a** | 4.1 | 19 | | | enriched in r diads^{c} |
| 157 | **40a** | 58 | 269 | 785 | 3.5 | enriched in meso diads^{c} |
| 158 | **42a** | 57 | 265 | 800 | 2.8 | enriched in meso diads^{c} |
| 159 | **46a** | 6 | 29 | 1,980 | 1.4 | highly isotactic |
| 160 | **47b** | 15 | 70 | 1,200 | 6.3 | enriched in meso diads^{c} |
| 161 | **48a** | 48 | 221 | 1,660 | 8.3 | enriched in meso diads^{c} |
| 162 | **49a** | 9 | 42 | 12,200 | 2.6 | enriched in meso diads^{c} |

| | | | | | | |
|---|---|---|---|---|---|---|
| ^{a}Number of turnovers: TO = (moles styrene consumed, as determined by the weight of the isolated polymer) divided by (moles catalyst). | | | | | | |
| ^{b}Mₙ (GPC, TCB, 120°C, polystyrene standards). | | | | | | |
| ^{c} According to ¹³C NMR spectroscopy (CDCl₃) and relative to atactic polystyrene. | | | | | | |
| ^{d}60°C. | | | | | | |
| ^{e}No polymer was isolated. | | | | | | |

**Table 8**

| Styrene (S) and Norbornene (N) Copolymerizations | | | | | | | |
|---|---|---|---|---|---|---|---|
| | | | TO^{a} | | | | |
| Ex. | Cmpd | Yield (%) | S | N | Mₙ^{b} | PDI | % S |
| 163 | **1a** | 4.7 | c | 47 | 5,330 | 5 | <5 |
| 164 | **2a** | 40 | c | 400 | 11,000 | 3.9 | <5 |
| 165 | **4a** | 43 | 44 | 390 | 7,630 | 3.3 | 11 |
| 166 | **5a** | 27 | 15 | 251 | 5,430 | 2.3 | 6.7 |
| 167 | **8a** | 14 | c | 141 | 14,300 | 2.7 | <5 |
| 168 | **9a** | 8.5 | c | 84 | 8,100 | 2.3 | <5 |
| 169 | **14a** | 32 | 17 | 303 | 7,290 | 3.4 | 5.8 |
| 170 | **15a** | 36 | 29 | 329 | 7,140 | 3.0 | 9 |
| 171 | **16a** | 8.8 | c | 92 | 6,920 | 2.6 | <5 |
| 172 | **17a** | 7.8 | c | 78 | 7,060 | 2.3 | <5 |
| 173 | **21a** | 44 | 41 | 400 | 2,730 | 3.8 | 10.2 |
| 174 | **22a** | 47 | 45 | 425 | 3,340 | 3.3 | 10.5 |
| 175 | **24a** | 15 | 5 | 142 | 5,800 | 3.0 | 3.7 |
| 176 | **28a** | 45 | 47 | 415 | 2,680 | 3.9 | 11.2 |
| 177 | **31a** | 30 | c | 300 | 4,700 | 2.4 | <5 |
| 178 | **32a** | 26 | 19 | 236 | 5,670 | 2.2 | 7.5 |
| 179 | **33a** | 31 | 13 | 300 | 5,940 | 2.6 | 4.5 |
| 180 | **35a** | 7.4 | c | 74 | 20,500 | 2.4 | <5 |
| 181 | **39a** | 3.0 | c | 262 | 5,054 | 2.9 | <5 |
| 182 | **40a** | 18 | 5 | 172 | 5,960 | 2.9 | 3.0 |
| 183 | **42a** | 43 | 31 | 398 | 2,470 | 4.4 | 8.7 |
| 184 | **46a** | 38 | c | 379 | 14,800 | 2.8 | <5 |
| 185 | **47b** | 17 | c | 170 | 4,570 | 6.2 | <5 |
| 186 | **48a** | 8 | c | 78 | 17,600 | 2.5 | <5 |
| 187 | **49a** | 15 | c | 145 | 7,500 | 2.3 | <5 |

| | | | | | | | |
|---|---|---|---|---|---|---|---|
| ^{*a*}Number of turnovers: TO = (moles monomer consumed, as determined by the weight of the isolated polymer) divided by (moles catalyst). | | | | | | | |
| ^{*b*}Mₙ (GPC, TCB, 120°C, polystyrene standards). | | | | | | | |
| ^{*c*}Low styrene incorporation (<5%) precluded calculation of the styrene turnover numbers. | | | | | | | |

### Examples 188 - 194

### Norbornene Homopolymerizations and Norbornene/Functionalized-Norbornene Copolymerizations

### Example 188

### Norbornene Homopolymerization Catalyzed by 52/B(C₆F₅)₃.

In a 20 mL scintillation vial under nitrogen, compound **52** (0.010 g, 0.021 mmol) and norbornene (1.00 g, 10.62 mmol) were dissolved in 5 mL of toluene to give an orange solution. To this was added B(C₆F₅)₃ (0.011 g, 0.022 mmol). After 30 min at ambient temperature, more B(C₆F₅)₃ was added to the reaction mixture (0.110 g, 0.214 mmol). An extremely viscous, yellow suspension formed very rapidly and within minutes the reaction mixture could no longer be stirred. Twenty-three h after the initial addition of B(C₆F₅)₃, the reaction mixture was quenched by addition of methanol under air. Further workup afforded 0.93 g of polymer. ¹H NMR (1,1,2,2-tetrachloroethane-*d*₂, 120°C) indicated that the polymer was the addition polymer of norbornene formed without double bond ring-opening.

### Example 189

### Copolymerization of Norbornene with the Dimethyl Ester of endo-5-Norbornene-2,3-Dicarboxylic Acid Catalyzed by 21a/B (C₆F₅)₃ (Copolymer: ~30 Mole % Dimethyl Ester)

In a 20 mL scintillation vial under nitrogen, compound **21a** (0.015 g, 0.029 mmol), norbornene (0.500 g, 5.31 mmol), and the dimethyl ester of 5-norbornene-2,3-dicarboxylic acid (1.00 g, 4.76 mmol) were dissolved in 10 mL of toluene. To this solution was added solid B (C₆F₅) ₃ (0.029 g, 0.058 mmol) . The resulting solution was stirred initially at ambient temperature by means of a magnetic stirbar; however, after several minutes the reaction mixture consisted of a viscous, solvent-swollen polymer that could not be stirred. Twenty-seven h after the addition of B(C₆F₅)₃, the reaction mixture was quenched by addition of the solvent-swollen reaction mixture to methanol under air. The precipitated polymer was filtered off, washed with methanol, and dried to afford 0.810 g of addition copolymer. ¹H NMR (CD₂Cl₂, 25 °C) indicated the following composition: norbornene (74 mole %), dimethyl ester (26 mole %). Quantitative ¹³C NMR (trichlorobenzene-*d*₃, 100°C) indicated the following composition: norbornene (70.8 mole %), dimethyl ester (29.2 mole %).

### Example 190

### Copolymerization of Norbornene with the Dimethyl Ester of endo-5-Norbornene-2,3-Dicarboxylic Acid Catalyzed by 21a/B(C₆F₅)₃ (Copolymer: ~22 Mole % Dimethyl Ester)

A reaction identical to that above in Example 189, but run in CH₂Cl₂ instead of toluene gave the following results: Yield = 0.63 g. ¹H NMR (CDCl₃, 25 °C) indicated the following composition: norbornene (81%), dimethyl ester (19%). Quantitative ¹³C NMR (trichlorobenzene-*d*₃, 100 °C): norbornene (78.11 mole %), dimethyl ester (21.89 mole %).

### Example 191

### Copolymerization of Norbornene with the Dimethyl Ester of endo-5-Norbornene-2,3-Dicarboxylic Acid Catalyzed by 21a/B (C₆F₅) ₃ (Copolymer: ~11 mole % Dimethyl Ester)

In a 20 mL scintillation vial under nitrogen, compound **21a** (0.015 g, 0.029 mmol), norbornene (3.00 g, 31.86 mmol), the dimethyl ester of 5-norbornene-2,3-dicarboxylic acid (1.00 g, 4.76 mmol), and B (C₆F₅)₃ (0.029 g, 0.058 mmol) were dissolved in 10 mL of toluene. The resulting yellow solution was stirred initially at ambient temperature by means of a magnetic stirbar; however, within 15 minutes an extremely rapid, highly exothermic reaction ensued. The reaction mixture setup and could not be stirred after this point. Three h after the addition of B (C₆F₅) ₃, the reaction mixture was quenched by addition of the solvent-swollen reaction mixture to methanol under air. Further workup afforded 3.75 g of addition copolymer. ¹H NMR (CDCl₃, 25 °C) indicated the following composition: norbornene (90 mole %), dimethyl ester (10 mole %). Quantitative ¹³C NMR (trichlorobenzene-*d*₃, 100 °C) indicated the following composition: norbornene (89.05 mole%), dimethyl ester (10.95 mole%).

### Example 192

### Copolymerization of Norbornene with the Dimethyl Ester of endo-5-Norbornene-2,3-Dicarboxylic Acid Catalyzed by 21a / B(C₆F₅)₃ (Copolymer: ∼6 mole % Dimethyl Ester)

A reaction identical to that above in Example 191, but run in CH₂Cl₂ instead of toluene gave the following results: Yield = 3.12 g. ¹H NMR (CDCl₃, 25°C) indicated the following composition: norbornene (96 mole %), dimethyl ester (4 mole %). Quantitative ¹³C NMR (trichlorobenzene-*d*₃, 100°C): norbornene (94.19 mole %), dimethyl ester (5.81 mole %).

### Example 193

### Copolymerization of Norbornene with the t-Bu Ester of 5-Norbornene-2-Carboxylic Acid Catalyzed by 50/B(C₆F₅)₃ (Copolymer: ∼30 mole % t-Bu Ester)

In a 20 mL scintillation vial under nitrogen, compound **50** (0.010 g, 0.020 mmol), norbornene (0.500 g, 5.31 mmol), and the t-Bu ester of 5-norbornene-2-carboxylic acid (1.00 g, 5.15 mmol) were dissolved in 5 mL toluene. To this solution was added B (C₆F₅)₃ (0.102 g, 0.200 mmol). The resulting yellow solution was stirred at ambient temperature for 16 h. The reaction mixture was quenched and the copolymer precipitated by addition of methanol under air. Further workup afforded 0.664 g of addition copolymer. Quantitative ¹³C NMR (trichlorobenzene-*d*₃, 100 °C) indicated the following composition: norbornene (70.4 mole %), t-Bu ester (29.6 mole %).

### Example 194

### Copolymerization of Norbornene with the Dimethyl Ester of endo-5-Norbornene-2,3-Dicarboxylic Acid Catalyzed by 52/B(C₆F₅)₃ (Copolymer: ~32 mole % Dimethyl Ester)

In a 20 mL scintillation vial under nitrogen, compound **52** (0.010 g, 0.021 mmol), norbornene (0.500 g, 5.31 mmol), and the dimethyl ester of 5-norbornene-2,3-dicarboxylic acid (1.00 g, 4.76 mmol) were dissolved in 5 mL of toluene. To this solution was added a suspension of B(C₆F₅)₃ (0.029 g, 0.058 mmol) in 5 mL of toluene. The resulting orange solution was stirred initially at ambient temperature by means of a magnetic stirbar; however, after several minutes the reaction mixture consisted of a viscous, solvent-swollen polymer that could not be stirred. Twenty-two h after the addition of B(C₆F₅)₃, the reaction mixture was quenched by addition of the solvent-swollen reaction mixture to methanol under air. The precipitated polymer was filtered off, washed with methanol, and dried to afford 0.930 g of addition copolymer. ¹H NMR (CDCl₃, 25 °C) indicated the following composition: norbornene (68 mole %), dimethyl ester (32 mole %).

### Examples 195 - 366

### Ethylene Polymerizations General Procedure for Ethylene Polymerizations of Table 9

### Pressure Tube Loaded Outside of the Drybox Under a Nitrogen Purge

(In the ethylene polymerization reactions of Tables 2, 3, and 4, the glass inserts were also loaded in the pressure reactor tube outside of the drybox under a nitrogen purge.) Procedure. In the drybox, a glass insert was loaded with the isolated allyl initiator (0.06 mmol). The insert was cooled to -35°C in the drybox freezer, 5 mL of C₆D₆ was added to the cold insert, and the insert was cooled again. A Lewis acid cocatalyst [typically BPh₃ or B(C₆F₅)₃] was added to the cold solution, and the insert was then capped and sealed. Outside of the drybox, the cold insert was placed under a nitrogen purge into the pressure tube. The pressure tube was sealed, placed under ethylene (6.9 MPa), and allowed to warm to rt as it was shaken mechanically for approximately 18 h. An aliquot of the solution was used to acquire a ¹H NMR spectrum. The remaining portion was added to ~20 mL of MeOH in order to precipitate the polymer. The polyethylene was isolated and dried under vacuum.

### General Procedure for Ethylene Polymerizations of Tables 10-14:

### Pressure Tube Loaded and Sealed in the Drybox under a Nitrogen Atmosphere

Procedure. In the drybox, a glass insert was loaded with the nickel compound. Often, a Lewis acid (typically BPh₃ or B(C₆F₅)₃) was also added to the glass insert. Next, 5 mL of a solvent (typically 1,2,4-trichlorobenzene although *p*-xylene, cyclohexane, etc. were also used at times) was added to the glass insert and the insert was capped. The glass insert was then loaded in the pressure tube inside the drybox. The pressure tube containing the glass insert was then sealed inside of the drybox, brought outside of the drybox, connected to the pressure reactor, placed under the desired ethylene pressure and shaken mechanically. After the stated reaction time, the ethylene pressure was released and the glass insert was removed from the pressure tube. The polymer was precipitated by the addition of MeOH (∼20 mL) and concentrated HCl (~1-3 mL). The polymer was then collected on a frit and rinsed with HCl, MeOH, and acetone. The polymer was transferred to a pre-weighed vial and dried under vacuum overnight. The polymer yield and characterization were then obtained.

**Table 9**

| Ethylene Polymerization (6.9 MPa, C₆D₆ (5 mL), 0.06 mmol Cmpd, 18 h) | | | | | | | |
|---|---|---|---|---|---|---|---|
| Ex. | Cmpd | Temp (°C) | Lewis Acid^{a} | PE(g) | PE(TO)^{b} | M.W.^{c} (MI, GPC, and/or ¹H NMR) | Total Me^{d} |
| 195 | **21a** | 25 | BPh₃ | 3.34 | 2,000 | MI: 79.5; Mₙ(¹H): 3,670 | 36.9 |
| 196 | **21a** | 80 | B(C₆F₅)₃ | e | e | | |
| 197 | **22a** | 25 | BPh₃ | 4.41 | 2,600 | MI > 200; Mₙ(¹H): 1,890 | 85.5 |
| 198 | **22a** | 80 | B(C₆F₅)₃ | 0.04 | 20 | | |
| 199 | **24a** | 25 | BPh₃ | 15.3 | 9,100 | MI<0.01;Mₙ(¹H):no olefins | 4.5 |
| 200 | **24a** | 80 | B(C₆F₅)₃ | 0.15 | 91 | | |
| 201 | **28a** | 25 | BPh₃ | 0.30 | 180 | Mₙ(¹H): 18,900 | 67.1 |
| 202 | **28a** | 80 | B(C₆F₅)₃ | 0.04 | 24 | | |
| 203 | **31a** | 25 | BPh₃ | f | f | | |
| 204 | **31a** | 80 | B(C₆F₅)₃ | f | f | | |
| 205 | **32a** | 25 | BPh₃ | 0.01^{e} | 7^{e} | | |
| 206 | **32a** | 80 | B(C₆F₅)₃ | e | e | | |
| 207 | **33a** | 25 | BPh₃ | 0.21 | 120 | | |
| 208 | **33a** | 80 | B(C₆F₅)₃ | 1.60 | 950 | MI: 79.5; Mₙ(¹H): 1,390 | 51.2 |
| 209 | **35a** | 25 | BPh₃ | 0.19^{e} | 110^{e} | | |
| 210 | **35a** | 80 | B(C₆F₅)₃ | 0.11^{e} | 68^{e} | | |
| 211 | **37a** | 25 | BPh₃ | 0.02^{e} | 10^{e} | | |
| 212 | **38a** | 80 | B(C₆F₅)₃ | 0.07^{e} | 42^{e} | | |
| 213 | **39a** | 25 | BPh₃ | f | f | | |
| 214 | **39a** | 80 | B(C₆F₅)₃ | 2.11 | 1,300 | MI: 105; Mₙ(¹H): 5,200 | 21.5 |
| 215 | **40a** | 25 | BPh₃ | 0.08^{e} | 50^{e} | | |
| 216 | **40a** | 80 | B(C₆F₅)₃ | e | e | | |
| 217 | **42a** | 25 | BPh₃ | e | e | | |
| 218 | **42a** | 80 | B(C₆F₅)₃ | e | e | | |
| 219 | **46a** | 25 | BPh₃ | f | f | | |
| 220 | **46a** | 80 | B(C₆F₅)₃ | 0.11 | 65 | | |
| 221 | **47b** | 25 | BPh₃ | 0.12^{e} | 71^{e} | | |
| 222 | **47b** | 80 | B(C₆F₅)₃ | e | e | | |
| 223 | **47b** | 25 | BPh₃ | 0.15 | 91 | | |
| 224 | **47b** | 80 | B(C₆F₅)₃ | 0.09 | 52 | | |
| 225 | **48a** | 25 | BPh₃ | 0.07 | 43 | | |
| 226 | **48a** | 80 | B(C₆F₅)₃ | 0.22^{e} | 132^{e} | | |
| 227 | **49a** | 25 | BPh₃ | 0.10 | 59 | | |
| 228 | **49a** | 80 | B(C₆F₅)₃ | 0.06^{e} | 34^{e} | | |

| | | | | | | | |
|---|---|---|---|---|---|---|---|
| ^{a}Two equiv. | | | | | | | |
| ^{b}TO: number of turnovers per metal center = (moles ethylene consumed, as determined by the weight of the isolated polymer or oligomers) divided by (moles catalyst). | | | | | | | |
| ^{c}M.W.: Molecular weight of the polymer or oligomers as determined by melt index (MI: g/10 min at 190°C), GPC (molecular weights are reported versus polystyrene standards; conditions: Waters 150C, trichlorobenzene at 150°C, Shodex columns at -806MS 4G 734/602005, RI detector), and/or ¹H NMR (olefin end group analysis). | | | | | | | |
| ^{*d*}*Total* number of methyl groups per 1000 methylene groups as determined by ¹H NMR analysis. | | | | | | | |
| ^{e1}H NMR: oligomers and/or (CH₂)ₙ peak observed. | | | | | | | |
| ^{*f*}PE was not obtained in isolable quantities. ^{*g*}The general procedure for the screening of ethylene polymerizations by nickel allyl initiators at 6.9 MPa ethylene (*see above*) was followed. | | | | | | | |

**Table 10**

| Ethylene Polymerizations at 6.9 MPa: Pressure Tube Loaded in the Drybox under N₂ Atmosphere (Trichlorobenzene (5 mL), 18 h) | | | | | | | |
|---|---|---|---|---|---|---|---|
| Ex. | Cmpd^{f} | Temp (°C) | Lewis Acid^{a} | PE(g) | PE(TO)^{b} | M.W.^{c}(MI,GPC, and/or ¹H NMR) | Total Me^{d} |
| 229 | 3a | 25 | BPh₃ | 8,31 | 12,900 | MI: 5.5; Mₙ(¹H): 18,100 | 36.9 |
| 230 | 3a^{g} | 25 | BPh₃ | 1.54 | 3,660 | MI:40; Mₙ(¹H): no olefins | 20.0 |
| 231 | 3a^{g} | 25 | B(C₆F₅)₃ | 0.701 | 1,460 | MI: 22.9, Mₙ(¹H): 99,900 | 22.9 |
| 232 | 3a | 80 | BPh₃ | 4.29 | 6,640 | MI > 200; Mₙ(¹H): 4,770 | 56.2 |
| 233 | 3a | 80 | B(C₆F₅)₃ | 0.203 | 359 | Mn (¹H): 2,500 | 47.7 |
| 234 | 4a | 25 | BPh₃ | 2.44 | 4.630 | MI: 126; Mₙ(¹H): 10,300 | 43.5 |
| 235 | 4a | 80 | BPh₃ | 2.19 | 3,640 | MI > 200; Mₙ(¹H): 2,270 | 75.5 |
| 236 | 4a | 80 | B(C₆F₅)₃ | 0.096 | 190 | Mn(¹H): 3,260 | 29.3 |
| 237 | 5a | 25 | BPh₃ | 4.98 | 8,630 | M_{W}(GPC): 14,100; PDI:6.7 | 93.2 |
| 238 | 5a | 80 | BPh₃ | 2.72 | 4,710 | M_{w}(GPC): 2,850; PDI: 3.7 | 137.3 |
| 239 | 6a | 25 | BPh₃ | 4.44 | 7,730 | MI:0.85 | |
| 240 | 6aⁱ | 25 | B(C₆F₅)₃ | 6.18 | 5,490 | MI: 1.2; Mₙ(¹H): 9,620 | 28.5 |
| 241 | 6a^{g} | 80 | BPh₃ | 4.13 | 10,500 | MI: 21; Mₙ(¹H): 12,200 | 28.4 |
| 242 | 6a^{h} | 80 | BPh₃ | 13.1 | 7,800 | MI > 200; Mₙ(¹H): 3,030 | 79.0 |
| 243 | 6a | 80 | B(C₆F₅)₃ | 3.31 | 5,990 | MI: 81; Mₙ(¹H): 5,920 | 34.7 |
| 244 | 6a^{g} | 80 | B(C₆F₅)₃ | 3.14 | 7,300 | MI: 45 | |
| 245 | 6a^{h} | 80 | B(C₆F₅)₃ | 8.92 | 5,300 | MI > 200; Mₙ(¹H): 1,420 | 89.1 |
| 246 | 7a | 25 | BPh₃ | 0.93 | 1,350 | MI<0.01;Mₙ(¹H):no olefins | 2.1 |
| 247 | 7a | 25 | B(C₆F₅)₃ | 2.19 | 3,790 | Mₙ(¹H): 4,160 | 11.6 |
| 248 | 7a | 80 | B(C₆F₅)₃ | 1.36 | 2,030 | MI: 35; M,(¹H): 1,370 | 35.7 |
| 249 | 8a | 25 | BPh₃ | 3.88 | 6,400 | MI: 120; Mₙ(¹H): 2,990 | 57.2 |
| 250 | 8a | 25 | B(C₆F₅)₃ | 6.95 | 11,800 | MI: 2.6; Mₙ(¹H): 6,770 | 57.5 |
| 251 | 8a | 80 | B(C₆F₅)₃ | 3.99 | 7,220 | MI:132 | |
| 252 | 9a | 25 | BPh₃ | 6.35 | 11,200 | MI > 200; Mₙ(¹H): 4,910 | 64.9 |
| 253 | 9a | 25 | B(C₆F₅)₃ | 6.32 | 9.900 | MI:102; Mₙ(¹H): 5,380 | 91.3 |
| 254 | 9a | 80 | B(C₆F₅)₃ | 4.8 | 8,000 | Mₙ(¹H): 1,410 | 134.6 |
| 255 | 10a | 25 | BPh₃ | 0.18 | 320 | | |
| 256 | 10a | 80 | BPh₃ | 1.33 | 2,140 | MI: 63; Mₙ(¹H): 11,100 | 38.3 |
| 257 | 10a | 80 | B(C₆F₅)₃ | 0.097 | 170 | Mₙ(¹H): 2.010 | 30.4 |
| 258 | 11a | 25 | BPh₃ | 0.11 | 190 | | |
| 259 | 11a | 80 | BPh₃ | 1.33 | 2,140 | MI: 160; Mₙ(¹H): 7,990 | 40.5 |
| 260 | 12a | 25 | BPh₃ | 3.68 | 6,040 | MI < 0.01 | |
| 261 | 12a | 80 | BPh₃ | 4.22 | 7,260 | MI: 74; Mₙ(¹H): 9,260 | 33.0 |
| 262 | 12a | 80 | B(C₆F₅)₃ | 1.61 | 2,870 | MI: 30; Mₙ(¹H): 12,700 | 19.3 |
| 263 | 13a | 25 | BPh₃ | 0.99 | 1,690 | MI: 0.04; Mₙ(¹H): 23,400 | 20.5 |
| 264 | 13a | 80 | BPh₃ | 3.73 | 6,580 | MI: 147; Mₙ(¹H): 4,890 | 40.2 |
| 265 | 13a | 80 | B(C₆F₅)₃ | 0.93 | 1,560 | MI > 200; Mₙ(¹H): 4,520 | 36.5 |
| 266 | 14a | 25 | BPh₃ | 1.68 | 3,120 | MI: 0.3; Mₙ(¹H): 18,300 | 24.2 |
| 267 | 14a | 25 | B(C₆F₅)₃ | 4.28 | 7,010 | MI: 6; Mₙ(¹H): 5,820 | 42.6 |
| 268 | 14a | 80 | B(C₆F₅)₃ | 1.52 | 2,760 | MI: 117; Mₙ(¹H): 3,080 | 54.0 |
| 269 | 15a | 25 | BPh₃ | 0.265 | 468 | | |
| 270 | 15a | 25 | BPh₃ | 1.75 | 3,060 | MI: 0.1; Mₙ(¹H): 1,900 | 30.7 |
| 271 | 15a | 80 | B(C₆F₅)₃ | 0.399 | 705 | Mₙ(¹H): 1,700 | 43.7 |
| 272 | 17a | 25 | BPh₃ | 0.191 | 346 | Mₙ(¹H): 23,500 | 21.1 |
| 273 | 17a | 25 | B(C₆F₅)₃ | 5.69 | 10,100 | MI < 0.01; Mₙ(¹H): 24,600 | 10.4 |
| 274 | 17a | 80 | B(C₆F₅)₃ | 1.59 | 2,540 | MI: 0.08; Mₙ(¹H): 7,130 | 24.1 |
| 275 | 18a | 25 | BPh₃ | e | e | | |
| 276 | 18a | 80 | B(C₆F₅)₃ | 0.046 | 76 | Mₙ(¹H): 4,690 | 27.4 |
| 277 | 19a | 25 | BPh₃ | 0.289 | 544 | Mₙ(¹H): 4,450 | 36.2 |
| 278 | 19a | 25 | B(C₆F₅)₃ | 0.223 | 399 | Mₙ(¹H): 1,660 | 27.8 |
| 279 | 19a | 80 | BPh₃ | 0.077 | 128 | Mₙ(¹H): 2,550 | 39.3 |
| 280 | 19a | 80 | B(C₆F₅)₃ | 0.224 | 399 | Mₙ(¹H): 839 | 52.5 |
| 281 | 21a | 25 | BPh₃ | 6.48 | 10,700 | MI: 42; Mₙ(¹H): 21,400 | 24.9 |
| 282 | 21a | 80 | BPh₃ | 3.44 | 6,090 | MI > 200; Mₙ(¹H): 4,010 | 52.5 |
| 283 | 21a | 80 | B(C₆F₅)₃ | 0.123 | 226 | | |
| 284 | 21b | 25 | BPh₃ | 4.15 | 6,060 | MI: 16.5; Mₙ(¹H): 21,600 | 19.9 |
| 285 | 21b | 25 | B(C₅F₅)₃ | 0.024 | 31 | Mₙ(¹H): 1,570 | 34.2 |
| 286 | 21b | 80 | BPh₃ | 3.39 | 5,640 | MI > 200; Mₙ(¹H): 3,730 | 55.2 |
| 287 | 21b | 80 | B(C₆F₅)₃ | 0.030 | 48 | Mₙ(¹H): 2,190 | 38.7 |
| 288 | 22a | 25 | BPh₃ | 10.1 | 17,900 | MI > 200; Mₙ(¹H): 2,600 | 85.1 |
| 289 | 22a | 80 | BPh₃ | 4.11 | 7,120 | Mn (¹H): 1,630 | 92.6 |
| 290 | 22a | 80 | B(C₆F₅)₃ | 0.15 | 260 | Mₙ(¹H): no olefins | 23.7 |
| 291 | 23a | 25 | BPh₃ | 2.93 | 4,770 | MI > 200; Mₙ(¹H): 7,220 | 59.1 |
| 292 | 23a | 25 | BPh₃ | 2.90 | 4,960 | MI: 120; Mₙ(¹H): 8,950 | 56.5 |
| 293 | 23a | 80 | B(C₆F₅)₃ | e | e | | |
| 294 | 24a | 25 | BPh₃ | 1.73 | 3,250 | MI<0.01;Mₙ(¹H):no olefins | 8.6 |
| 295 | 24a | 80 | BPh₃ | 1.95 | 3,340 | MI: 29; Mₙ(¹H): 8,110 | 28.6 |
| 296 | 24a | 80 | B(C₆F₅)₃ | 1.16 | 2,060 | MI: 70; Mₙ(¹H): 8,540 | 23.0 |
| 297 | 25a | 25 | BPh₃ | 9.07 | 17,000 | MI: 1.4 | |
| 298 | 25a | 80 | BPh₃ | 3.64 | 6,450 | Mi > 200; Mₙ(¹H): 3,310 | 54.2 |
| 299 | 25a | 80 | B(C₆F₅)₃ | 0.025 | 47 | Mₙ(¹H): 3,140 | 31.6 |
| 300 | 26a | 25 | BPh₃ | 7.89 | 13,700 | MI > 200; Mₙ(¹H): 3,250 | 69.2 |
| 301 | 26a | 25 | BPh₃ | 11.7 | 17,900 | MI > 200; Mₙ(¹H): 3,930 | 66.6 |
| 302 | 26a | 80 | B(C₆F₅)₃ | e | e | | |
| 303 | 27a | 25 | BPh₃ | 4.47 | 7,800 | MI: 210; Mₙ(¹H): 8,040 | 52.7 |
| 304 | 27a | 25 | BPh₃ | 7.03 | 11,500 | MI: 108; Mₙ(¹H): 8,230 | 50.9 |
| 305 | 27a | 80 | B(C₆F₅)₃ | 0.009 | 17 | Mₙ(¹H): 5,070 | 27.9 |
| 306 | 28a | 25 | BPh₃ | 0.761 | 1,300 | MI: 60; Mₙ(¹H): 19,900 | 37.1 |
| 307 | 28a | 25 | BPh₃ | 0.271 | 481 | Mₙ(¹H): 26,700 | 31.3 |
| 308 | 28a | 80 | B(C₆F₅)₃ | 0.006 | 10 | Mₙ(¹H): 6,630 | 19.8 |
| 309 | 29a | 25 | B(C₆F₅)₃ | 0.573 | 994 | MI: 0.12; Mₙ(¹H): 4,010 | 16.2 |
| 310 | 29a | 25 | BPh₃ | e | e | | |
| 311 | 29a | 80 | B(C₆F₅)₃ | 0.199 | 360 | Mₙ(¹H): 1,650 | 35.3 |
| 312 | 30a | 25 | B(C₆F₅)₃ | 2.45 | 4,160 | MI<0.01; Mₙ(¹H): 8,300 | 8.1 |
| 313 | 30a | 25 | BPh₃ | e | e | | |
| 314 | 30a | 80 | B(C₆F₅)₃ | 1.64 | 2,610 | MI: 17; Mₙ(¹H): 3,600 | 23.0 |
| 315 | 33a | 25 | BPh₃ | 0.431 | 768 | Mₙ(¹H):21,300 | 3.4 |
| 316 | 33a | 25 | B(C₆F₅)₃ | 2.35 | 4,070 | MI: 0.13; Mₙ(¹H): 4,270 | 37.1 |
| 317 | 33a | 80 | B(C₆F₅)₃ | 0.915 | 1,540 | MI: 36.8: Mₙ(¹H):1,860 | 36.1 |
| 318 | 34a | 25 | B(C₆F₅)₃ | 7.53 | 11,600 | Mₙ (¹H): 3,450 | 72.4 |
| 319 | 34a | 80 | B(C₆F₅)₃ | 5.35 | 7,570 | M_{w}(GPC): 29,100; PDI: 46 | 113.2 |
| 320 | 36a | 25 | BPh₃ | 9.31 | 15,300 | M_{w}(GPC):461,000;PDI:3.3 | 8.0 |
| 321 | 36a | 80 | BPh₃ | 0.353 | 564 | M_{w}(GPC):30,000; PDI:4.0 | 25.8 |
| 322 | 37a | 25 | BPh₃ | 0.919 | 1,650 | MI: 0.06; Mₙ(¹H):no olefins | 14.4 |
| 323 | 37a | 25 | BPh₃ | 0.299 | 434 | Mₙ(¹H): 31,100 | 15.0 |
| 324 | 37a | 80 | B(C₆F₅)₃ | 0.269 | 434 | Mₙ(¹H): 5,200 | 40.4 |
| 325 | 38a | 25 | BPh₃ | 1.43 | 2,470 | MI: 111; Mₙ(¹H): 6,150 | 65.8 |
| 326 | 38a | 80 | BPh₃ | 1.55 | 2,580 | MI > 200; Mₙ(¹H): 2,780 | 99.2 |
| 327 | 39a | 25 | B(C₆F₅)₃ | 0.414 | 814 | Mₙ(¹H): 10,700 | 7.7 |
| 328 | 39a | 25 | BPh₃, | e | e | | |
| 329 | 39a | 25 | BPh₃ | e | e | | |
| 330 | 39a | 80 | B(C₆F₅)₃ | 0.758 | 1,290 | MI: 80; Mₙ(¹H): 5,190 | 20.1 |
| 331 | 41a | 25 | BPh₃ | 0.316 | 586 | Mₙ(¹H): no olefins | 11.5 |
| 332 | 41a | 25 | B(C₆F₅)₃ | 4.08 | 6,690 | MI < 0.01; Mn (¹H): 30,700 | 30.9 |
| 333 | 41a | 80 | B(C₆F₅)₃ | 2.26 | 3,730 | MI: 180; Mₙ(¹H): 9,960 | 36.9 |
| 334 | 43a | 25 | BPh₃ | e | e | | |
| 335 | 43a | 25 | B(C₆F₅)₃ | 0.53 | 918 | Mₙ(¹H): 3,600 | 36.6 |
| 336 | 43a | 80 | BPh₃ | e | e | | |
| 337 | 43a | 80 | B(C₆F₅)₃ | 0.054 | 93 | Mₙ(¹H): 2.960 | 32.0 |
| 338 | 44a | 25 | B(C₆F₅)₃ | 0.167 | 291 | M_{w}(GPC): 136,000; PDI:18 | 25.4 |
| 339 | 44a | 80 | B(C₆F₅)₃ | 0.019 | 34 | Mn(¹H): 5,150 | 43.3 |
| 340 | 45a | 25 | B(C₆F₅)₃ | 0.026 | 43 | Mₙ(¹H):5,150 | 8.6 |
| 341 | 45a | 80 | B(C₆F₅)₃ | trace | trace | Mₙ(¹H): 6,310 | 14.2 |

| | | | | | | | |
|---|---|---|---|---|---|---|---|
| ^{*a*}Two equiv. | | | | | | | |
| ^{*b*}TO: number of turnovers per metal center = (moles ethylene consumed, as determined by the weight of the isolated polymer or oligomers) divided by (moles catalyst). Calculations are based upon the exact amount of catalyst used. | | | | | | | |
| ^{*c*}M.W.: Molecular weight of the polymer or oligomers as determined by melt index (MI: g/10 min at 190 °C), GPC (molecular weights are reported versus polystyrene standards; conditions: Waters 150C, trichlorobenzene at 150 °C, Shodex columns at-806MS 4G 734/602005, RI detector), and/or ¹H NMR (olefin end group analysis). | | | | | | | |
| ^{*d*}total number of methyl groups per 1000 methylene groups as determined by ¹H NMR analysis. | | | | | | | |
| ^{*e*}PE was not obtained in isolable quantities. | | | | | | | |
| ^{*f*}0.02 mmol unless noted otherwise. | | | | | | | |
| ^{*g*}0.015mmol. | | | | | | | |
| ^{*h*}0.06 mmol. | | | | | | | |

**Table 11**

| Ethylene Polymerizations at 6.9 MPa Pressure Tube Loaded in the Drybox under N₂ Atmosphere (*p*-Xylene, (5 mL), 18 h) | | | | | | | |
|---|---|---|---|---|---|---|---|
| Ex. | Cmpd^{f} | Temp (°C) | Lewis Acid^{a} | PE(g) | PE(TO)^{b} | M.W.^{c}(MI, GPC, and/or ¹H NMR) | Total Me^{d} |
| 342 | 3a^{h} | 25 | BPh₃ | 20.2 | 12,000 | MI:2.6 | |
| 343 | 3a^{g} | 25 | BPh₃ | 0.19 | 366 | | |
| 344 | 3a^{g} | 25 | B(C₆F₅)₃ | 0.48 | 1,160 | M,(¹H): 27,100 | 24.4 |
| 345 | 6a^{h} | 80 | BPh₃ | 11.1 | 6,610 | MI: 105; Mₙ(¹H): 9,090 | 31.1 |
| 346 | 6a^{h} | 80 | B(C₆F₅)₃ | 6.90 | 4,100 | MI: >200; Mₙ(¹H): 3,170 | 63.4 |
| 347 | 6a^{g} | 80 | B(C₆F₅)₃ | 3.47 | 8,470 | MI: 58.8; Mₙ(¹H): 6,880 | 34.5 |

| | | | | | | | |
|---|---|---|---|---|---|---|---|
| ^{*a*}Two equiv. | | | | | | | |
| ^{*b*}TO: number of turnovers per metal center = (moles ethylene consumed, as determined by the weight of the isolated polymer or oligomers) divided by (moles catalyst). Calculations are based upon the exact amount of catalyst used. | | | | | | | |
| ^{*c*}M.W.: Molecular weight of the polymer or oligomers as determined by melt index (MI: g/10 min at 190 °C), GPC (molecular weights are reported versus polystyrene standards; conditions: Waters 150C, trichlorobenzene at 150 °C, Shodex columns at-806MS 4G 734/602005, RI detector), and/or ¹H NMR (olefin end group analysis). | | | | | | | |
| ^{*d*}Total number of methyl groups per 1000 methylene groups as determined by ¹H NMR analysis. ^{*e*}PE was not obtained in isolable quantities. | | | | | | | |
| ^{*f*}0.02 mmol unless noted otherwise. | | | | | | | |
| ^{*g*}0.015 mmol. | | | | | | | |
| ^{*h*}0.06 mmol. | | | | | | | |

**Table 12**

| Ethylene Polymerizations at 6.9 MPa Pressure Tube Loaded in the Drybox under N₂ Atmosphere (Cyclohexane, (5 mL), 18 h) | | | | | | | |
|---|---|---|---|---|---|---|---|
| Ex. | Cmpd ^{f} | Temp (°C) | Lewis Acid ^{a} | PE(g) | PE(TO)^{b} | M. W.^{c} (MI, GPC, and/or ¹H NMR) | Total Me^{d} |
| 348 | 3a^{g} | 25 | BPh₃ | 0.52 | 1,160 | | |
| 349 | 6a^{h} | 80 | BPh₃ | 10.6 | 6,270 | MI: 135; Mₙ(¹H): 7,410 | 33.8 |
| 350 | 6a^{g} | 80 | BPh₃ | 7.07 | 16,810 | MI: 129; Mₙ(¹H): 8,800 | 27.7 |

| | | | | | | | |
|---|---|---|---|---|---|---|---|
| ^{*a*}Two equiv. | | | | | | | |
| ^{*b*}TO: number of turnovers per metal center = (moles ethylene consumed, as determined by the weight of the isolated polymer or oligomers) divided by (moles catalyst). Calculations are based upon the exact amount of catalyst used. | | | | | | | |
| ^{*c*}M.W.: Molecular weight of the polymer or oligomers as determined by melt index (MI: g/10 min at 190 °C), GPC (molecular weights are reported versus polystyrene standards; conditions: Waters 150C, trichlorobenzene at 150 °C, Shodex columns at-806MS 4G 734/602005, RI detector), and/or ¹H NMR (olefin end group analysis). | | | | | | | |
| ^{*d*}Total number of methyl groups per 1000 methylene groups as determined by ¹H NMR analysis. ^{*e*}PE was not obtained in isolable quantities. | | | | | | | |
| ^{*f*}0.02 mmol unless noted otherwise. | | | | | | | |
| ^{*g*}0.015 mmol. | | | | | | | |
| ^{*h*}0.06 mmol. | | | | | | | |

**Table 13**

| Ethylene Polymerizations at 1.4 MPa Pressure Tube Loaded in the Drybox under N₂ Atmosphere (Trichlorobenzene (5 mL), 0.02 mmol Cmpd. 18 h) | | | | | | | |
|---|---|---|---|---|---|---|---|
| Ex. | Cmpd | Temp (°C) | Lewis Acid^{a} | PE(g) | PE(TO)^{b} | M.W.^{c}(MI,GPC, and/or ¹H NMR) | Total Me^{d} |
| 351 | 3a | 25 | BPh₃ | 1.71 | 2,540 | MI: 0.26; Mₙ(¹H): 64,600 | 17.3 |
| 352 | 4a | 25 | BPh₃ | 2.87 | 5,000 | MI: 92; Mₙ(¹H): 15,300 | 40.3 |
| 353 | 6a | 25 | B(C₆F₅)₃ | 2.31 | 3,760 | MI: 1; Mₙ(¹H): 11,700 | 55.0 |
| 354 | 8a | 25 | B(C₆F₅)₃ | 2.10 | 3,440 | MI: 123; Mₙ(¹H):5,570 | 81.5 |
| 355 | 9a | 25 | B(C₆F₅)₃ | 1.53 | 2,730 | Mₙ(¹H): 4,850 | 112.3 |
| 356 | 14a | 25 | B(C₆F₅)₃ | 1.18 | 2,080 | MI: 7.5; Mₙ(¹H): 4,730 | 53.3 |
| 357 | 21a | 25 | BPh₃ | 1.58 | 2,670 | MI: 1.5; Mₙ(¹H): 14,700 | 39.9 |
| 358 | 22a | 25 | BPh₃ | 2.94 | 4740 | MI > 200; Mₙ(¹H): 4,580 | 73.7 |
| 359 | 25a | 25 | BPh₃ | 1.18 | 2,060 | MI: 6.6; Mₙ(¹H): 5,020 | 110.6 |
| 360 | 26a | 25 | BPh₃ | 2.41 | 4,040 | MI > 200; Mₙ(¹H): 3,870 | 73.6 |

| | | | | | | | |
|---|---|---|---|---|---|---|---|
| ^{a}Two equiv. | | | | | | | |
| ^{*b*}TO: number of turnovers per metal center = (moles ethylene consumed, as determined by the weight of the isolated polymer or oligomers) divided by (moles catalyst). Calculations are based upon the exact amount of catalyst used. | | | | | | | |
| ^{c}M.W.: Molecular weight of the polymer or oligomers as determined by melt index (MI: g/10 min at 190°C), GPC (molecular weights are reported versus polystyrene standards; conditions: Waters 150C, trichlorobenzene at 150 °C, Shodex columns at-806MS 4G 734/602005, RI detector), and/or ¹H NMR (olefin end group analysis). | | | | | | | |
| ^{*d*}Total number of methyl groups per 1000 methylene groups as determined by ¹H NMR analysis. ^{*e*}PE was not obtained in isolable quantities. | | | | | | | |

**Table 14**

| Ethylene Polymerizations Using Nickel Methyl Initiators: Effect of Lewis Acid on Initiation/Productivity (6.9 MPa, 0.02 mmol Cmpd, Trichlorobenzene (5 mL), 18 h) | | | | | | | |
|---|---|---|---|---|---|---|---|
| Ex. | Cmpd | Temp (°C) | Lewis Acid (equiv) | PE(g) | PE(TO)^{a} | M.W.^{b} (MI, GPC, and/or ¹H NMR) | Total Me^{c} |
| 361 | 50^{d} | 25 | none | trace | trace | | |
| 362 | 50^{d} | 80 | none | 0.189 | 307 | Mₙ(¹H): 5,840 | 39.4 |
| 363 | 50 | 25 | BPh₃ (2) | 0.126 | 201 | | |
| 364 | 50 | 80 | B(C₆F₅)₃ (2) | 0.074 | 124 | | |
| 365 | 50 | 25 | BPh₃ (10) | 4.41 | 7,590 | | |
| 366 | 50 | 80 | BPh₃ (10) | 3.01 | 5,220 | M_{w}(GPC): 17,900;PDI:6 | |

| | | | | | | | |
|---|---|---|---|---|---|---|---|
| ^{*a*}TO: number of turnovers per metal center = (moles ethylene consumed, as determined by the weight of the isolated polymer or oligomers) divided by (moles catalyst). Calculations are based upon the exact amount of catalyst used. | | | | | | | |
| ^{*b*}M.W.: Molecular weight of the polymer or oligomers as determined by melt index (MI: g/10 min at 190 °C), GPC (molecular weights are reported versus polystyrene standards; conditions: Waters 150C, trichlorobenzene at 150°C, Shodex columns at- 806MS 4G 734/602005, RI detector), and/or ¹H NMR (olefin end group analysis). | | | | | | | |
| ^{c}Total number of methyl groups per 1000 methylene groups as determined by ¹H NMR analysis. | | | | | | | |
| ^{d}Under the same reaction conditions (e.g., no Lewis acid present), nickel compounds **51 - 54** gave analogous results: no polymer was isolated, but the H NMR spectra showed a -(CH₂)ₙ- resonance. | | | | | | | |

### Examples 367 - 369

### Cyclopentene Oligomerizations

General Procedure for Cyclopentene Oligomerizations. In the drybox under a nitrogen atmosphere, the nickel compound (0.03 mmol) was placed in a vial. Next, 5 mL of toluene was added to the vial followed by 1.3 mL of cyclopentene. B(C₆F₅)₃ (40 mg) was then added to the vial. The reaction mixture was mixed for 3 d on a vortexer and then removed from the drybox and added to 100 mL of stirring methanol. No polymer precipitated. GC analysis was carried out on the organic layer. The results are reported in Table 15 below.

**Table 15**

| **Cyclopentene Oligomerizations** | | |
|---|---|---|
| Ex. | Cmpd | GC Analysis |
| 367 | 31a | dimers through heptamers observed |
| 368 | 32a | dimers through heptamers observed |
| 369 | 47b | dimers through heptamers observed |

### Examples 370 - 375

### Ethylene/Ethyl 4-Pentenoate Polymerizations

General Procedure for Ethylene/Ethyl 4-Pentenoate Polymerizations. In a nitrogen-filled drybox, the nickel compound (0.06 mmol) and the Lewis acid (5 equiv) were placed together in a glass insert. The insert was cooled to -30 °C in the drybox freezer. 5 mL of cold ethyl 4-pentenoate was added to the cold insert, and the insert was recooled in the drybox freezer. The cold inserts were removed from the drybox and placed under a nitrogen purge in a pressure tube, which was then sealed and pressurized to 6.9 MPa of ethylene and mechanically shaken for 18 h. The pressure was then released and the glass insert was removed from the pressure tube and the polymer was precipitated in MeOH, collected on a frit, and dried in vacuo. Characteristic NMR resonances of the copolymer include the 4.01 OC*H*_{*2*} resonance in the ¹H NMR and the 59.7 O*C*H₂ resonance and ∼172.4 *C*=O resonances in the ¹³C NMR spectrum.

**Table 16**

| Ethylene/Ethyl 4-Pentenoate (E-4-P) Polymerizations | | | | | |
|---|---|---|---|---|---|
| Ex. | Cmpd | Lewis Acid | Temp (°C) | Polymer (g) | DSC/GPC |
| 370 | **3a** | BPh₃ | 25 | 2.35 | DSC: Tₘ = 111°C |
| | | ¹³_{C} NMR: 0.59 mole percent E-4-P Incorp.; Branching per 1000 CH₂'s: Total methyls (27.3), Methyl (23.7), Ethyl (1.7), Propyl (0), Butyl (0.8), Amyl (2.4), Hex and greater and end of chains (5.4), Am and greater and end of chains (1.9), Bu and greater and end of chains (1.8) | | | |
| 371 | **21a** | BPh₃ | 25 | 0.586 | DSC: Tₘ = 115 °C |
| | | ¹³C NMR: 0.26 mole percent E-4-P Incorp.; Branching per 1000 CH₂'s: Total methyls (25.0), Methyl (19.3), Ethyl (2.1), Propyl (0.0), Butyl (1.2), Amyl (0.3), Hex and greater and end of chains (3. 1). Am and greater and end of chains (4.1), Bu and greater and end of chains 3.6) | | | |
| 372 | **8a** | B(C₆F₅)₃ | 25 | 0.254 | DSC: Tₘ = 124°C, 96 °C |
| | | ¹³C NMR: 0.64 mole percent E-4-P Incorp.; Branching per 1000 CH₂'s: Total methyls (47.8), Methyl (35.8), Ethyl (3.7), Propyl (0.0), Butyl (3.1), Amyl (3.8), Hex and greater and end of chains (8.7), Am and greater and end of chains (10.2), Bu and greater and end of chains (8.3) | | | |
| 373 | **3a** | BPh₃ | 80 | 0.468 | |
| | | ¹³C NMR: 1.94 mole percent E-4-P Incorp.; Branching per 1000 CH₂'s: Total methyls (67.0), Methyl (50.7), Ethyl (6.3), Propyl (0.0), Butyl (3.7), Amyl (7.3), Hex and greater and end of chains (18.6), Am and greater and end of chains (8.4), Bu and greater and end of chains (9.9) | | | |
| 374 | **21a** | BPh₃ | 80 | 0.312 | |
| | | ¹³CNMR: 1.67 mole percent E-4-P Incorp.; Branching per 1000 CH₂'s: Total methyls (73.9), Methyl (47.9), Ethyl (9.2), Propyl (0.0), Butyl (0.0), Amyl (6.1), Hex and greater and end of chains (16.2), Am and greater and end of chains (15.7), Bu and greater and end of chains (16.8) | | | |
| 375 | **8a** | B(C₆F₅)₃ | 80 | 0.232 | GPC (THF, 35 °C): M_{w}=5,130, PDI= 1.7; DSC: Tₘ: 117°C, 46°C |
| | | ¹H NMR: 1.6 mole percent E-4-P Incorp.; Branching per 1000 CH₂'s: Total methyls (94) | | | |

### Examples 376-381

### General Procedure for Ethylene Polymerizations of Table 17:

### Ethylene Polymerizations in the Parr® Reactor

Procedure. Prior to conducting the polymerization, the Parr® reactor flushed with nitrogen, heated under vacuum overnight, and then allowed to cool to room temperature. In the drybox, a glass vial was loaded with the nickel compound, Lewis acid and solvent and then capped with a rubber septum. The solution of the nickel complex and Lewis acid was then transferred to a 100 mL Parr reactor under vacuum, and the reactor was pressurized with ethylene and the reaction mixture was mechanically stirred. After the stated reaction time, the ethylene pressure was released, and the polymer was precipitated by adding the reaction mixture to a solution of MeOH (~100 mL) and concentrated HCl (~1-3 mL). The polymer was then collected on a frit and rinsed with HCl, MeOH, and acetone. The polymer was transferred to a pre-weighed vial and dried under vacuum overnight. The polymer yield and characterization were then obtained.

**Table 17**

| Ethylene Polymerizations in the Parr Reactor (0.02 mmol Cmpd, Trichlorobenzene (35 mL)) | | | | | | | | |
|---|---|---|---|---|---|---|---|---|
| Ex. | Cmpd | Time (h) | Press. (MPa) | LewisAcid (equiv) | PE(g) | PE(TO)^{a} | M.W.^{b} (MI, GPC, and/or ¹H NMR) | Total Me^{c} |
| 376 | **6a** | 9.9 | 5.5 | B(C₆F₅)₃/2 | 7.46 | 12,400 | Mₙ(¹H):no olefins | 34.4 |
| 377 | **6a** | 0.5 | 5.5 | B(C₆F₅)₃/2 | 3.5 | 5,940 | Mₙ(¹H):no olefins | 40.3 |
| 378 | **6a** | 6.5 | 5.5 | B(C₆F₅/₃2 | 9.30 | 15,500 | Mₙ(¹H):no olefins | 39.2 |
| 379 | **6a** | 4.8 | 1.4 | B(C₆F₅)₃/2 | 0.26 | 394 | Mₙ(¹H):no olefins | 32.6 |
| 380 | **3a** | 6.0 | 3.5 | BPh₃/5 | 3.57 | 5,560 | Mₙ(¹H):no olefins | 19.1 |
| 381 | **6a** | 6.6 | 3.5 | B(C₆F₅)₃/5 | 1.52 | 2,480 | Mₙ(¹H):no olefins | 29.0 |

| | | | | | | | | |
|---|---|---|---|---|---|---|---|---|
| ^{a}TO: number of turnovers per metal center = (moles ethylene consumed, as determined by the weight of the isolated polymer or oligomers) divided by (moles catalyst). Calculations are based upon the exact amount of catalyst used. | | | | | | | | |
| ^{b}M.W.: Molecular weight of the polymer or oligomers as determined by melt index (MI: g/10 min at 190°C), GPC (molecular weights are reported versus polystyrene standards; conditions: Waters 150C, trichlorobenzene at 150°C, Shodex columns at- 806MS 4G 734/602005, RI detector), and/or ¹H NMR (olefin end group analysis). | | | | | | | | |
| ^{c}Total number of methyl groups per 1000 methylene groups as determined by ¹H NMR analysis. ^{d}Under the same reaction conditions (e.g., no Lewis acid present), nickel compounds **51 - 54** gave analogous results: no polymer was isolated, but the ¹H NMR spectra showed a -(CH₂)ₙ- resonance. | | | | | | | | |

### Examples 382-437

### General Procedure for Ethylene (28-35 kPa) Polymerizations of Table 18

Procedure. In the drybox, a glass Schlenk flask was loaded with the nickel compound, Lewis acid, solvent and a stir bar. The flask was then capped with a rubber septum and the stopcock was closed prior to removing the flask from the drybox. The flask was then attached to the ethylene line where it was evacuated and backfilled with ethylene. The reaction mixture was stirred under ethylene for the stated reaction time, the ethylene pressure was then released, and the polymer was precipitated by adding the reaction mixture to a solution of MeOH (∼100 mL) and concentrated HCl (∼1-3 mL). The polymer was then collected on a frit and rinsed with MeOH. The polymer was transferred to a pre-weighed vial and dried under vacuum overnight. The polymer yield and characterization were then obtained.

**Table 18**

| Ethylene Polymerizations at 28-35 kPa Ethylene | | | | | | |
|---|---|---|---|---|---|---|
| Ex. | Cmpd | Lewis Acid (equiv) | Time (h) | Solvent (mL)^{b} | PE(g) | PE(TO) |
| 382 | **1a** | B(C₆F₅)₃/20 | 25.0 | Toluene (35) | *a* | *a* |
| 383 | **1a** | MAO-IP/90 | 0.5 | Toluene (35) | 0.924 | 1,040 |
| Description: White rubbery solid. | | | | | | |
| 384 | **2a** | MAO-IP/86 | 0.5 | Toluene (35) | 0.534 | 577 |
| Description: White, soft, slightly rubbery solid. | | | | | | |
| 385 | **3a** | BPh₃/5 | 26.3 | Toluene (35) | *a* | *a* |
| 386 | **3a** | BPh₃/20 | 23.5 | Toluene (35) | 0.662 | 787 |
| Description: Slightly sticky, clear, colorless amorphous solid. | | | | | | |
| ¹H NMR (C₆D₆, rt) 99.6 total Me/1000CH₂ | | | | | | |
| 387 | **3a** | BPh₃/50 | 23.5 | Toluene (35) | 0.110 | 131 |
| Description: Clear, colorless sticky viscous oil. | | | | | | |
| ¹H NMR (C₆D₆, rt) 98.6 total Me/1000CH₂ | | | | | | |
| 388 | **3a** | BPh₃/100 | 23.5 | Toluene (35) | 0.021 | 25 |
| Description: Light yellow, clear amorphous solid/oil. | | | | | | |
| ¹H NMR (C₆D₆, rt) 102.7 total Me/1000CH₂ | | | | | | |
| 389 | **3a** | B(C₆F₅)₃/20 | 32.4 | Toluene (35) | 0.042 | 50 |
| Description: White powder. | | | | | | |
| 390 | **3a** | BF₃·Et₂O/50 | 25.5 | Toluene (35) | *a* | *a* |
| 391 | **4a** | BPh₃/50 | 23.5 | Toluene (35) | 0.261 | 310 |
| Description: Clear, amorphous gummy solid. | | | | | | |
| ¹H NMR (C₆D₆, rt) 107.4 total Me/1000CH₂ | | | | | | |
| 392 | **4a** | BPh₃/100 | 37.2 | Toluene (35) | 0.797 | 950 |
| Description: Soft, white powder/solid. | | | | | | |
| 393 | **5a** | BPh₃/5 | 26.3 | Toluene (35) | *a* | *a* |
| 394 | **5a** | BPh₃/50 | 23.5 | Toluene (35) | 0.054 | 64 |
| Description: White slightly sticky, partially amorphous solid. | | | | | | |
| 395 | **5a** | BPh₃/50 | 26.4 | Toluene (35) | 0.065 | 77 |
| | | Description: Clear/white partial powder/partial amorphous solid. | | | | |
| 396 | **6a** | B(C₆F₅)₃/5 | 26.4 | Toluene (35) | 0.15 | 180 |
| Description: Brown sticky amorphous solid. ¹H NMR (C₆D₆, rt): 105.4 Total | | | | | | |
| Me/1000 CH₂ | | | | | | |
| 397 | **6a** | B(C₆F₅)₃/20 | 26.4 | Toluene (35) | 7.38 | 8,760 |
| Description: White, slightly rubbery or spongy powder. ¹³C NMR: Branching per 1000 CH₂'s. Total methyls (59.8), methyl (38.5), ethyl (10.4), propyl (1.6), butyl (2.4), hexyl and greater and end of chains (7.0), amy and greater and end of chains (7.9), butyl and greater and end of chains (9.3). | | | | | | |
| 398 | **6a** | BPh₃/100 | 17.0 | Toluene (35) | 0.17 | 200 |
| Description: White soft powder. | | | | | | |
| 399 | **6a** | BF₃^{.}Et₂O/50 | 25.5 | Toluene (35) | *a* | *a* |
| 400 | **6a** | Al(O-*i*-Pr)₃/20 | 22.4 | Toluene (35) | *a* | *a* |
| 401 | **6a** | PMAO-IP/28 | 25.1 | Toluene (35) | *a* | *a* |
| 402 | **7a** | B(C₆F₅)₃/20 | 24.1 | Toluene (35) | 1.04 | 1,180 |
| Description: White powder. | | | | | | |
| 403 | **8a** | B(C₆F₅)₃/5 | 25.4 | Toluene (35) | 1.45 | 1,730 |
| Soft partial powder/partial amorphous solid. ¹H NMR (C₆D₆, rt): 118.3 Total Me/1000 | | | | | | |
| CH₂ | | | | | | |
| 404 | **8a** | BPh₃/100 | 17.0 | Toluene (35) | 0.016 | 19 |
| Description: Tan powder. | | | | | | |
| 405 | **8a** | B(C₆F₅)₃/20 | 23.1 | Toluene (35) | 2.82 | 3,350 |
| Description: Brown amorphous sticky solid. ¹H NMR (C₆D₆, rt): 143.0 Total | | | | | | |
| Me/ 1000 CH₂ | | | | | | |
| 406 | **9a** | B(C₆F₅)₃/5 | 26.4 | Toluene (35) | 0.018 | 21 |
| Description: Brown partial oil, partial amorphous solid. | | | | | | |
| 407 | **9a** | B(C₆F₅)₃/20 | 23.1 | Toluene (35) | 6.99 | 8,300 |
| Description: Brown amorphous sticky solid. ¹H NMR (C₆D₆, rt): 174.4 Totalt | | | | | | |
| Me/1000 CH₂ | | | | | | |
| 408 | **10a** | BPh₃/20 | 24.2 | Toluene (35) | 1.43 | 1,700 |
| Description: White powder. | | | | | | |
| 409 | **12a** | BPh₃/20 | 25.0 | Toluene (35) | 0.749 | 890 |
| Description: White powder. | | | | | | |
| 410 | **12a** | B(C₆F₅)₃/10 | 22.5 | Toluene (35) | a | a |
| 411 | **14a** | B(C₆F₅)₃/20 | 25.5 | Toluene (35) | 0.97 | 1,150 |
| Description: White, stringy powder | | | | | | |
| 412 | **15a** | B(C₆F₅)₃/10 | 22.5 | Toluene (35) | 0.106 | 126 |
| Description: Slightly rubbery off-white solid. | | | | | | |
| 413 | **17a** | B(C₆F₅)₃/20 | 25.5 | Toluene (35) | 2.08 | 2,470 |
| Description: Soft white powder. | | | | | | |
| 414 | **21a** | BPh₃/5 | 25.4 | Toluene (35) | 1.88 | 2,230 |
| Description: White, somewhat rubbery powder. | | | | | | |
| 415 | **21a** | BPh₃/20 | 26.4 | Toluene (35) | 1.73 | 2,060 |
| Description: White powder. | | | | | | |
| 416 | **21a** | BPh₃/50 | 26.4 | Toluene (35) | 0.631 | 750 |
| Description: White powder. | | | | | | |
| 417 | **21a** | BPh₃/100 | 21.4 | 1,2,4-TCB (20) | 0.474 | 563 |
| Description: White powder. | | | | | | |
| 418 | **21b** | BPh₃/100 | 21.4 | 1,2,4-TCB (20) | 0.156 | 185 |
| 419 | **22a** | BPh₃/100 | 37.2 | Toluene (35) | 0.777 | 920 |
| Description: White powder. | | | | | | |
| 420 | **23a** | BPh₃/20 | 26.0 | Toluene (35) | 0.409 | 473 |
| Description: Almost clear, sticky amorphous oil/solid. | | | | | | |
| 421 | **24a** | B(C₆F₅)₃/10 | 22.5 | Toluene (35) | *a* | *a* |
| 422 | **24a** | BPh₃/50 | 22.4 | Toluene (35) | 0.374 | 444 |
| Description: White powder. | | | | | | |
| 423 | **25a** | BPh₃/50 | 24.2 | Toluene (35) | 1.47 | 1,750 |
| Description: White, slightly rubbery powder. | | | | | | |
| 424 | **27a** | BPh₃/20 | 26.0 | Toluene (35) | 0.856 | 992 |
| Description: Amorphous, slightly sticky, waxy, clear solid. | | | | | | |
| ¹H NMR (C₆D₆, rt) 90.0 total Me/ 1000CH₂ | | | | | | |
| 425 | **30a** | B(C₆F₅)₃/20 | 24.2 | Toluene (25) | 0.65 | 770 |
| Description: White powder. | | | | | | |
| 426 | **34a** | B(C₆F₅)₃/20 | 23.1 | Toluene (35) | 5.75 | 6,830 |
| Description: Tan amorphous solid. ¹H NMR (C₆D₆, rt) 182.0 Total Me/1000 CH₂. | | | | | | |
| Mn ∼1,980 | | | | | | |
| 427 | **36a** | BPh₃/20 | 25.6 | Toluene (35) | 1.32 | 1,570 |
| Description: White powder. | | | | | | |
| 428 | **36a** | B(C₆F₅)₃/10 | 24.3 | Toluene (35) | 0.11 | 130 |
| Description: Tan powder. | | | | | | |
| 429 | **37a** | BPh₃/20 | 23.8 | Toluene (35) | 0.486 | 576 |
| 430 | **38a** | BPh₃/20 | 26.0 | Toluene (35) | 0.024 | 28 |
| Description: Clear, amorphous, very slightly sticky solid | | | | | | |
| ¹H NMR (C₆D₆, rt) 91.2 total Me/1000CH₂ | | | | | | |
| 431 | **39a** | B(C₆F₅)₃/20 | 22.6 | Toluene (20) | 0.244 | 290 |
| Description: White powder. | | | | | | |
| 432 | **39a** | BPh₃/200 | 23.8 | Toluene (35) | *a* | *a* |
| 433 | **41a** | B(C₆F₅)₃/5 | 25.4 | Toluene (35) | 0.059 | 70 |
| Description: White powder. | | | | | | |
| 434 | **41a** | B(C₆F₅)₃/20 | 22.4 | Toluene (35) | 1.73 | 2,060 |
| Description: White powder. | | | | | | |
| 435 | **50** | BPh₃/100 | 21.4 | 1,2,4-TCB (20) | 1.06 | 1,260 |
| Description: White powder. | | | | | | |
| 436 | **52** | BPh₃/100 | 21.4 | 1,2,4-TCB (20) | 1.21 | 1,440 |
| Description: White powder. | | | | | | |
| 437 | **52** | BPh₃/100 | 23.1 | Toluene (35) | 1.40 | 1,660 |
| Description: White powder. | | | | | | |

| | | | | | | |
|---|---|---|---|---|---|---|
| ^{a}Only a trace of polymer or no polymer was isolated. | | | | | | |
| ^{b}1,2,4-Trichlorobenzene is abbreviated as 1,2,4-TCB. | | | | | | |

### Example 438

### Ethylene Polymerization Using (acac)Ni(Et)PPh₃ Precursor at 6.9 MPa

In the drybox, a glass insert was loaded with (acac)Ni(Et)PPh₃ (26.9 mg, 0.06 mmol) and [2-(NaO)-3,5-(*t*-Bu)₂-C₆H₂-C(Me)=NAr (Ar = 2,6-(*i*-Pr)₂-C₆H₃) 0.5 THF] (25.8 mg, 1 equiv). The insert was cooled to -35 °C in the drybox freezer, 5 mL of C₆D₆ was added to the cold insert, and the insert was cooled again. BPh₃ (29.1 mg, 2 equiv) was added to the cold solution, and the insert was then capped and sealed and cooled again. Outside of the drybox, the cold insert was placed under a nitrogen purge into the pressure tube. The pressure tube was sealed, placed under ethylene (6.9 MPa), and allowed to warm to rt as it was shaken mechanically for approximately 18 h. Polyethylene (16.5 g, 9,820 TO) was isolated as a powder following precipitation from methanol.

### Example 439

### Ethylene Polymerization Using NiBr₂ Precursor at 28-35 MPa

The sodium salt of the ligand of Example 1 (1.01 q, 2.23 mmol), e.g. 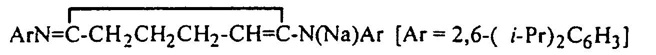 was placed in a round bottom flask in the drybox together with 487 mg (2.23 mmol) of NiBr₂. THF (20 mL) was added and the solution was stirred for ∼2 months. The THF was removed in vacuo and the product was dissolved in CH₂Cl₂ and the resulting solution was filtered. The solvent was removed and the product was dried in vacuo. An orange powder (488 mg) was isolated. (In addition to CD₂Cl₂, the product was also soluble in C₆D₆. ¹H NMR spectra in both solvents were complex.)

In the drybox, a glass Schlenk flask was loaded with the resulting orange nickel compound (17 mg, ∼0.03 mmol), 35 mL of toluene and a stir bar. The flask was then capped with a rubber septum and the stopcock was closed prior to removing the flask from the drybox. The flask was then attached to the ethylene line where it was evacuated and backfilled with ethylene. MAO-IP (2 mL, ∼94 equiv) was added to the flask via cannula. The reaction mixture was stirred under ethylene for 3.5 h, the ethylene pressure was then released, and the polymer was precipitated by adding the reaction mixture to a solution of MeOH (~100 mL) and concentrated HCl (∼1-3 mL). The polymer was then collected on a frit and rinsed with MeOH. The polymer was transferred to a pre-weighed vial and dried under vacuum overnight. A white polyethylene film (5.09 g, ∼6050 TO) was isolated.

### Examples 440-468

### Ligand Syntheses

Ligand syntheses and deprotonations were carried out according to the general procedures given below and under Examples 1-16 (see above) unless stated otherwise.

### Example 440

### [2-(OH)-3,5-Cl₂-C₆H₂]-C(Me)=NAr [Ar = 2,6-(i-Pr)₂-C₆H₃]

The general procedure for imine synthesis was followed using 10.03 g (48.9 mmol) of 3',5'-dichloro-2'-hydroxyacetophenone and 11.27 g (1.30 equiv) of 2,6-diisopropylaniline. A yellow powder (15.35 g, 86.2%) was isolated: ¹H NMR (CDCl₃) δ 7.46 (d, 1, Ar': H), 7.44 (d, 1, Ar': H), 7.14 (m, 3, Ar: H), 2.64 (septet, 2, C*H*Me₂), 2.12 (s, 3, N=C(*Me*)), 1.08 and 1.04 (d, 6 each, CH*MeMe'*).

The sodium salt was synthesized according to the above general procedure: ¹H NMR *(THF-d*_{*8*}*):* 0.59 equiv of THF coordinated.

### Example 441

### [2- (OH) -3, 5-Cl₂-C₆H₂] -C (Me) =NAr [Ar = 2,6-Me₂-C₆H₃]

The general procedure for imine synthesis was followed using 10.681 g (52.1 mmol) of 3',5'-dichloro-2'-hydroxyacetophenone and 8.21 g (1.30 equiv) of 2,6-dimethylaniline. A yellow powder (7.61 g, 47.4) was isolated: ¹H NMR (CDCl₃) δ 7.57 (d, 1, Ar': H), 7.52 (d, 1, Ar': H), 7.15 (d, 2, Ar: H_{*m*}), 7.08 (t, 1, Ar: H_{*p*}), 2.21 (s, 3, N=C*Me*), 2.10 (s, 6, Ar: *Me*).

The sodium salt was synthesized according to the above general procedure: ¹H NMR *(THF-d*_{*8*}*):* 0.59 equiv of THF coordinated.

### Example 442

### [2-(OH)-3,5-Cl₂-C₆H₂]-C(Me)=NAr [Ar = 2-(t-Bu)-C₆H₄]

The general procedure for imine synthesis was followed using 10.41 g (50.8 mmol) of 3',5'-dichloro-2'-hydroxyacetophenone and 9.85 g (1.30 equiv) of 2-*t*-butylaniline. A yellow powder (15.30 g, 89.6%, 2 crops) was isolated: ¹H NMR (CDCl₃) δ 7.55 (d, 1, Ar': H), 7.52 (d, 1, Ar': H), 7.50 (d, 1, Ar: H), 7.25 (t, 1, Ar: H), 7.22 (t, 1, Ar: H), 6.52 (d, 1, Ar: H), 2.31 (s, 3, Me), 1.36 (s, 9, CMe₃).

The sodium salt was synthesized according to the above general procedure: ¹H NMR (THF-*d*_{*8*}): 0.16 equiv of THF coordinated.

### Example 443

### [2-(OH)-3,5-Br₂-C₆H₂]-CH=NAr [Ar = 2,6-(i-Pr)₂-C₆H₃]

The general procedure for imine synthesis was followed using 3.23 g (11.5 mmol) of 3,5-dibromosalicylaldehyde and 2.66 g (1.30 equiv) of 2,6-diisopropylaniline. A yellow powder (3.10 g, 61.4%) was isolated: ¹H NMR (CDCl₃ 8.21 (s, 1, N=C*H*), 7.81 (d, 1, Ar': H), 7.45 (d, 1, Ar': H), 7.22 (s, 3, Ar: H), 2.94 (septet, 2, C*H*Me₂), 1.18 (d, 12, CH*Me*_{*2*}).

The sodium salt was synthesized according to the above general procedure: ¹H NMR (THF-d_{*8*}): 0.7 equiv of THF coordinated.

### Example 444

### [2-(OH)-3,5-Br₂-C₆H₂]-C(Me)=NAr [ Ar = 2,6-(i-Pr)₂-C₆H₃]

The general procedure for imine synthesis was followed using 10.84 g (36.9 mmol) of 3',5'-dibromo-2'-hydroxyacetophenone and 8.50 g (1.30 equiv) of 2,6-diisopropylaniline. A yellow powder (13.16 g, 78.7%) was isolated: ¹H NMR (CDCl₃) δ 7.83 (d, 1, Ar': H), 7.73 (d, 1, Ar': H), 7.26 (m, 3, Ar: H), 2.76 (septet, 2, C*H*Me₂), 2.24 (s, 3, *Me*), 1.19 and 1.18 (d, 6 each, CH*MeMe'*).

The sodium salt was synthesized according to the above general procedure: ¹H NMR (THF-*d*_{*8*}): 0.54 equiv of THF coordinated.

### Example 445

### [2-(OH)-3,5-Br₂-C₆H₂]-C(Me)=NAr [Ar = 2,6-Me₂-C₆H₃]

The general procedure for imine synthesis was followed using 10.43 g (35.5 mmol) of 3',5'-dibromo-2'-hydroxyacetophenone and 5.59 g (1.30 equiv) of 2,6-dimethylaniline. A yellow powder (11.6 g) was isolated. The ¹H NMR spectrum of the initially isolated product showed that it was contaminated by the hydroxyacetophenone. The product was repurified by washing with more methanol, dissolving in CH₂Cl₂ and drying over Na₂SO₄, filtering and evaporating the solvent. A yellow powder (5.60 g) was isolated. The product mixture was now 12.7% of the starting aldehyde. The remainder is the desired imine product: ¹H NMR (CDCl₃) δ 7.78 (d, 1, Ar' : H), 7.71 (d, 1, Ar' : H), 7.11 (d, 2, Ar: H_{*m*}), 7.05 (t, 1, Ar: H_{*p*}), 2.18 (s, 3, N=C*Me*), 2.05 (s, 6, Ar: *Me*).

The sodium salt was synthesized according to the above general procedure and is clean and consistent with the desired product (no hydroxyacetophenone impurities present): ¹H NMR (THF*-d*_{*8*}): 0.81 equiv of THF coordinated.

### Example 446

### [2-(OH)-3,5-Br₂-C₆H₂]-C(Me)=NAr [Ar = 2-(t-Bu)-C₆H₄]

The general procedure for imine synthesis was followed using 10.04 g (34.2 mmol) of 3',5'-dibromo-2'-hydroxyacetophenone and 6.63 g (1.30 equiv) of 2-*t*-butylaniline. A yellow powder (12.63 g, 86.9%) was isolated: ¹H NMR (CDCl₃) δ 7.81 (d, 1, Ar': H), 7.72 (d, 1, Ar': H), 7.51 (d, 1, Ar: H), 7.27 (t, 1, Ar: H), 7.22 (t, 1, Ar: H), 6.51 (d, 1, Ar: H), 2.31 (s, 3, N*=*C*Me*), 1.37 (s, 9, C*Me*_{*3*}).

The sodium salt was synthesized according to the above general procedure: ¹H NMR (THF-*d*_{*8*}): trace THF coordinated.

### Example 447

### [2-(OH)-3,5-(t-Bu)₂C₆H₂]-CH=NAr [Ar = 2-(t-Bu)-C₆H₄]

The general procedure for imine synthesis was followed using 4.12 g (17.6 mmol) of 3,5,-di-*t*-butyl-2-hydroxybenzaldehyde and 3.15 g (1.20 equiv) of 2-*t*-butylaniline. The desired imine product was isolated as a yellow powder: ¹H NMR (CDCl₃) δ 8.36 (s, 1, N=C*H*), 7.40 (d, 1, Ar': H), 7.36 (d, 1, Ar: H), 7.18 (t, 1, Ar: H), 7.15 (d, 1, Ar': H), 7.13 (t, 1, Ar: H), 6.80 (d, 1, Ar: H), 1.42, 1.37 and 1.26 (s, 9 each, C*Me*_{*3*}*,* C'*Me*_{*3*}, C''*Me*_{*3*}).

The sodium salt was synthesized according to the above general procedure: ¹H NMR (THF-d₈): ~1 equiv of THF coordinated.

### Example 448

### [2-(OH)-3,5-(t-Bu)₂C₆H₂]-CH=NAr [Ar = 2-Aza-C₅H₄]

The general procedure for imine synthesis was followed using 3.02 g (12.9 mmol) of 3,5-di-*t*-butyl-2-hydroxybenzaldehyde and 1.46 g (1.20 equiv) of 2-aminopyridine. An orange powder (0.552 g, 13.8%) was isolated: ¹H NMR (CDCl₃) δ 9.47 (s, 1, N=C*H*) , 8.51 (m, 1, Py: H), 7.77 (m, 1, Py: H), 7.48 (d, 1, Ar': H), 7.35 (d, 1, Ar': H), 7.33 (m, 1, Py: H), 7.20 (m, 1, Py: H), 1.48 (s, 9, C*Me*₃), 1.34 (s, 9, C'*Me*_{*3*}).

The sodium salt was synthesized according to the above general procedure: ¹H NMR (THF-d₈): 0.2 equiv of THF coordinated.

### Example 449

### [2-(OH)-3,5-(t-Bu)₂C₆H₂]-CH=NAr [Ar = 2-Aza-6-Me-C₅H₃]

The general procedure for imine synthesis was followed using 3.46 g (14.7 mmol) of 3,5-di-*t*-butyl-2-hydroxybenzaldehyde and 1.91 g (1.20 equiv) of 2-amino-3-picoline. The first crop isolated as a precipitate from methanol was an orange powder (1.18 g). This crop was not clean and was discarded, although some of the desired product was present as a minor component. The remaining methanol solution was allowed to slowly evaporate to give orange crystals. The methanol was decanted off of the crystals and the standard work-up procedure was followed. An orange powder (0.813 g) was isolated, and the NMR spectrum of this second crop was clean and consistent with the desired product: ¹H NMR (CDCl₃) δ 9.45 (s, 1, N=C*H*), 8.34 (d, 1, Py: H), 7.60 (d, 1, Py: H), 7.48 (d, 1, Ar': H), 7.38 (d, 1, Ar': H), 7.13 (dd, 1, Py: H), 2.49 (s, 3, *Me*), 1.5 (s, 9, C*Me*_{*3*})*,* 1.34 (s, 9, C'*Me*_{*3*}).

The sodium salt was synthesized according to the above general procedure: ¹H NMR (THF-*d*_{*8*}): 0.4 equiv of THF coordinated.

### Example 450

### [2-(OH)-3,5-(t-Bu)₂C₆H₂]-CH=NCHPh₂

The general procedure for imine synthesis was followed using 3.00 g (12.8 mmol) of 3,5-di-*t*-butyl-2-hydroxybenzaldehyde and 2.60 g (1.11 equiv) of aminodiphenylmethane. A yellow powder (2.85 g, 55.7%) was isolated: ¹H NMR (CDCl₃) δ 8.50 (s, 1, N=C*H*) , 7.42 (d, 1, Ar': H), 7.40 - 7.23 (m, 10, C*Ph*_{*2*})*,* 7.11 (d, 1, Ar': H), 5.63 (s, 1, C*H*Ph₂), 1.48 and 1.32 (s, 9 each, C*Me*_{*3*} and C'*Me*_{*3*}).

The sodium salt was synthesized according to the above general procedure: ¹H NMR (THF-*d*_{*8*}): 1 equiv of THF coordinated.

### Example 451

### [2-(OH)-3,5-(t-Bu)₂C₆H₂]-CH=NR [R = 1,2,3,4-tetrahydro-1-naphthyl]

The general procedure for imine synthesis was followed using 3.08 g (13.1 mmol) of 3,5-di-t-butyl-2-hydroxybenzaldehyde and 2.32 g (1.20 equiv) of 1,2,3,4-tetrahydro-1-naphthylamine. A yellow powder (3.97 g, 83.4%) was isolated: ¹H NMR (CDCl₃) δ 8.45 (s, 1, N=CH), 7.39 (d, 1, Ar': H), 7.22 - 7.04 (m, 5, Ar: H, Ar': H), 4.53 (m, 1, NCHCH₂CH₂CH₂), 2.88 (m, 2, NCHCH₂CH₂CH₂), 2. 14 - 1.79 (m, 4, NCHCH₂CH₂CH₂) , 1.42 (s, 9, C*Me*_{*3*})*,* 1.32 (s, 9, C'*Me*_{*3*}).

The sodium salt was synthesized according to the above general procedure: ¹H NMR (THF-d₈): 0.6 equiv of THF coordinated.

### Example 452

### [2-(OH)-3,5-(NO₂)₂C₆H₂]-CH=NAr [Ar = 2-(t-Bu)-C₆H₄]

The general procedure for imine synthesis was followed using 3.05 g (14.4 mmol) of 3,5-dinitrosalicylaldehyde and 2.57 g (1.20 equiv) of 2-t-butylaniline. A yellow powder was isolated: ¹H NMR (CDCl₃) δ 8.94 (s, 1, N=C*H*), 8.54 (d, 1, Ar': H), 8.50 (d, 1, Ar': H), 7.49 (d, 1, Ar: H), 7.35 (t, 1, Ar: H), 7 .31 (t, 1, Ar: H), 7.02 (d, 1, Ar: H), 1.40 (s, 9 C*Me*_{*3*}).

The sodium salt was synthesized according to the above general procedure: ¹H NMR (THF-*d*_{*8*}): 0.79 equiv of THF coordinated.

### Example 453

### [2-(OH)-3,5-(NO₂)₂C₆H₂]-CH=NAr [Ar = 2-Me-6-Cl-C₆H₃]

The general procedure for imine synthesis was followed using 1.56 g (7.33 mmol) of 3,5-dinitrosalicylaldehyde and 1.25 g (1.20 equiv) of 2-chloro-6-methylaniline. An orange powder (1.35 g, 55.0%) was isolated: ¹H NMR (CDCl₃) δ 15.96 (br s, 1, O*H*)*,* 8.71 (s, 1, N=C*H*)*,* 8.60 (d, 1, H_{aryl}), 7.50 - 7.15 (m, 4, H_{aryl}), 2.36 (s, 1, *Me*).

The sodium salt was synthesized according to the above general procedure: ¹H NMR (THF-*d*_{*8*}): 0.14 equiv of THF coordinated.

### Example 454

### [2-(OH)-3,5-(NO₂)₂C₆H₂]-CH=NR [R = 1,2,3,4-tetrahydro-1-naphthyl]

The general procedure for imine synthesis was followed using 3.07 g (14.5 mmol) of 3,5-dinitrosalicylaldehyde and 2.55 g (1.20 equiv) of 1,2,3,4-tetrahydro-1-naphthylamine. A yellow powder (4.31 g, 87.1%) was isolated: ¹H NMR (CDCl₃) δ 8.98 (d, 1, Ar': H), 8. 36 (d, 1, Ar': H), 8.07 (d, 1, Ar: H), 7.36 (m, 1, Ar: H), 7.27 (m, 3, N=C*H* and Ar: H), 7.15 (d, 1, Ar: H), 5.04 (m, 1, NCHCH₂CH₂CH₂), 2.90 (m, 2, NCHCH₂CH₂CH₂) , 2.26, 1.97 and 1.87 (m's, 2, 1 and 1 each, NCHC*H*_{*2*}C*H*_{*2*}CH₂).

The sodium salt was synthesized according to the above general procedure: ¹H NMR (THF-*d*_{*8*}): 0.11 equiv of THF coordinated.

### Example 455

### [2-(OH)-3,5-I₂-C₆H₂]-CH=NAr [Ar = 2,6-(i-Pr)₂-C₆H₃]

The general procedure for imine synthesis was followed using 6.00 g (16.0 mmol) of 3,5-diiodosalicylaldehyde and 3.70 g (1.31 equiv) of 2,6-diisopropylaniline. A yellow powder (7.93 g, 93.0%) was isolated: ¹H NMR (CDCl₃) δ 8.14 (d, 1, Ar': H), 8.10 (s, 1, N=CH), 7.60 (d, 1, Ar': H), 7.20 (m, 3, Ar: H), 2.92 (septet, 2, C*H*Me₂), 1.18 (d, 12, CH*Me*_{*2*}).

The sodium salt was synthesized according to the above general procedure: ¹H NMR (THF-d₈) : 0.67 equiv of THF coordinated.

### Example 456

### [2-(OH)-4,6-(OMe)₂-C₆H₂]-CH=NAr [Ar = 2,6-(i-Pr)₂-C₆H₃]

The general procedure for imine synthesis was followed using 5.05 g (27.7 mmol) of 4,6-dimethoxysalicylaldehyde and 5.90 g (1.20 equiv) of 2,6-diisopropylaniline. A yellow powder (3.59 g, 38.0%) was isolated: ¹H NMR (CDCl₃) δ 8.58 (s, 1, N=C*H*) , 7.18 (s, 3, Ar: H), 6.13 (d, 1, Ar': H), 5.92 (d, 1, Ar': H), 3. 84 (s, 3, O*Me*) , 3.80 (s, 3, O*Me*') , 3.03 (septet, 1, C*H*Me₂), 1.19 (d, 12, CH*Me*_{*2*}).

The sodium salt was synthesized according to the above general procedure: ¹H NMR (THF-*d*_{*8*}): No THF coordinated.

### Example 457

### [2-Hydroxynaphthyl]-CH=NAr [Ar = 2,6-Br₂-4-F-C₆H₂]

The general procedure for imine synthesis was followed using 29.8 g (173 mmol) of 2-hydroxy-1-naphthaldehyde and 52.0 g (193 mmol) of 2,6-dibromo-4-fluoroaniline. A yellow powder (62.1 g, 84.9%, 2 crops) was isolated: ¹H NMR (CDCl₃ ) δ 9.40 (s, 1, N=C*H*), 8.09 (d, 1, Ar': H), 7.92 (d, 1, Ar': H), 7.81 (d, 1, Ar': H), 7.55 (t, 1, Ar': H), 7.43 (d, 2, Ar: H), 7.40 (t, 1, Ar': H), 7.25 (d, 1, Ar': H).

The sodium salt was synthesized according to the above general procedure: ¹H NMR (THF-*d*_{*8*}) : 0.66 equiv of THF coordinated.

### Example 458

### [2-Hydroxynaphthyl]-CH=NAr [Ar = 2-Aza-6-Me-C₅H₃]

The general procedure for imine synthesis was followed using 3.44 g (20.0 mmol) of 2-hydroxy-1-naphthaldehyde and 2.59 g (1.20 equiv) of 2-amino-3-picoline. A yellow-orange powder (4.51 g, 86.0%) was isolated: ¹H NMR (CDCl₃) δ 9.94 (d,1, H_{aryl}), 8.27 (d, 1, N=C*H*) , 8.09 (d, 1, H_{aryl}), 7.68 (d, 1, H_{aryl}), 7.54 (d, 1, H_{aryl}) , 7.51 (d, 1, H_{aryl}) , 7.44 (t, 1, H_{aryl}) , 7.24 (t, 1, H_{aryl}) , 7.02 (t, 1, H_{aryl}) , 6.85 (d, 1, H_{aryl}) , 2.44 (s, 3, *Me*).

The sodium salt was synthesized according to the above general procedure: ¹H NMR (THF*-d*_{*8*}): 0.1 equiv of THF coordinated.

### Example 459

### [2-Hydroxynaphthyl]-CH=NAr [Ar = 2-(t-Bu)-C₆H₄]

The general procedure for imine synthesis was followed using 10.19 g (59.2 mmol) of 2-hydroxy-1-naphthaldehyde and 10.60 g (1.20 equiv) of 2-*t*-butylaniline. A yellow powder (10.8 g, 60.4%) was isolated: ¹H NMR (CDCl₃) δ 9.27 (d, 1, N=C*H*), 8.18 (d, 1, H_{aryl}), 7.88 (d, 1, H_{aryl}) , 7.79 (d, 1, H_{aryl}) , 7.55 (t, 1, H_{aryl}), 7.52 (d, 1, H_{aryl}) , 7.39 (t, 1, H_{aryl}), 7.37 (t, 1, H_{aryl}), 7.30 (t, 1, H_{aryl}), 7.21 (d, 1, H_{aryl}), 7.19 (d, 1, H_{aryl}), 1.52 (s, 9, *CMe*_{*3*}) *.*

The sodium salt was synthesized according to the above general procedure: ¹H NMR (THF*-d*_{*8*}): 0.48 equiv of THF coordinated.

### Example 460

### (Ar) (H) N-C (Me) =CH-C(O)-Ph [Ar = 2,6-(i-Pr)₂-C₆H₃]

The general procedure for imine synthesis was followed using 5.17 g (31.9 mmol) of 1-benzoylacetone and 7.35 g (1.30 equiv) of 2,6-diisopropylaniline. After 2 days, no precipitate formed from the methanol solution. However, slow evaporation of the methanol yielded single crystals, which were isolated and washed with a small amount of additional methanol. The standard work-up procedure was then followed to yield a white powder (2.56 g, 25.0%): ¹H NMR (CDCl₃) δ 7.89 (d, 2, H_{aryl}), 7.38 (m, 3, H_{aryl}), 7.25 (t, 1, H_{aryl}), 7.12 (d, 1, H_{aryl}), 5.86 (s, 1, =C*H*) , 3.02 (septet, 2, C*H*Me₂), 1.71 (s, 3, N-C(*Me*)), 1.17 and 1.11 (d, 6 each, *CHMeMe');* ¹³C NMR (CDCl₃) δ 188.4 (*C*(O)), 165.0 (N-C(Me)), 146.2 (Ar: C_{*o*}), 140.0 and 133.5 (Ph: Cᵢₚₛₒ; Ar: Cᵢₚₛₒ), 130.8 and 128.3 (Ar: C_{*p*}; Ph: C_{*p*}), 128.1, 127.1 and 123.5 (Ph: C_{*o*}, C_{*m*}; Ar: C_{*m*}), 92.1 (C (Me) =C*H*), 28.5 (CHMe₂), 24.6 and 22.7 (CH*MeMe*'), 19.7 (N-C(*Me*)).

The sodium salt was synthesized according to the above general procedure: ¹H NMR (THF-*d*_{*8*}): 0.66 equiv of THF coordinated.

### Example 461

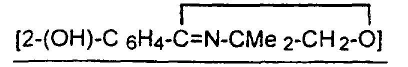

A 200 mL sidearm flask was charged with 5.0 g (42 mmol, 1.0 equiv) of 2-hydroxybenzonitrile, 5.6 g (63 mmol, 1.5 equiv) of 2-amino-2-methylpropanol, 0.29 g (2.1 mmol, 0.05 equiv) of ZnCl₂, and 90 mL of chlorobenzene. The reaction mixture was heated to reflux under N₂ atmosphere for 24 h. After this time, reflux was discontinued, the flask was cooled to ambient temperature, and most of the volatile materials were removed using a rotary evaporator. The resulting residue was dissolved in ∼100 mL of CH₂Cl₂, transferred to a separatory funnel, and washed with 3 x 50 mL of H₂O. The combined H₂O washings were back extracted with ∼30 mL of CH₂Cl₂, and the combined CH₂Cl₂ extracts were then dried over Na₂SO₄, filtered and evaporated to yield a brown oil which was purified by flash chromatography (SiO₂, eluting with 5:1 hexanes:EtOAc), to yield 6.3 g (78%) of the desired product: ¹H NMR (CDCl₃) δ 12.2 (br s, 1, O*H*), 7.6 (m, 1, Haryl) 7.4 (m, 1, H_{aryl}) , 7.06 (m, 1, H_{aryl}) , 6.92 (m, 1, H_{aryl}) , 4.14 (s, 2, C*H*_{*2*}), 1.44 (s, 6, C*Me*_{*2*}).

The sodium salt was synthesized according to the above general procedure: ¹H NMR (THF-d_{*8*}): 0.20 equiv of THF coordinated.

### Example 462

### (4-Me-C₆H₄-N=P(Ph)₂-CH₂-(Ph)₂P=N-C₆H₄-4-Me)

See *Phosphorus, Sulfur, and Silicon* **1990,** *47*, 401. A 100-mL 3-neck round-bottomed flask was fitted with a condenser, a nitrogen inlet and an addition funnel. It was charged with 2.64 g (6.87 mmol) of bis(diphenylphosphino)methane (DPPM) dissolved in 17 mL of benzene. The addition funnel was charged with 1.86 g (14.0 mmol) of 4-Me-C₆H₄-N₃ (prepared from *p*-toluidine hydrochloride, sodium nitrite and sodium azide, see Ugi, I; Perlinger, H.; Behringer, L. *Chemische Berichte* **1958,** *91*, 2330) dissolved in ca. 7-10 mL of benzene. The DPPM solution was heated to 60°C and the aryl azide solution slowly added to the reaction mixture. As the addition occurred, nitrogen was evolved. After the addition was completed, the reaction mixture was kept an additional 4 h at 60 °C. The solvent was then removed in vacuo, and the solid was collected, washed with 2 x 15mL of hexane and dried *in vacuo.* The yield was 3.75 g (92%): ¹H NMR (CDCl₃) δ 7.72 (m, 8, PPh₂: H_{*o*}) , 7.41 (t, 4, PPh₂: H_{*p*}), 7.29 (t, 3, PPh₂: H_{*m*}) , 6.83 (d, 4, NAr: H_{*m*}), 6.52 (d, 4, NAr: H_{*o*}), 3.68 (t, 2, J_{HP} = 14.2, PC*H*_{*2*}P) , 2.21 (s, 6, NAr: Me).

The sodium salt was synthesized according to the above general procedure: ¹H NMR (THF-d₈): 0.39 equiv of THF coordinated.

### Example 463

### (2-Me-C₆H₄-N=P(Ph)₂-CH₂-(Ph)₂P=N-C₆H₄-2-Me)

A 100-mL 3-neck round-bottomed flask was fitted with a condenser, a nitrogen inlet and an addition funnel. It was charged with 3.0 g (7.80 mmol) of bis(diphenylphosphino)methane (DPPM) dissolved in 20 mL of toluene. The addition funnel was charged with 2.11 g (15.8 mmol) of 2-Me-C₆H₄-N₃ (prepared from *o*-toluidine hydrochloride, sodium nitrite and sodium azide) dissolved in ca. 12 mL of toluene. The DPPM solution was heated to 60°C and the aryl azide solution slowly added to the reaction mixture. As the addition occurred, nitrogen was evolved. After the addition was completed, the reaction mixture was kept an additional 4 h at 60°C. The solvent was then removed *in vacuo,* the solid was collected and recrystallized from Et₂O/hexane. The yield was 2.70 g (58%). ¹H NMR (CDCl₃) δ 7.68 (m, 8, PPh₂: H_{*o*}), 7.38 (t, 4, PPh₂: H_{*p*}), 7.25 (t, 8, PPh₂: H_{*m*}), 7.09 (d, 2, NAr: H_{*m*}'), 6.76 (t, 2, NAr: H_{*m*}), 6.23 (t, 2, NAr: H_{*p*}) , 6.23 (d, 2, NAr: H_{*o*}), 3.87 (t, 2, J_{HP} = 13.5, PC*H*_{*2*}P), 2.29 (s, 6, NAr: *Me*).

The sodium salt was synthesized according to the above general procedure: ¹H NMR (THF-d₈): 0.59 equiv of THF coordinated.

### Example 464

### Lithium 5-Methyl-2-Thiophenecarboxylate

The sodium salt was synthesized from commercially available 5-methyl-2-thiophenecarboxylic acid according to the above general procedure and the cation was exchanged with an excess of lithium chloride to improve product solubility: ¹H NMR (THF-*d*_{*8*}): 0.25 equiv of THF coordinated.

### Example 465

### Cy₂PCH₂CH(CH₃)SLi

A 100-mL Schlenk flask was charged with 1.28 g (6.28 mmol) of PCy₂Li (prepared from PCy₂H and n-BuLi) dissolved in 20 mL of THF. The flask was cooled to -78°C and propylene sulfide (520 mg, 7.01 mmol) was vacuum transferred onto the lithium salt solution. The reaction mixture was kept at -78°C for 45 min. The dry ice/acetone bath was then removed and the yellowish solution was allowed to warm to ambient temperature. After an additional 20 min, the solvent was removed in vacuo. The solid was washed three times with 30 mL of hexane and dried in vacuo. The yield was 1.37 g (78%). ¹H NMR (THF-*d*_{*8*}, 300 MHz, 23°C) δ 2.80 (m, 1, C*H*) , 1.31 (d, 3, J= 6 Hz,, C*H*₃), 1-2 (m, 24, *Cy*₂, PC*H*₂); ³¹P NMR: δ -7.6.

### Example 466

### Sodium 2,3,5,6-Tetrachloro-4-Pyridinethiolate

The sodium salt was synthesized according to the above general procedure from the commercially available 2,3,5,6-tetrachloro-4-pyridinethiol.

### Example 467

### Sodium 2,5-Dimethylpyrrole

The sodium salt was synthesized according to the above general procedure from the commercially available 2,5-dimethylpyrrole: ¹H NMR (THF-*d*_{*8*}): No THF coordinated.

### Example 468

### Sodium 2,6-Dibromo-4-Methylanilide

The sodium salt was synthesized according to the above general procedure from the commercially available 2,6-dibromo-4-methylaniline: ¹H NMR (THF-*d*_{*8*}): 0.5 equiv THF coordinated.

### Examples 469-498

Complexes **21** through **49** were synthesized according to the general procedure for the synthesis of allyl initiators (see above under Examples 17-40).

### Example 469

Complex **21a.** Two equiv (2.77 g, 6.45 mmol) of the sodium salt of the ligand were reacted with one equiv (1.53 g, 3.22 mmol) of [(allyl)Ni(µ-Br)]₂ (allyl = H₂CC(CO₂Me)CH₂) to give 2.94 g (87.4% yield) of a yellow powder: ¹H NMR (C₆D₆) δ 7.39 (d, 1, Ar': H), 7.29 (d, 1, Ar': H), 7.0 - 6.9 (m, 3, Ar: H), 4.00 (m, 1, *H*H'CC(CO₂Me)C'HH'), 3.57 and 2.86 (septet, 1 each, C*H*Me₂ and C'*H*Me₂), 3.25 (s, 3, O*Me*), 2.86 (s, 1, HH'CC(CO₂Me)C'*H*H'), 1.92 (m, 1, H*H'*CC(CO₂Me)C'HH'), 1.47 (s, 3, N=C*Me*), 1.34, 1.18, 0.89 and 0.79 (d, 3 each, CH*MeMe'* and C'H*MeMe'*), 1.12 (s, 1 each, HH'CC (CO₂Me)C'H*H'*).

### Example 470

Complex **21b.** Two equiv (720 mg, 1.68 mmol) of the sodium salt of the ligand were reacted with one equiv (225 mg, 0.834 mmol) of [(allyl)Ni(µ-Br)]₂ (allyl = H₂CCHCH₂) to give 599 mg (77.6% yield) of a yellow powder: ¹H NMR (CDCl₃) δ 7.39 (d, 1, Ar': H), 7.35 (d, 1, Ar': H), 7.13 - 7.00 (m, 3, Ar: H), 5.78 (m, 1, H₂CC*H*C'H₂), 3.66 and 3.07 (m, 1 each, C*H*Me₂ and C'*H*Me₂), 3.21, 2.64, 1.44 and 1.11 (d, 1 each, *HH'*CCHC'*HH'*), 1.99 (s, 3, N=C*Me*), 1.31, 1.26, 1.07 and 0.99 (d, 3 each, CH*MeMe*' and C'H*MeMe'*); ¹³C NMR (CDCl₃) δ 168.7 (N=*C*Me), 159.5, 150.0, 137.7, 137.0, 131.8, 128.7, 127.9, 125.9, 123.8, 123.3, 120.9, 117.1, 113.1 (Ar: C_{*o*}, C_{*o*}', C_{*m*}' C_{*m*}', C_{*p*}, Ar': C_{*o*}, C_{*o*}', C_{*m*}, C_{*m*}' C_{*p*}, H₂C*C*HCH₂), 59.4 and 52.8 (H₂*C*CH*C*'H₂), 28.3 and 27.8 (*C*HMe₂, *C'*HMe₂), 24.1, 23.64, 23.54 and 23.4 (CH*MeMe'* and C'H*MeMe'*), 20.3 (N=C*Me*).

### Example 471

Complex **22a**. Two equiv (809 mg, 2.14 mmol) of the sodium salt of the ligand were reacted with one equiv (508 mg, 1.07 mmol) of [(allyl)Ni(µ-Br)]₂ (allyl = H₂CC(CO₂Me)CH₂) to give 792 mg (79.6% yield) of a yellow powder: ¹H NMR (C₆D₆) δ 7.57 (d, 1, Ar': H), 7.30 (d, 1, Ar': H) , 7.0 - 6.9 (m, 3, Ar: H), 4.10 (m, 1, *H*H'CC(CO₂Me)C'HH'), 3.38 (s, 3, O*Me*), 2.69 (s, 1, HH'CC(CO₂Me)C'*H*H'), 2.02 and 2.12 (s, 3, Ar: *Me, Me'*)*,* 1.89 (m, 1, H*H'*CC(CO₂Me)C'HH'), 1.45 (s, 3, N=C*Me*), 1.35 (s, 1, HH'CC(CO₂Me)C'H*H'*).

### Example 472

Complex **23a.** Two equiv (1.56 g, 4.20 mmol) of the sodium salt of the ligand were reacted with one equiv (1.00 g, 2.10 mmol) of [(allyl)Ni(µ-Br)]₂ (allyl = H₂CC(CO₂Me)CH₂) to give 1.35 g (65.3% yield) of a yellow powder: According to the ¹H NMR spectrum, two isomers (t-Bu group positioned syn and anti to the CO₂Me group) are present in a 1:1 ratio. ¹H NMR (C₆D₆) δ 7.6 - 6.5 (m, 8, H_{aryl}) , 4.16 and 4.01 (s, 1 each, *H*H'CC(CO₂Me)C'HH' of each isomer), 3.42 and 3.42 (s, 3 each, O*Me* of each isomer), 2.82 and 2.74 (s, 1 each, HH'CC(CO₂Me)C'*H*H' of each isomer), 2.22 and 2.02 (s, 1 each, H*H'*CC (CO₂Me) C' HH' of each isomer), 1.63 and 1.56 (s, 3 each, N=C*Me* of each isomer), 1.56 and 1.38 (s, 9 each, C*Me*_{*3*} of each isomer), 1.54 and 1.36 (s, 1 each, HH'CC(CO₂Me)C'H*H'* of each isomer).

### Example 473

Complex **24a.** Two equiv (1.10 g, 2.15 mmol) of the sodium salt of the ligand were reacted with one equiv (512 mg, 1.08 mmol) of [(allyl)Ni(µ-Br)]₂ (allyl = H₂CC(CO₂Me)CH₂) to give 635 mg (49.5% yield) of a yellow powder: ¹H NMR (CDCl₃) δ 7.72 (s, 1, N=C*H*) , 7.70 (d, 1, Ar': H), 7.20 - 7.08 (m, 4, Ar: H, Ar': H), 3.90 (d, 1, *H*H'CC(CO₂Me)C'HH'), 3.80 (s, 3, O*Me*), 3.73 and 2.92 (septet, 1 each, C*H*Me₂ and C'*H*Me₂), 2.99 (s, 1, HH'CC(CO₂Me)C'*H*H'), 2.03 (m, 1, H*H'*CC(CO₂Me)C'HH'), 1.56 (s, 1, HH'CC(CO₂Me)C'H*H'*), 1.30, 1.22, 1.08 and 0.93 (d, 3 each, CH*MeMe'* and C'H*MeMe'*).

### Example 474

Complex **25a.** Two equiv (3.25 g, 6.31 mmol) of the sodium salt of the ligand were reacted with one equiv (1.50 g, 3.16 mmol) of [(allyl)Ni(µ-Br)]₂ (allyl = H₂CC(CO₂Me)CH₂) to give 3.49 g (90.6% yield) of a yellow powder: ¹H NMR spectrum is clean and consistent with the desired product.

### Example 475

Complex **26a.** Two equiv (2.01 g, 4.20 mmol) of the sodium salt of the ligand were reacted with one equiv (1.00 g, 2.10 mmol) of [(allyl)Ni(µ-Br)]₂ (allyl = H₂CC(CO₂Me)CH₂) to give 1.51 g (64.9% yield) of a golden brown powder: ¹H NMR (C₆D₆) δ 7.64 (s, 1, Ar': H), 7.25 (s, 1, Ar': H), 6.70 (, 3, Ar: H), 3.85 (s, 1, *H*H'CC(CO₂Me)C'HH'), 3.15 (s, 3, O*Me*), 2.44 (s, 1, HH'CC(CO₂Me)C*H*H'), 1.97 and 1.85 (s, 3 each, Ar: *Me*, *Me'),* 1.60 (s, 1, H*H'*CC(CO₂Me)C'HH'), 1.20 (s, 3, N=C*Me),* 1.11 (s, 1, HH'CC(CO₂Me)C'H*H'*); ¹³ C NMR (C₆D₆, selected resonances) δ 59.5, 52.7, and 51.3 (H₂*C*C (CO₂*Me*) *C'* H₂) , 19.0, 18.6, and 18.0 (N=C*Me*, Ar: *Me*, *Me'*).

### Example 476

Complex **27a.** Two equiv (951 mg, 2.13 mmol) of the sodium salt of the ligand were reacted with one equiv (505 mg, 1.06 mmol) of [(allyl)Ni(µ-Br)]₂ (allyl = H₂CC(CO₂Me)CH₂ to give 851 mg (68.6% yield) of a yellow powder: ¹H NMR spectrum in C₆D₆ is clean and consistent with the desired product. The product exists as a 1:1 ratio of isomers (*t*-Bu group positioned syn or anti to the CO₂Me group.)

### Example 477

Complex **28a.** Two equiv (983 mg, 2.14 mmol) of the sodium salt of the ligand were reacted with one equiv (509 mg, 1.07 mmol) of [(allyl)Ni(µ-Br)]₂ (allyl = H₂CC(CO₂Me)CH₂) to give 1.02 g (90.9% yield) of a green powder: ¹H NMR spectrum in C₆D₆ is broad, but consistent with the desired product. The product exists as an ~1:1 ratio of isomers (*t*-Bu group positioned syn or anti to the CO₂Me group.)

### Example 478

Complex **29a.** Two equiv (550 mg, 1.59 mmol) of the sodium salt of the ligand were reacted with one equiv (308 mg, 0.647 mmol) of [(allyl)Ni(µ-Br)]₂ (allyl = H₂CC(CO₂Me)CH₂) to give 478 mg (64.3% yield) of a dark brown powder.

### Example 479

Complex **30a.** Two equiv (478 mg, 1.27 mmol) of the sodium salt of the ligand were reacted with one equiv (303 mg, 0.637 mmol) of [(allyl)Ni(µ-Br)]₂ (allyl = H₂CC(CO₂Me)CH₂) to give 375 mg (61.4% yield) of a dark brown powder:

### Example 480

Complex **31a.** Two equiv (1.04 g, 2.10 mmol) of the sodium salt of the ligand were reacted with one equiv (500 mg, 1.05 mmol) of [(allyl)Ni(µ-Br)]₂ (allyl = H₂CC(CO₂Me)CH₂) to give 948 mg (81.1% yield) of a green-yellow powder: ¹H NMR (THF-*d*_{*8*}) δ 7.85, 7.34, 7.14, 7.12, 6.63 and 6.43 (N=C*H*, H_{aryl}, C*H*Ph₂), 3.72 (s, 3, O*Me*), 3.80, 2.78, 2.78, and 1.48 (s, 1 each, *HH'*CC(CO₂Me)C'*HH'*), 1.37 and 1.18 (CMe₃, C'Me₃) ; ¹³C NMR (THF-*d*_{*8*}) δ 166.9 (N=*C*H), 167.1, 166.9, 164.1, 142.3, 142.0, 140.9, 135.8, 130.3, 130.0, 129.4, 129.3, 129.27, 128.3, 118.3, 110.4 (C_{aryl} and H₂C*C*(*C*O₂Me)CH₂), 81.0 (*C*HPh₂), 59.1 and 45.8 (H₂*C*C(CO₂Me)*C*H₂), 52.6 (O*Me*), 35.9 and 34.3 (*C*Me₃, *C*'Me₃), 31.7 and 29.7 (C*Me*_{*3*}, C'*Me*_{*3*}).

### Example 481

Complex **32a.** Two equiv (923 mg, 2.15 mmol) of the sodium salt of the ligand were reacted with one equiv (512 mg, 1.08 mmol) of [(allyl)Ni(µ-Br)]₂ (allyl = H₂CC(CO₂Me)CH₂) to give 907 mg (81.1% yield) of a brown-orange powder.

### Example 482

Complex **33a.** Two equiv (891 mg, 2.11 mmol) of the sodium salt of the ligand were reacted with one equiv (502 mg, 1.06 mmol) of [(allyl)Ni(µ-Br)]₂ (allyl = H₂CC(CO₂Me)CH₂) to give 940 mg (89.1% yield) of a gold powder. Two major isomers are present in a 1.18:1 ratio. There is a very small amount of a third product or isomer present. The ¹H NMR assignments of the two major isomers follow: ¹H NMR (CDCl₃) δ 8.30 and 8.25 (d, 1 each, Ar' : H of each isomer), 7.48 and 7.32 (d, 1 each, Ar': H of each isomer), 6.94 and 6.78 (s, 1 each, N=C*H* of each isomer), 7.05 (m, 2, H_{aryl}) , 6.83 (m, 1, H_{aryl}), 6.80 (m, 3, H_{aryl}), 6.58 (d, 1, H_{aryl}), 6.45 (m, 1, H_{aryl}) , 3.90 and 3.73 (m, 1 each, *H*H'CC(CO₂Me)C'HH' of each isomer), 3.18 and 3.09 (s, 3 each, O*Me* of each isomer), 2.43 and 2.41 (s, 1 each, HH'CC(CO₂Me)C'*H*H' of each isomer), 2.26 and 2.08 (m, 1 each, H*H'*CC(CO₂Me)C'HH' of each isomer), 1.30 and 1.17 (s, 1 each, HH'CC(CO₂Me)C'H*H'* of each isomer), 1.18 and 1.03 (s, 9 each, C*Me*_{*3*} of each isomer).

### Example 483

Complex **34a.** Two equiv (719 mg, 1.95 mmol) of the sodium salt of the ligand were reacted with one equiv (464 mg, 0.977 mmol) of [(allyl)Ni(µ-Br)]₂ (allyl = H₂CC(CO₂Me)CH₂) to give 928 mg (96.6% yield) of a yellow powder. The ¹H NMR spectrum indicates that two isomers (Cl group positioned syn and anti to the CO₂Me group) are present in a 2.5 to 1 ratio. Major Isomer: ¹H NMR (C₆D₆) δ 8.50 (d, 1, Ar': H), 7.52 (d, 1, Ar': H) , 7.10 (d, 1, Ar: H), 6.75 (t, 1, Ar: H), 6.72 (s, 1, N=C*H*), 6.70 (d, 1, Ar: H), 4.02 (d, 1, *H*H'CC(CO₂Me)C'HH'), 3.28 (s, 3, O*Me*), 2.60 (s, 1, HH'CC(CO₂Me)C'*H*H'), 2.18 (d, 1, H*H*'CC (CO₂Me) C'HH'), 1.99 (s, 3, Ar: *Me*), 1.63 (s, 1, HH'CC(CO₂Me)C'H*H*'); Minor Isomer: ¹H NMR (C₆D₆) δ 8.50 (d, 1, Ar': H), 7.53 (d, 1, Ar': H), 7.06 (d, 1, Ar: H), 6.8 - 6.7 (m, 3, N=C*H*, Ar: H), 4.10 (d, 1, *H*H'CC(CO₂Me)C'HH'). 3.39 (s, 3, O*Me*), 2.57 (s, 1, HH'CC(CO₂Me)C'*H*H'), 2.19 (d, 1, H*H*' CC (CO₂Me) C' HH'), 1.98 (s, 3, Ar: *Me*), 1.35 (s, 1, HH' CC (CO₂Me) C' H*H'*).

### Example 484

Complex **35a.** Two equiv (765 mg, 2.11 mmol) of the sodium salt of the ligand were reacted with one equiv (500 mg, 1.05 mmol) of [(allyl)Ni(µ-Br)]₂ (allyl = H₂CC(CO₂Me)CH₂) to give 890 mg (84.7% yield) of a red powder.

### Example 485

Complex **36a.** Two equiv (1.22 g, 2.10 mmol) of the sodium salt of the ligand were reacted with one equiv (500 mg, 1.05 mmol) of [(allyl)Ni(µ-BHr)]₂ (allyl = H₂CC(CO₂Me)CH₂) to give 1.18 g (81.7% yield) of a yellow powder: ¹H NMR (CD₂Cl₂) δ 8.03 (d, 1, Ar': H), 7.70 (s, 1, N=C*H*) , 7.36 (d, 1, Ar': H), 7 .20 - 7.07 (m, 3, Ar: H), 3.88 (m, 1, *H*H' C (CO₂Me ) C' HH' ) , 3.78 (s, 3, O*Me*) , 3.71 (septet, 1, C*H*Me₂), 2.97 (s, 1, HH'CC(CO₂Me)C'*H*H'), 2.90 (septet, 1, C' *H*Me₂) , 1.96 (m, 1, H*H*'CC(CO₂Me)C'HH'), 1.57 (s, 1 HH"CC(CO₂Me)CH*H*'), 1.28, 1.20, 1.04 and 0.90 (d, 3 each, CH*MeMe*', *C'*H*MeMe'* ) ; ¹³C NMR (CD₂Cl₂) δ 166.8 (N=CH), 167.9, 164.6, 153.2, 151.6, 144.4, 141.4, 140.6, 128.4, 125.3, 125.2, 121.1, 114.2, 98.2 and 75.2 (Ar: C_{*o*}, C_{*o*}', C_{*m*}, C_{*m*}', C_{*p*}; Ar': C_{*o*}, C_{*o*}', C_{*m*}, C_{*m*}', *C*_{*p*}; H₂C*C*(*C*O₂Me) C'H₂) , 62.7, 54.5 and 50.2 (H₂*C*C (CO₂*Me*) *C'*H₂) , 30.2 and 29. 8 (*C*HMe₂, *C'*HMe₂), 26.7, 26. 5, 24.2 and 23.7 (CH*MeMe*', C'H*MeMe'*)*.*

### Example 486

Complex **37a.** Two equiv (771 mg, 2.12 mmol) of the sodium salt of the ligand were reacted with one equiv (504 mg, 1.06 mmol) of [(allyl)Ni(µ-Br)]₂ (allyl = H₂CC(CO₂Me)CH₂) to give 992 mg (93.9% yield) of a green powder: ¹H NMR (CD₂Cl₂) δ 8.25 (s, 1, N=C*H*) , 7.18 (m, 3, Ar: H), 6.02 (d, 2, Ar': H), 5.68 (d, 2, Ar': H), 3.90 (septet, 1, C*H*Me₂), 3.84, 3.78 and 3.71 (s, 3 each, Ar: O*Me* and *OMe';* CO₂*Me*) , 3.65 (s, 1, *H*H'CC(CO₂Me)C'HH'), 3.03 (septet, 1, C'*H*Me₂), 2.80 (s, 1, HH'CC(CO₂Me)C'*H*H'), 1.88 (s, 1, H*H*'CC(CO₂Me)C'HH'), 1.46 (s, 1, HH'CC(CO₂Me)C'H*H*'), 1.36, 1.28, 1.14 and 0.99 (d, 3 each, CH*MeMe'*, C'H*MeMe'*)*.*

### Example 487

Complex **38a.** Two equiv (1.04 g, 2.12 mmol) of the sodium salt of the ligand were reacted with one equiv (503 mg, 1.06 mmol) of ((allyl)Ni(µL-Br)]₂ (allyl = H₂CC(CO₂Me)CH₂) to give 1.10 g (89.3% yield) of a green-yellow powder: ¹H NMR (CD₂Cl₂) δ 8.65 (s, 1, N=C*H*), 7.84 (d, 1, Ar': H), 7.77 (d, 1, Ar': H), 7.68 (t, 1, Ar': H), 7.48 (m, 2, Ar: H), 7.44 (t, 1, Ar': H), 7.27 (t, 1, Ar': H), 7.07 (d, 1, Ar': H), 3.86 (s, 3, OMe), 3.80, 2.84, 2.05 and 1.91 (s, 1 each, *HH'* CC (CO₂Me) C' *HH'*).

### Example 488

Complex **39a.** Two equiv (614 mg, 2.10 mmol) of the sodium salt of the ligand were reacted with one equiv (500 mg, 1.05 mmol) of [(allyl)Ni(µ-Br)]₂ (allyl = H₂CC (CO₂Me) CH₂) to give 683 mg (77.6% yield) of a green-yellow powder: ¹H NMR (C₆D₆) δ 9.17 (s, 1, N=C*H*), 8.39 (d, 1, H_{aryl}), 7.62 (d, 1, H_{aryl}), 7.52 (d, 1, H_{aryl}) 7.52 (d, 1, H_{aryl}) , 7.44 (d, 1, H_{aryl}) , 7.24 (t, 1, H_{aryl}) , 7.18 ( t, 1, H_{aryl}) , 7.11 (d, 1, H_{aryl}), 6.75 (dd, 1, H_{aryl}), 4.19 (br s, 1, *H*H'CC(CO₂Me)C'HH'), 3.43 (s, 3, O*Me*), 2.67 (br s, 1, HH'CC(CO₂Me)C'*H*H'), 2.32 (br s, 1, H*H'*CC(CO₂Me)C'HH') , 2.24 (s, 3, Ar: *Me*), 1.65 (s, 1, HH' CC (CO₂Me) C'H*H*').

### Example 489

Compound **40a.** Two equiv (765 mg, 2.12 mmol) of the sodium salt of the ligand were reacted with one equiv (505 mg, 1.06 mmol) of [(allyl) Ni (µ-Br)]₂ (allyl = H₂CC(CO₂Me)CH₂) to give 925 mg (95.1% yield) of a green powder: Three isomers or products are present in a 1.35 to 1.02 to 1.00 ratio. ¹H NMR (CDCl₃, selected resonances only) δ 8.84, 8.72 and 8.20 (N=C*H* of the 3 products), 3.29 (O*Me* of the 3 products--all overlapping), 1.81, 1.45 and 1.25 (C*Me*_{*3*} of the 3 products).

### Example 490

Complex **41a.** Two equiv (867 mg, 2.22 mmol) of the sodium salt of the ligand were reacted with one equiv (527 mg, 1.11 mmol) of [(allyl)Ni(µ-Br)]₂ (allyl = H₂CC(CO₂Me)CH₂) to give 782 mg (77.1% yield) of a golden brown powder: ¹H NMR (C₆D₆) δ 8.08 (d, 2, Ph: C_{*o*}), 7.27 (m, 6, Ph: C_{*m*}, C_{*p*}; Ar: C_{*m*}, C_{*p*}), 6.01 (s, 1, PhCC*H*CMe), 4.23 (s, 1, *H*H' CC (CO₂Me) C'HH'), 4.03 (septet, 1, C*H*Me₂), 3.45 (s, 3, O*Me*), 3.33 (septet, 1, C'*H*Me₂), 3. 04 (s, 1, HH' CC (CO₂Me) C'*H*H'), 2. 18 (s, 1, H*H*'CC (CO₂Me) CHH'), 1.69 (s, 3, C*Me*NAr) , 1.38 (s, 1, H*H*'CC(CO₂Me)CH*H'*), 1.54, 1.41, 1.29 and 1.18 (d, 3 each, CH*MeMe'* and C'H*MeMe'*).

### Example 491

Complex **42a.** Two equiv (495 mg, 2.17 mmol) of the sodium salt of the ligand were reacted with one equiv (516 mg, 1.09 mmol) of [(allyl)Ni(µ-Br)]₂ (allyl = H₂CC(CO₂Me)CH₂) to give 434 mg (57.4% yield) of a yellow powder: ¹H NMR (C₆D₆) δ 7.82 (d, 1, H_{aryl}) , 7.20 (d, 1, H_{aryl}), 7.10 (t, 1, H_{aryl}), 6.47 (t, 1, H_{aryl}), 4.10 (s, 1, *H*H'CC(CO₂Me)C'HH'), 3.27 (s, 3, O*Me*) , 3.27 (s, 2, OC*H*_{*2*}, overlaps with OMe), 3.02, 2.73 and 1.11 (s, 1 each, H*H'*CC(CO₂Me)C'*HH'*), 0.81 and 0.73 (s, 3 each, C*MeMe'*).

### Example 492

Complex **43a.** Two equiv (586 mg, 0.909 mmol) of the sodium salt of the ligand were reacted with one equiv (216 mg, 0.455 mmol) of [(allyl)Ni ((µ-Br) ]₂ (allyl = H₂CC (CO₂Me) CH₂) to give 506 mg (74.1% yield) of a dark red powder: ¹H NMR (THF-*d*_{*8*}) δ 7.50 (m, 8, PPh: H_{*o*}) , 7.20 (t, 4, PPh: H_{*p*}), 7.10 (t, 8, PPh: H_{*m*}) , 6.65 (d, 4, NAr: H_{*m*}), 6.59 (d, 4, NAr: H_{*o*}), 3.52 (s, 3, O*Me*), 2.77 (s, 2, *H*H'CC(CO₂Me)C*H*H'), 2.03 (s, 6, NAr: *Me*), 2.03 or 1.81 (m, 1, PC*H*P), 1.72 (s, 2, H*H*'CC (CO₂Me) CH*H*' ) ; ¹³C NMR (THF-*d*₈, selected resonances only) δ 50.9 and 47.9 (H₂*C*C (CO₂*Me*)*C*H₂) , 19.5 (NAr: *Me*), 12.0 (t, J_{cp} = 109 Hz, PCHP) .

### Example 493

Complex **44a.** Two equiv (343 mg, 0.520 mmol) of the sodium salt of the ligand were reacted with one equiv (124 mg, 0.260 mmol) of [(allyl)Ni(µ-Br)]₂ (allyl = H₂CC(CO₂Me)CH₂) to give 128 mg (32.8% yield) of an orange powder. The ¹H NMR spectrum is consistent with the presence of one major symmetrical isomer; some of the ligand is also present along with some impurities and possibly the presence of other isomers. (The three possible isomers include the isomer with both methyl groups anti to the CO₂Me group, the isomer with both methyl groups syn to the CO₂Me group, and the isomer with one Me group anti and one Me group syn to the CO₂Me group). The nonaromatic resonances of the major symmetrical isomer follow: ¹H NMR (THF-*d*_{*8*}) δ 3.60 (s, *3,* O*Me*), 2.77 (s, 2, *H*H' CC(CO₂Me)C*H*H'), 3.47 or 2.01 (m, 1, PCHP), 1.88 (s, 6, Ar: *Me*), 1.75 (s, 2, H*H*' CC (CO₂Me) CH*H*') .

### Example 494

Complex **45a.** Two equiv (349 mg, 2.10 mmol) of the lithium salt of the ligand were reacted with one equiv (500 mg, 1.05 mmol) of [(allyl)Ni(µ-Br)]₂ (allyl = H₂CC(CO₂Me)CH₂) to give 255 mg (40.7% yield) of an brown-yellow powder. ¹H NMR (C₆D₆/THF-*d*_{*8*}) δ 6.02 (s, 1, Thiophene: H), 5.23 (s, 1, Thiophene: H), 3.78 (br s, 1, *H*H'CC(CO₂Me)C'HH'), 3.40 and 3.38 (s, 3 each, Thiophene: *Me* and CO₂*Me*), 2.41 (s, 2, H*H'* CC (CO₂Me) C' *H*H'), 2.02 (s, 1, HH' CC (CO₂Me) CH*H*').

### Example 495

Complex **46a.** Two equiv (587 mg, 2.11 mmol) of the lithium salt of the ligand were reacted with one equiv (501 mg, 1.05 mmol) of [(allyl)Ni(µ-Br)]₂ (allyl = H₂CC(CO₂Me)CH₂) to give 765 mg (84.5% yield) of an orange powder. ¹H NMR spectrum in C₆D₆ is complex. Peaks consistent with two different isomers of the product are present.

### Example 496

Complex **47a.** Two equiv (607 mg, 2.24 mmol) of the sodium salt of the ligand were reacted with one equiv (303 mg, 1.12 mmol) of [(allyl)Ni(µ-Br)]₂ (allyl = H₂CCHCH₂) to give 482 mg (61.8% yield) of a red powder.

### Example 497

Complex **48a.** Two equiv (149 mg, 1.27 mmol) of the sodium salt of the ligand were reacted with one equiv (302 mg, 0.635 mmol) of [(allyl)Ni(µ-Br)]₂ (allyl = H₂CC(CO₂Me)CH₂) to give 146 mg (45.7% yield) of a red powder.

### Example 498

Complex **49a.** Two equiv (700 mg, 2.17 mmol) of the sodium salt of the ligand were reacted with one equiv (515 mg, 1.08 mmol) of [(allyl)Ni(µ-Br)]₂ (allyl = H₂CC(CO₂Me)CH₂) to give 779 mg (685.2% yield) of an orange powder. ¹H NMR spectrum in THF-*d*_{*8*} is complex.

### Examples 499-503

The following complexes of Examples through were synthesized by mixing the protonated form of the hydroxy-imine ligand with a base (e.g., pyridine, lutidine, acetonitrile, etc.) in an Et₂O solution and cooling this solution to -35 °C. The cold Et₂O solution was then added to a cold flask containing (tmeda)NiMe₂. [For the preparation of (tmeda)NiMe₂ please see: Kaschube, W.; Porschke, K. R.; Wilke, G. *J. Organomet.* Chem. **1988,** *355*, 525 - 532.] The reaction mixture was stirred for ∼ 4 h. The solution was then filtered though a frit with dry Celite®. The solvent was removed and the product was dried in vacuo.

### Example 499

Complex **50.** One equiv of [2-(OH)-3,5-Cl₂-C₆H₂-C(Me)=NAr [Ar = 2,6-(*i*-Pr)₂-C₆H₃] (356 mg, 0.976 mmol) was reacted with (tmeda)NiMe₂ (200 mg, 0.976 mmol) and pyridine (772 mg, 9.76 mmol) to yield an orange-red powder: ¹H NMR (C₆D₆) δ 8.66 (d, 2, Py: H_{*o*}) , 7.50 (d, 1, Ar': H), 7.31 (d, 1, Ar': H), 7.09 (m, 3, Ar: H), 6.63 (t, 1, Py: H_{*p*}), 6.29 (t, 1, Py: H_{*m*}), 3.98 (septet, 2, C*H*Me₂) , 1.68 (d, 6, CH*Me*Me'), 1.51 (s, 3, N=C*Me*), 1.04 (d, 6, CHMe*Me*'), -0.92 (s, 3, Ni*Me*).

### Example 500

Complex **51.** One equiv of [2-(OH)-3,5-Cl₂-C₆H₂-C(Me)=NAr [Ar = 2,6-(i-Pr)₂-C₆H₃] (88.8 mg, 0.244 mmol) was reacted with (tmeda)NiMe₂ (50 mg, 0.244 mmol) and lutidine (26.2 mg, 0.244 mmol) to yield an orange powder: ¹H NMR (C₆D₆) δ 7.35 (d, 1, Ar': H), 7.28 (d, 1, Ar': H), 7.01 (s, 3, Ar: H), 6.64 (t, 1, Lutidine: H_{*p*}), 6.28 (d, 2, Lutidine: H_{*m*}), 3.91 (septet, 2, C*H*Me₂), 3.72 (s, 6, Lutidine: *Me*), 1.52 (d, 6, CH*Me*Me'), 1.46 (s, 3, N=C*Me*), 0.98 (d, 6, CHMe*Me*'), -1.42 (s, 3, Ni*Me*).

### Example 501

Complex 52. One equiv of [2-(OH)-3,5-Cl₂-C₆H₂-C(Me)=NAr [Ar = 2,6-(i-Pr)₂-C₆H₃] (370 mg, 1.02 mmol) was reacted with (tmeda)NiMe₂ (209 mg, 1.02 mmol) and acetonitrile (10 mL) to yield a yellow-orange powder: ¹H NMR (CD₂Cl₂) δ 7.23 (d, 1, Ar' : H), 7.10 (t, 1, Ar: H_{*p*}), 7.04 (d, 2, Ar: Hₘ), 6.95 (d, 1, Ar': H), 4.34 (septet, 2, C*H*Me₂), 1.89 (s, 3, N=C*Me*), 1.70 (s, 3, NC=*Me*), 1.33 (d, 6, CH*Me*Me'), 1.15 (d, 6, CHMe*Me*'), 0.80 (s, 3, Ni*Me*).

### Example 502

Complex **53.** One equiv of [2-(OH)-3,5-Cl₂-C₆H₂-C(Me)=NAr [Ar = 2,6-(*i*-Pr)₂-C₆H₃] (88.8 mg, 0.244 mmol) was reacted with (tmeda)NiMe₂ (50 mg, 0.244 mmol) and *p*-tolunitrile (28.6 mg, 0.244 mmol) to yield a brown powder: ¹H NMR (CD₂Cl₂) δ 7.74 (d, 2, Nitrile: H), 7.23 (d, 1, Ar' : H), 7.21 (d, 2, Nitrile: H), 7.10 (t, 1, Ar: H_{*p*}), 7.04 (d, 1, Ar: H_{*m*}), 6.95 (d, 1, Ar': H), 4.34 (septet, 2, C*H*Me₂), 2.33 (s, 3, Nitrile: *Me*), 1.70 (s, 3, N=C*Me*), 1.33 (d, 6, CH*Me*Me'), 1.15 (d, 6, CHMe*Me*'), 0.80 (s, 3, Ni*Me*).

### Example 503

Complex **54.** One equiv of [2-(OH)-3,5-Br₂-C₆H₂-C(Me)=NAr [Ar = 2,6-(*i*-Pr)₂-C₆H₃] (111 mg, 0.244 mmol) was reacted with (tmeda)NiMe₂ (50 mg, 0.244 mmol) and pyridine (200 mg) to yield a yellow-orange powder: ¹H NMR (C₆D₆) δ 8.77 (d, 2, Py: H_{*o*}), 7.60 (t, 1, Py: H_{*p*}), 7.52 (d, 1, Ar': H), 7.44 (d, 1, Ar': H), 7.13 (t, 2, Py: H_{*m*}) , 7.10 (s, 3, Ar: H), 3.85 (septet, 2, C*H*Me₂), 1.82 (s, 3, N=C*Me*), 1.51 (d, 6, CH*Me*Me'), 1.07 (d, 6, CHMe*Me'*) , -1.42 (s, 3, Ni*Me*).

### Examples 504-509

### General Procedure for Ethylene(28-35 kPa)/α-Olefin Copolymerizations of Table 19

In the drybox, a glass Schlenk flask was loaded with the nickel compound, Lewis acid, solvent, comonomer, and a stir bar. The flask was then capped with a rubber septum and the stopcock was closed prior to removing the flask from the drybox. The flask was then attached to the ethylene line where it was evacuated and backfilled with ethylene. The reaction mixture was stirred under ethylene for the stated reaction time, the ethylene pressure was then released, and the polymer was precipitated by adding the reaction mixture to a solution of MeOH (~100 mL) and concentrated HCl (~1-3 mL). The solid polymer was then collected on a frit and rinsed with MeOH. For amorphous polymers, the MeOH was decanted off of the polymer. Often, the amorphous polymer was dissolved in hexane and reprecipitated in methanol. The polymer was transferred to a pre-weighed vial and dried under vacuum overnight. The polymer yield and characterization were then obtained.

For Example 505 the following quantitative ¹³C NMR (TCB, 120-140°C) was obtained: Branching per 1000 CH₂' S; total methyls (98.4), methyl (54.5), ethyl (13.1), propyl (3.2), butyl (14.4), amyl (4.9), hexyl and greater and end of chains (11.1), amyl and greater and end of chains (13.7), butyl and greater and end of chains (27.6)

For Example 506 the following quantitative ¹³C NMR (TCB, 120-140°C) was obtained: Branching per 1000 CH₂'s; total methyls (115.4), methyl (61.5), ethyl (12.8), propyl (3.8), butyl (21.3), amyl (4.0), hexyl and greater and end of chains (14.4), amyl and greater and end of chains (16.3), butyl and greater and end of chains (37.2)

**Table 19**

| Ethylene/α-Olefin Copolymerizations at 28-35 kPa Ethylene | | | | | | |
|---|---|---|---|---|---|---|
| Ex. | Cmpd | Lewis Acid (equiv) | Time (h) | Toluene (mL) | Comonomer (mL) | Polymer (g) |
| 504 | **3a** | BPh₃/20 | 32 | 30 | 1 -Hexene (10) | 0.633 |
| Description: Viscous clear oil. ¹H NMR (C₆D₆, rt): 198.2 Total Me/1000 CH₂ | | | | | | |
| 505 | **6a** | B(C₆F₅)₃/20 | 24.2 | 30 | 1 -Hexene (5) | 7.31 |
| Description: Tough, rubbery, amorphous light tan solid. ¹H NMR (C₆D₆, rt): 116.1 | | | | | | |
| Total Me/1000 CH₂ | | | | | | |
| 506 | **6a** | B(C₆F₅)₃/20 | 24.2 | 25 | 1 -Hexene (10) | 6.26 |
| Description: Rubbery, slightly sticky amorphous light tan solid. ¹H NMR (C₆D₆, rt): | | | | | | |
| 129.9 Total Me/ 1000 CH₂ | | | | | | |
| 507 | **6a** | B(C₆F₅)₃/20 | 24.2 | 20 | 1 -Hexene (15) | 4.18 |
| Description: Sticky, very viscous oil--almost a solid. ¹H NMR (C₆D₆, rt): 167.9 Total | | | | | | |
| Me/ 1000 CH₂ | | | | | | |
| 508 | **6a** | B(C₆F₅)₃/20 | 33.3 | 30 | 1 -Octene (5) | 5.65 |
| Description: Tough, amorphous rubbery solid. ¹H NMR (C₆D₆, rt): 112.0 Total | | | | | | |
| Me/1000 CH₂ | | | | | | |
| 509 | **9a** | B(C₆F₅)₃/20 | 27.3 | 30 | 1 -Octene (5) | 7.53 |
| Description: Sticky, amorphous light tan solid. ¹H NMR (C₆D₆, rt): 178.7 Me/1000 | | | | | | |
| CH₂ | | | | | | |

### Examples 510-512

### General Procedure for Homopolymerizations of 1-Hexene, 1-Octene, and Cyclopentene by Cmpd 6a (Table 20)

In the drybox, the nickel compound, Lewis acid, solvent, monomer and stir bar were placed together in a round bottom flask. The reaction mixture was stirred for the stated amount of time. The flask was removed from the drybox and water and concentrated hydrochloric acid were added. The product was extracted with toluene and/or hexane and the solution was filtered through a frit containing a layer of neutral alumina on top of a layer of silica gel. The solvent was then evaporated and the product was dried in vacuo.

**Table 20**

| Ethylene/α-Olefin Copolymerizations at 28-35 kPa Ethylene | | | | | | |
|---|---|---|---|---|---|---|
| Ex. | Cmpd | Lewis Acid (equiv) | Time (weeks) | Toluene (mL) | Comonomer (mL) | Polymer (g) |
| 510 | **6a** | B(C₆F₅)₃/20 | ~2 | 5 | 1-Hexene (10) | 0.511 |
| Description: Viscous oil. ¹H NMR (C₆D₆, rt): 152.2 Total Me per 1000 Carbon | | | | | | |
| Atoms; DP ∼ 17.4; Mₙ ~ 1,460 | | | | | | |
| 511 | **6a** | B(C₆F₅)₃/20 | ∼2 | 5 | 1-Octene (10) | 1.83 |
| Description: Free-flowing, slightly viscous oil. ¹H NMR (C₆D₆, rt): 122.8 Total Me | | | | | | |
| per 1000 Carbon Atoms; DP ∼ 10.5; Mₙ ~ 1,180 | | | | | | |
| 512 | **6a** | B(C₆F₅)₃/20 | ∼2 | 5 | Cyclopentene (10) | 1.57 |
| Description: Partial viscous oil/partial solid. ¹H NMR (C₆D₆) indicates | | | | | | |
| polycyclopentene formation with olefinic end groups present. | | | | | | |

## Claims

1. A process for the polymerization of an olefin selected from one or more of R⁶⁷ CH=CH₂, cyclopentene, a styrene, a norbornene or H₂C=CH (CH₂)ₛCO₂R⁷⁷, comprising, contacting, at a temperature of about -100°C to about +200°C, R⁶⁷CH=CH₂, cyclopentene, a styrene, a norbornene, or H₂C=CH (CH₂)ₛCO₂R⁷⁷, optionally a Lewis acid, and a compound of the formula: 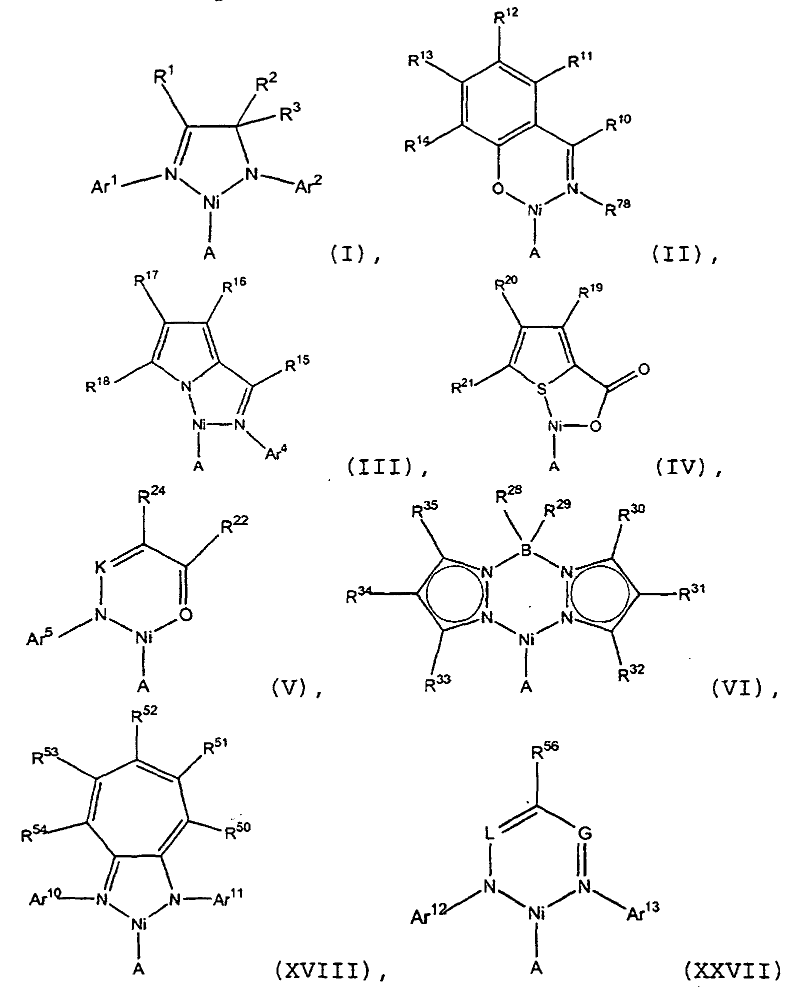 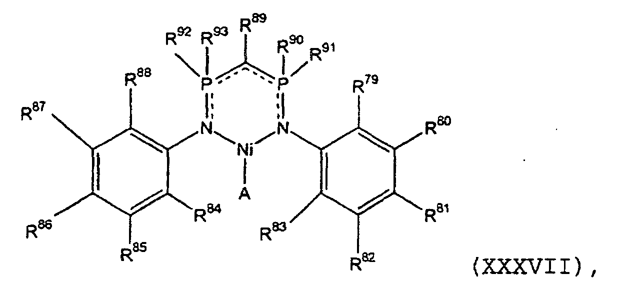 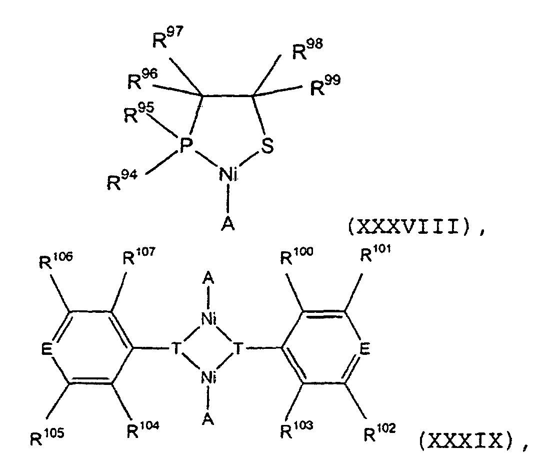 or 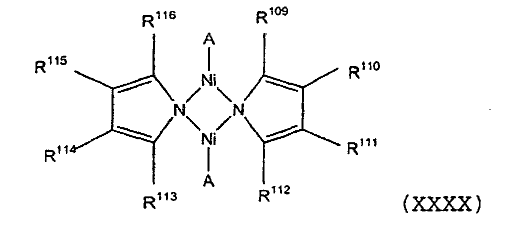 wherein:
Ar¹, Ar², Ar⁴, Ar⁵, Ar¹⁰, Ar¹¹, Ar¹² and Ar¹³ are each independently aryl or substituted aryl;
R¹ and R² are each independently hydrogen, hydrocarbyl, substituted hydrocarbyl, or R¹ and R² taken together form a ring, and R³ is hydrogen, hydrocarbyl or substituted hydrocarbyl or R¹, R² and R³ taken together form a ring;
A is a π-allyl or π-benzyl group;
R¹⁰ and R¹⁵ are each independently hydrogen, hydrocarbyl or substituted hydrocarbyl;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵ R⁵⁰, R⁵¹, R⁵², R⁵³ and R⁵⁴ are each independently hydrogen, hydrocarbyl, substituted hydrocarbyl, an inert functional group, and provided that any two of these groups vicinal to one another taken together may form a ring;
K is N or CR²⁷;
R²² is hydrocarbyl, substituted hydrocarbyl, -SR¹¹⁷, -OR¹¹⁷, or -NR¹¹⁸ ₂, R²⁴ is hydrogen, a functional group, hydrocarbyl or substituted hydrocarbyl, and R²⁷ is hydrocarbyl or substituted hydrocarbyl, and provided that R²² and R²⁴ or R²⁴ and R²⁷ taken together may form a ring;
R¹¹⁷ is hydrocarbyl or substituted hydrocarbyl;
each R¹¹⁸ is independently hydrogen, hydrocarbyl or substituted hydrocarbyl;
G and L are both N or G is CR⁵⁷ and L is CR⁵⁵_{;}
R⁵⁵, R⁵⁶ and R⁵⁷ are each independently hydrogen, hydrocarbyl or substituted hydrocarbyl, or any two of R⁵⁵, R⁵⁶ and R⁵⁷ taken together form a ring;
R⁶⁷ is hydrogen, alkyl or substituted alkyl;
R⁷⁷ is hydrocarbyl or substituted hydrocarbyl;
R⁷⁸ is hydrocarbyl or substituted hydrocarbyl;
R⁷⁹, R⁸⁰, R⁸¹, R⁸², R⁸³, R⁸⁴, R⁸⁵, R⁸⁶, R⁸⁷, R⁸⁸ and R⁸⁹ are each independently hydrogen, hydrocarbyl, substituted hydrocarbyl, or a functional group;
R⁹⁰, R⁹¹, R⁹² and R⁹³ are each independently hydrocarbyl or substituted hydrocarbyl;
R⁹⁴ and R⁹⁵ are each independently hydrocarbyl or substituted hydrocarbyl;
R⁹⁶, R⁹⁷, R⁹⁸, and R⁹⁹ are each independently hydrogen, hydrocarbyl, substituted hydrocarbyl or a functional group;
both of T are S (sulfur) or NH (amino);
each E is N (nitrogen) or CR¹⁰⁸ wherein R¹⁰⁸ is hydrogen, hydrocarbyl, substituted hydrocarbyl or a functional group;
R¹⁰⁰, R¹⁰¹, R¹⁰² , R¹⁰³, R¹⁰⁴, R¹⁰⁵, R¹⁰⁶, and R¹⁰⁷ are each independently hydrogen, hydrocarbyl, substituted hydrocarbyl, or a functional group;
R¹⁰⁹, R¹¹⁰, R¹¹¹, R¹¹², R¹¹³, R¹¹⁴ , R¹¹⁵ and R¹¹⁶ are each independently hydrogen, hydrocarbyl, substituted hydrocarbyl or a functional group;
s is an integer of 1 or more; and
R²⁸ and R²⁹ are each independently hydrogen, hydrocarbyl or substituted hydrocarbyl;
and provided that when H₂C=CH(CH₂)ₛCO₂R⁷⁷ is present, R⁶⁷ CH=CH₂ is also present.

2. A process for the polymerization of an olefin selected from one or more of R⁶⁷CH=CH₂, cyclopentene a styrene, a norbornene or H₂C=CH(CH₂)ₛCO₂R⁷⁷, comprising, contacting, at a temperature of about -100°C to about +200°C, R⁶⁷CH=CH₂, cyclopentene, a styrene, a norbornene, or H₂C=CH(CH₂)ₛCO₂R⁷⁷, optionally a Lewis acid, and a compound of the formula: 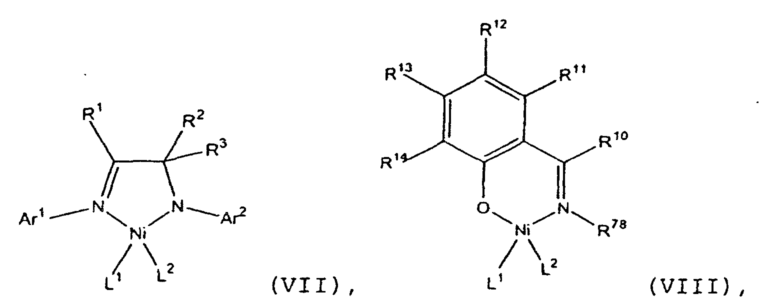 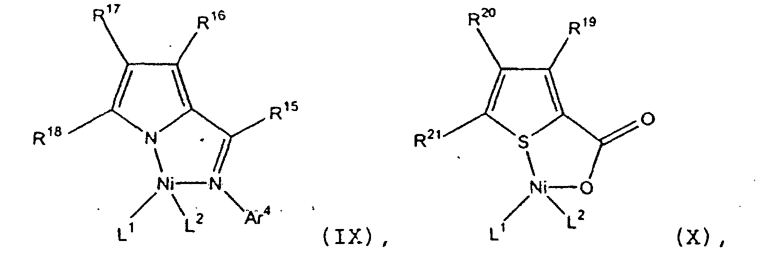 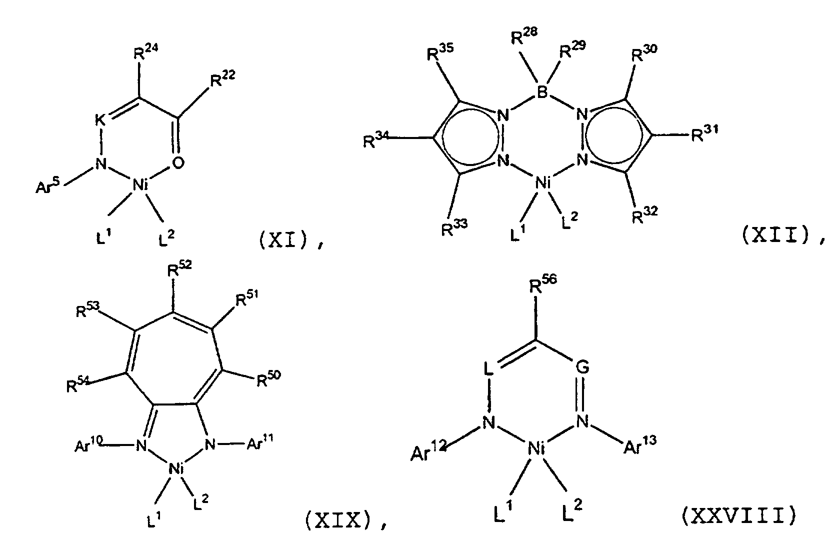 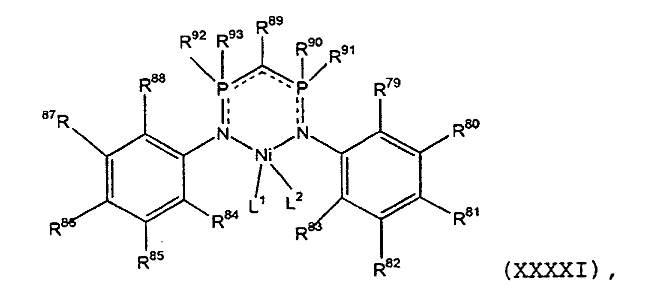 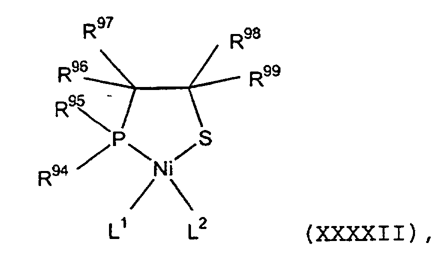 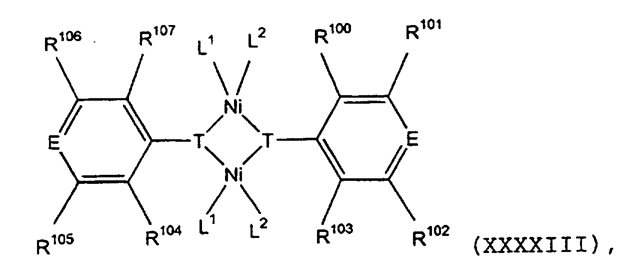 or 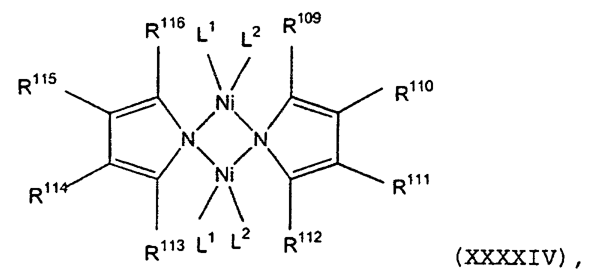 wherein:
L¹ is a neutral monodentate ligand which may be displaced by said olefin, and L² is a monoanionic monodentate ligand, or L¹ and L² taken together are a monoanionic bidentate ligand, provided that said monoanionic monodentate ligand or said monoanionic bidentate ligand may add to said olefin;
Ar¹, Ar², Ar⁴, Ar⁵, Ar¹⁰, Ar¹¹, Ar¹² and Ar¹³ are each independently aryl or substituted aryl;
R¹ and R² are each independently hydrogen, hydrocarbyl, substituted hydrocarbyl, or R¹ and R² taken together form a ring, and R³ is hydrogen, hydrocarbyl or substituted hydrocarbyl or R¹, R² and R³ taken together form a ring;
R¹⁰ and R¹⁵ are each independently hydrogen, hydrocarbyl or substituted hydrocarbyl;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ and R⁵⁴ are each independently hydrogen, hydrocarbyl, substituted hydrocarbyl, an inert functional group, and provided that any two of these groups vicinal to one another taken together may form a ring;
K is N or CR²⁷_{;}
R²² is hydrocarbyl, substituted hydrocarbyl, - SR¹¹⁷ , -OR¹¹⁷, or -NR¹¹⁸₂, R²⁴ is hydrogen, a functional group, hydrocarbyl or substituted hydrocarbyl, and R²⁷ is hydrocarbyl or substituted hydrocarbyl, and provided that R²² and R²⁴ or R²⁴ and R²⁷ taken together may form a ring;
R¹¹⁷ is hydrocarbyl or substituted hydrocarbyl;
each R¹¹⁸ is independently hydrogen, hydrocarbyl or substituted hydrocarbyl;
G and L are both N or G is CR⁵⁷ and L is CR⁵⁵;
R⁵⁵ , R⁵⁶ and R⁵⁷ are each independently hydrogen, hydrocarbyl or substituted hydrocarbyl, or any two of R⁵⁵, R⁵⁶ and R⁵⁷ taken together form a ring;
R⁶⁷ is hydrogen, alkyl or substituted alkyl;
R⁷⁷ is hydrocarbyl or substituted hydrocarbyl;
R⁷⁸ is hydrocarbyl or substituted hydrocarbyl; R⁷⁹ , R⁸⁰ , R⁸¹ , R⁸², R⁸³, R⁸⁴, R⁸⁵, R⁸⁶ , R⁸⁷, R⁸⁸ and R⁸⁹ are each independently hydrogen, hydrocarbyl, substituted hydrocarbyl, or a functional group;
R⁹⁰, R⁹¹, R⁹² and R⁹³ are each independently hydrocarbyl or substituted hydrocarbyl;
R⁹⁴ and R⁹⁵ are each independently hydrocarbyl or substituted hydrocarbyl;
R⁹⁶, R⁹⁷, R⁹⁸, and R⁹⁹ are each independently hydrogen, hydrocarbyl, substituted hydrocarbyl or a functional group;
both of T are S (sulfur) or NH (amino);
each E is N (nitrogen) or CR¹⁰⁸ wherein R¹⁰⁸ is hydrogen, hydrocarbyl, substituted hydrocarbyl or a functional group;
R¹⁰⁰, R¹⁰¹, R¹⁰², R¹⁰³, R¹⁰⁴, R¹⁰⁵, R¹⁰⁶, and R¹⁰⁷ are each independently hydrogen, hydrocarbyl, substituted hydrocarbyl, or a functional group;
R¹⁰⁹, R¹¹⁰, R¹¹¹, R¹¹², R¹¹³, R¹¹⁴, R¹¹⁵ and R¹¹⁶ are each independently hydrogen, hydrocarbyl, substituted hydrocarbyl or a functional group;
s is an integer of 1 or more; and
R²⁸ and R²⁹ are each independently hydrogen, hydrocarbyl or substituted hydrocarbyl;
and provided that when H₂C=CH(CH₂)ₛCO₂R⁷⁷ is present, R⁶⁷ CH=CH₂ is also present.

3. The process as recited in claim 1 or 2 wherein said temperature is about 0°C to about 150°C.

4. The process as recited in claim 1 or 2 wherein said temperature is about 25°C to about 100°C.

5. The process as recited in claim 1 or 2 wherein said Lewis acid is present.

6. The process as recited in claim 1 or 2 wherein said Lewis acid is not present.

7. The process as recited in claim 2 wherein said compound is (VII).

8. The process as recited in claim 2 wherein said compound is (VIII).

9. The process as recited in claim 8 wherein:
R¹⁰ is hydrogen or methyl;
R⁷⁸ is Ar³, which is aryl or substituted aryl; and
R¹¹, R¹², R¹³ and R¹⁴ are each independently chloro, bromo, iodo, alkyl, alkoxy, hydrogen or nitro, or R¹¹ and R¹² taken together form a 6-membered carbocyclic ring and R¹³ and R¹⁴ are hydrogen.

10. The process as recited in claim 2 wherein said compound is (IX).

11. The process as recited in claim 2 wherein said compound is (X).

12. The process as recited in claim 2 wherein said compound is (XI).

13. The process as recited in claim 2 wherein said compound is (XII).

14. The process as recited in claim 2 wherein said compound is (XIX).

15. The process as recited in claim 2 wherein said compound is (XXVIII).

16. The process as described in claim 2 wherein said compound is (XXXXI).

17. The process as described in claim 2 wherein said compound is (XXXXII).

18. The process as recited in claim 2 wherein said compound is (XXXXIII).

19. The process as recited in claim 2 wherein said compound is (XXXXIV).

20. The process as recited in claim 2 wherein L¹ is a nitrile, pyridine or substituted pyridine, and L² is methyl.

21. The process as recited in claim 1 or claim 2 wherein said olefin or olefins are: ethylene; a styrene; a norbornene; an α-olefin; cyclopentene; H₂C=CH (CH₂) ₛCO₂R⁷⁷ and ethylene; ethylene and an α-olefin; a styrene and a norbornene; and 2 or more norbornenes.

22. A compound of the formula: 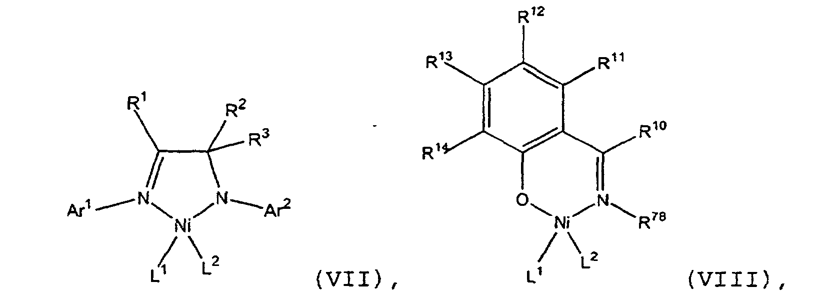 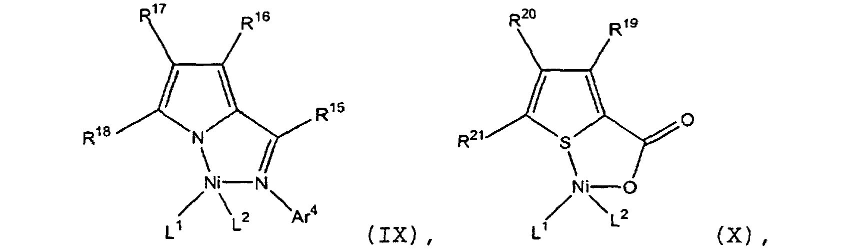 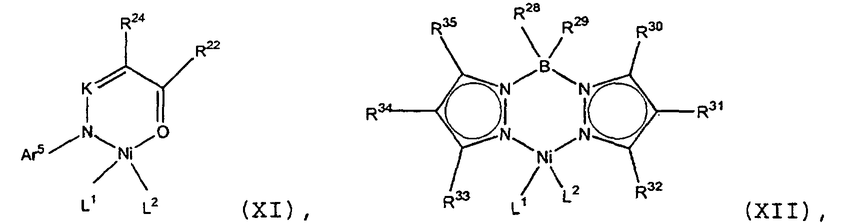 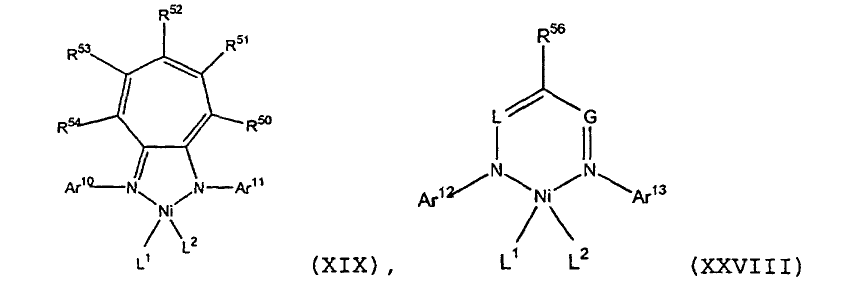 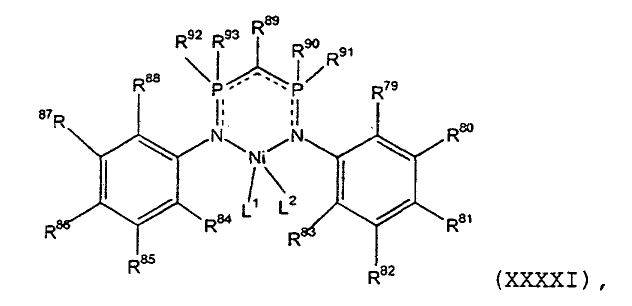 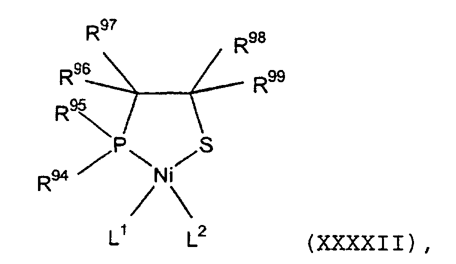 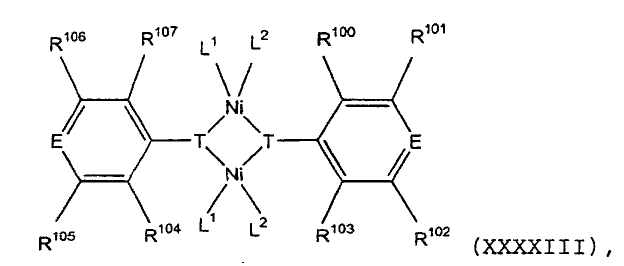 or 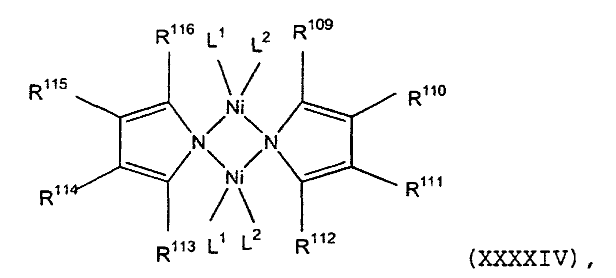 wherein:
L¹ is a neutral monodentate ligand which may be displaced by said olefin, and L² is a monoanionic monodentate ligand, or L¹ and L² taken together are a monoanionic bidentate ligand, provided that said monoanionic monodentate ligand or said monoanionic bidentate ligand may add to said olefin;
Ar¹, Ar², Ar⁴, Ar⁵, Ar¹⁰, Ar¹¹, Ar¹² and Ar¹³ are each independently aryl or substituted aryl;
R¹ and R² are each independently hydrogen, hydrocarbyl, substituted hydrocarbyl, or R¹ and R² taken together form a ring, and R³ is hydrogen, hydrocarbyl or substituted hydrocarbyl or R¹, R² and R³ taken together form a ring;
R¹⁰ and R¹⁵ are each independently hydrogen, hydrocarbyl or substituted hydrocarbyl;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ and R⁵⁴ are each independently hydrogen, hydrocarbyl, substituted hydrocarbyl, an inert functional group, and provided that any two of these groups vicinal to one another taken together may form a ring;
K is N or CR²⁷;
R²² is hydrocarbyl, substituted hydrocarbyl, -SR¹¹⁷, -OR¹¹⁷, or -NR¹¹⁸₂, R²⁴ is hydrogen, a functional group, hydrocarbyl or substituted hydrocarbyl, and R²⁷ is hydrocarbyl or substituted hydrocarbyl, and provided that R²² and R²⁴ or R²⁴ and R²⁷ taken together may form a ring;
R¹¹⁷ is hydrocarbyl or substituted hydrocarbyl;
each R¹¹⁸ is independently hydrogen, hydrocarbyl or substituted hydrocarbyl;
G and L are both N or G is CR⁵⁷ and L is CR⁵⁵;
R⁵⁵, R⁵⁶ and R⁵⁷ are each independently hydrogen, hydrocarbyl or substituted hydrocarbyl, or any two of R⁵⁵, R⁵⁶ and R⁵⁷ taken together form a ring;
R⁷⁸ is hydrocarbyl or substituted hydrocarbyl;
R⁷⁹, R⁸⁰, R⁸¹, R⁸², R⁸³, R⁸⁴, R⁸⁵, R⁸⁶, R⁸⁷, R⁸⁸ and R⁸⁹ are each independently hydrogen, hydrocarbyl, substituted hydrocarbyl, or a functional group;
R⁹⁰, R⁹¹, R⁹² and R⁹³ are each independently hydrocarbyl or substituted hydrocarbyl;
R⁹⁴ and R⁹⁵ are each independently hydrocarbyl or substituted hydrocarbyl;
R⁹⁶, R⁹⁷, R⁹⁸, and R⁹⁹ are each independently hydrogen, hydrocarbyl, substituted hydrocarbyl or a functional group;
both of T are S (sulfur) or NH (amino);
each E is N (nitrogen) or CR¹⁰⁸ wherein R¹⁰⁸ is hydrogen, hydrocarbyl, substituted hydrocarbyl or a functional group;
R¹⁰⁰, R¹⁰¹, R¹⁰², R¹⁰³, R¹⁰⁴, R¹⁰⁵, R¹⁰⁶, and R¹⁰⁷ are each independently hydrogen, hydrocarbyl, substituted hydrocarbyl, or a functional group;
R¹⁰⁹, R¹¹⁰, R¹¹¹, R¹¹², R¹¹³, R¹¹⁴, R¹¹⁵ and R¹¹⁶ are each independently hydrogen, hydrocarbyl, substituted hydrocarbyl or a functional group; and
R²⁸ and R²⁹ are each independently hydrogen, hydrocarbyl or substituted hydrocarbyl.

23. The compound as recited in claim 22 which is (VII).

24. The compound as recited in claim 22 which is (VIII).

25. The compound as recited in claim 24 wherein:
R¹⁰ is hydrogen or methyl;
R⁷⁸ is Ar³, which is aryl or substituted aryl; and
R¹¹, R¹², R¹³ and R¹⁴ are each independently chloro, bromo, iodo, alkyl, alkoxy, hydrogen or nitro, or R¹¹ and R¹² taken together form a 6-membered carbocyclic ring and R¹³ and R¹⁴ are hydrogen.

26. The compound as recited in claim 22 which is (IX).

27. The compound as recited in claim 22 which is (X).

28. The compound as recited in claim 22 which is (XI).

29. The compound as recited in claim 22 which is (XII).

30. The compound as recited in claim 22 which is (XIX).

31. The compound as recited in claim 22 which is (XXVIII).

32. The compound as described in claim 22 which is (XXXXI).

33. The compound as described in claim 22 which is (XXXXII).

34. The compound as recited in claim 22 which is (XXXXIII).

35. The compound as recited in claim 22 which is (XXXXIV).

36. The compound as recited in claim 22 wherein L¹ is a nitrile, pyridine, or a substituted pyridine, and L² is methyl.

37. The compound as recited in claim 22 wherein L¹ and L² taken together are not π-allyl or π-benzyl.

38. A compound of the formula 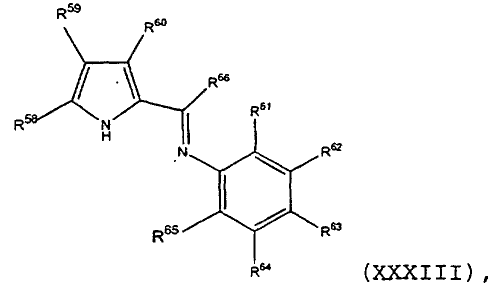 wherein:
R⁵⁸, R⁵⁹, R⁶⁰, R⁶², R⁶³ and R⁶⁴ are each independently hydrogen, hydrocarbyl, substituted hydrocarbyl, or a functional group, and provided that any two of these groups vicinal to one another taken together may form a ring, or if vicinal to R⁶¹ or R⁶⁵ form a ring with them;
R⁶⁶ is hydrogen, hydrocarbyl or substituted hydrocarbyl; and
R⁶¹ and R⁶⁵ are each independently hydrocarbyl containing 2 or more carbon atoms, or substituted hydrocarbyl containing 2 or more carbon atoms, and provided that R⁶¹ and R⁶⁵ may form a ring with any group vicinal to it.

39. A compound of the formula 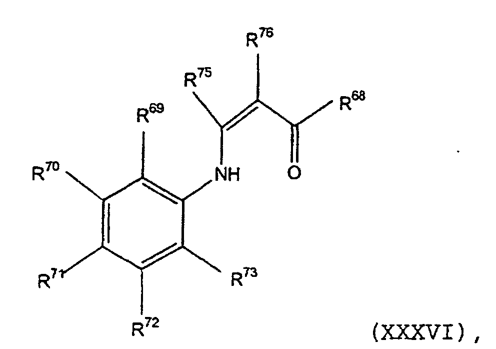 wherein:
R⁶⁸ is hydrocarbyl, substituted hydrocarbyl, - SR¹¹⁷, -OR¹¹⁷, or -NR¹¹⁸₂, R⁷⁶ is hydrogen, a functional group, hydrocarbyl or substituted hydrocarbyl, and R⁷⁵ is hydrocarbyl or substituted hydrocarbyl, and provided that R⁶⁸ and R⁷⁶ or R⁷⁵ and R⁷⁶ taken together may form a ring;
R¹¹⁷ is hydrocarbyl or substituted hydrocarbyl;
each R¹¹⁸ is independently hydrogen, hydrocarbyl or substituted hydrocarbyl;
R⁷⁰, R⁷¹ and R⁷² are each independently hydrogen, hydrocarbyl, substituted hydrocarbyl or a functional group;
R⁶⁹ and R⁷³ are hydrocarbyl containing 3 or more carbon atoms, substituted hydrocarbyl containing 3 or more carbon atoms or a functional group;
and provided that any two of R⁷⁰, R⁷¹, R⁷², R⁶⁹ and R⁷³ vicinal to one another together may form a ring.

## Patentansprüche

1. Verfahren zur Polymerisation eines Olefins, das aus einem oder mehreren von R⁶⁷CH=CH₂, Cylcopenten, einem Styren, einem Norbornen oder H₂C=CH(CH₂)ₛCO₂R⁷⁷ ausgewählt ist, umfassend Kontaktieren, bei einer Temperatur von ca. -100°C bis +200°C, R⁶⁷CH=CH₂, Cyclopenten, ein Styren, ein Norbomen oder H₂C=CH(CH₂)ₛCO₂R⁷⁷, optional eine Lewis-Säure und eine Verbindung der Formel: 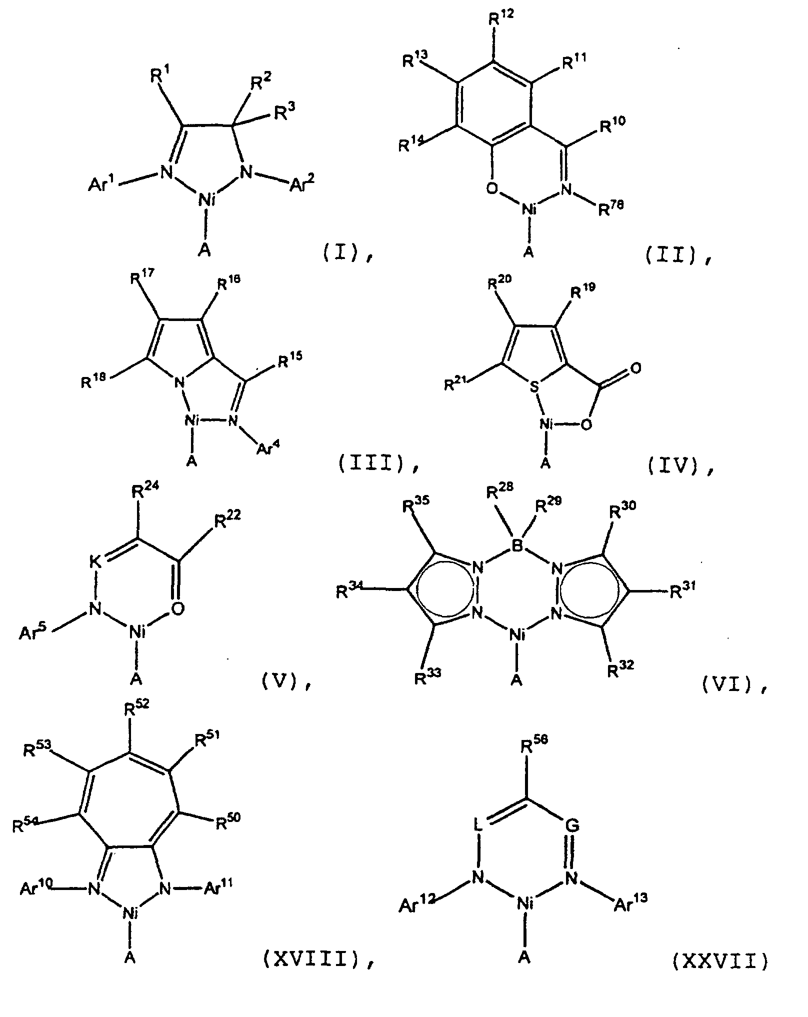 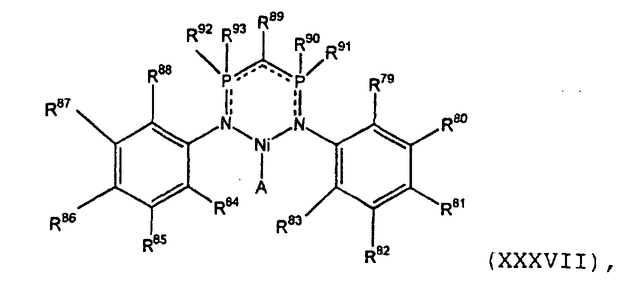 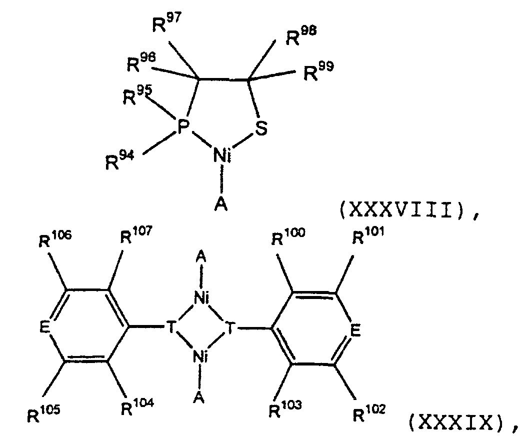 oder 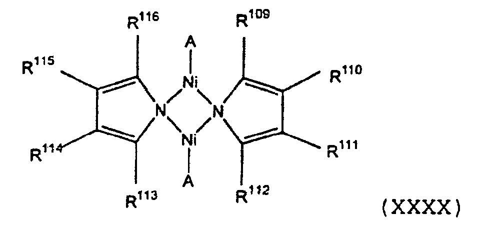 worin:
Ar¹, Ar², Ar⁴, Ar⁵, Ar¹⁰, Ar¹¹, Ar¹² und Ar¹³ jeweils unabhängig Aryl oder substituiertes Aryl darstellen;
R¹ und R² jeweils unabhängig Wasserstoff, Hydrocarbyl, substituiertes Hydrocarbyl darstellen oder R¹ und R² zusammen genommen einen Ring bilden und R³ Wasserstoff, Hydrocarbyl oder substituiertes Hydrocarbyl darstellt oder R¹, R² und R³ zusammen genommen einen Ring bilden;
A eine π-Allyl- oder π-Benzylgruppe darstellt;
R¹⁰ und R¹⁵ jeweils unabhängig Wasserstoff, Hydrocarbyl oder substituiertes Hydrocarbyl darstellen;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ und R⁵⁴ jeweils unabhängig Wasserstoff, Hydrocarbyl, substituiertes Hydrocarbyl, eine inerte funktionelle Gruppe darstellen und vorausgesetzt, dass jedwede zwei dieser Gruppen vicinal zueinander zusammen genommen einen Ring bilden können;
K für N oder CR²⁷ steht;
R²² Hydrocarbyl, substituiertes Hydrocarbyl, -SR¹¹⁷, -OR¹¹⁷ oder -NR¹¹⁸₂ darstellt, R²⁴ Wasserstoff, eine funktionelle Gruppe, Hydrocarbyl oder substituiertes Hydrocarbyl darstellt und R²⁷ Hydrocarbyl oder substituiertes Hydrocarbyl darstellt und vorausgesetzt, dass R²² und R²⁴ oder R²⁴ und R²⁷ zusammen genommen einen Ring bilden können;
R¹¹⁷ Hydrocarbyl oder substituiertes Hydrocarbyl darstellt;
jedes R¹¹⁸ unabhängig Wasserstoff, Hydrocarbyl oder substituiertes Hydrocarbyl darstellt;
G und L beide N darstellen oder G für CR⁵⁷ steht und L für CR⁵⁵ steht;
R⁵⁵, R⁵⁶ und R⁵⁷ jeweils unabhängig Wasserstoff, Hydrocarbyl oder substituiertes Hydrocarbyl darstellen oder jedwede zwei von R⁵⁵, R⁵⁶ und R⁵⁷ zusammen genommen einen Ring bilden;
R⁶⁷ Wasserstoff, Alkyl oder substituiertes Alkyl darstellt;
R⁷⁷ Hydrocarbyl oder substituiertes Hydrocarbyl darstellt;
R⁷⁸ Hydrocarbyl oder substituiertes Hydrocarbyl darstellt;
R⁷⁹, R⁸⁰, R⁸¹, R⁸², R⁸³, R⁸⁴, R⁸⁵, R⁸⁶, R⁸⁷, R⁸⁸ und R⁸⁹ jeweils unabhängig Wasserstoff, Hydrocarbyl, substituiertes Hydrocarbyl oder eine funktionelle Gruppe darstellen;
R⁹⁰, R⁹¹, R⁹² und R⁹³ jeweils unabhängig Hydrocarbyl oder substituiertes Hydrocarbyl darstellen;
R⁹⁴ und R⁹⁵ jeweils unabhängig Hydrocarbyl oder substituiertes Hydrocarbyl darstellen;
R⁹⁶, R⁹⁷, R⁹⁸ und R⁹⁹ jeweils unabhängig Wasserstoff, Hydrocarbyl, substituiertes Hydrocarbyl oder eine funktionelle Gruppe darstellen;
beide T für S (Schwefel) oder NH (Amino) stehen;
jedes E für N (Stickstoff) oder CR¹⁰⁸ steht, worin R¹⁰⁸ Wasserstoff, Hydrocarbyl, substituiertes Hydrocarbyl oder eine funktionelle Gruppe darstellt;
R¹⁰⁰, R¹⁰¹, R¹⁰², R¹⁰³, R¹⁰⁴, R¹⁰⁵, R¹⁰⁶ und R¹⁰⁷ jeweils unabhängig Wasserstoff, Hydrocarbyl, substituiertes Hydrocarbyl oder eine funktionelle Gruppe darstellen;
R¹⁰⁹, R¹¹⁰, R¹¹¹, R¹¹², R¹¹³, R¹¹⁴, R¹¹⁵ und R¹¹⁶ jeweils unabhängig Wasserstoff, Hydrocarbyl, substituiertes Hydrocarbyl oder eine funktionelle Gruppen darstellen;
s eine ganze Zahl von 1 oder mehr darstellt; und
R²⁸ und R²⁹ jeweils unabhängig Wasserstoff, Hydrocarbyl oder substituiertes Hydrocarbyl darstellen;
und vorausgesetzt, dass wenn H₂C=CH(CH₂)ₛCO₂R⁷⁷ anwesend ist, R⁶⁷CH=CH₂ auch anwesend ist.

2. Verfahren zur Polymerisation eines Olefins, das aus einem oder mehreren R⁶⁷CH=CH₂, Cyclopenten, einem Styren, einem Norbornen oder H₂C=CH(CH₂)ₛCO₂R⁷⁷ ausgewählt ist, umfassend Kontaktieren bei einer Temperatur von ca. -100°C bis +200°C, R⁶⁷CH=CH₂, Cyclopenten, ein Styren, ein Norbornen oder H₂C=CH(CH₂)ₛCO₂R⁷⁷, optional eine Lewis-Säure und eine Verbindung der Formel: 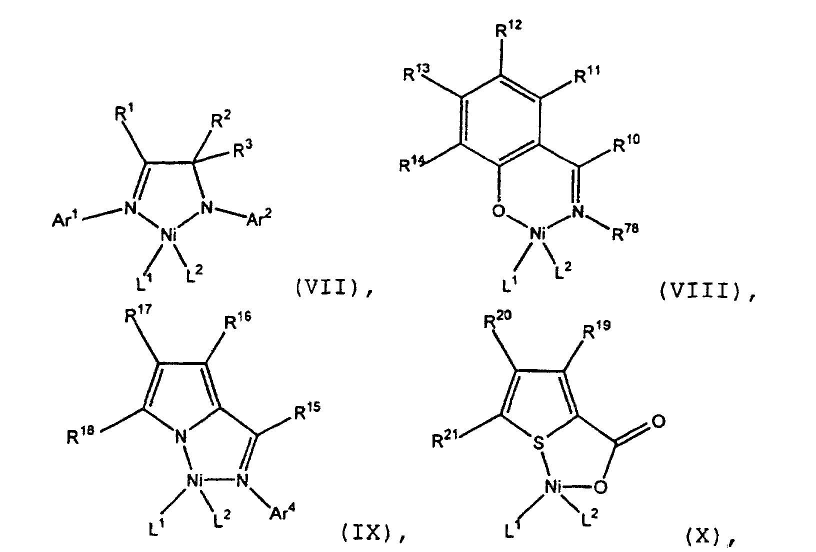 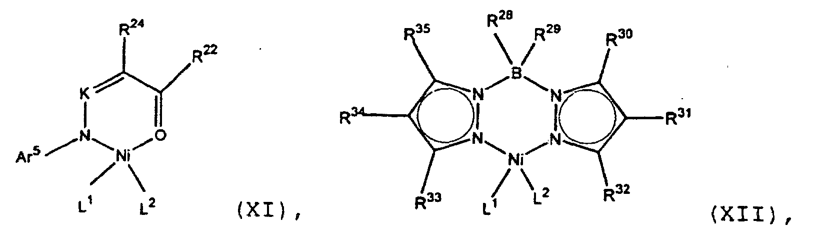 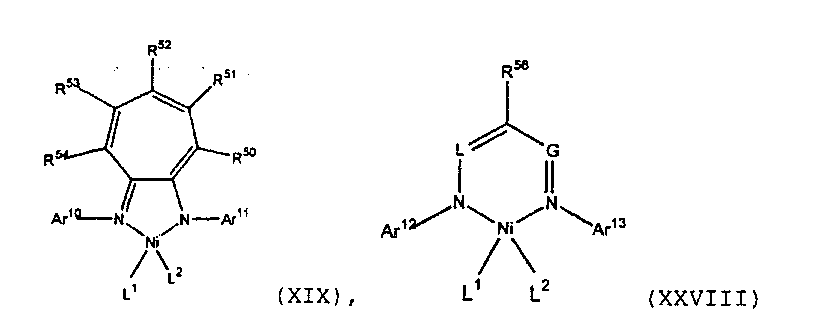 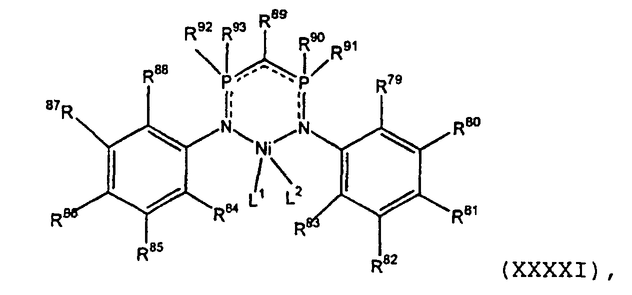 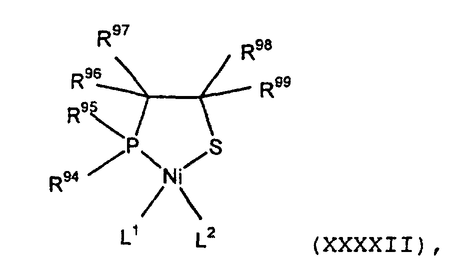 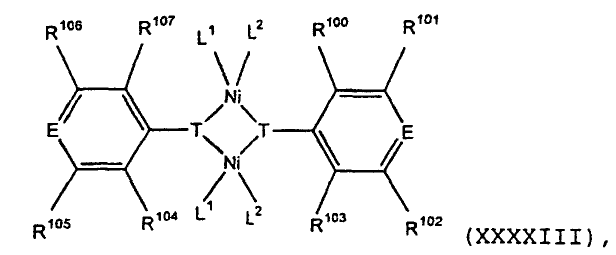 oder 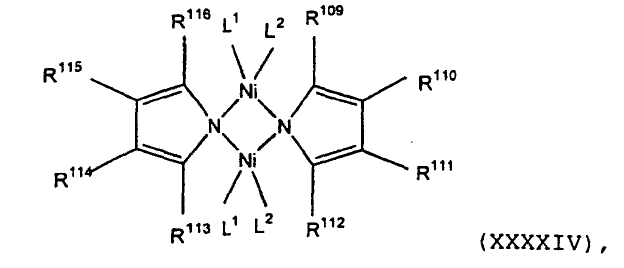 worin:
L¹ einen neutralen Monodentat-Liganden darstellt, der durch das Olefin verdrängt werden kann und L² einen monoanionischen Monodentat-Liganden darstellt oder L¹ und L² zusammen genommen einen monoanionischen Bidentat-Liganden darstellen, vorausgesetzt, dass der monanionische Monodentat-Ligand oder der monoanionische Bidentat-Ligand an das Olefin addieren kann;
Ar¹, Ar², Ar⁴, Ar⁵, Ar¹⁰, Ar¹¹, Ar¹² und Ar¹³ jeweils unabhängig Aryl oder substituiertes Aryl darstellen;
R¹ und R² jeweils unabhängig Wasserstoff, Hydrocarbyl, substituiertes Hydrocarbyl darstellen oder R¹ und R² zusammen genommen einen Ring bilden und R³ Wasserstoff, Hydrocarbyl oder substituiertes Hydrocarbyl darstellt oder R¹, R² und R³ zusammen genommen einen Ring bilden;
R¹⁰ und R¹⁵ jeweils unabhängig Wasserstoff, Hydrocarbyl oder substituiertes Hydrocarbyl darstellen;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ und R⁵⁴ jeweils unabhängig Wasserstoff, Hydrocarbyl, substituiertes Hydrocarbyl, eine inerte funktionelle Gruppe darstellen und vorausgesetzt, dass jedwede zwei dieser Gruppen vicinal zueinander zusammen genommen einen Ring bilden können;
K für N oder CR²⁷ steht;
R²² Hydrocarbyl, substituiertes Hydrocarbyl, -SR¹¹⁷, -OR¹¹⁷ oder -NR¹¹⁸₂ darstellt, R²⁴ Wasserstoff, eine funktionelle Gruppe, Hydrocarbyl oder substituiertes Hydrocarbyl darstellt und R²⁷ Hydrocarbyl oder substituiertes Hydrocarbyl darstellt und vorausgesetzt, dass R²² und R²⁴ oder R²⁴ und R²⁷ zusammen genommen einen Ring bilden können;
R¹¹⁷ Hydrocarbyl oder substituiertes Hydrocarbyl darstellt;
jedes R¹¹⁸ unabhängig Wasserstoff, Hydrocarbyl oder substituiertes Hydrocarbyl darstellt;
G und L beide N darstellen oder G für CR⁵⁷ steht und L für CR⁵⁵ steht;
R⁵⁵, R⁵⁶ und R⁵⁷ jeweils unabhängig Wasserstoff, Hydrocarbyl oder substituiertes Hydrocarbyl darstellen oder jedwede zwei von R⁵⁵, R⁵⁶ und R⁵⁷ zusammen genommen einen Ring bilden;
R⁶⁷ Wasserstoff, Alkyl oder substituiertes Alkyl darstellt;
R⁷⁷ Hydrocarbyl oder substituiertes Hydrocarbyl darstellt;
R⁷⁸ Hydrocarbyl oder substituiertes Hydrocarbyl darstellt;
R⁷⁹, R⁸⁰, R⁸¹, R⁸², R⁸³, R⁸⁴, R⁸⁵, R⁸⁶, R⁸⁷, R⁸⁸ und R⁸⁹ jeweils unabhängig Wasserstoff, Hydrocarbyl, substituiertes Hydrocarbyl oder eine funktionelle Gruppe darstellen;
R⁹⁰, R⁹¹, R⁹² und R⁹³ jeweils unabhängig Hydrocarbyl oder substituiertes Hydrocarbyl darstellen;
R⁹⁴ und R⁹⁵ jeweils unabhängig Hydrocarbyl oder substituiertes Hydrocarbyl darstellen;
R⁹⁶, R⁹⁷, R⁹⁸ und R⁹⁹ jeweils unabhängig Wasserstoff, Hydrocarbyl, substituiertes Hydrocarbyl oder eine funktionelle Gruppe darstellen;
beide T für S (Schwefel) oder NH (Amino) stehen;
jedes E für N (Stickstoff) oder CR¹⁰⁸ steht, worin R¹⁰⁸ Wasserstoff, Hydrocarbyl, substituiertes Hydrocarbyl oder eine funktionelle Gruppe darstellt;
R¹⁰⁰, R¹⁰¹, R¹⁰², R¹⁰³, R¹⁰⁴, R¹⁰⁵, R¹⁰⁶ und R¹⁰⁷ jeweils unabhängig Wasserstoff, Hydrocarbyl, substituiertes Hydrocarbyl oder eine funktionelle Gruppe darstellen;
R¹⁰⁹, R¹¹⁰, R¹¹¹, R¹¹², R¹¹³, R¹¹⁴, R¹¹⁵ und R¹¹⁶ jeweils unabhängig Wasserstoff, Hydrocarbyl, substituiertes Hydrocarbyl oder eine funktionelle Gruppen darstellen;
s eine ganze Zahl von 1 oder mehr darstellt; und
R²⁸ und R²⁹ jeweils unabhängig Wasserstoff, Hydrocarbyl oder substituiertes Hydrocarbyl darstellen;
und vorausgesetzt, dass wenn H₂C=CH(CH₂)ₛCO₂R⁷⁷ anwesend ist, R⁶⁷CH=CH₂ auch anwesend ist.

3. Verfahren nach Anspruch 1 oder 2, worin die Temperatur ca. 0°C bis ca. 150°C beträgt.

4. Verfahren nach Anspruch 1 oder 2, worin die Temperatur ca. 25°C bis ca. 100°C beträgt.

5. Verfahren nach Anspruch 1 oder 2, worin die Lewis-Säure anwesend ist.

6. Verfahren nach Anspruch 1 oder 2, worin die Lewis-Säure nicht anwesend ist.

7. Verfahren nach Anspruch 2, worin die Verbindung (VII) darstellt.

8. Verfahren nach Anspruch 2, worin die Verbindung (VIII) darstellt.

9. Verbindung nach Anspruch 8, worin:
R¹⁰ Wasserstoff oder Methyl darstellt;
R⁷⁸ für Ar³ steht, das Aryl oder substituiertes Aryl darstellt; und
R¹¹, R¹², R¹³ und R¹⁴ jeweils unabhängig Chloro, Bromo, Iodo, Alkyl, Alkoxy, Wasserstoff oder Nitro darstellen oder R¹¹ und R¹² zusammen genommen einen 6-gliedrigen carbocyclischen Ring bilden und R¹³ und R¹⁴ Wasserstoff darstellen.

10. Verfahren nach Anspruch 2, worin die Verbindung (IX) darstellt.

11. Verfahren nach Anspruch 2, worin die Verbindung (X) darstellt.

12. Verfahren nach Anspruch 2, worin die Verbindung (XI) darstellt.

13. Verfahren nach Anspruch 2, worin die Verbindung (XII) darstellt.

14. Verfahren nach Anspruch 2, worin die Verbindung (XIX) darstellt.

15. Verfahren nach Anspruch 2, worin die Verbindung (XXVIII) darstellt.

16. Verfahren nach Anspruch 2, worin die Verbindung (XXXXI) darstellt.

17. Verfahren nach Anspruch 2, worin die Verbindung (XXXXII) darstellt.

18. Verfahren nach Anspruch 2, worin die Verbindung (XXXXIII) darstellt.

19. Verfahren nach Anspruch 2, worin die Verbindung (XXXXIV) darstellt.

20. Verfahren nach Anspruch 2, worin L¹ ein Nitril, Pyridin oder substituiertes Pyridin darstellt und L² Methyl darstellt.

21. Verfahren nach Anspruch 1 oder 2, worin das Olefin oder die Olefine Folgendes darstellen: Ethylen; ein Styren; ein Norbornen; ein α-Olefin; Cyclopenten; H₂C=CH(CH₂)ₛCO₂R ⁷⁷ und Ethylen; Ethylen und ein α-Olefin; ein Styren und ein Norbornen und 2 oder mehr Norbomene.

22. Verbindung der Formel: 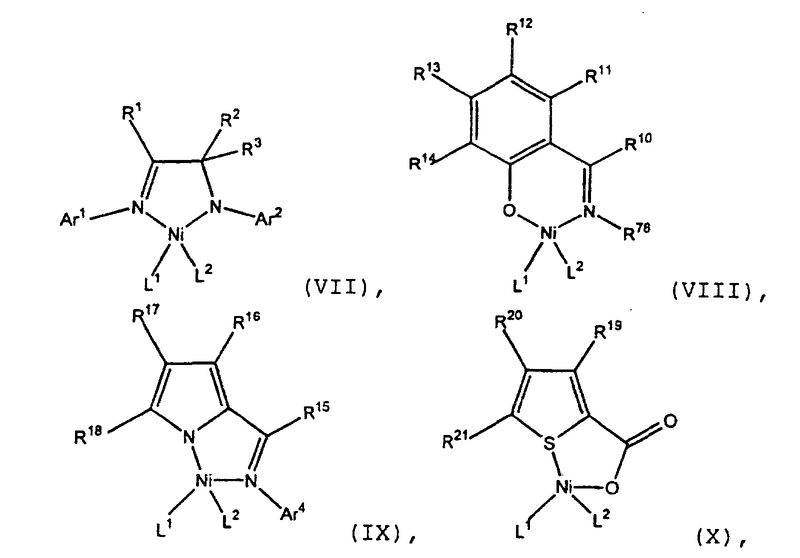 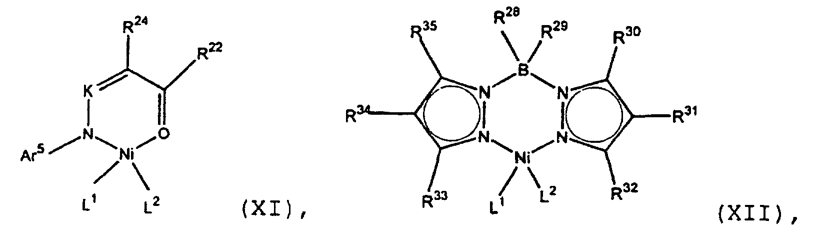 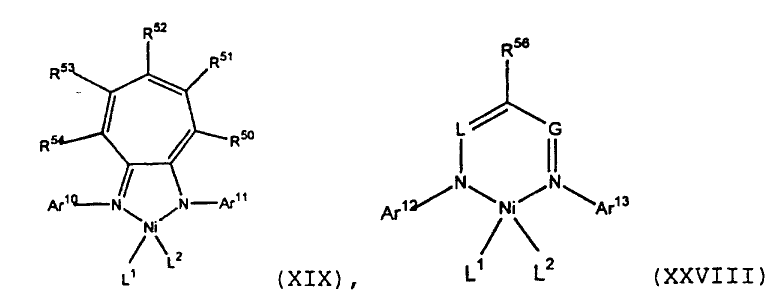 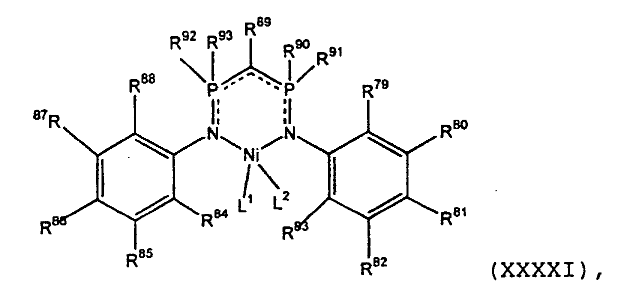 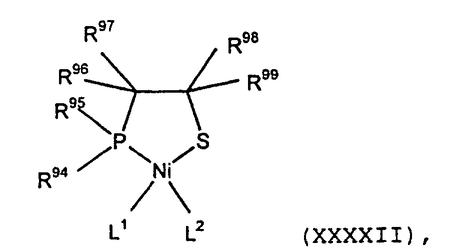 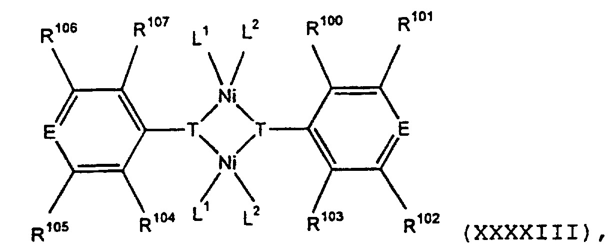 oder 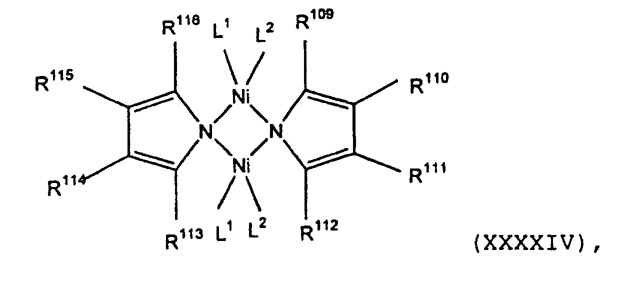 worin:
L¹ einen neutralen Monodentat-Liganden darstellt, der durch das Olefin verdrängt werden kann und L² einen monoanionischen Monodentat-Liganden darstellt oder L' und L² zusammen genommen einen monoanionischen Bidentat-Liganden darstellen, vorausgesetzt, dass der monanionische Monodentat-Ligand oder der monoanionische Bidentat-Ligand an das Olefin addieren kann;
Ar¹, Ar², Ar⁴, Ar⁵, Ar¹⁰, Ar¹¹, Ar¹² und Ar¹³ jeweils unabhängig Aryl oder substituiertes Aryl darstellen;
R¹ und R² jeweils unabhängig Wasserstoff, Hydrocarbyl, substituiertes Hydrocarbyl darstellen oder R¹ und R² zusammen genommen einen Ring bilden und R³ Wasserstoff, Hydrocarbyl oder substituiertes Hydrocarbyl darstellt oder R¹, R² und R³ zusammen genommen einen Ring bilden;
R¹⁰ und R¹⁵ jeweils unabhängig Wasserstoff, Hydrocarbyl oder substituiertes Hydrocarbyl darstellen;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵¹, R⁵², R⁵³ und R⁵⁴ jeweils unabhängig Wasserstoff, Hydrocarbyl, substituiertes Hydrocarbyl, eine inerte funktionelle Gruppe darstellen und vorausgesetzt, dass jedwede zwei dieser Gruppen vicinal zueinander zusammen genommen einen Ring bilden können;
K für N oder CR²⁷ steht;
R²² Hydrocarbyl, substituiertes Hydrocarbyl, -SR¹¹⁷, -OR¹¹⁷ oder -NR¹¹⁸₂ darstellt, R²⁴ Wasserstoff, eine funktionelle Gruppe, Hydrocarbyl oder substituiertes Hydrocarbyl darstellt und R²⁷ Hydrocarbyl oder substituiertes Hydrocarbyl darstellt und vorausgesetzt, dass R²² und R²⁴ oder R²⁴ und R²⁷ zusammen genommen einen Ring bilden können;
R¹¹⁷ Hydrocarbyl oder substituiertes Hydrocarbyl darstellt;
jedes R¹¹⁸ unabhängig Wasserstoff, Hydrocarbyl oder substituiertes Hydrocarbyl darstellt;
G und L beide N darstellen oder G für CR⁵⁷ steht und L für CR⁵⁵ steht;
R⁵⁵, R⁵⁶ und R⁵⁷ jeweils unabhängig Wasserstoff, Hydrocarbyl oder substituiertes Hydrocarbyl darstellen oder jedwede zwei von R⁵⁵, R⁵⁶ und R⁵⁷ zusammen genommen einen Ring bilden;
R⁷⁸ Hydrocarbyl oder substituiertes Hydrocarbyl darstellt;
R⁷⁹, R⁸⁰, R⁸¹, R⁸², R⁸³, R⁸⁴, R⁸⁵, R⁸⁶, R⁸⁷, R⁸⁸ und R⁸⁹ jeweils unabhängig Wasserstoff, Hydrocarbyl, substituiertes Hydrocarbyl oder eine funktionelle Gruppe darstellen;
R⁹⁰, R⁹¹, R⁹² und R⁹³ jeweils unabhängig Hydrocarbyl oder substituiertes Hydrocarbyl darstellen;
R⁹⁴ und R⁹⁵ jeweils unabhängig Hydrocarbyl oder substituiertes Hydrocarbyl darstellen;
R⁹⁶, R⁹⁷, R⁹⁸ und R⁹⁹ jeweils unabhängig Wasserstoff, Hydrocarbyl, substituiertes Hydrocarbyl oder eine funktionelle Gruppe darstellen;
beide T für S (Schwefel) oder NH (Amino) stehen;
jedes E für N (Stickstoff) oder CR¹⁰⁸ steht, worin R¹⁰⁸ Wasserstoff, Hydrocarbyl, substituiertes Hydrocarbyl oder eine funktionelle Gruppe darstellt;
R¹⁰⁰, R¹⁰¹, R¹⁰² , R¹⁰³, R¹⁰⁴, R¹⁰⁵, R¹⁰⁶ und R¹⁰⁷ jeweils unabhängig Wasserstoff, Hydrocarbyl, substituiertes Hydrocarbyl oder eine funktionelle Gruppe darstellen;
R¹⁰⁹, R¹¹⁰, R¹¹¹, R¹¹², R¹¹³, R¹¹⁴, R¹¹⁵ und R¹¹⁶ jeweils unabhängig Wasserstoff, Hydrocarbyl, substituiertes Hydrocarbyl oder eine funktionelle Gruppe darstellen; und
R²⁸ und R²⁹ jeweils unabhängig Wasserstoff, Hydrocarbyl oder substituiertes Hydrocarbyl darstellen.

23. Verbindung nach Anspruch 22, die (VII) darstellt.

24. Verbindung nach Anspruch 22, die (VIII) darstellt.

25. Verbindung nach Anspruch 24, worin:
R¹⁰ Wasserstoff oder Methyl darstellt;
R⁷⁸ für Ar³ steht, das Aryl oder substituiertes Aryl darstellt;
und
R¹¹, R¹², R¹³ und R¹⁴ jeweils unabhängig Chloro, Bromo, Iodo, Alkyl, Alkoxy, Wasserstoff oder Nitro darstellen oder R¹¹ und R¹² zusammen genommen einen 6-gliedrigen carbocyclischen Ring bilden und R¹³ und R¹⁴ Wasserstoff darstellen.

26. Verbindung nach Anspruch 22, die (IX) darstellt.

27. Verbindung nach Anspruch 22, die (X) darstellt.

28. Verbindung nach Anspruch 22, die (XI) darstellt.

29. Verbindung nach Anspruch 22, die (XII) darstellt.

30. Verbindung nach Anspruch 22, die (XIX) darstellt.

31. Verbindung nach Anspruch 22, die (XXVIII) darstellt.

32. Verbindung nach Anspruch 22, die (XXXXI) darstellt.

33. Verbindung nach Anspruch 22, die (XXXXII) darstellt.

34. Verbindung nach Anspruch 22, die (XXXXIII) darstellt.

35. Verbindung nach Anspruch 22, die (XXXXIV) darstellt.

36. Verbindung nach Anspruch 22, worin L¹ ein Nitril, Pyridin oder ein substituiertes Pyridin darstellt und L² Methyl darstellt.

37. Verbindung nach Anspruch 22, worin L¹ und L² zusammen genommen nicht π-Allyl oder π-Benzyl darstellen.

38. Verbindung der Formel 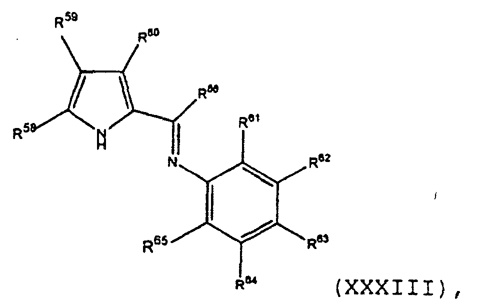 worin:
R⁵⁸, R⁵⁹, R⁶⁰, R⁶² , R⁶³ und R⁶⁴ jeweils unabhängig Wasserstoff, Hydrocarbyl, substituiertes Hydrocarbyl oder eine funktionelle Gruppe darstellen und vorausgesetzt, dass jedwede zwei dieser Gruppen vicinal zueinander zusammen genommen einen Ring bilden können oder wenn sie sich vicinal zu R⁶¹ oder R⁶⁵ befinden, einen Ring mit ihnen bilden;
R⁶⁶ Wasserstoff, Hydrocarbyl oder substituiertes Hydrocarbyl darstellen; und
R⁶¹ und R⁶⁵ jeweils unabhängig Hydrocarbyl, enthaltend 2 oder mehr Kohlenstoffatome oder substituiertes Hydrocarbyl, enthaltend 2 oder mehr Kohlenstoffatome darstellen und vorausgesetzt, dass R⁶¹ und R⁶⁵ einen Ring mit jedweder Gruppe vicinal zu ihm bilden können.

39. Verbindung der Formel 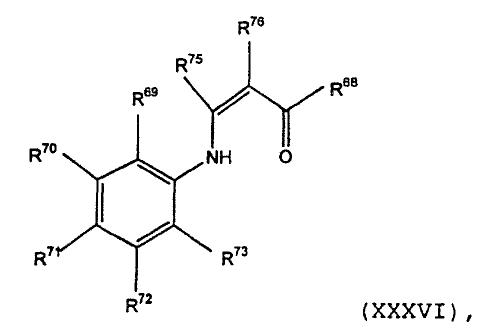 worin:
R⁶⁸ Hydrocarbyl, substituiertes Hydrocarbyl, -SR¹¹⁷, -OR¹¹⁷ oder -NR¹¹⁸₂ darstellt, R⁷⁶ Wasserstoff, eine funktionelle Gruppe, Hydrocarbyl oder substituiertes Hydrocarbyl darstellt und R⁷⁵ Hydrocarbyl oder substituiertes Hydrocarbyl darstellt und vorausgesetzt, dass R⁶⁸ und R⁷⁶ oder R⁷⁵ und R⁷⁶ zusammen genommen einen Ring bilden können;
R¹¹⁷ Hydrocarbyl oder substituiertes Hydrocarbyl darstellt;
jedes R¹¹⁸ unabhängig Wasserstoff, Hydrocarbyl oder substituiertes Hydrocarbyl darstellt;
R⁷⁰, R⁷¹ und R⁷² jeweils unabhängig Wasserstoff, Hydrocarbyl, substituiertes Hydrocarbyl oder eine funktionelle Gruppe darstellen;
R⁶⁹ und R⁷³ Hydrocarbyl, enthaltend 3 oder mehr Kohlenstoffatome, substituiertes Hydrocarbyl, enthaltend 3 oder mehr Kohlenstoffatome oder eine funktionelle Gruppe darstellen;
und vorausgesetzt, dass jedwede zwei von R⁷⁰, R⁷¹, R⁷², R⁶⁹ und R⁷³ vicinal zueinander zusammen einen Ring bilden können.

## Revendications

1. Procédé pour la polymérisation d'une oléfine choisie parmi un ou plusieurs d'entre du R⁶⁷CH=CH₂, du cyclopentène, un styrène, un norbornène ou du H₂C=CH(CH₂)ₛCO₂R⁷⁷, comprenant, mettre en contact, à une température d'environ - 100°C à environ +200°C, du R⁶⁷CH=CH₂, du cyclopentène, un styrène, un norbornène ou du H₂C=CH(CH₂)ₛCO₂R⁷⁷, de manière optionnelle un acide de Lewis, et un composé de formule: 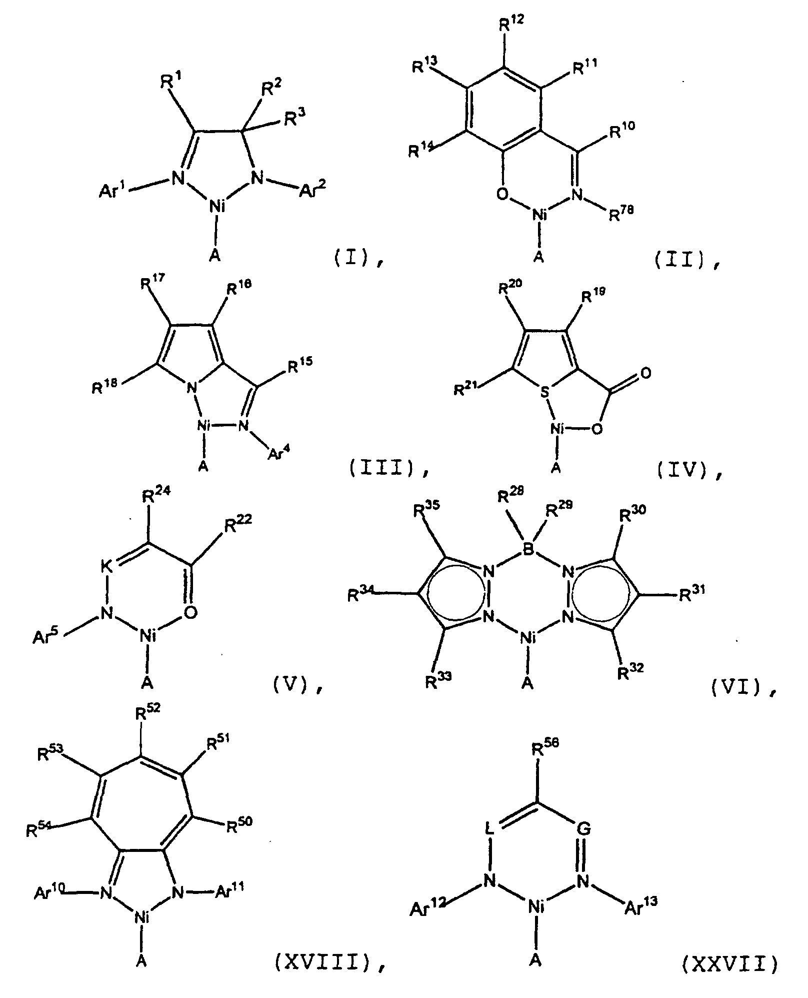 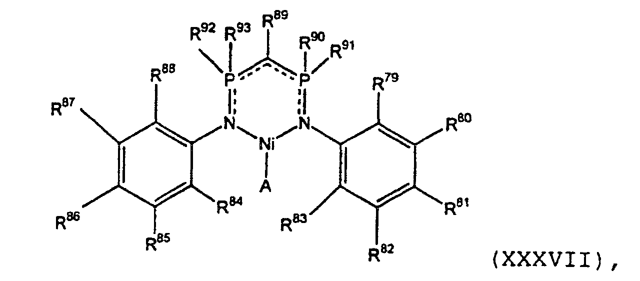 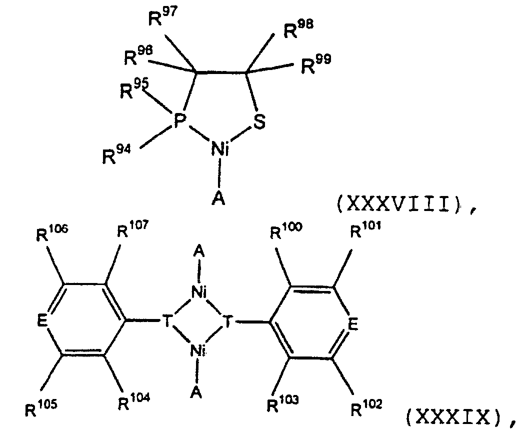 ou 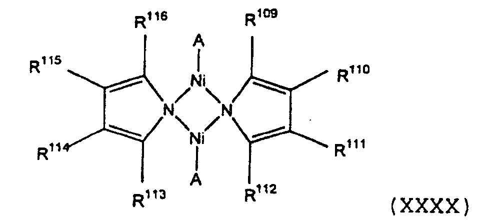 dans lesquelles:
Ar¹, Ar², Ar⁴, Ar⁵, Ar¹⁰, Ar¹¹, Ar¹² et Ar¹³ sont chacun indépendamment de l'aryle ou de l'aryle substitué;
R¹ et R² sont chacun indépendamment de l'hydrogène, de l'hydrocarbyle, de l'hydrocarbyle substitué, ou R¹ et R² pris ensemble forment un cycle, et R³ est de l'hydrogène, de l'hydrocarbyle ou de l'hydrocarbyle substitué ou R¹, R² et R³ pris ensemble forment un cycle;
A est un groupe π-allyle ou π-benzyle;
R¹⁰ et R¹⁵ sont chacun indépendamment de l'hydrogène, de l'hydrocarbyle ou de l'hydrocarbyle substitué;
R¹¹, R¹², R¹³ , R¹⁴ , R¹⁶ , R¹⁷ , R¹⁸ , R¹⁹ , R²⁰ , R²¹ , R³⁰ , R³¹ , R³² , R³³ , R³⁴ , R³⁵ , R⁵⁰ , R⁵² R⁵³, et R⁵⁴ sont chacun indépendamment de l'hydrogène, de l'hydrocarbyle, de l'hydrocarbyle substitué, un groupe fonctionnel inerte, et pourvu que deux quelconques de ces groupes à proximité l'un de l'autre pris ensemble puissent former un cycle;
K est N ou CR²⁷;
R²² est de l'hydrocarbyle, de l'hydrocarbyle substitué, -SR¹¹⁷, -OR¹¹⁷, ou -NR¹¹⁸₂, R²⁴ est de l'hydrogène, un groupe fonctionnel, de l'hydrocarbyle ou de l'hydrocarbyle substitué, et R²⁷ est de l'hydrocarbyle ou de l'hydrocarbyle substitué, et pourvu que R²² et R²⁴ ou R²⁴ et R²⁷ pris ensemble puissent former un cycle;
R¹¹⁷ est de l'hydrocarbyle ou de l'hydrocarbyle substitué;
chaque R¹¹⁸ est indépendamment de l'hydrogène, de l'hydrocarbyle ou de l'hydrocarbyle substitué;
G et L sont tous deux N ou G est CR⁵⁷ et L est CR^{55;}
R⁵⁵, R⁵⁶ et R⁵⁷ sont chacun indépendamment de l'hydrogène, de l'hydrocarbyle ou de l'hydrocarbyle substitué, ou deux quelconques de R⁵⁵, R⁵⁶ et R⁵⁷ pris ensemble forment un cycle;
R⁶⁷ est de l'hydrogène, de l'alkyle ou de l'alkyle substitué;
R⁷⁷ est de l'hydrocarbyle ou de l'hydrocarbyle substitué;
R⁷⁸ est de l'hydrocarbyle ou de l'hydrocarbyle substitué;
R⁷⁹, R⁸⁰, R⁸¹, R⁸² , R⁸³, R⁸⁴ , R⁸⁵, R⁸⁶, R⁸⁷, R⁸⁸ et R⁸⁹ sont chacun indépendamment de l'hydrogène, de l'hydrocarbyle, de l'hydrocarbyle substitué, ou un groupe fonctionnel;
R⁹⁰, R⁹¹, R⁹² et R⁹³ sont chacun indépendamment de l'hydrocarbyle ou de l'hydrocarbyle substitué;
R⁹⁴ et R⁹⁵ sont chacun indépendamment de l'hydrocarbyle ou de l'hydrocarbyle substitué;
R⁹⁶, R⁹⁷, R⁹⁸ et R⁹⁹ sont chacun indépendamment de l'hydrogène, de l'hydrocarbyle, de l'hydrocarbyle substitué ou un groupe fonctionnel;
les deux T sont des S (soufre) ou des NH (amine);
chaque E est N (azote) ou CR¹⁰⁸ dans lequel CR¹⁰⁸ est de l'hydrogène, de l'hydrocarbyle, de l'hydrocarbyle substitué ou un groupe fonctionnel;
R¹⁰⁰, R¹⁰¹, R¹⁰², R¹⁰³, R¹⁰⁴, R¹⁰⁵, R¹⁰⁶ et R¹⁰⁷ sont chacun indépendamment de l'hydrogène, de l'hydrocarbyle, de l'hydrocarbyle substitué, ou un groupe fonctionnel;
R¹⁰⁹, R¹¹⁰, R¹¹¹, R¹¹², R¹¹³ , R¹¹⁴ , R¹¹⁵ et R¹¹⁶ sont chacun indépendamment de l'hydrogène, de l'hydrocarbyle, de l'hydrocarbyle substitué, ou un groupe fonctionnel;
s est un nombre entier égal ou supérieur à 1; et
R²⁸ et R²⁹ sont chacun indépendamment de l'hydrogène, de l'hydrocarbyle ou de l'hydrocarbyle substitué;
et pourvu que lorsque H₂C=CH(CH₂)ₛCO₂R⁷⁷ est présent, R⁶⁷CH=CH₂ est aussi présent.

2. Procédé pour la polymérisation d'une oléfine choisie parmi un ou plusieurs d'entre du R⁶⁷CH=CH₂, du cyclopentène, un styrène, un norbornène ou du H₂C=CH(CH₂)ₛCO₂R⁷⁷, comprenant, mettre en contact, à une température d'environ - 100°C à environ +200°C, du R⁶⁷CH=CH₂, du cyclopentène, un styrène, un norbomène ou du H₂C=CH(CH₂)ₛCO₂R⁷⁷, de manière optionnelle un acide de Lewis, et un composé de formule: 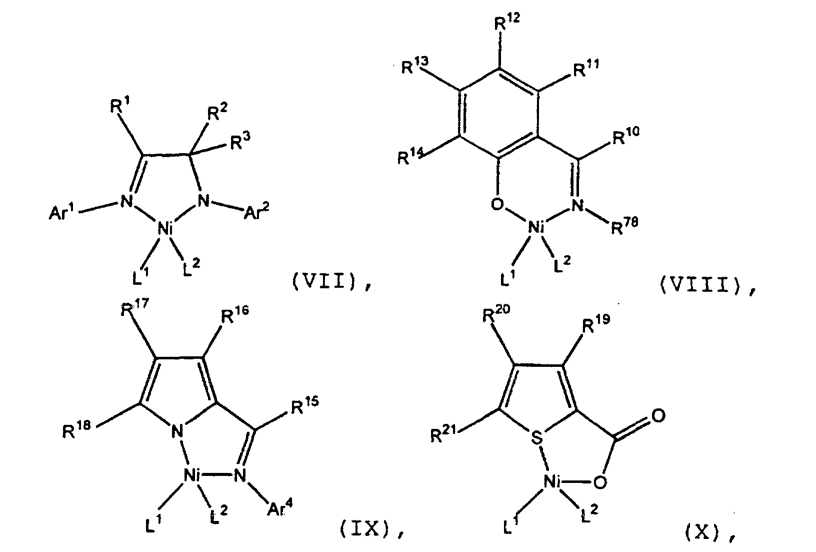 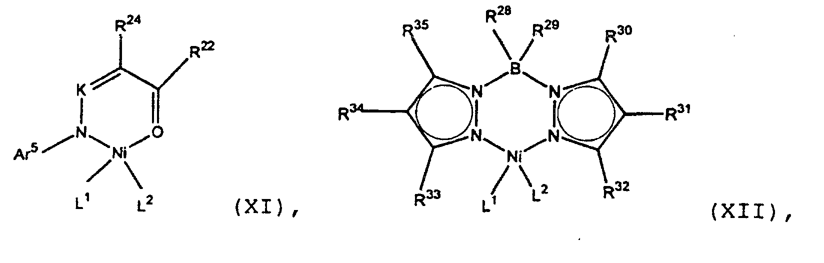 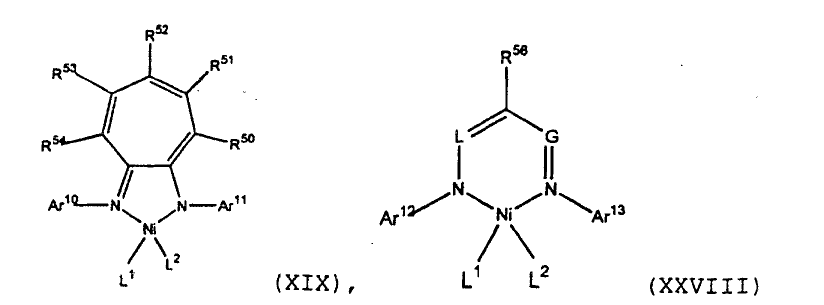 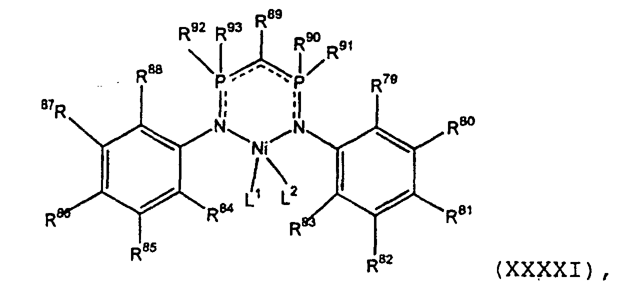 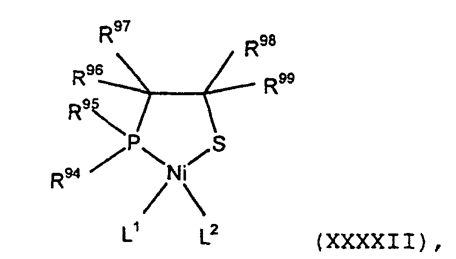 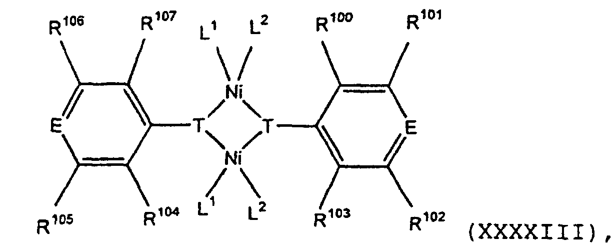 ou 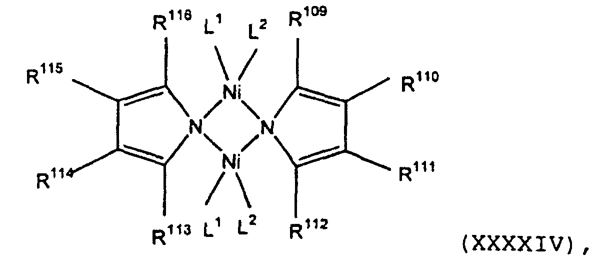 dans lesquelles:
L¹ est un ligand monodenté neutre lequel peut être déplacé par ladite oléfine, et L² est un ligand monoanionique monodenté, ou L¹ et L² pris ensemble sont un ligand monoanionique bidenté, pourvu que ledit ligand monoanionique monodenté ou ledit ligand monoanionique bidenté puisse s'ajouter à ladite oléfine;
Ar¹, Ar², Ar⁴, Ar⁵, Ar¹⁰, Ar¹¹, Ar¹² et Ar¹³ sont chacun indépendamment de l'aryle ou de l'aryle substitué;
R¹ et R² sont chacun indépendamment de l'hydrogène, de l'hydrocarbyle, de l'hydrocarbyle substitué, ou R¹ et R² pris ensemble forment un cycle, et R³ est de l'hydrogène, de l'hydrocarbyle ou de l'hydrocarbyle substitué ou R¹, R² et R³ pris ensemble forment un cycle;
R¹⁰ et R¹⁵ sont chacun indépendamment de l'hydrogène, de l'hydrocarbyle ou de l'hydrocarbyle substitué;
R¹¹, R¹², R¹³ , R¹⁴ , R¹⁶ , R¹⁷, R¹⁸, R¹⁹, R²⁰, R²¹, R³⁰, R³¹, R³², R³³, R³⁴, R³⁵ , R⁵⁰, R⁵², R⁵³, et R⁵⁴ sont chacun indépendamment de l'hydrogène, de l'hydrocarbyle, de l'hydrocarbyle substitué, un groupe fonctionnel inerte, et pourvu que deux quelconques de ces groupes à proximité l'un de l'autre pris ensemble puissent former un cycle;
K est N ou CR²⁷;
R²² est de l'hydrocarbyle, de l'hydrocarbyle substitué, -SR¹¹⁷, -OR¹¹⁷, ou -NR¹¹⁸₂, R²⁴ est de l'hydrogène, un groupe fonctionnel, de l'hydrocarbyle ou de l'hydrocarbyle substitué, et R²⁷ est de l'hydrocarbyle ou de l'hydrocarbyle substitué, et pourvu que R²² et R²⁴ ou R²⁴ et R²⁷ pris ensemble puissent former un cycle;
R¹¹⁷ est de l'hydrocarbyle ou de l'hydrocarbyle substitué;
chaque R¹¹⁸ est indépendamment de l'hydrogène, de l'hydrocarbyle ou de l'hydrocarbyle substitué;
G et L sont tous deux N ou G est CR⁵⁷ et L est CR^{55;}
R⁵⁵, R⁵⁶et R⁵⁷sont chacun indépendamment de l'hydrogène, de l'hydrocarbyle ou de l'hydrocarbyle substitué, ou deux quelconques de R⁵⁵, R⁵⁶ et R⁵⁷ pris ensemble forment un cycle;
R⁶⁷ est de l'hydrogène, de l'alkyle ou de l'alkyle substitué;
R⁷⁷ est de l'hydrocarbyle ou de l'hydrocarbyle substitué;
R⁷⁸ est de l'hydrocarbyle ou de l'hydrocarbyle substitué;
R⁷⁹, R⁸⁰, R⁸¹, R⁸², R⁸³, R⁸⁴, R⁸⁵, R⁸⁶, R⁸⁷, R⁸⁸et R⁸⁹ sont chacun indépendamment de l'hydrogène, de l'hydrocarbyle, de l'hydrocarbyle substitué, ou un groupe fonctionnel;
R⁹⁰, R⁹¹, R⁹² et R⁹³ sont chacun indépendamment de l'hydrocarbyle ou de l'hydrocarbyle substitué;
R⁹⁴ et R⁹⁵ sont chacun indépendamment de l'hydrocarbyle ou de l'hydrocarbyle substitué;
R⁹⁶, R⁹⁷, R⁹⁸ et R⁹⁹ sont chacun indépendamment de l'hydrogène, de l'hydrocarbyle, de l'hydrocarbyle substitué ou un groupe fonctionnel;
les deux T sont des S (soufre) ou des NH (amine);
chaque E est N (azote) ou CR¹⁰⁸ dans lequel CR¹⁰⁸ est de l'hydrogène, de l'hydrocarbyle, de l'hydrocarbyle substitué ou un groupe fonctionnel;
R¹⁰⁰, R¹⁰¹, R¹⁰², R¹⁰³, R¹⁰⁴, R¹⁰⁵, R¹⁰⁶ et R¹⁰⁷ sont chacun indépendamment de l'hydrogène, de l'hydrocarbyle, de l'hydrocarbyle substitué, ou un groupe fonctionnel;
R¹⁰⁹, R¹¹⁰, R¹¹¹, R¹¹², R¹¹³, R¹¹⁴, R¹¹⁵ et R¹¹⁶ sont chacun indépendamment de l'hydrogène, de l'hydrocarbyle, de l'hydrocarbyle substitué, ou un groupe fonctionnel;
s est un nombre entier égal ou supérieur à 1; et
R²⁸ et R²⁹ sont chacun indépendamment de l'hydrogène, de l'hydrocarbyle ou de l'hydrocarbyle substitué;
et pourvu que lorsque H₂C=CH(CH₂)ₛCO₂R⁷⁷ est présent, R⁶⁷CH=CH₂ est aussi présent.

3. Procédé comme énuméré dans la revendication 1 ou 2 dans lequel ladite température est d'environ 0°C à environ 150°C.

4. Procédé comme énuméré dans la revendication 1 ou 2 dans lequel ladite température est d'environ 25°C à environ 100°C.

5. Procédé comme énuméré dans la revendication 1 ou 2 dans lequel ledit acide de Lewis est présent.

6. Procédé comme énuméré dans la revendication 1 ou 2 dans lequel ledit acide de Lewis n'est pas présent.

7. Procédé comme énuméré dans la revendication 2 dans lequel ledit composé est (VII).

8. Procédé comme énuméré dans la revendication 2 dans lequel ledit composé est (VIII).

9. Procédé comme énuméré dans la revendication 8 dans lequel:
R¹⁰ est de l'hydrogène ou du méthyle;
R⁷⁸ est Ar³, lequel est de l'aryle ou de l'aryle substitué; et
R¹¹, R¹², R¹³ et R¹⁴ sont chacun indépendamment du chloro, bromo, iodo, alkyle, alcoxy, hydrogène ou nitro, ou R¹¹ et R¹² pris ensemble forment un cycle carbocyclique à 6 chaînons et R¹³ et R¹⁴ sont de l'hydrogène.

10. Procédé comme énuméré dans la revendication 2 dans lequel ledit composé est (IX).

11. Procédé comme énuméré dans la revendication 2 dans lequel ledit composé est (X).

12. Procédé comme énuméré dans la revendication 2 dans lequel ledit composé est (XI).

13. Procédé comme énuméré dans la revendication 2 dans lequel ledit composé est (XII).

14. Procédé comme énuméré dans la revendication 2 dans lequel ledit composé est (XIX).

15. Procédé comme énuméré dans la revendication 2 dans lequel ledit composé est (XXVIII).

16. Procédé comme décrit dans la revendication 2 dans lequel ledit composé est (XXXXI).

17. Procédé comme décrit dans la revendication 2 dans lequel ledit composé est (XXXXII).

18. Procédé comme énuméré dans la revendication 2 dans lequel ledit composé est (XXXXIII).

19. Procédé comme énuméré dans la revendication 2 dans lequel ledit composé est (XXXXIV).

20. Procédé comme énuméré dans la revendication 2 dans lequel L¹ est un nitrile, une pyridine ou une pyridine substituée, et L² est du méthyle.

21. Procédé comme énuméré dans la revendication 1 ou la revendication 2 dans lequel ladite oléfine ou les oléfines sont: de l'éthylène; un styrène; un norbomène; une α-oléfine; du cyclopéntène; du H₂C=CH(CH₂)ₛCO₂R⁷⁷ et de l'éthylène; de l'éthylène et une α-oléfine; un styrène et un norbomène; et 2 norbonènes ou plus.

22. Composé de formule: 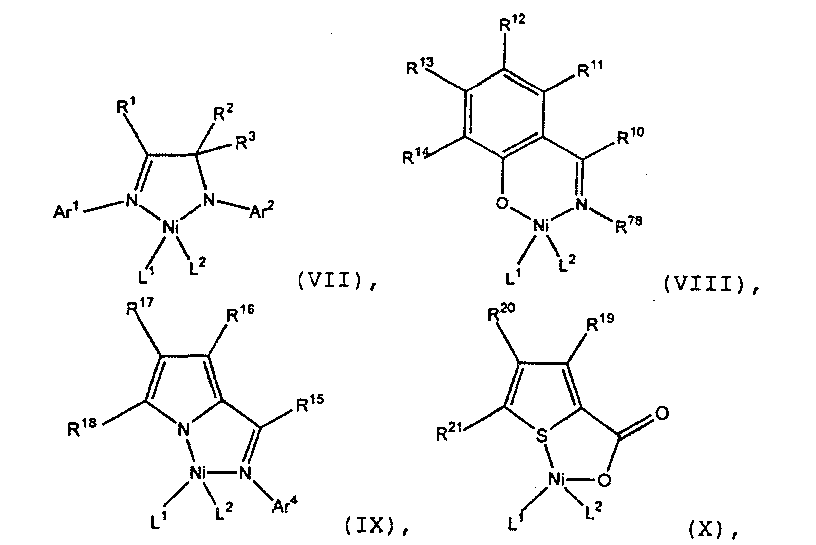 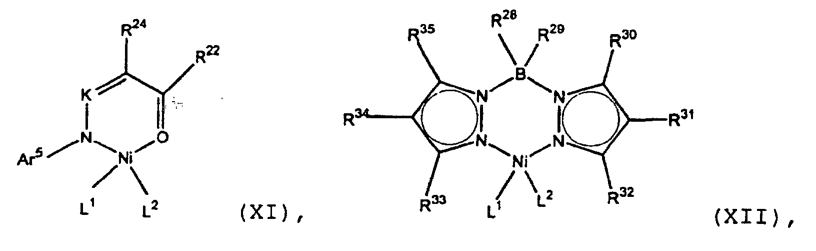 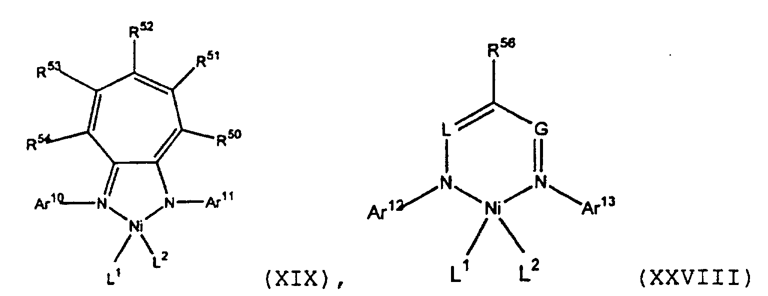 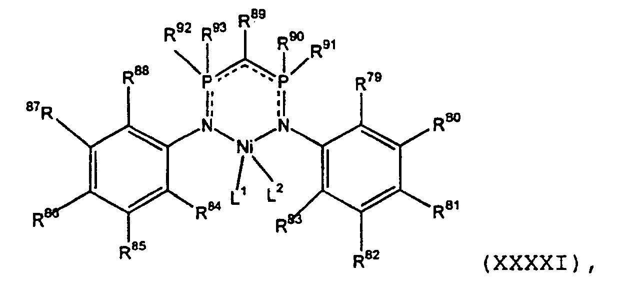 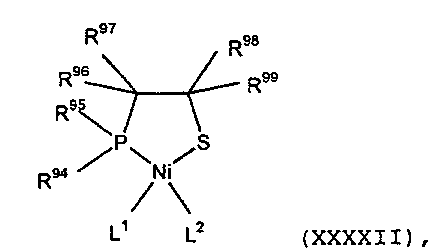 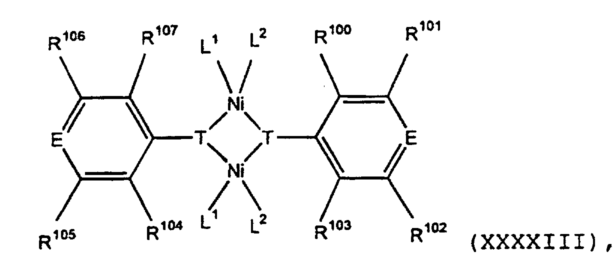 ou 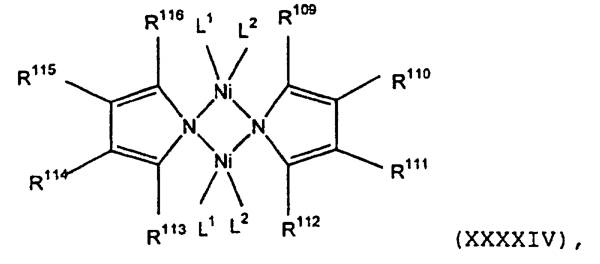 dans lesquelles:
L¹ est un ligand monodenté neutre lequel peut être déplacé par ladite oléfine, et L² est un ligand monoanionique monodenté, ou L¹ et L² pris ensemble sont un ligand monoanionique bidenté, pourvu que ledit ligand monoanionique monodenté ou ledit ligand monoanionique bidenté puisse s'ajouter à ladite oléfine;
Ar¹, Ar², Ar⁴, Ar⁵, Ar¹⁰, Ar¹¹, Ar¹² et Ar¹³ sont chacun indépendamment de l'aryle ou de l'aryle substitué;
R¹ et R² sont chacun indépendamment de l'hydrogène, de l'hydrocarbyle, de l'hydrocarbyle substitué, ou R¹ et R² pris ensemble forment un cycle, et R³ est de l'hydrogène, de l'hydrocarbyle ou de l'hydrocarbyle substitué ou R¹, R² et R³ pris ensemble forment un cycle;
R¹⁰ et R¹⁵ sont chacun indépendamment de l'hydrogène, de l'hydrocarbyle ou de l'hydrocarbyle substitué;
R¹¹, R¹², R¹³, R¹⁴, R¹⁶, R¹⁷, R¹⁸, R¹⁹, R²⁰, R²¹, R³⁰, R³¹, R³², R³³, R³⁴, R³⁵, R⁵⁰, R⁵², R⁵³, et R⁵⁴ sont chacun indépendamment de l'hydrogène, de l'hydrocarbyle, de l'hydrocarbyle substitué, un groupe fonctionnel inerte, et pourvu que deux quelconques de ces groupes à proximité l'un de l'autre pris ensemble puissent former un cycle;
K est N ou CR²⁷;
R²² est de l'hydrocarbyle, de l'hydrocarbyle substitué, -SR¹¹⁷, -OR¹¹⁷, ou -NR¹¹⁸₂, R²⁴ est de l'hydrogène, un groupe fonctionnel, de l'hydrocarbyle ou de l'hydrocarbyle substitué, et R²⁷ est de l'hydrocarbyle ou de l'hydrocarbyle substitué, et pourvu que R²² et R²⁴ ou R²⁴ et R²⁷ pris ensemble puissent former un cycle;
R¹¹⁷ est de l'hydrocarbyle ou de l'hydrocarbyle substitué;
chaque R¹¹⁸ est indépendamment de l'hydrogène, de l'hydrocarbyle ou de l'hydrocarbyle substitué;
G et L sont tous deux N ou G est CR⁵⁷ et L est CR^{55;}
R⁵⁵, R⁵⁶ et R⁵⁷ sont chacun indépendamment de l'hydrogène, de l'hydrocarbyle ou de l'hydrocarbyle substitué, ou deux quelconques de R⁵⁵, R⁵⁶ et R⁵⁷ pris ensemble forment un cycle;
R⁷⁸ est de l'hydrocarbyle ou de l'hydrocarbyle substitué;
R⁷⁹, R⁸⁰, R⁸¹, R⁸², R⁸³, R⁸⁴, R⁸⁵, R⁸⁶, R⁸⁷, R⁸⁸ et R⁸⁹ sont chacun indépendamment de l'hydrogène, de l'hydrocarbyle, de l'hydrocarbyle substitué, ou un groupe fonctionnel;
R⁹⁰, R⁹¹, R⁹² et R⁹³ sont chacun indépendamment de l'hydrocarbyle ou de l'hydrocarbyle substitué;
R⁹⁴ et R⁹⁵ sont chacun indépendamment de l'hydrocarbyle ou de l'hydrocarbyle substitué;
R⁹⁶, R⁹⁷, R⁹⁸ et R⁹⁹ sont chacun indépendamment de l'hydrogène, de l'hydrocarbyle, de l'hydrocarbyle substitué ou un groupe fonctionnel;
les deux T sont des S (soufre) ou des NH (amine);
chaque E est N (azote) ou CR¹⁰⁸ dans lequel CR¹⁰⁸ est de l'hydrogène, de l'hydrocarbyle, de l'hydrocarbyle substitué ou un groupe fonctionnel;
R¹⁰⁰,R¹⁰¹, R¹⁰², R¹⁰³, R¹⁰⁴, R¹⁰⁵, R¹⁰⁶ et R¹⁰⁷ sont chacun indépendamment de l'hydrogène, de l'hydrocarbyle, de l'hydrocarbyle substitué, ou un groupe fonctionnel;
R¹⁰⁹, R¹¹⁰, R¹¹¹, R¹¹² , R¹¹³ , R¹¹⁴, R¹¹⁵ et R¹¹⁶ sont chacun indépendamment de l'hydrogène, de l'hydrocarbyle, de l'hydrocarbyle substitué, ou un groupe fonctionnel; et
R²⁸ et R²⁹ sont chacun indépendamment de l'hydrogène, de l'hydrocarbyle ou de l'hydrocarbyle substitué.

23. Composé comme énuméré dans la revendication 22 lequel est (VII).

24. Composé comme énuméré dans la revendication 22 lequel est (VIII).

25. Composé comme énuméré dans la revendication 24 dans lequel:
R¹⁰ est de l'hydrogène ou du méthyle;
R⁷⁸ est Ar³, lequel est de l'aryle ou de l'aryle substitué; et
R¹¹, R¹², R¹³ et R¹⁴ sont chacun indépendamment du chloro, bromo, iodo, alkyle, alcoxy, hydrogène ou nitro, ou R¹¹ et R¹² pris ensemble forment un cycle carbocyclique à 6 chaînons et R¹³ et R¹⁴ sont de l'hydrogène.

26. Composé comme énuméré dans la revendication 22 lequel est (IX).

27. Composé comme énuméré dans la revendication 22 lequel est (X).

28. Composé comme énuméré dans la revendication 22 lequel est (XI).

29. Composé comme énuméré dans la revendication 22 lequel est (XII).

30. Composé comme énuméré dans la revendication 22 lequel est (XIX).

31. Composé comme énuméré dans la revendication 22 lequel est (XXVIII).

32. Composé comme décrit dans la revendication 22 lequel est (XXXXI).

33. Composé comme décrit dans la revendication 22 lequel est (XXXXII).

34. Composé comme énuméré dans la revendication 22 lequel est (XXXXIII).

35. Composé comme énuméré dans la revendication 22 lequel est (XXXXIV).

36. Composé comme énuméré dans la revendication 22 dans lequel L¹ est un nitrile, une pyridine ou une pyridine substituée, et L² est du méthyle.

37. Composé comme énuméré dans la revendication 22 dans lequel L¹ et L² pris ensemble ne sont pas du π-allyle ou du π-benzyle.

38. Composé de formule 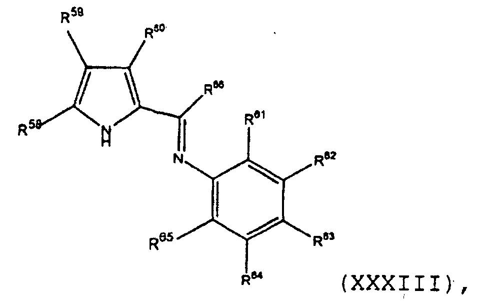 dans laquelle:
R⁵⁸, R⁵⁹, R⁶⁰, R⁶², R⁶³ et R⁶⁴ sont chacun indépendamment de l'hydrocarbyle, de l'hydrocarbyle substitué, ou un groupe fonctionnel, et pourvu que deux quelconques de ces groupes à proximité l'un de l'autre pris ensemble puissent former un cycle, ou s'ils sont à proximité de R⁶¹ ou R⁶⁵ former un cycle avec eux;
R⁶⁶ est de l'hydrogène, de l'hydrocarbyle, de l'hydrocarbyle substitué; et
R⁶¹ et R⁶⁵ sont chacun indépendamment de l'hydrocarbyle contenant 2 atomes de carbone ou plus, ou de l'hydrocarbyle substitué contenant 2 atomes de carbone ou plus, et pourvu que R⁶¹ et R⁶⁵ puissent former un cycle avec n'importe quel groupe à proximité de lui.

39. Composé de formule 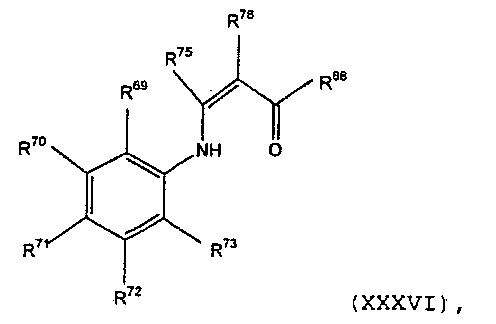 dans laquelle:
R⁶⁸ est de l'hydrocarbyle, de l'hydrocarbyle substitué, -SR^{117,} -OR¹¹⁷, ou -NR¹¹⁸₂, R⁷⁶ est de l'hydrogène, un groupe fonctionnel, de l'hydrocarbyle ou de l'hydrocarbyle substitué, et R⁷⁵ est de l'hydrocarbyle ou de l'hydrocarbyle substitué, et pourvu que R⁶⁸ et R⁷⁶ ou R⁷⁵ et R⁷⁶ pris ensemble puissent former un cycle;
R¹¹⁷ est de l'hydrocarbyle ou de l'hydrocarbyle substitué;
chaque R¹¹⁸ est indépendamment de l'hydrogène, de l'hydrocarbyle ou de l'hydrocarbyle substitué;
R⁷⁰, R⁷¹ et R⁷² sont chacun indépendamment de l'hydrogène, de l'hydrocarbyle, de l'hydrocarbyle substitué ou un groupe fonctionnel;
R⁶⁹ et R⁷³ sont de l'hydrocarbyle contenant 3 atomes de carbone ou plus, de l'hydrocarbyle substitué contenant 3 atomes de carbone ou plus ou un groupe fonctionnel;
et pourvu que deux quelconques de R⁷⁰, R⁷¹, R⁷², R⁶⁹, et R⁷³ à proximité l'un de l'autre puissent former un cycle ensemble.
